# EUROPEAN PATENT SPECIFICATION

(11) **EP 1 506 214 B1**
(45) Date of publication and mention of the grant of the patent: **20.01.2010**
(21) Application number: 03733983.5
(22) Date of filing: 09.05.2003
(51) Int. Cl.: C07H 17/08, A61K 31/7048, A61P 31/04

(54) **6-11 BICYCLIC KETOLIDE DERIVATIVES**
6-11 BICYCLISCHE KETOLIDDERIVATE
DERIVES DE CETOLIDE 6-11 BICYCLIQUES

(30) Priority: 13.05.2002 US 144558; 05.05.2003 US 429485
(43) Date of publication of application: 16.02.2005
(73) Proprietor: Enanta Pharmaceuticals, Inc., Watertown, MA 02472 (US)
(72) Inventor: OR, Yat, Sun, Watertown, MA 02472 (US); WANG, Guoqiang, Belmont, MA 02478 (US); PHAN, Ly, Tam, Quincy, MA 02170 (US); NIU, Deqiang, Lexington, MA 02421 (US); VO, Nha Huu, Southborough, MA 01772 (US); QIU, Yao-Ling, Andover, MA 01810 (US); WANG, Yanchun, Massachusetts 02461 (US); BUSUYEK, Marina, Natick, MA 01760 (US); HOU, Ying, Everett, MA 02149 (US); PENG, Yulin, Belmont, MA 02478 (US); KIM, Heejin, Allston, MA 02134 (US); LIU, Tongzhu, Auburndale, MA 02466 (US); FARMER, Jay Judson, New Haven, CT 06511 (US); XU, Guoyou, Auburndale, MA 02466 (US)
(74) Representative: Chapman, Paul William
(86) International application number: PCT/US2003/014669
(87) International publication number: WO 2003/097659

(56) References cited:
- US-A- 5 866 549

## Description

### TECHNICAL FIELD

The present invention relates to novel semisynthetic macrolides having antibacterial activity and useful in the treatment and prevention of bacterial infections. More particularly, the invention relates to 6-11 bicyclic ketolide derivatives, compositions containing such compounds and methods for using the same, as well as processes for making such compounds.

### BACKGROUND OF THE INVENTION

Macrolide antibiotics play a therapeutically important role, particularly with the emergence of new pathogens. Structural differences are related to the size of the lactone ring and to the number and nature (neutral or basic) of the sugars. Macrolides are classified according to the size of the lactone ring (12,14, 15 or 16 atoms). The macrolide antibiotic family (14-, 15-and 16-membered ring derivatives) shows a wide range of characteristics (antibacterial spectrum, side-effects and bioavailability). Among the commonly used macrolides are erythromycin, clarithromycin, and azithromycin.
Macrolides possessing a 3- oxo moiety in place of the 3-cladinose sugar are known as ketolides and have shown enhanced activity towards gram-negative bacteria and macrolide resistant gram-positive bacteria. The search for macrolide compounds which are active against MLSB-resistant strains (MLS_{B} = Macrolides-Lincosamides-type B Streptogramines) has become a major goal, together with retaining the overall profile of the macrolides in terms of stability, tolerance and pharmacokinetics. US 5,866,549 discloses 6-0-substituted erythromycin derivatives with reportedly increased stability and enhanced activity towards macrolide-resistant gram positive bacteria.

### SUMMARY OF THE INVENTION

The present invention provides a novel class of C6-C11 bridged erythromycin derivatives which possess antibacterial activity.

In one aspect of the present invention there are provided novel bridged ketolide compounds, or their pharmaceutically acceptable salts, esters or produrugs, represented by formula I as illustrated below: 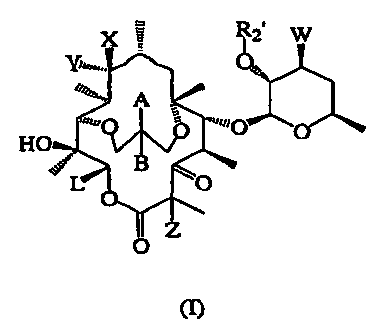 or its pharmaceutically acceptable salt or ester wherein
A is selected from:
a) -OH;
b) -ORₚ, where Rₚ is a hydroxy protecting group;
c) -R₁, where R₁ is independently selected from:
   (1) aryl;
   (2) substituted aryl;
   (3) heteroaryl; and
   (4) substituted heteroaryl;
d)-OR₁, where R₁ is as previously defined;
e)-R₂, where R₂ is selected from:
   (1) hydrogen;
   (2) halogen;
   (3) C₁-C₁₂ alkyl optionally containing 0,1, 2, or 3 heteroatoms selected from O, S or N, optionally substituted with one or more substituents selected from halogen, aryl, substituted aryl, heteroaryl, and substituted heteroaryl;
   (4) C₂-C₁₂ alkenyl optionally containing 0, 1, 2, or 3 heteroatoms selected from O, S and N, optionally substituted with one or more substituents selected from halogen, aryl, substituted aryl, heteroaryl, and substituted heteroaryl; and
   (5) C₂-C₁₂ alkynyl optionally containing 0,1, 2, or 3 heteroatoms selected from O**,** S and N, optionally substituted with one or more substituents selected from halogen, aryl, substituted aryl, heteroaryl, and substituted heteroaryl;
f)-OR₂, where R₂ is independently previously defined;
g) -S(O)ₙR₁₁, where n = 0,1 or 2, and R₁₁ is independently hydrogen, R₁ or R₂,
   where R₁ and R₂ are as previously defined;
h) -NHC(O)R₁₁, where R₁₁) is as previously defined;
i) -NHC(O)NHR₁₁, where R₁₁ is as previously defined;
j) -NHS(O)₂R₁₁, where R₁₁ is as previously defined;
k) -NR₁₄R₁₅, where R₁₄ and R₁₅ are each independently R₁₁, where R₁₁ is as previously defined; and
l) -NHR₃, where R₃ is an amino protecting group; .
B is selected from:
a) hydrogen;
b) deuterium;
c) halogen;
d) -OH;
e) R₁, where R₁ is as previously defined;
f) R₂, where R₂ is as previously defined; and
g) -ORₚ, where Rₚ is as previously defined,
h) provided that when B is halogen, -OH, or -ORₚ, A is R₁ or R₂;
or alternatively, A and B taken together with the carbon atom to which they are attached are selected from:
a) C=O;
b) C(OR₂)₂, where R₂ is as previously defined;
c) C(SR₂)₂, where R₂ is as previously defined;
d) C[-O(CH₂)ₘ]₂, where m = 2 or 3;
e) C[-S(CH₂)ₘ]₂, where m is as previously defined;
f) C=CHR₁₁, where R₁₁ is as previously defined;
g) C=N-O-R₁₁, where R₁₁ is as previously defined;
h) C=N-O-Ar₁-M-Ar₂, wherein
   (1) -Ar₁- is R₃₁, where R₃₁ is independently selected from:
      (a) R₁, where R₁ is as previously defined;
      (b) C₁-C₁₂ alkyl optionally containing 0, 1, 2, or 3 heteroatoms selected from O, S or N, optionally substituted with one or more substituents selected from halogen, aryl, substituted aryl, heteroaryl, and substituted heteroaryl;
      (c) C₂-C₁₂ alkenyl optionally containing 0, 1, 2, or 3 heteroatoms selected from O, S and N, optionally substituted with one or more substituents selected from halogen, aryl, substituted aryl, heteroaryl, and substituted heteroaryl; or
      (d) C₂-C)₁₂ alkynyl optionally containing 0, 1, 2, or 3 heteroatoms selected from O, S and N, optionally substituted with one or more substituents selected from halogen, aryl, substituted aryl, heteroaryl, and substituted heteroaryl;
   (2) -M- is absent or selected from:
      (a) -C₁-C₁₂ alkyl optionally containing:
         0-3 heteroatoms selected from O, S or N; and
         0-3 groups selected from -C=N-, -N=N, -C(O)-;
      (b) -C₂-C₁₂ alkenyl optionally containing:
         0-3 heteroatoms selected from O, S or N; and
         0-3 groups selected from -C=N-, -N=N, -C(O);
      (c) -C₂-C₁₂ alkynyl optionally containing;
         0-3 heteroatoms selected from O, S or N;
         0-3 groups selected from -C=N-, -N=N-, -C(O)-;
      (d) substituted aryl;
      (e) substituted heteroaryl; or
      (f) substituted heterocyclo alkyl; and
   (3) -Ar₂ is selected from:
      (a) aryl;
      (b) substituted aryl;
      (c) heteroaryl; or
      (d) substituted heteroaryl;
i) C=NNHR₁₁, where R₁₁ is as previously defined;
j) C=NNHC(O)R₁₁, where R₁₁ is as previously defined;
k) C=NNHC(O)NHR₁₁, where R₁₁ is as previously defined;
l) C=NNHS(O)₂R₁₁, where R₁₁ is as previously defined;
m) C=NNHR₃, where R₃ is as previously defined;
n) C=NR₁₁, where R₁₁ is as previously defined; or
o) C=N-N=CHR₁₁, where R₁₁ is as previously defined;
one of X and Y is hydrogen and the other is selected:
a) hydrogen;
b) deuterium;
c) -OH;
d) -ORₚ, where Rₚ is as previously defined;
e) -NR₄R₅, where R₄ and R₅ are each independently selected from:
   (1) hydrogen;
   (2) C₁-C₁₂ alkyl, optionally substituted with one or more substituents selected from halogen, aryl, substituted aryl, heteroaryl and substituted heteroaryl; or
   (3) R₄ and R₅, taken together with the nitrogen atom to which they are attached form a 3-10 membered heteroalkyl ring containing 0-2 additional hetero atoms selected from O, S and N; or
alternatively, X and Y taken together with the carbon atom to which they are attached are selected from:
a) C=O;
b) C=N-Q, wherein Q is selected from:
   (1) -R₁₁, where R₁₁ is as previously defined;
   (2) amino protecting group;
   (3) -C(O)R₁₁, where R₁₁ is as previously defined;
   (4) -OR₆, where R₆ is independently selected from:
      (a) hydrogen;
      (b) -CH₂O(CH₂)₂OCH₃,
      (c) -CH₂O(CH₂O)ₙCH₃, where n is as previously defined;
      (d) -C₁-C₁₂ alkyl, optionally substituted with one or more substituents selected from aryl, substituted aryl, heteroaryl and substituted heteroaryl;
      (e) -C₃-C₁₂ cycloalkyl;
      (f) -C(O)- C₁-C₁₂ alkyl;
      (g) -C(O)-C₃-C₁₂ cycloalkyl;
      (h) -C(O)-R₁, where R₁ is as previously defined; or
      (i) -Si(Rₐ)(R_{b})(R_{c}), wherein Rₐ, R_{b} and R_{c} are each independently selected from C₁-C₁₂ alkyl, aryl and substituted aryl; or
   (5) O-C(R₇)(R₈)-O-R₆, where R₆ is as previously defined, provided that R₆ is not C(O)- C₁-C₁₂ alkyl, C(O)-C₃-C₁₂ cycloalkyl, or C(O)-R₁, and R₇ and R₈ taken together with the carbon atom to which they are attached form a C₃-C₁₂ cycloalkyl group or each independently is selected from:
      (1) hydrogen; or
      (2) C₁-C₁₂ alkyl;
L is selected from:
a) -CH₃;
b) -CH₂CH₃;
c) -CH(OH)CH₃;
d) C₁-C₆ alkyl, optionally substituted with one or more substituents selected from aryl, substituted aryl, heteroaryl, and substituted heteroaryl;
e) C₂-C₆ alkenyl, optionally substituted with one or more substituents selected from aryl, substituted aryl, heteroaryl, and substituted heteroaryl; or
f) C₂-C₆ alkynyl, optionally substituted with one or more substituents selected from aryl, substituted aryl, heteroaryl, and substituted heteroaryl;
W is -NR₂₀R₂₁, where R₂₀ and R₂₁, are each independently selected from:
a) hydrogen;
b) C₁-C₁₂ alkyl, optionally substituted with one or more substituents selected from halogen, aryl, substituted aryl, heteroaryl and substituted heteroaryl;
c) C₂-C₁₂ alkenyl, optionally substituted with one or more substituents selected from halogen, aryl, substituted aryl, heteroaryl and substituted heteroaryl;
d) C₂-C₁₂ alkynyl, optionally substituted with one or more substituents selected from halogen, aryl, substituted aryl, heteroaryl and substituted heteroaryl; or
e) R₂₀ and R₂₁, taken together with the nitrogen atom to which they are attached form a heterocycloalkyl moiety; or
Z is selected from:
a) hydrogen;
b) methyl; or
c) halogen; and
R₂' is hydrogen or Rₚ, where Rₚ, is as previously defined.

In another embodiment of the present invention there are disclosed pharmaceutical compositions comprising a therapeutically effective amount of any compound of the present invention in combination with a pharmaceutically acceptable carrier or excipient. Yet another embodiment of the invention comprises the use of any compound of the present invention for the manufacture of a medicament for the control of a bacterial infection in a subject in need of such treatment. Suitable carriers and methods of formulation are also disclosed.

In a further aspect of the present invention there are provided processes for the preparation of 6,11-3C-bridged ketolide derivatives of formula (I) via any synthetic route delineated herein.

### DETAILED DESCRIPTION OF THE INVENTION

A first embodiment of the present invention is a compound of formula I as defined herein, or its pharmaceutically acceptable salt or ester.

Representative subgenera of the present invention are:
A compound according to claim 1 which is represented by the formula 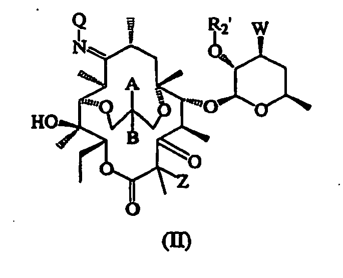 wherein A, B, R₂, Q, W, and Z are as defined in claim;
A compound according to claim 1 which is represented by the formula 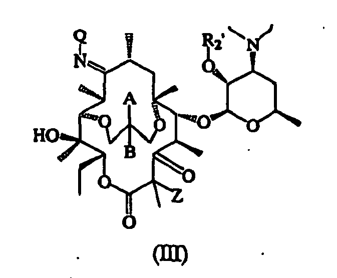 wherein A, B, R₂, Q, and Z are as defined in claim 1;
A compound according to claim 1 which is represented by the formula 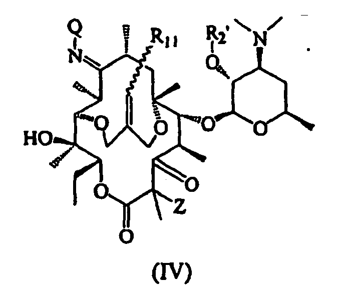 wherein A, B, R₂', Q, and Z are as previously defined;
A compound which is represented by the formula 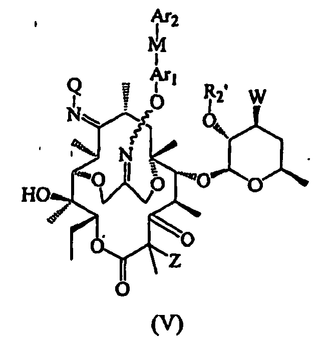 wherein Ar₁, Ar₂, R₂, M, Q, W, and Z are as previously defined;
A compound which is represented by the formula: 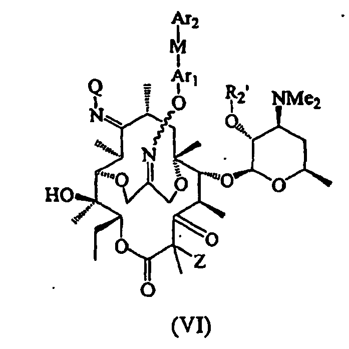 wherein Ar₁, Ar₂, R_{2'}, M, Q, and Z are as previously defined;
A compound which is represented by the formula: 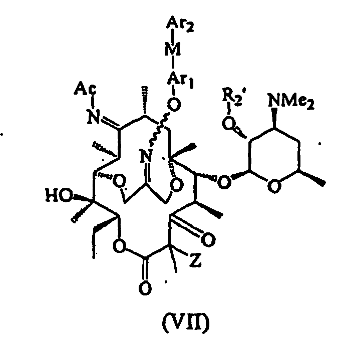 wherein Ar₁, Ar₂, R_{2'}, M, and Z are as previously defined; or
A compound which is represented by the formula: 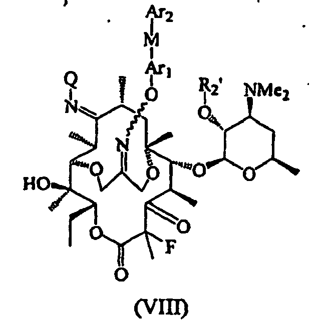 wherein Ar₁, Ar₂, R_{2'}, M, and Q are as previously defined.

Representative species of the present invention are:
Example 1. Compound of formula I: A and B taken together with the carbon atom to which they attached are C=CH₂, X and Y taken together with the carbon atom to which they are attached are C=N-Ac, L = CH₂CH₃, Z = H, and R₂' =Ac;
Example 2. Compound of formula I: A and B taken together with the carbon atom to which they are attached are C=CH₂, X and Y taken together with the carbon atom to which they are attached are C=N-Ac, L = CH₂CH₃, Z = H and R₂' = H;
Example 3. Compound of formula I: A and B taken together with the carbon atom to which they are attached are C=O, X and Y taken together with the carbon atom to which they attached are C=N-Ac, L = CH₂CH₃, Z = H and R₂' = H;
Example 4. Compound of formula I: A and B taken together with the carbon atom to which they are attached are C=N-O-CH₂-Ph, X and Y taken together with the carbon atom to which they are attached are C=N-Ac, L = CH₂CH₃, Z = H and R₂' = H;
Example 5. Compound of formula I: A and B taken together with the carbon atom to which they are attached are C=N-O-CH₂-(3-pyridyl), X and Y taken together with the carbon atom to which they are attached are C=N-Ac, L = CH₂CH₃, Z = H and R₂' = H;
Example 6. Compound of formula I: A and B taken together with the carbon atom to which they are attached are C=N-O-CH₂-(2-pyridyl), X and Y taken together with the carbon atom to which they are attached are C=N-Ac, L = CH₂CH₃, Z = H and R₂' = H;
Example 7. Compound of formula I: A and B taken together with the carbon atom to which they are attached are C=N-O-CH₂-(3-quinolyl), X and Y taken together with the carbon atom to which they are attached are C=N-Ac, L = CH₂CH₃, Z = H and R₂' =H;
Example 8. Compound of formula I: A and B taken together with the carbon atom to which they are attached are C=N-O-CH₂-(2-quinolyl), X and Y taken together with the carbon atom to which they are attached are C=N-Ac, L = CH₂CH₃, Z = H and R₂' = H;
Example 9. Compound of formula I: A and B taken together with the carbon atom to which they are attached are C=N-O-CH₂(5-pyridin-2-ylthiophen-2yl), X and Y taken together with the carbon atom to which they are attached are C=N-Ac, L = CH₂CH₃, Z = H and R₂' = H;
Example 10. Compound of formula I: A and B taken together with the carbon atom to which they are attached are C=N-O-[3-(pyrimidin-2-yl)prop-2-ynyl], X and Y taken together with the carbon atom to which they are attached are C=N-Ac, L = CH₂CH₃, Z = H and R₂' = H;
Example 11. Compound of formula I: A and B taken together with the carbon atom to which they are attached are C=N-O-Ph, X and Y taken together with the carbon atom to which they are attached are C=N-Ac, L = CH₂CH₃, Z = H and R₂' = H;
Example 12. Compound of formula I: A = NHCH₂-Ph, B = H, X and Y taken together with the carbon atom to which they are attached are C=N-Ac, L = CH₂CH₃, Z = H and R₂' = H;
Example 13. Compound of formula I: A = NHCH2CH₂-Ph, B = H, X and Y taken together with the carbon atom to which they are attached are C=N-Ac, L = CH₂CH₃, Z = H and R₂' = H;
Example 14. Compound of formula I: A and B taken together with the carbon atom to which they are attached are C=CH₂, X and Y taken together with the carbon atom to which they are attached are C=N-O-CH₂-O-CH₃, L = CH₂CH₃, Z = H, and R₂' = Ac;
Example 15. Compound of formula I: A and B taken together with the carbon atom to which they are attached are C=CH₂, X and Y taken together with the carbon atom to which they are attached are C=N-O-CH₂-O-CH₃, L = CH₂CH₃, Z = H and R₂' = H;
Example 16. Compound of formula I: A and B taken together with the carbon atom to which they are attached are C=O, X and Y taken together with the carbon atom to which they are attached are C=N-O-CH₂-O-CH₃, L = CH₂CH₃, Z = H and R₂' = H;
Example 17. Compound of formula I: A and B taken together with the carbon atom to which they are attached are C=NOCH₂-Ph, X and Y taken together with the carbon atom to which they are attached are C=N-O-CH₂-O-CH₃, L = CH₂CH₃, Z = H and R₂' = H;
Example 18. Compound of formula **I:** A and B taken together with the carbon atom to which they are attached are C=O, X and Y taken together with the carbon atom to which they are attached are C=O, L = CH₂CH₃, Z = H, and R₂' = Ac;
Example 19. Compound of formula I: A and B taken together with the carbon atom to which they are attached are C=CH₂; X and Y taken together with the carbon atom to which they are attached are C=O, L = CH₂CH₃, Z = H, and R₂' = H;
Example 20. Compound of formula I: A and B taken together with the carbon atom to which they are attached are C=O, X and Y taken together with the carbon atom to which they are attached are C=O, L = CH₂CH₃, Z = H, and R₂' = H;
Example 21. Compound of formula I: A and B taken together with the carbon atom to which they are attached are C=N-O-CH₂-Ph, X and Y taken together with the carbon atom to which they are attached are C=O, L = CH₂CH₃, Z = H, and R₂**'** = H;
Example 22. Compound of formula I: A and B taken together with the carbon atom to which they are attached are C=CH₂, X and Y taken together with the carbon atom to which they are attached are C=NH, L = CH₂CH₃, Z = H, and R₂' =H;
Example 23. Compound of formula I: A and B taken together with the carbon atom to which they are attached are C=CH-O-CH₂-*p*-NO₂-Ph, X and Y taken together with the carbon atom to which they are attached are C=N-Ac, L = CH₂CH₃, Z = H, and R₂' =H;
Example 24. Compound of formula I: A and B taken together with the carbon atom to which they are attached are C=N-O-(CH₂)₂-Ph, X and Y taken together with the carbon atom to which they are attached are C=N-Ac, L = CH₂CH₃, Z = H, and R₂' =H;
Example 25. Compound of formula I: A and B taken together with the carbon atom to which they are attached are C=N-O-(CH₂)₃-Ph, X and Y taken together with the carbon atom to which they are attached are C=N-Ac, L = CH₂CH₃, Z = H, and R₂' = H;
Example 26. Compound of formula I: A and B taken together with the carbon atom to which they are attached are C=N-O-CH₂-CH=CH-Ph, X and Y taken together with the carbon atom to which they are attached are C=N-Ac, L = CH₂CH₃, Z = H, and R₂' = H;
Example 27. Compound of formula I: A is NH-(CH₂)₃-Ph, B is H, X and Y taken together with the carbon atom to which they are attached are C=N-Ac, L = CH₂CH₃, Z = H, and R₂' =H;
Example 28. Compound of formula I: A is NH-(CH₂)₄-Ph, B is H, X and Y taken together with the carbon atom to which they are attached are C=N-Ac, L = CH₂CH₃, Z = H, and R₂' = H;
Example 29. Compound of formula I: A is CH₂-CH=CH₂, B is OH, X and Y taken together with the carbon atom to which they are attached are C=N-Ac, L = CH₂CH₃, Z = H, and R₂' = H;
Example 30. Compound of formula I: A is CH₂-Ph, B is OH, X and Y taken together with the carbon atom to which they are attached are C=N-Ac, L = CH₂CH₃, Z = H, and R₂' = H;
Example 31. Compound of formula I: A is Ph, B is OH, X and Y taken together with the carbon atom to which they are attached are C=N-Ac, L = CH₂CH₃, Z = H, and R₂' = H;
Example 32. Compound of formula I: A is Ph, B is OH, X and Y taken together with the carbon atom to which they are attached are C=N-Ac, L = CH₂CH₃, Z = H, and R₂' = H;
Example 33. Compound of formula I: A is CH₂-CH=CH-Ph, B is OH, X and Y taken together with the carbon atom to which they are attached are C=N-Ac, L = CH₂CH₃, Z = H, and R₂' = H;
Example 34. Compound of formula I: A is (CH₂)₃-Ph, B is OH, X and Y taken together with the carbon atom to which they are attached are C=N-Ac, L = CH₂CH₃, Z = H, and R₂' = H;
Example 35. Compound of formula I: A and B taken together with the carbon atom to which they are attached are C=CH-CH=CH-Ph, X and Y taken together with the carbon atom to which they are attached are C=N-Ac, L = CH₂CH₃, Z = H, and R₂' = H;
Example 36. Compound of formula I: A is (CH₂)₃-Ph, B is H, X and Y taken together with the carbon atom to which they are attached are C=N-Ac, L = CH₂CH₃, Z = H, and R₂' = H;
Example 37. Compound of formula I: A and B taken together with the carbon atom to which they are attached are C=CH-CH=CH-3-pyridyl, X and Y taken together with the carbon atom to which they are attached are C=N-Ac, L = CH₂CH₃, Z = H, and R₂' = H;
Example 38. Compound of formula I: A and B taken together with the carbon atom to which they are attached are C=CH-CH=CH-3-quinolyl, X and Y taken together with the carbon atom to which they are attached are C=N-Ac, L = CH₂CH₃, Z = H, and R₂' = H;
Example 39. Compound of formula I: A and B taken together with the carbon atom to which they are attached are C=CH-2-quinolyl, X and Y taken together with the carbon atom to which they are attached are C=N-Ac, L = CH₂CH₃, Z = H, and R₂' = H;
Example 40. Compound of formula I: A and B taken together with the carbon atom to which they are attached are C=CH-2-quinolyl, X and Y taken together with the carbon atom to which they are attached are C=N-H, L = CH₂CH₃, Z = H; and R₂' =H;
Example 41. Compound of formula I: A and B taken together with the carbon atom to which they are attached are C=CH-4-biphenyl, X and Y taken together with the carbon atom to which they are attached are C=N-Ac, L = CH₂CH₃, Z = H, and R₂' = H;
Example 42. Compound of formula I: A and B taken together with the carbon atom to which they are attached are C=CH-3-biphenyl, X and Y taken together with the carbon atom to which they are attached are C=N-Ac, L = CH₂CH₃, Z = H, and R₂' = H;
Example 43. Compound of formula I: A and B taken together with the carbon atom to which they are attached are C=CH-4-phenoxyphenyl, X and Y taken together with the carbon atom to which they are attached are C=N-Ac, L = CH₂CH₃, Z = H, and R₂' = H;
Example 44. Compound of formula I: A and B taken together with the carbon atom to which they are attached are C=CH-Ph, X and Y taken together with the carbon atom to which they are attached are C=N-Ac, L = CH₂CH₃, Z = H, and R₂' = H;
Example 45. Compound of formula **I:** A and B taken together with the carbon atom to which they are attached are C=CH-2-(2pyridyl)-thiophen-5-yl, X and Y taken together with the carbon atom to which they are attached are C=N-Ac, L = CH₂CH₃, Z = H, and R₂' = H; or
Example 46. Compound of formula I: A and B taken together with the carbon atom to which they are attached are C=O, X and Y taken together with the carbon atom to which they are attached are C=N-Ac, L = CH₂CH₃, Z = F, and R₂' = Ac.

Further representative species of the present invention are:
Example compounds 47-114 of the formula **A:** 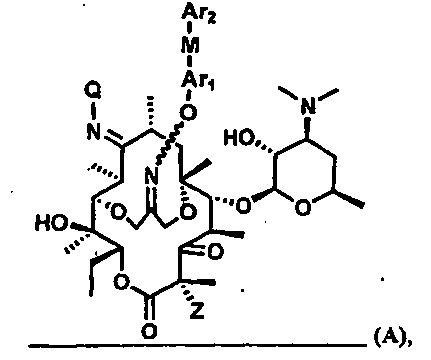 wherein Ar₁, Ar₂, M, Q, and Z are delineated for each example in Table A:

**TABLE A**

| Example | **Q** | **-Ar₁-M-Ar₂** | **Z** |
|---|---|---|---|
| Example 47. | Ac | 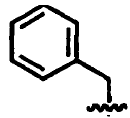 | H |
| Example 48. | Ac | 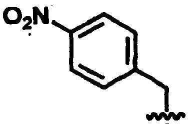 | H |
| Example 49. | Ac | 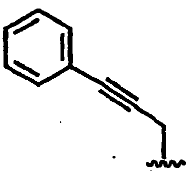 | H |
| Example 50. | Ac | 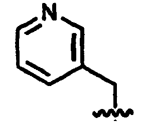 | H |
| Example 51. | Ac | 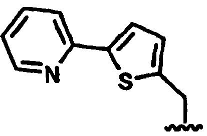 | H |
| Example 52. | Ac | 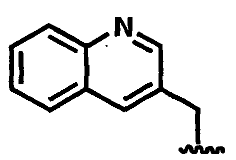 | H |
| Example 53. | Ac | 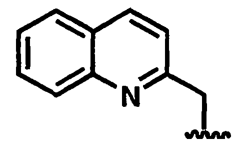 | H |
| Example 54. | Ac | 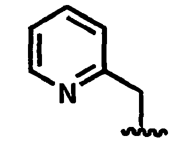 | H |
| Example 55. | MOM | 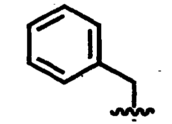 | H |
| Example 56. | Ac | 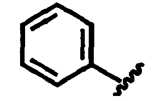 | H |
| Example 57. | Ac | 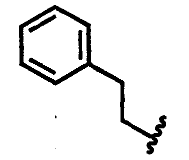 | H |
| Example 58. | Ac | 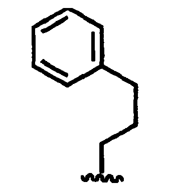 | H |
| Example 59. | Ac | 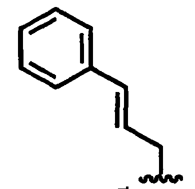 | H |
| Example 60. | Ac | 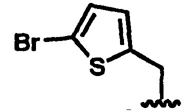 | H |
| Example 61. | Ac | 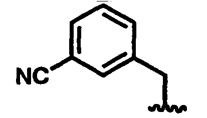 | H |
| Example 62. | Ac | 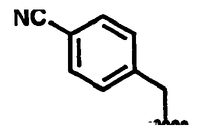 | H |
| Example 63. | Ac | 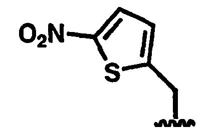 | H |
| Example 64. | Ac | 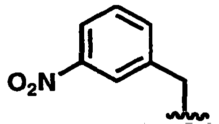 | H |
| Example 65. | Ac | 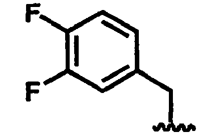 | H |
| Example 66. | Ac | 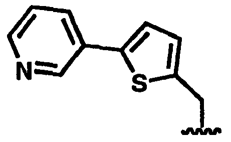 | H |
| Example 67. | Ac | 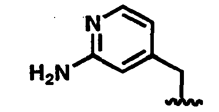 | H |
| Example 68. | Ac | 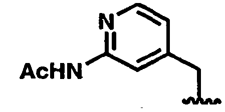 | H |
| Example 69. | Ac | 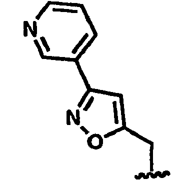 | H |
| Example 70. | Ac | 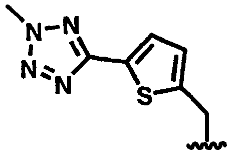 | H |
| Example 71. | Ac | 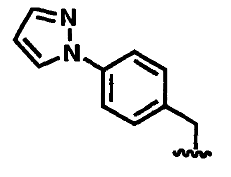 | H |
| Example 72. | Ac | 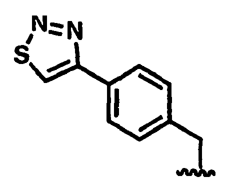 | H |
| Example 73. | Ac | 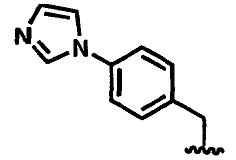 | H |
| Example 74. | Ac | 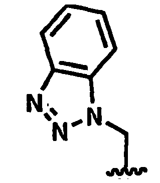 | H |
| Example 75. | Ac | 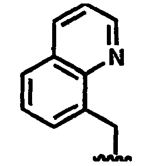 | H |
| Example 76. | Ac | 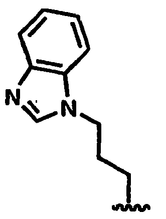 | H |
| Example 77. | Ac | 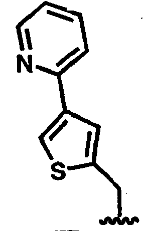 | H |
| Example 78. | Ac | 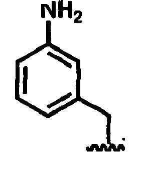 | H |
| Example 79. | Ac | 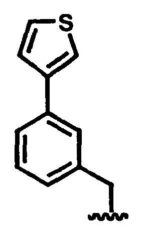 | H |
| Example 80. | Ac | 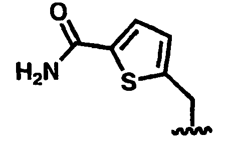 | F |
| Example 81. | H | 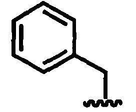 | H |
| Example 82. | Ac | 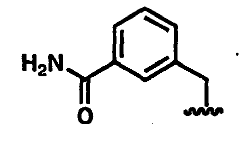 | H |
| Example 83. | Ac | 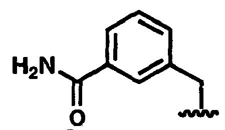 | F |
| Example 84. | Ac | 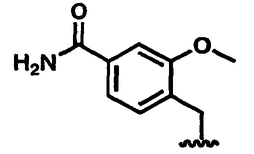 | H |
| Example 85. | Ac | 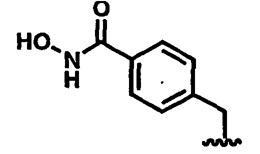 | H |
| Example 86. | Ac | 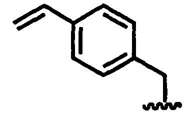 | H |
| Example 87. | Ac | 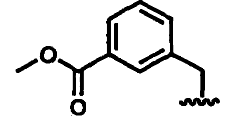 | H |
| Example 88. | Ac | 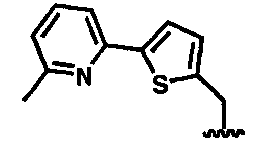 | H |
| Example 89. | Ac | 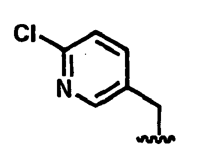 | H |
| Example 90. | H | 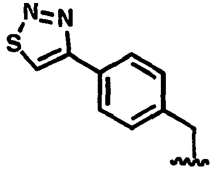 | H |
| Example 91. | H | 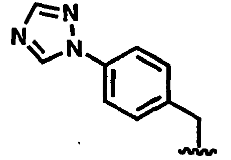 | H |
| Example 92. | OMe | 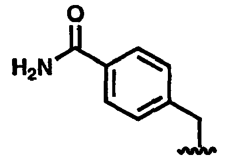 | H |
| Example 93. | MOM | 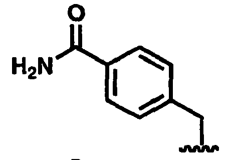 | H |
| Example 94. | -OCH₂CN | 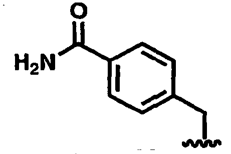 | H |
| Example 95. | -OCH₂CH₂OH | 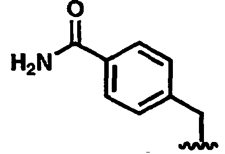 | H |
| Example 96. | H | 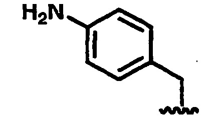 | H |
| Example 97. | Ac | 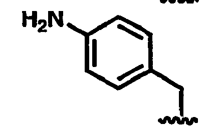 | H |
| Example 98. | Ac | 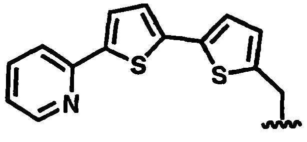 | H |
| Example 99. | Ac | 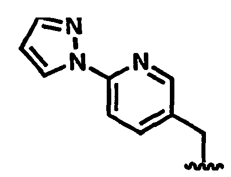 | H |
| Example 100. | Ac | 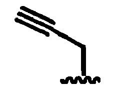 | H |
| Example 101. | Ac | 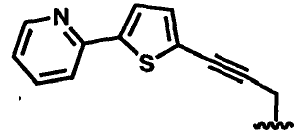 | H |
| Example 102. | Ac | 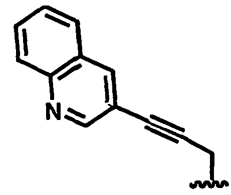 | H |
| Example 103. | Ac | 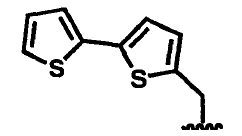 | H |
| Example 104. | Ac | 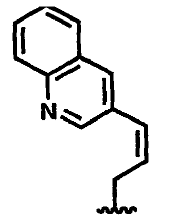 | H |
| Example 105. | Ac | 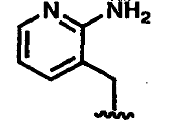 | H |
| Example 106. | Ac | 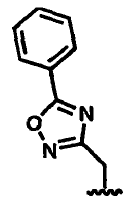 | H |
| Example 107. | Ac | 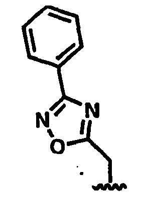 | H |
| Example 108. | Ac | 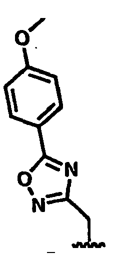 | H |
| Example 109. | Ac | 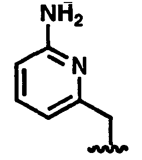 | H |
| Example 110. | Ac | 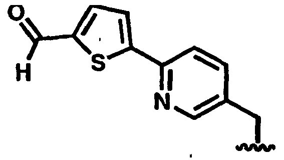 | H |
| Example 111. | Ac | 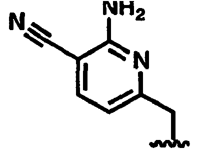 | H |
| Example 112. | Ac | 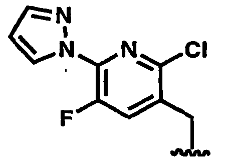 | H |
| Example 113. | Ac | 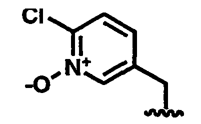 | H |
| Example 114. | Ac | 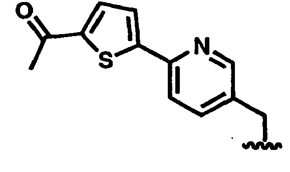 | H |

Example compounds 115-262 of formula **A1:** 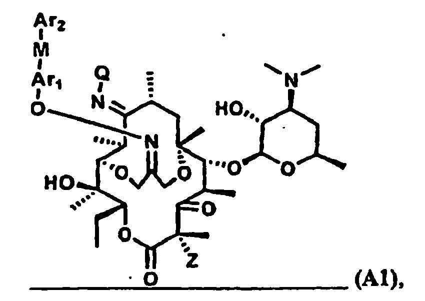 wherein Ar₁, Ar₂, M, Q, and Z are delineated for each example in Table A1:

**TABLE A1.**

| Example | **Q** | **-Ar₁-M-Ar₂** | **Z** |
|---|---|---|---|
| Example 115. | Ac | 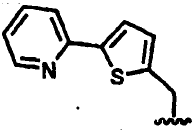 | H |
| Example 116. | Ac | 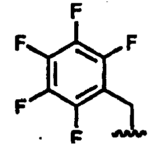 | H |
| Example 117. | Ac | 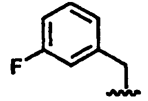 | H |
| Example 118. | Ac | 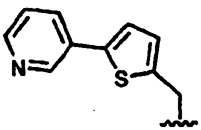 | H |
| Example 119. | Ac | 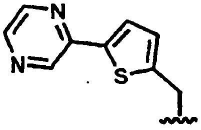 | H |
| Example 120. | Ac | 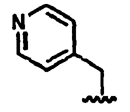 | H |
| Example 121. | Ac | 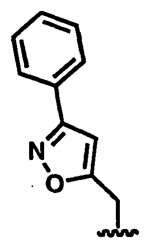 | H |
| Example 122. | Ac | 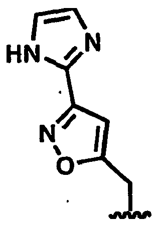 | H |
| Example 123. | Ac | 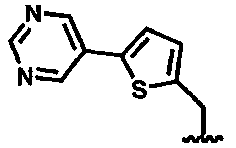 | H |
| Example 124. | Ac | 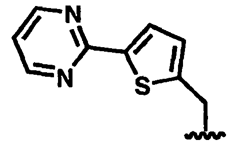 | H |
| Example 125. | Ac | 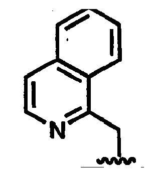 | H |
| Example 126. | Ac | 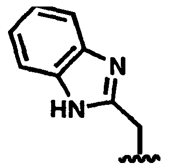 | H |
| Example 127. | Ac | 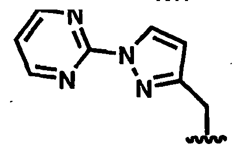 | H |
| Example 128. | Ac | 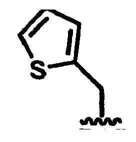 | H |
| Example 129. | Ac | 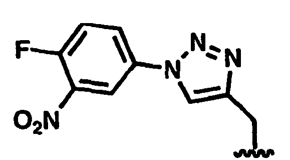 | H |
| Example 130. | Ac | 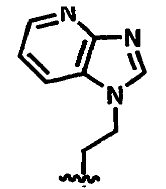 | H |
| Example 131. | Ac | 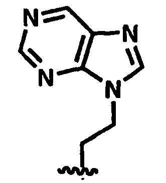 | H |
| Example 132. | Ac | 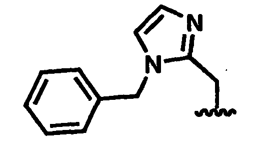 | H |
| Example 133. | Ac | 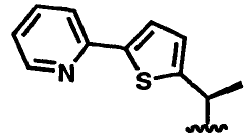 | H |
| Example 134. | Ac | 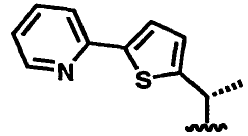 | H |
| Example 135. | Ac | 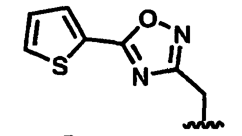 | H |
| Example 136. | Ac | 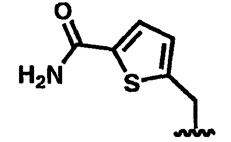 | H |
| Example 137. | Ac | 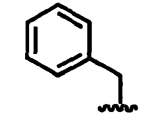 | H |
| Example 138. | Ac | 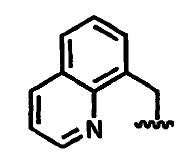 | F |
| Example 139. | Ac | 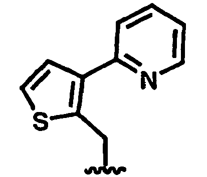 | H |
| Example 140. | Ac | 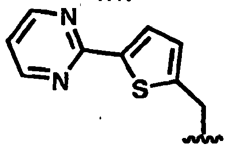 | F |
| Example 141. | Ac | 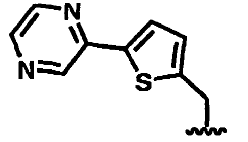 | F |
| Example 142. | Ac | 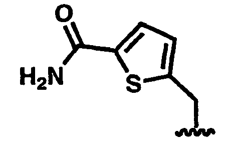 | F |
| Example 143. | Ac | 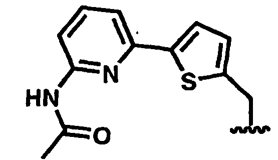 | H |
| Example 144. | Ac | 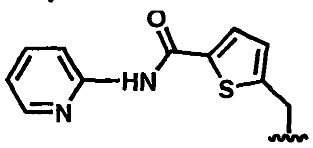 | H |
| Example 145. | Ac | 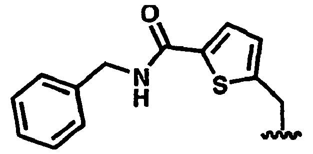 | H |
| Example 146. | Ac | 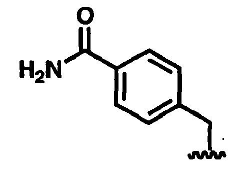 | H |
| Example 147. | Ac | 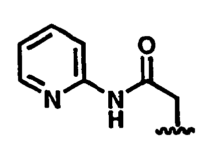 | H |
| Example 148. | Ac | 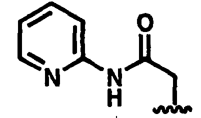 | F |
| Example 149. | Ac | 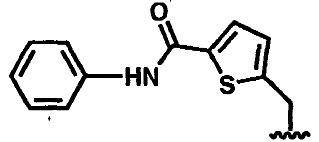 | H |
| Example 150. | Ac | 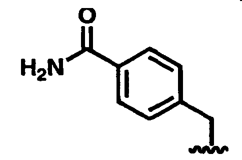 | F |
| Example 151. | Ac | 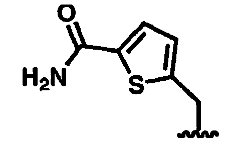 | H |
| Example 152. | Ac | 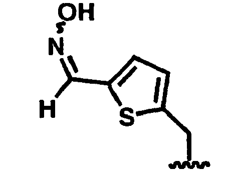 | H |
| Example 153. | Ac | 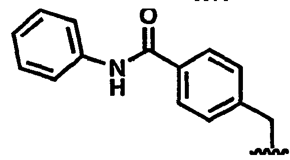 | H |
| Example 154. | Ac | 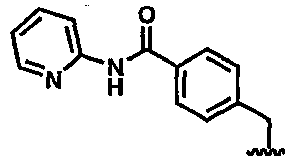 | H |
| Example 155. | Ac | 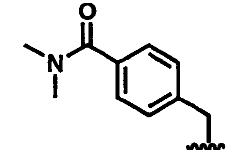 | H |
| Example 156. | Ac | 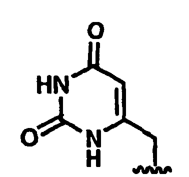 | H |
| Example 157. | Ac | 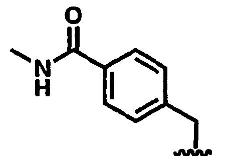 | H |
| Example 158. | Ac | 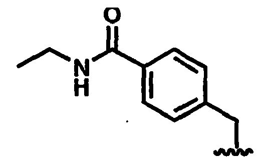 | H |
| Example 159. | Ac | 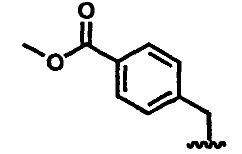 | H |
| Example 160. | Ac | 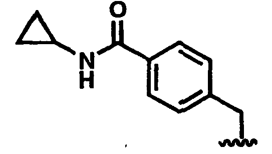 | H |
| Example 161. | Ac | 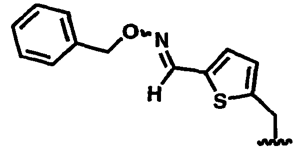 | H |
| Example 162. | Ac | 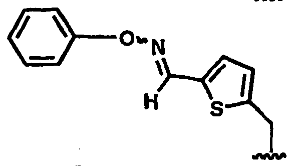 | H |
| Example 163. | Ac | 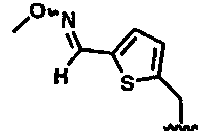 | H |
| Example 164. | Ac | 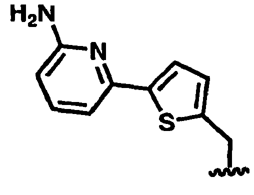 | H |
| Example 165. | Ac | 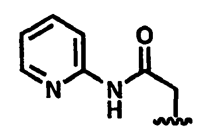 | H |
| Example 166. | Ac | 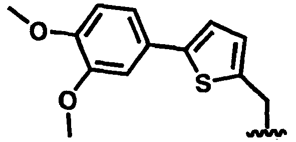 | H |
| Example 167. | Ac | 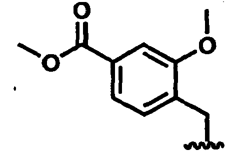 | H |
| Example 168. | Ac | 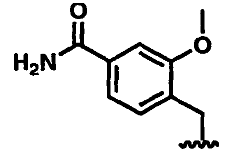 | H |
| Example 169. | Ac | 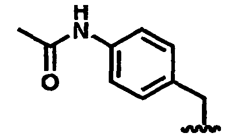 | H |
| Example 170. | Ac | 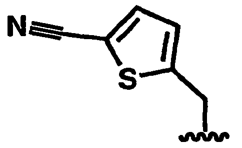 | H |
| Example 171. | Ac | 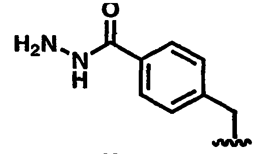 | H |
| Example 172. | Ac | 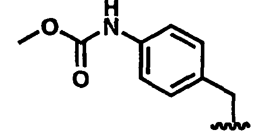 | H |
| Example 173. | Ac | 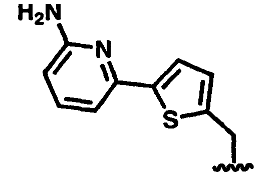 | F |
| Example 174. | Ac | 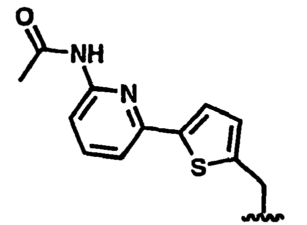 | F |
| Example 175. | Ac | 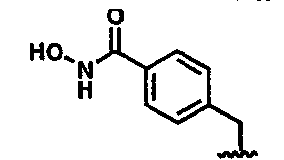 | H |
| Example 176. | Ac | 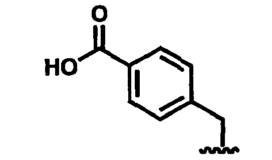 | H |
| Example 177. | Ac | 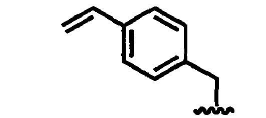 | H |
| Example 178. | Ac | 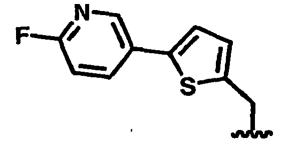 | H |
| Example 179. | Ac | 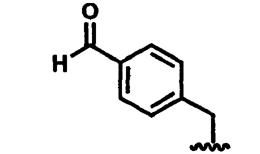 | H |
| Example 180. | H | 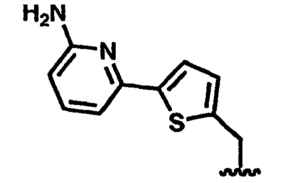 | H |
| Example 181. | Ac | 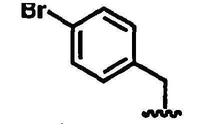 | H |
| Example 182. | Ac | 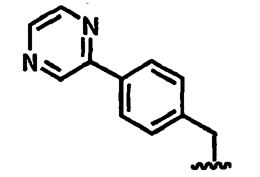 | H |
| Example 183. | Ac | 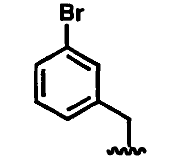 | H |
| Example 184. | Ac | 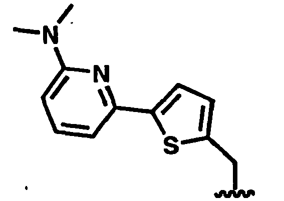 | F |
| Example 185. | Ac | 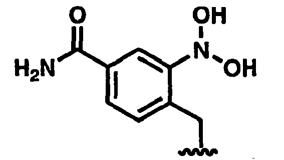 | H |
| Example 186. | Ac | 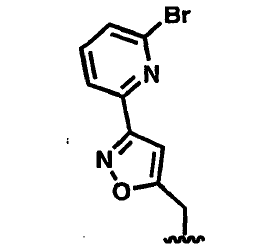 | H |
| Example 187. | Ac | 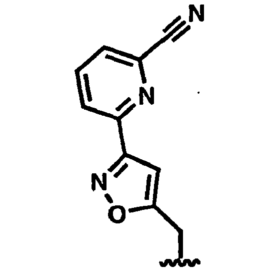 | H |
| Example 188. | Ac | 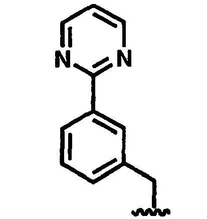 | H |
| Example 189. | Ac | 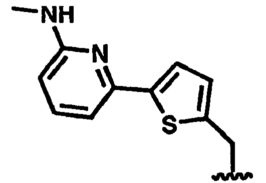 | F |
| Example 190. | Ac | 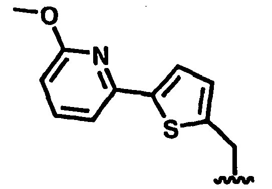 | H |
| Example 191. | H | 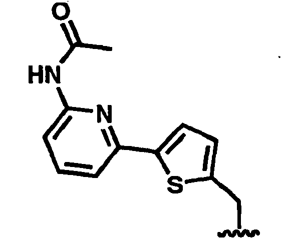 | H |
| Example 192. | Ac | 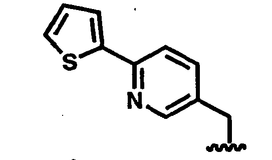 | H |
| Example 193. | Ac | 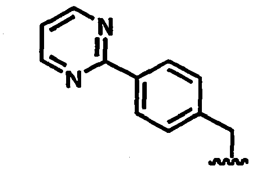 | H |
| Example 194. | H | 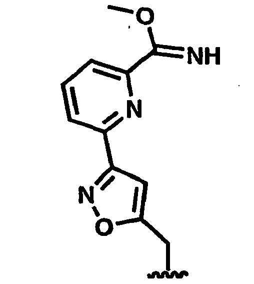 | H |
| Example 195. | Ac | 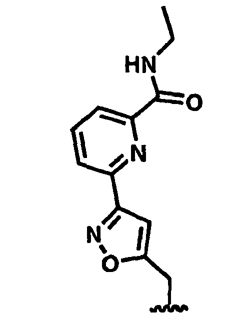 | H |
| Example 196. | H | 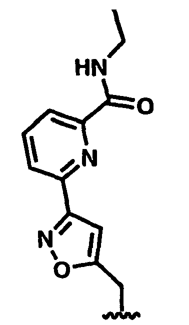 | H |
| Example 197. | H | 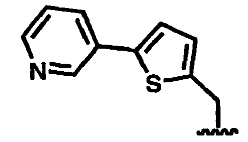 | H |
| Example 198. | H | 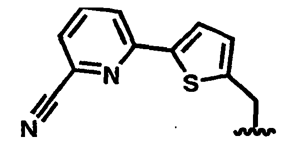 | H |
| Example 199. | Ac | 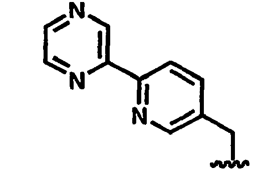 | H |
| Example 200. | Ac | 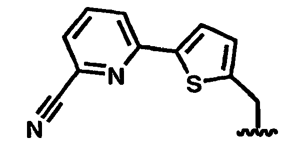 | H |
| Example 201. | Ac | 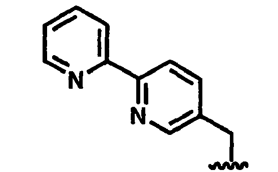 | H |
| Example 202. | H | 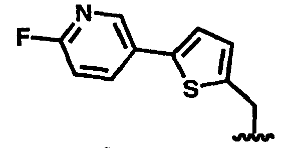 | H |
| Example 203. | H | 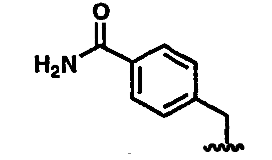 | H |
| Example 204. | H | 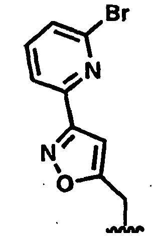 | H |
| Example 205. | Ac | 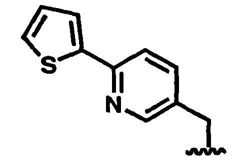 | H |
| Example 206. | H | 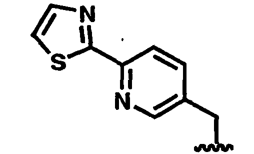 | H |
| Example 207. | Ac | 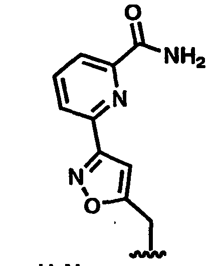 | H |
| Example 208. | Ac | 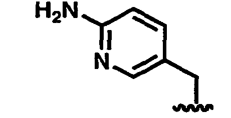 | H |
| Example 209. | Ac | 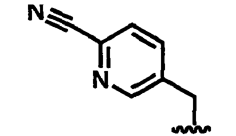 | H |
| Example 210. | Ac | 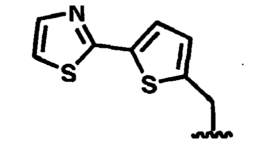 | H |
| Example 211. | Ac | 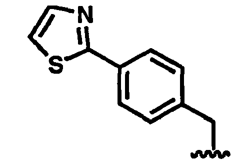 | F |
| Example 212. | H | 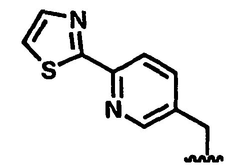 | F |
| Example 213. | H | 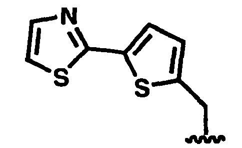 | H |
| Example 214. | Ac | 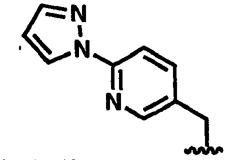 | F |
| Example 215. | Ac | 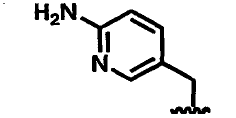 | F |
| Example 216. | Ac | 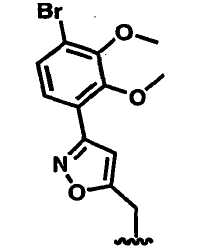 | H |
| Example 217. | Ac | 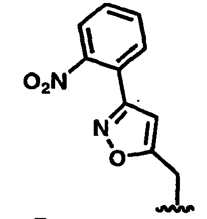 | H |
| Example 218. | Ac | 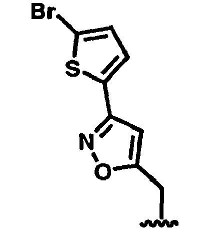 | H |
| Example 219. | Ac | 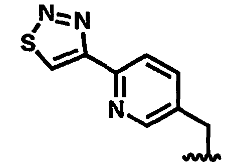 | F |
| Example 220. | Ac | 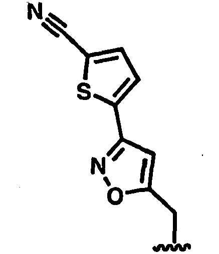 | H |
| Example 221. | Ac | 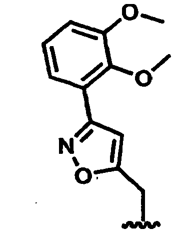 | H |
| Example 222. | Ac | 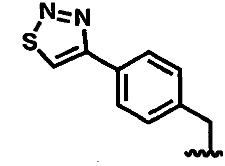 | H |
| Example 223. | Ac | 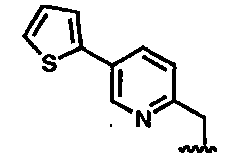 | H |
| Example 224. | Ac | 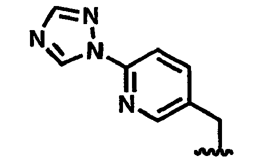 | H |
| Example 225. | Ac | 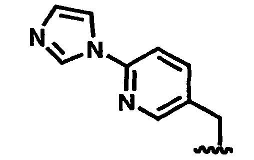 | H |
| Example 226. | Ac | 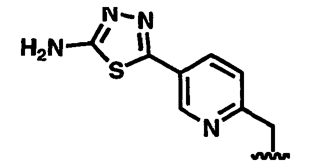 | H |
| Example 227. | Ac | 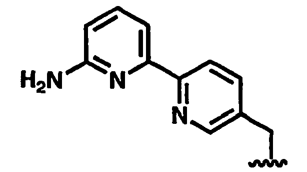 | H |
| Example 228. | Ac | 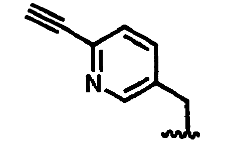 | H |
| Example 229. | H | 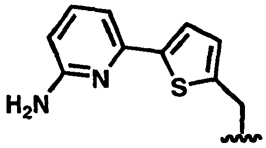 | F |
| Example 230. | H | 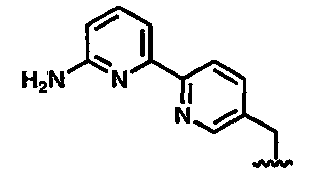 | H |
| Example 231. | Ac | 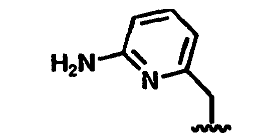 | H |
| Example 232. | Ac | 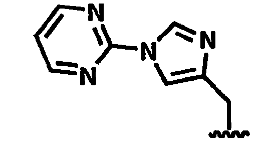 | H |
| Example 233. | Ac | 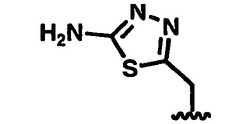 | H |
| Example 234. | Ac | 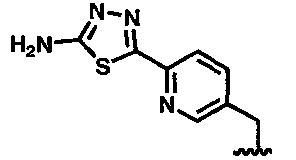 | H |
| Example 235. | Ac | 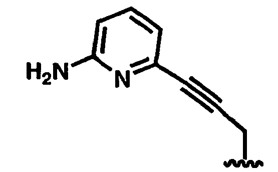 | H |
| Example 236. | Ac | 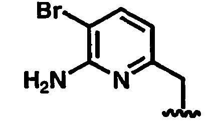 | H |
| Example 237. | Ac | 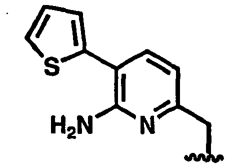 | H |
| Example 238. | Ac | 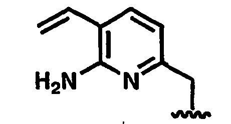 | H |
| Example 239. | Ac | 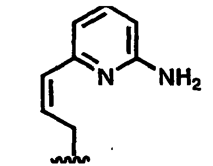 | H |
| Example 240. | Ac | 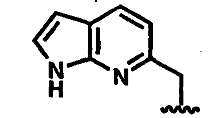 | H |
| Example 241. | Ac | 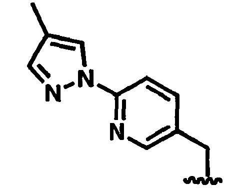 | H |
| Example 242. | Ac | 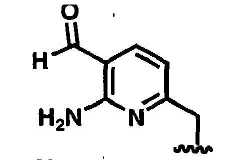 | H |
| Example 243. | Ac | 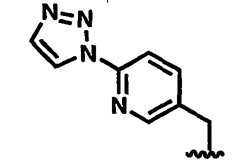 | H |
| Example 244. | Ac | 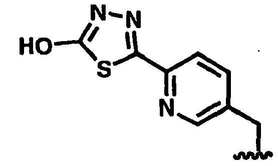 | H |
| Example 245. | Ac | 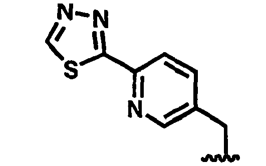 | H |
| Example 246. | Ac | 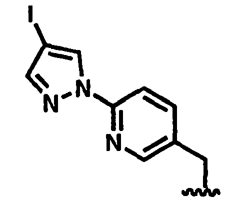 | H |
| Example 247. | Ac | 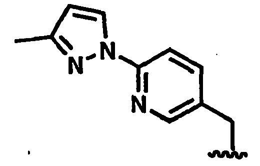 | H |
| Example 248. | Ac | 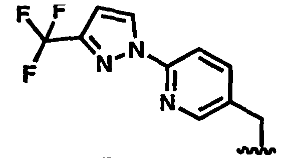 | H |
| Example 249. | Ac | 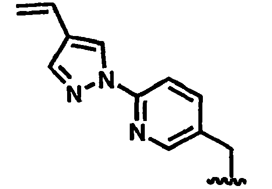 | H |
| Example 250. | Ac | 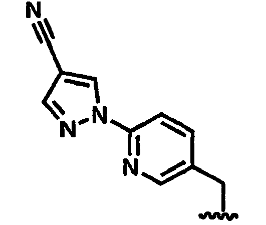 | H |
| Example 251. | Ac | 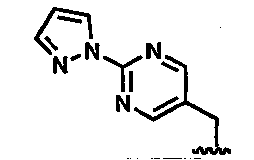 | H |
| Example 252. | Ac | 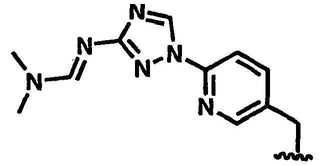 | H |
| Example 253. | Ac | 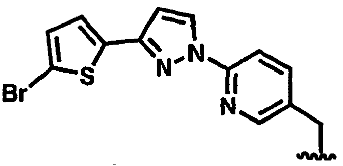 | H |
| Example 254. | Ac | 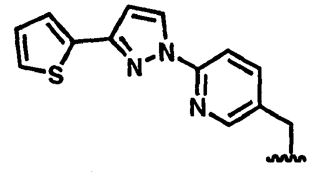 | H |
| Example 255. | Ac | 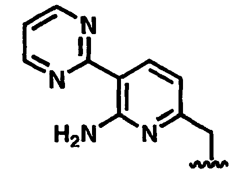 | H |
| Example 256. | H | 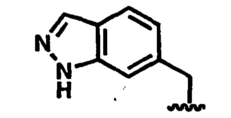 | H |
| Example 257. | Ac | 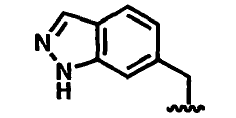 | H |
| Example 258. | Ac | 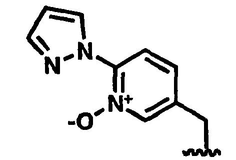 | H |
| Example 259. | Ac | 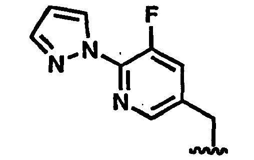 | H |
| Example 260. | -COCH₂CH₃ | 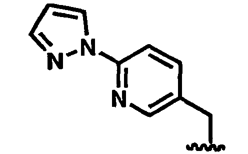 | H |
| Example 261. | Ac | 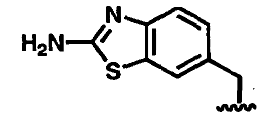 | H |
| Example 262. | Ac | 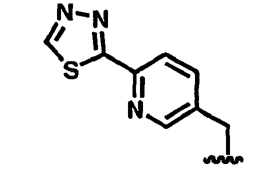 | H |

Example compounds 263-337 of formula **A2:** 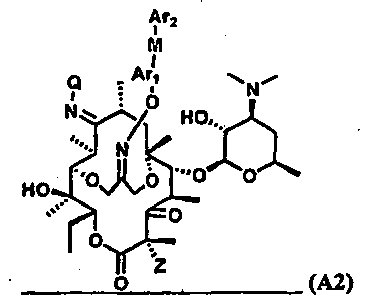 wherein Ar₁, Ar₂, M, Q, and Z are delineated for each example in Table A2:

**TABLE A2**

| Example | **Q** | **-Ar₁-M-Ar₂** | **Z** |
|---|---|---|---|
| Example 263. | Ac | 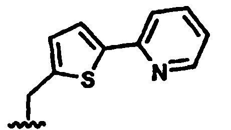 | H |
| Example 264. | Ac | 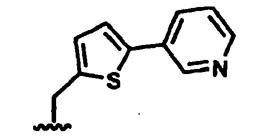 | H |
| Example 265. | Ac | 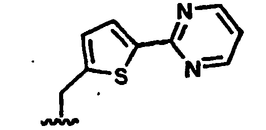 | H |
| Example 266. | Ac | 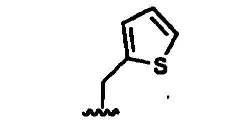 | H |
| Example 267. | Ac | 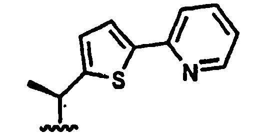 | H |
| Example 268. | Ac | 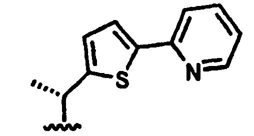 | H |
| Example 269. | Ac | 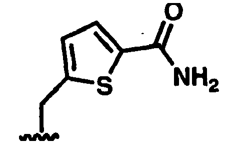 | H |
| Example 270. | Ac | 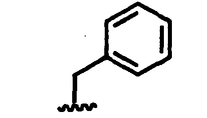 | H |
| Example 271. | Ac | 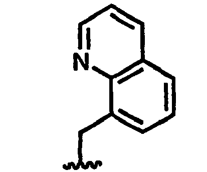 | F |
| Example 272. | Ac | 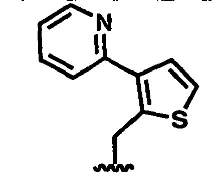 | H |
| Example 273. | Ac | 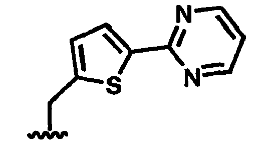 | F |
| Example 274. | Ac | 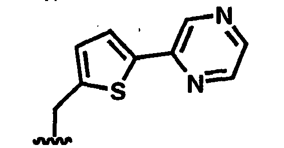 | F |
| Example 275. | Ac | 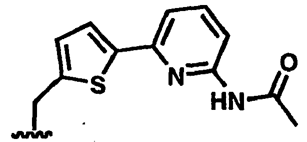 | H |
| Example 276. | Ac | 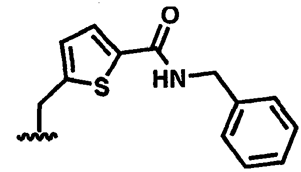 | H |
| Example 277. | Ac | 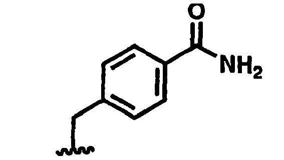 | H |
| Example 278. | Ac | 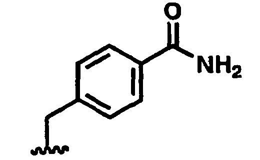 | F |
| Example 279. | Ac | 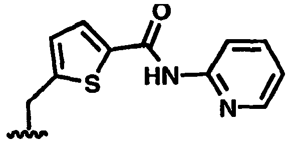 | H |
| Example 280. | Ac | 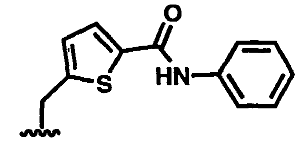 | H |
| Example 281. | Ac | 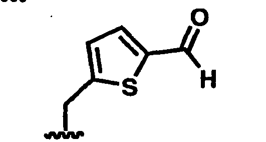 | H |
| Example 282. | Ac | 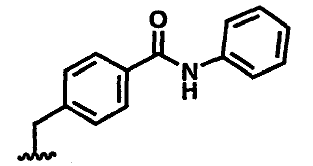 | H |
| Example 283. | Ac | 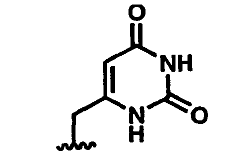 | H |
| Example 284. | Ac | 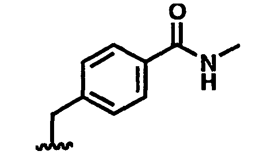 | H |
| Example 285. | Ac | 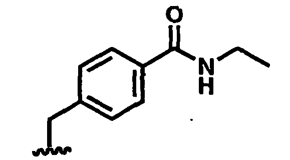 | H |
| Example 286. | Ac | 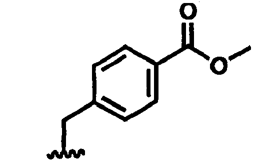 | H |
| Example 287. | Ac | 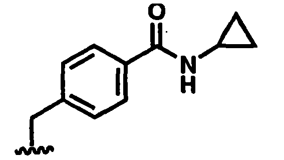 | H |
| Example 288. | Ac | 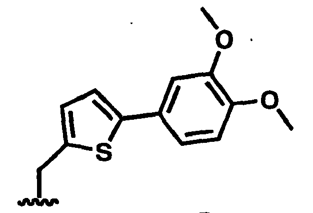 | H |
| Example 289. | Ac | 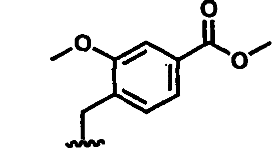 | H |
| Example 290. | Ac | 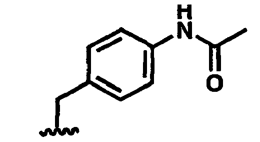 | H |
| Example 291. | Ac | 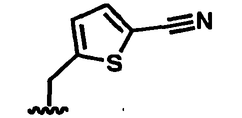 | H |
| Example 292. | Ac | 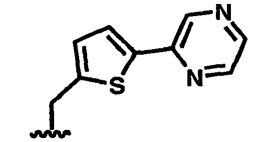 | H |
| Example 293. | Ac | 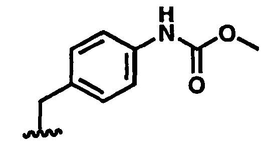 | H |
| Example 294. | Ac | 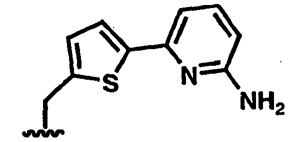 | F |
| Example 295. | Ac | 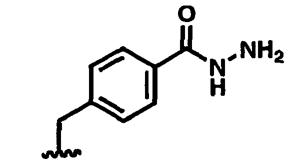 | H |
| Example 296. | Ac | 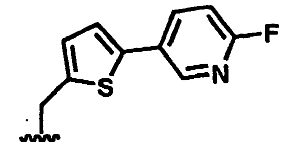 | H |
| Example 297. | Ac | 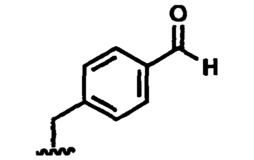 | H |
| Example 298. | Ac | 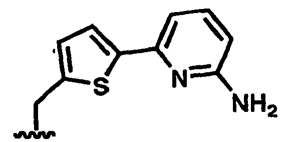 | H |
| Example 299. | H | 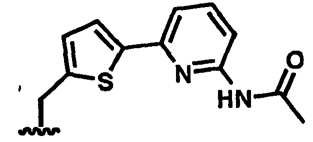 | H |
| Example 300. | Ac | 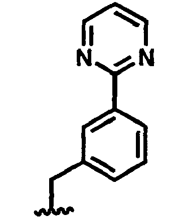 | H |
| Example 301. | Ac | 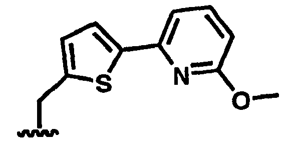 | H |
| Example 302. | H | 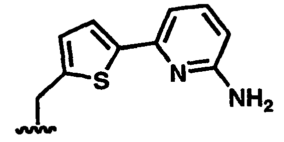 | H |
| Example 303. | H | 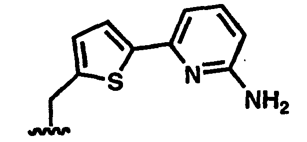 | F |
| Example 304. | Ac | 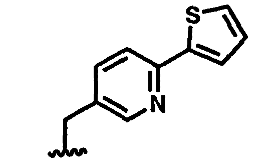 | H |
| Example 305. | Ac | 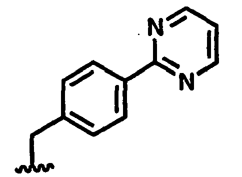 | H |
| Example 306. | Ac | 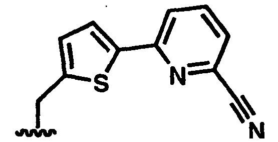 | H |
| Example 307. | Ac | 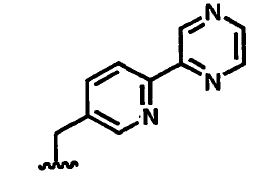 | H |
| Example 308. | Ac | 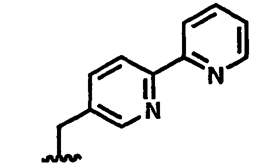 | H |
| Example 309. | Ac | 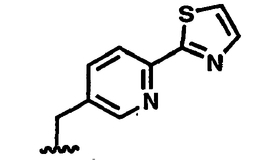 | H |
| Example 310. | Ac | 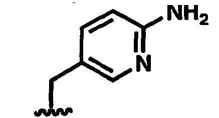 | H |
| Example 311. | Ac | 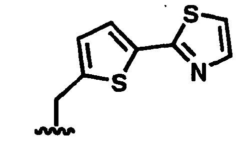 | H |
| Example 312. | Ac | 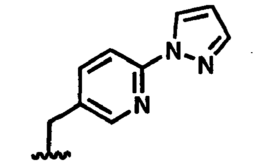 | F |
| Example 313. | Ac | 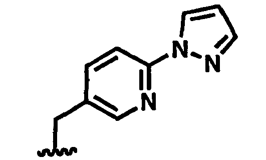 | H |
| Example 314. | Ac | 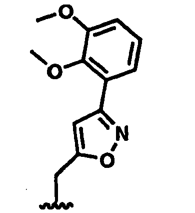 | H |
| Example 315. | Ac | 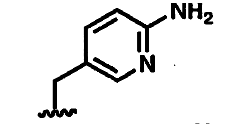 | F |
| Example 316. | Ac | 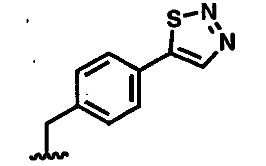 | H |
| Example 317. | Ac | 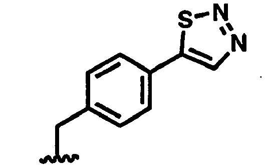 | F |
| Example 318. | Ac | 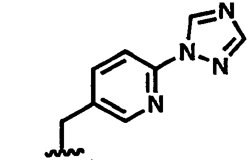 | H |
| Example 319. | Ac | 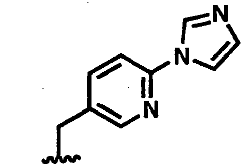 | H |
| Example 320. | Ac | 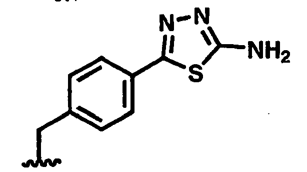 | H |
| Example 321. | Ac | 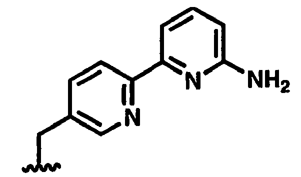 | H |
| Example 322. | Ac | 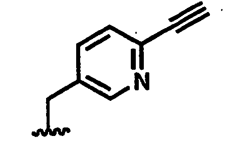 | H |
| Example 323. | Ac | 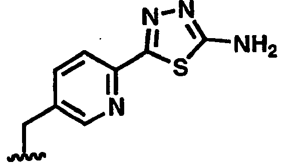 | H |
| Example 324. | Ac | 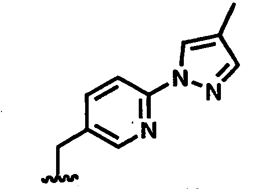 | H |
| Example 325. | Ac | 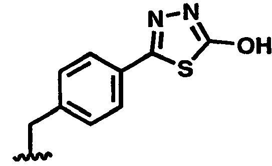 | H |
| Example 326. | Ac | 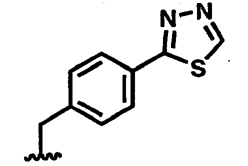 | H |
| Example 327. | Ac | 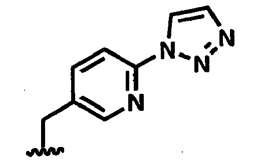 | H |
| Example 328. | Ac | 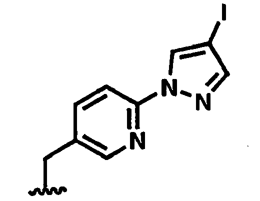 | H |
| Example 329. | Ac | 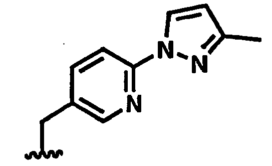 | H |
| Example 330. | Ac | 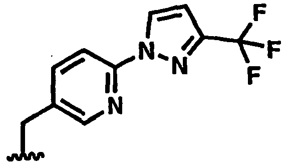 | H |
| Example 331. | Ac | 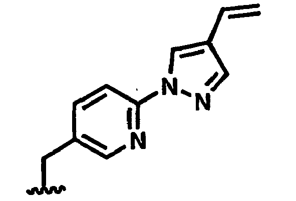 | H |
| Example 332. | Ac | 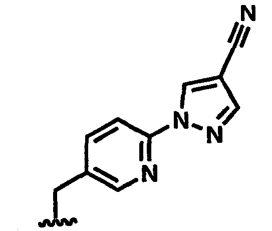 | H |
| Example 333. | Ac | 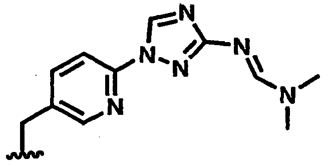 | H |
| Example 334. | Ac | 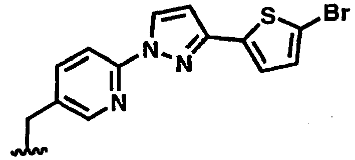 | H |
| Example 335. | Ac | 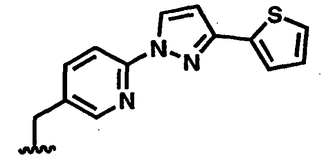 | H |
| Example 336. | H | 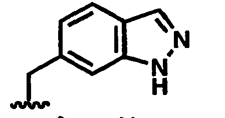 | H |
| Example 337. | Ac | 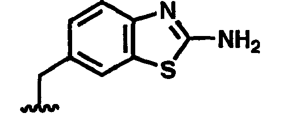 | H |

Example compounds 338-352 of formula **B:** 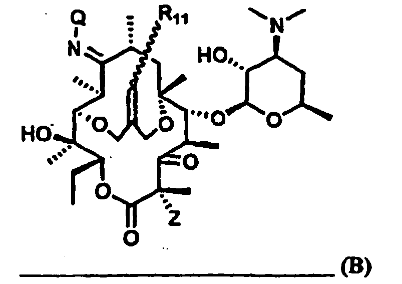 wherein R₁₁, Q, and Z are delineated for each example in Table B:

**Table B**

| Example | **Q** | **R₁₁** | **Z** |
|---|---|---|---|
| Example 338. | H | H | H |
| Example 339. | MOM | H | H |
| Example 340. | MOM | 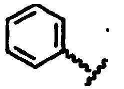 | H |
| Example 341. | Ac | 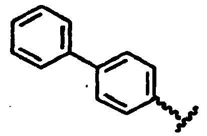 | H |
| Example 342. | Ac | 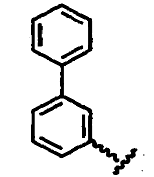 | H |
| Example 343. | Ac | 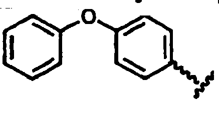 | H |
| Example 344. | Ac | 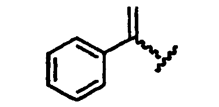 | H |
| Example 345. | Propionyl | 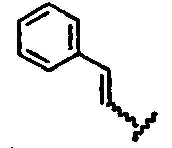 | H |
| Example 346. | Ac | 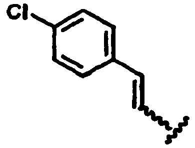 | H |
| Example 347. | Methyl carbamate | 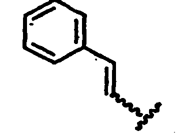 | H |
| Example 348. | urea | H | H |
| Example 349. | Me | H | H |
| Example 350. | BOM | H | H |
| Example 351. | Ac | 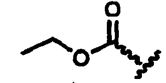 | H |
| Example 352. | Ac | 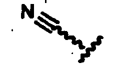 | H |

Example compounds 353-375 of formula **B1:** 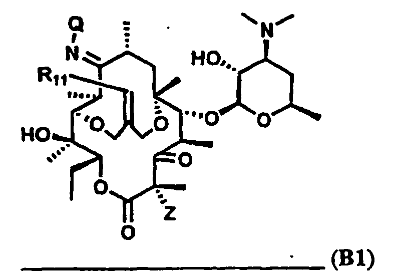 wherein R₁₁, Q, and Z are delineated for each example in Table B1:

| Examples | **Q** | **R₁₁** | **Z** |
|---|---|---|---|
| Example 353. | Ac | 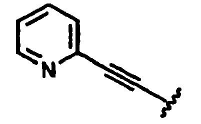 | H |
| Example 354. | Ac | 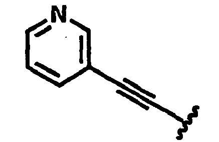 | H |
| Example 355. | Ac | 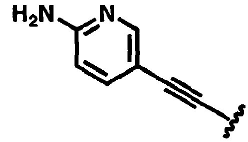 | H |
| Example 356. | Ac | 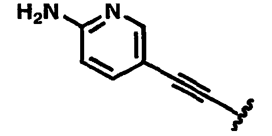 | H |
| Example 357. | Ac | 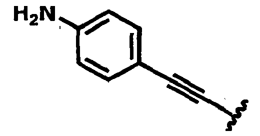 | H |
| Example 358. | Ac | 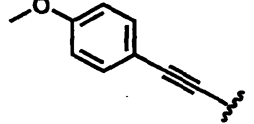 | H |
| Example 359. | Ac | 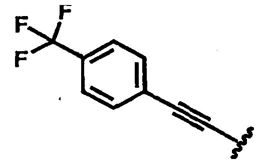 | H |
| Example 360. | Ac | 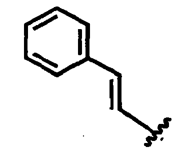 | F |
| Example 361. | Ac | 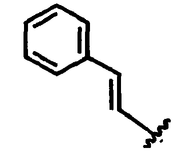 | H |
| Example 362. | Ac | 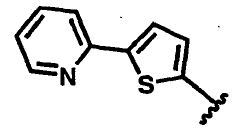 | H |
| Example 363. | Ac | 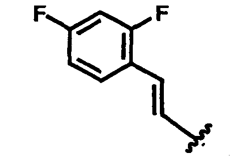 | H |
| Example 364. | 2-methoxyacetamide | 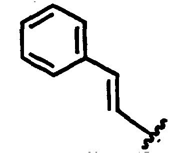 | H |
| Example 365. | 2-O-acyl-acetamide | 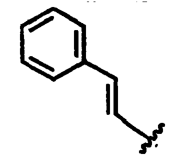 | H |
| Example 366. | 2-Fmoc-acetamide | 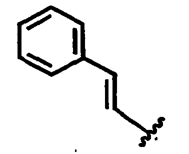 | H |
| Example 367. | Ac | 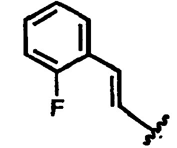 | H |
| Example 368. | Ac | 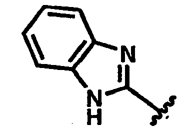 | H |
| Example 369. | 2-hydroxy acetyl | 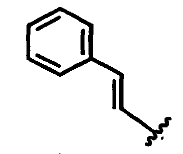 | H |
| Example 370. | 2-aminoacetyl | 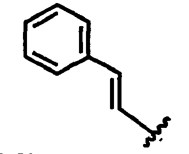 | H |
| Example 371. | Ac | 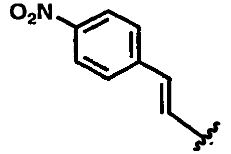 | H |
| Example 372. | Ac | 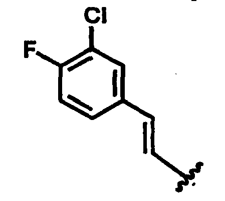 | H |
| Example 373. | Ac | 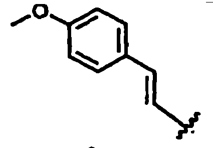 | H |
| Example 374. | Ac | 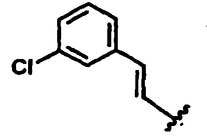 | H |
| Example 375. | Ac | 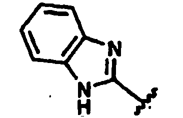 | H |

Example compounds 376-384 of formula **B2:** 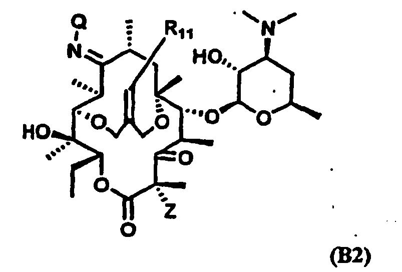 wherein R₁₁, Q, and Z are delineated for each example in Table B2:

**Table B2**

| Example | Q | **R₁₁** | **Z** |
|---|---|---|---|
| Example 376. | Ac | 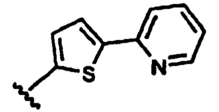 | H |
| Example 377. | Ac | 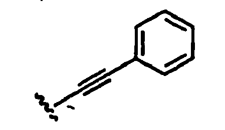 | H |
| Example 378. | Ac | 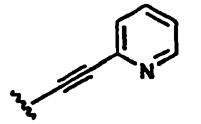 | H |
| Example 379. | Ac | 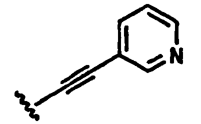 | H |
| Example 380. | Ac | 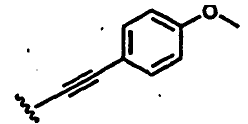 | H |
| Example 381. | Ac | 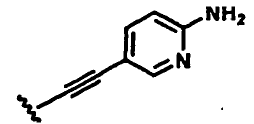 | H |
| Example 382. | Ac | 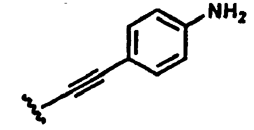 | H |
| Example 383. | Ac | 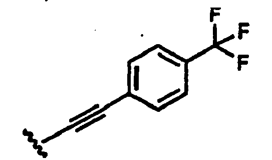 | H |
| Example 384. | Ac | 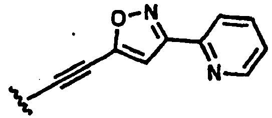 | H |

Example compounds 385-391 of formula **C:** 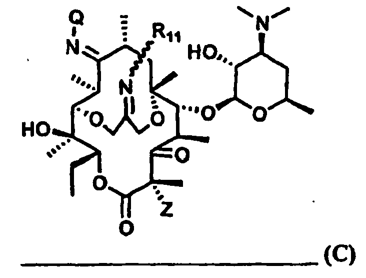 wherein R₁₁, Q, and Z are delineated for each example in Table C:

**Table C**

| Example | **Q** | **R₁₁** | **Z** |
|---|---|---|---|
| Example 385. | Ac | 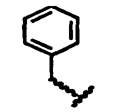 | H |
| Example 386. | Ac | 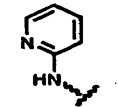 | H |
| Example 387. | Ac | 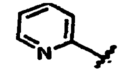 | H |
| Example 388. | Ac | 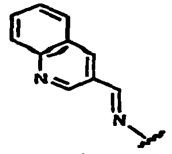 | H |
| Example 389. | Ac | 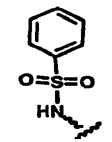 | H |
| Example 390. | Ac | 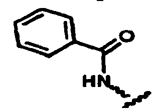 | H |
| Example 391. | Ac | 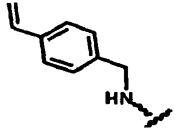 | H |

In another aspect, the present invention relates to the use of any compound of the present invention for the manufacture of a medicament for the control of a bacterial infection in a subject in need of such treatment. Identifying a subject in need of such treatment can be in the judgment of a subject or a health care professional and can be subjective (e. g. opinion) or objective (e. g. measurable by a test or diagnostic method).

The product may be packaged. The packaged product includes a container, one of the aforementioned compounds in the container, and a legend (e. g. , a label or an insert) associated with the container and indicating administration of the compound for treating a disorder associated with bacterial infection, including the diseases delineated herein.

In a further aspect of the present invention is a process of making any compound delineated herein via any synthetic route delineated herein.

### Definitions

Listed below are definitions of various terms used to describe this invention. These definitions apply to the terms as they are used throughout this specification and claims, unless otherwise limited in specific instances, either individually or as part of a larger group.

The terms"C1-C3 alkyl,""Cl-C6 alkyl" or "Cl-C12 alkyl, "as used herein, refer to saturated, straight-or branched-chain hydrocarbon radicals containing between one and three, one and twelve, or one and six carbon atoms, respectively. Examples of Cl-C3 alkyl radicals include methyl, ethyl, propyl and isopropyl radicals; examples of Cl-C6 alkyl radicals include, but are not limited to, methyl, ethyl, propyl, isopropyl, n-butyl, tert-butyl, neopentyl and n-hexyl radicals; and examples of Cl-C12 alkyl radicals include, but are not limited to, ethyl, propyl, isopropyl, n-hexyl, octyl, decyl, dodecyl radicals.

The term "substituted alkyl," as used herein, refers to a "C₂-C₁₂ alkyl" or "C₁-C₆ alkyl" group as previously defined, substituted by independent replacement or one, two, or three of the hydrogen atoms thereon with substituents including, but not limited to, -F, -Cl, -Br, -I, -OH, protected hydroxy, -NO₂, -CN, -C₁-C₁₂-alkyl optionally substituted with halogen, C₂-C₁₂-alkenyl optionally substituted with halogen, -C₂-C₁₂-alkynyl optionally substituted with halogen, -NH₂, protected amino, -NH -C₁-C₁₂-alkyl, -NH -C₂-C₁₂-alkenyl, -NH -C₂-C₁₂-alkenyl, -NH -C₃-C₁₂-cycloalkyl, -NH -aryl, -NH -heteroaryl, -NH-heterocycloalkyl; -dialkylamino, -diarylamino, -diheteroarylamino, -O-C₁-C₁₂-alkyl, -O-C₂-C₁₂-alkenyl, -O-C₂-C₁₂-alkenyl, -O-C₃-C₁₂-cycloalkyl, -O-aryl, -O-heteroaryl, -O-heterocycloalkyl, -C(O)-C₁-C₁₂-alkyl, -C(O)- C₂-C₁₂-alkenyl, -C(O)-C₂-C₁₂-alkenyl, -C(O)-C₃-C₁₂-cycloalkyl, -C(O)-aryl, -C(O)-heteroaryl, -C(O)-heterocycloalkyl, -CONH₂, -CONH- C₁-C₁₂-alkyl, -CONH- C₂-C₁₂-alkenyl, -CONH- C₂-C₁₂-alkenyl, -CONH-C₃-C₁₂-cycloalkyl, -CONH-aryl, -CONH-heteroaryl, -CONH-heterocycloalkyl, -OCO₂- C-C₁₂-alkyl, -OCO₂- C₂-C₁₂-alkenyl, -OCO₂-C₂-C₁₂-alkenyl, -OCO₂-C₃-C₁₂-cycloalkyl, -OCO₂-aryl, -OCO₂-heteroaryl, -OCO₂-heterocycloalkyl, -OCONH₂, -OCONH- C₁-C₁₂-alkyl, -OCONH- C₂-C₁₂-alkenyl, -OCONH- C₂-C₁₂-alkenyl, -OCONH- C₃-C₁₂-cycloalkyl, -OCONH- aryl, -OCONH- heteroaryl, -OCONH- heterocycloalkyl, -NHC(O)- C₁-C₁₂-alkyl, -NHC(O)-C₂-C₁₂-alkenyl, -NHC(O)-C₂-C₁₂-alkenyl, -NHC(O)-C₃-C₁₂-cycloalkyl, -NHC(O)-aryl, -NHC(O)-heteroaryl, -NHC(O)-heterocycloalkyl, -NHCO₂-C₁-C₁₂-alkyl, -NHCO₂-C₂-C₁₂-alkenyl, -NHCO₂-C₂-C₁₂-alkenyl, -NHCO₂-C₃-C₁₂-cycloalkyl, -NHCO₂-aryl, -NHCO₂-heteroaryl, -NHCO₂-heterocycloalkyl, -NHC(O)NH₂, NHC(O)NH- C₁-C₁₂-alkyl, -NHC(O)NH-C₂-C₁₂-alkenyl, -NHC(O)NH-C₂-C₁₂-alkenyl, -NHC(O)NH-C₃-C₁₂-cycloalkyl, -NHC(O)NH-aryl, -NHC(O)NH-heteroaryl, -NHC(O)NH-heterocycloalkyl, NHC(S)NH₂, NHC(S)NH- C₁-C₁₂-alkyl, -NHC(S)NH-C₂-C₁₂-alkenyl, -NHC(S)NH-C₂-C₁₂-alkenyl, -NHC(S)NH-C₃-C₁₂-cycloalkyl, -NHC(S)NH-aryl, -NHC(S)NH-heteroaryl, -NHC(S)NH-heterocycloalkyl, -NHC(NH)NH₂, NHC(NH)NH- C₁-C₁₂-alkyl, -NHC(NH)NH-C₂-C₁₂-alkenyl, -NHC(NH)NH-C₂-C₁₂-alkenyl, -NHC(NH)NH-C₃-C₁₂-cycloalkyl, -NHC(NH)NH-aryl, -NHC(NH)NH-heteroaryl, -NHC(NH)NH-heterocycloalkyl, NHC(NH)-C₁-C₁₂-alkyl, NHC(NH)-C₂-C₁₂-alkenyl, -NHC(NH)-C₂-C₁₂-alkenyl, -NHC(NH)-C₃-C₁₂-cycloalkyl, -NHC(NH)-aryl, -NHC(NH)-heteroaryl, -NHC(NH)-heterocycloalkyl, -C(NH)NH-C₁-C₁₂-alkyl, -C(NH)NH-C₂-C₁₂-alkenyl, -C(NH)NH-C₂-C₁₂-alkenyl, -C(NH)NH-C₃-C₁₂-cycloalkyl, -C(NH)NH-aryl, -C(NH)NH-heteroaryl, -C(NH)NH-heterocycloalkyl, -S(O)-C₁-C₁₂-alkyl, - S(O)-C₂-C₁₂-alkenyl, -S(O)-C₂-C₁₂-alkenyl, -S(O)-C₃-C₁₂-cycloalkyl, - S(O)-aryl, - S(O)-heteroaryl, - S(O)-heterocycloalkyl -SO₂NH₂, -SO₂NH- C₁-C₁₂-alkyl, -SO₂NH-C₂-C₁₂-alkenyl, -SO₂NH-C₂-C₁₂-alkenyl, -SO₂NH-C₃-C₁₂-cycloalkyl, -SO₂NH- aryl, -SO₂NH- heteroaryl, -SO₂NH-heterocycloalkyl, -NHSO₂-C₁-C₁₂-alkyl, -NHSO₂-C₂-C₁₂-alkenyl, -NHSO₂-C₂-C₁₂-alkenyl, -NHSO₂-C₃-C₁₂-cycloalkyl, -NHSO₂-aryl, -NHSO₂-heteroaryl, -NHSO₂-heterocycloalkyl, -CH₂NH₂, -CH₂SO₂CH₃, -aryl, -arylalkyl, -heteroaryl, -heteroarylalkyl, -heterocycloalkyl, -C₃-C₁₂-cycloalkyl, -methoxymethoxy, -methoxyethoxy, -SH, -S-C₁-C₁₂-alkyl, -S-C₂-C₁₂-alkenyl, -S-C₂-C₁₂-alkenyl, -S-C₃-C₁₂-cycloalkyl, -S-aryl, -S-heteroaryl, -S-heterocycloalkyl, or methylthiomethyl.

The terms "C₂-C₁₂ alkenyl" or "C₂-C₆ alkenyl," as used herein, denote a monovalent group derived from a hydrocarbon moiety containing from two to twelve or two to six carbon atoms having at least one carbon-carbon double bond by the removal of a single hydrogen atom. Alkenyl groups include, but are not limited to, for example, ethenyl, propenyl, butenyl, 1-methyl-2-buten-1-yl, and the like.

The term "substituted alkenyl," as used herein, refers to a "C₂-C₁₂ alkenyl" or "C₂-C₆ alkenyl" group as previously defined, substituted by independent replacement or one, two, or three of the hydrogen atoms thereon with substituents including, but not limited to, -F, -Cl, -Br, -I, -OH, protected hydroxy, -NO₂, -CN, -C₁-C₁₂-alkyl optionally substituted with halogen, C₂-C₁₂-alkenyl optionally substituted with halogen, -C₂-C₁₂-alkynyl optionally substituted with halogen, -NH₂, protected amino, -NH -C₁-C₁₂-alkyl, -NH-C₂-C₁₂-alkenyl, -NH -C₂-C₁₂₋alkenyl, -NH -C₃-C₁₂-cycloalkyl, -NH -aryl, -NH -heteroaryl, -NH - heterocycloalkyl, -dialkylamino, -diarylamino, -diheteroarylamino, -O-C₁-C₁₂-alkyl, -O-C₂-C₁₂-alkenyl, -O-C₂-C₁₂-alkenyl, -O-C₃-C₁₂-cycloalkyl, -O-aryl, -0-heteroaryl, -O-heterocycloalkyl, -C(O)- C₁-C₁₂-alkyl, -C(O)- C₂-C₁₂-alkenyl, -C(O)- C₂-C₁₂-alkenyl, -C(O)-C₃-C₁₂-cycloalkyl, -C(O)-aryl, -C(O)-heteroaryl, -C(O)-heterocycloalkyl, -CONH₂, -CONH- C₁-C₁₂-alkyl, -CONH- C₂-C₁₂-alkenyl, -CONH- C₂-C₁₂-alkenyl, -CONH-C₃-C₁₂-cycloalkyl, -CONH-aryl, -CONH-heteroaryl, -CONH-heterocycloalkyl, -OCO₂-C₁-C₁₂-alkyl, -OCO₂-C₂-C₁₂-alkenyl, -OCO₂-C₂-C₁₂-alkenyl, -OCO₂-C₃-C₁₂-cycloalkyl, -OCO₂-aryl, -OCO₂-heteroaryl, -OCO₂-heterocycloalkyl, -OCONH₂, -OCONH- C₁-C₁₂-alkyl, -OCONH- C₂-C₁₂-alkenyl, -OCONH- C₂-C₁₂-alkenyl, -OCONH-C₃-C₁₂-cycloalkyl, -OCONH- aryl, -OCONH- heteroaryl, -OCONH-heterocycloalkyl, -NHC(O)- C₁-C₁₂-alkyl, -NHC(O)-C₂-C₁₂-alkenyl, -NHC(O)-C₂-C₁₂-alkenyl, -NHC(O)-C₃-C₁₂-cycloalkyl, -NHC(O)-aryl, -NHC(O)-heteroaryl, -NHC(O)-heterocycloalkyl, -NHCO₂-C₁-C₁₂-alkyl, -NHCO₂-C₂-C₁₂-alkenyl, -NHCO₂-C₂-C₁₂-alkenyl, -NHCO₂-C₃-C₁₂-cycloalkyl, -NHCO₂-aryl, -NHCO₂-heteroaryl, -NHCO₂-heterocycloalkyl, -NHC(O)NH₂, NHC(O)NH- C₁-C₁₂-alkyl, -NHC(O)NH-C₂-C₁₂-alkenyl, -NHC(O)NH-C₂-C₁₂-alkenyl, -NHC(O)NH-C₃-C₁₂-cycloalkyl, -NHC(O)NH-aryl, -NHC(O)NH-heteroaryl, -NHC(O)NH-heterocycloalkyl, NHC(S)NH₂, NHC(S)NH- C₁-C₁₂-alkyl, -NHC(S)NH-C₂-C₁₂-alkenyl, -NHC(S)NH-C₂-C₁₂-alkenyl, -NHC(S)NH-C₃-C₁₂-cycloalkyl, -NHC(S)NH-aryl, -NHC(S)NH-heteroaryl, -NHC(S)NH-heterocycloalkyl, -NHC(NH)NH₂, NHC(NH)NH- C₁-C₁₂-alkyl, -NHC(NH)NH-C₂-C₁₂-alkenyl, NHC(NH)NH-C₂-C₁₂-alkenyl, -NHC(NH)NH-C₃-C₁₂-cycloalkyl, -NHC(NH)NH-aryl, -NHC(NH)NH-heteroaryl, -NHC(NH)NH-heterocycloalkyl, NHC(NH)-C₁-C₁₂-alkyl, -NHC(NH)-C₂-C₁₂-alkenyl, -NHC(NH)-C₂-C₁₂-alkenyl, -NHC(NH)-C₃-C₁₂-cycloalkyl, -NHC(NH)-aryl, -NHC(NH)-heteroaryl, -NHC(NH)-heterocycloalkyl, -C(NH)NH-C₁-C₁₂-alkyl, -C(NH)NH-C₂-C₁₂-alkenyl, -C(NH)NH-C₂-C₁₂-alkenyl, -C(NH)NH-C₃-C₁₂-cycloalkyl, -C(NH)NH-aryl, -C(NH)NH-heteroaryl, -C(NH)NH-heterocycloalkyl, -S(O)-C₁-C₁₂-alkyl, -S(O)-C₂-C₁₂-alkenyl, -S(O)-C₂-C₁₂-alkenyl, -S(O)-C₃-C₁₂-cycloalkyl, -S(O)-aryl, - S(O)-heteroaryl, - S(O)-heterocycloalkyl -SO₂NH₂, -SO₂NH-C₁-C₁₂-alkyl, -SO₂NH- C₂-C₁₂-alkenyl, -SO₂NH- C₂-C₁₂-alkenyl, -SO₂NH-C₃-C₁₂-cycloalkyl, -SO₂NH-aryl, -SO₂NH-heteroaryl, -SO₂NH-heterocycloalkyl, -NHSO₂-C₁-C₁₂-alkyl, -NHSO₂-C₂-C₁₂-alkenyl, -NHSO₂-C₂-C₁₂-alkenyl, -NHSO₂-C₃-C₁₂-cycloalkyl, -NHSO₂-aryl, -NHSO₂-heteroaryl, -NHSO₂-heterocycloalkyl, -CH₂NH₂, -CH₂SO₂CH₃, -aryl, -arylalkyl, -heteroaryl, -heteroarylalkyl, -heterocycloalkyl, -C₃-C₁₂-cycloalkyl, -methoxymethoxy, -methoxyethoxy, -SH, -S-C₁-C₁₂-alkyl, -S-C₂-C₁₂-alkenyl, -S-C₂-C₁₂-alkenyl, -S-C₃-C₁₂-cycloalkyl, -S-aryl, -S-heteroaryl, -S-heterocycloalkyl, or methylthiomethyl.

The terms "C₂-C₁₂ alkynyl" or "C₂-C₆ alkynyl," as used herein, denote a monovalent group derived from a hydrocarbon moiety containing from two to twelve or two to six carbon atoms having at least one carbon-carbon triple bond by the removal of a single hydrogen atom. Representative alkynyl groups include, but are not limited to, for example, ethynyl, 1-propynyl, 1-butynyl, and the like.

The term "substituted alkynyl," as used herein, refers to a "C₂-C₁₂ alkynyl" or "C₂-C₆ alkynyl" group as previously defined, substituted by independent replacement or one, two, or three of the hydrogen atoms thereon with substituents including, but not limited to, -F, -Cl, -Br, -I, -OH, protected hydroxy, -NO₂, -CN, -C₁-C₁₂-alkyl optionally substituted with halogen, C₂-C₁₂₋alkenyl optionally substituted with halogen, -C₂-C₁₂-alkynyl optionally substituted with halogen, -NH₂, protected amino, -NH -C₁-C₁₂-alkyl, -NH -C₂-C₁₂-alkenyl, -NH -C₂-C₁₂-alkenyl, -NH -C₃-C₁₂-cycloalkyl, -NH-aryl, -NH-heteroaryl, -NH-heterocycloalkyl, -dialkylamino, -diarylamino, -diheteroarylamino, -O-C₁-C₁₂-alkyl, -O-C₂-C₁₂-alkenyl, -O-C₂-C₁₂-alkenyl, -O-C₃-C₁₂-cycloalkyl, -O-aryl, -O-heteroaryl, -O-heterocycloalkyl, -C(O)-C₁-C₁₂-alkyl, -C(O)-C₂-C₁₂-alkenyl, -C(O)- C₂-C₁₂-alkenyl, -C(O)-C₃-C₁₂-cycloalkyl, -C(O)-aryl, -C(O)-heteroaryl, -C(O)-heterocycloalkyl, -CONH₂, -CONH- C₁-C₁₂-alkyl, -CONH- C₂-C₁₂-alkenyl, -CONH- C₂-C₁₂-alkenyl, -CONH-C₃-C₁₂-cycloalkyl, -CONH-aryl, -CONH-heteroaryl, -CONH-heterocycloalkyl, -OCO₂-C₁-C₁₂-alkyl, -OCO₂-C₂-C₁₂-alkenyl, -OCO₂-C₂-C₁₂-alkenyl, -OCO₂-C₃-C₁₂-cycloalkyl, -OCO₂-aryl, -OCO₂-heteroaryl, -OCO₂-heterocycloalkyl, -OCONH₂, -OCONH- C₁-C₁₂-alkyl, -OCONH- C₂-C₁₂-alkenyl, -OCONH- C₂-C₁₂-alkenyl, -OCONH- C₃-C₁₂-cycloalkyl, -OCONH- aryl, -OCONH- heteroaryl, -OCONH- heterocycloalkyl, -NHC(O)- C₁-C₁₂-alkyl, -NHC(O)-C₂-C₁₂-alkenyl, -NHC(O)-C₂-C₁₂-alkenyl, -NHC(O)-C₃-C₁₂-cycloalkyl, -NHC(O)-aryl, -NHC(O)-heteroaryl, -NHC(O)-heterocycloalkyl, -NHCO₂-C₁-C₁₂-alkyl, -NHCO₂-C₂-C₁₂-alkenyl, -NHCO₂-C₂-C₁₂-alkenyl, -NHCO₂-C₃-C₁₂-cycloalkyl, -NHCO₂-aryl, -NHCO₂- heteroaryl, -NHCO₂- heterocycloalkyl, -NHC(O)NH₂, NHC(O)NH- C₁-C₁₂-alkyl, -NHC(O)NH-C₂-C₁₂₋alkenyl, -NHC(O)NH-C₂-C₁₂-alkenyl, -NHC(O)NH-C₃-C₁₂-cycloalkyl, -NHC(O)NH-aryl, -NHC(O)NH-heteroaryl, -NHC(O)NH-heterocycloalkyl, NHC(S)NH₂, NHC(S)NH- C₁-C₁₂-alkyl, -NHC(S)NH-C₂-C₁₂-alkenyl, -NHC(S)NH-C₂-C₁₂-alkenyl, -NHC(S)NH-C₃-C₁₂-cycloalkyl, -NHC(S)NH-aryl, -NHC(S)NH-heteroaryl, -NHC(S)NH-heterocycloalkyl, -NHC(NH)NH₂, NHC(NH)NH- C₁-C₁₂-alkyl, -NHC(NH)NH-C₂-C₁₂-alkenyl, -NHC(NH)NH-C₂-C₁₂-alkenyl, -NHC(NH)NH-C₃-C₁₂-cycloalkyl, -NHC(NH)NH-aryl, -NHC(NH)NH-heteroaryl, -NHC(NH)NH-heterocycloalkyl, NHC(NH)-C₁-C₁₂-alkyl, -NHC(NH)-C₂-C₁₂-alkenyl, -NHC(NH)-C₂-C₁₂-alkenyl, -NHC(NH)-C₃-C₁₂-cycloalkyl, -NHC(NH)-aryl, -NHC(NH)-heteroaryl, -NHC(NH)-heterocycloalkyl, -C(NH)NH-C₁-C₁₂-alkyl, -C(NH)NH-C₂-C₁₂-alkenyl, -C(NH)NH-C₂-C₁₂-alkenyl, -C(NH)NH-C₃-C₁₂-cycloalkyl, -C(NH)NH-aryl, -C(NH)NH-heteroaryl, -C(NH)NH-heterocycloalkyl, -S(O)-C₁-C₁₂-alkyl, -S(O)-C₂-C₁₂-alkenyl, -S(O)-C₂-C₁₂-alkenyl, -S(O)-C₃-C₁₂-cycloalkyl, -S(O)-aryl, - S(O)-heteroaryl, - S(O)-heterocycloalkyl -SO₂NH₂, -SO₂NH-C₁-C₁₂-alkyl, -SO₂NH-C₂-C₁₂-alkenyl, -SO₂NH-C₂-C₁₂-alkenyl, -SO₂NH-C₃-C₁₂-cycloalkyl, -SO₂NH-aryl, -SO₂NH-heteroaryl, -SO₂NH-heterocycloalkyl, -NHSO₂-C₁-C₁₂-alkyl, -NHSO₂-C₂-C₁₂-alkenyl, -NHSO₂-C₂-C₁₂-alkenyl, -NHSO₂-C₃-C₁₂-cycloalkyl, -NHSO₂-aryl, -NHSO₂-heteroaryl, -NHSO₂-heterocycloalkyl, -CH₂NH₂, -CH₂SO₂CH₃, -aryl, -arylalkyl, -heteroaryl, -heteroarylalkyl, -heterocycloalkyl, -C₃-C₁₂-cycloalkyl, -methoxymethoxy, -methoxyethoxy, -SH, -S-C₁-C₁₂-alkyl, -S-C₂-C₁₂-alkenyl, -S-C₂-C₁₂-alkenyl, -S-C₃-C₁₂-cycloalkyl, -S-aryl, -S-heteroaryl, -S-heterocycloalkyl, or methylthiomethyl.

The term "C₁-C₆ alkoxy," as used herein, refers to a C₁-C₆ alkyl group, as previously defined, attached to the parent molecular moiety through an oxygen atom. Examples of C₁-C₆-alkoxy include, but are not limited to, methoxy, ethoxy, propoxy, isopropoxy, *n*-butoxy, *tert*-butoxy, neopentoxy and n-hexoxy.

The terms "halo" and "halogen," as used herein, refer to an atom selected from fluorine, chlorine, bromine and iodine.

The term "aryl," as used herein, refers to a mono- or bicyclic carbocyclic ring system having one or two aromatic rings including, but not limited to, phenyl, naphthyl, tetrahydronaphthyl, indanyl, idenyl and the like.

The term "substituted aryl," as used herein, refers to an aryl group, as previously defined, substituted by independent replacement or one, two, or three of the hydrogen atoms thereon with substituents including, but not limited to, -F, -Cl, -Br, -I, -OH, protected hydroxy, -NO₂, -CN, -C₁-C₁₂-alkyl optionally substituted with halogen, C₂-C₁₂-alkenyl optionally substituted with halogen, -C₂-C₁₂-alkynyl optionally substituted with halogen, -NH₂, protected amino, -NH -C₁-C₁₂-alkyl, -NH -C₂-C₁₂-alkenyl, -NH -C₂-C₁₂-alkenyl, -NH -C₃-C₁₂-cycloalkyl, -NH -aryl, -NH -heteroaryl, -NH -heterocycloalkyl, -dialkylamino, -diarylamino, -diheteroarylamino, -O-C₁-C₁₂-alkyl, -O-C₂-C₁₂-alkenyl, -O-C₂-C₁₂-alkenyl, -O-C₃-C₁₂-cycloalkyl, -O-aryl, -O-heteroaryl, -O-heterocycloalkyl, -C(O)-C₁-C₁₂-alkyl, -C(O)- C₂-C₁₂-alkenyl, -C(O)- C₂-C₁₂-alkenyl, -C(O)-C₃-C₁₂-cycloalkyl, -C(O)-aryl, -C(O)-heteroaryl, -C(O)-heterocycloalkyl, -CONH₂, -CONH- C₁-C₁₂-alkyl, -CONH- C₂-C₁₂-alkenyl, -CONH- C₂-C₁₂-alkenyl, -CONH-C₃-C₁₂-cycloalkyl, -CONH-aryl, -CONH-heteroaryl, -CONH-heterocycloalkyl, -OCO₂-C₁-C₁₂-alkyl, -OCO₂-C₂-C₁₂-alkenyl, -OCO₂-C₂-C₁₂-alkenyl, -OCO₂-C₃-C₁₂-cycloalkyl, -OCO₂-aryl, -OCO₂-heteroaryl, -OCO₂-heterocycloalkyl, -OCONH₂, -OCONH- C₁-C₁₂-alkyl, -OCONH- C₂-C₁₂-alkenyl, -OCONH- C₂-C₁₂-alkenyl, -OCONH- C₃-C₁₂-cycloalkyl, -OCONH- aryl, -OCONH-heteroaryl, -OCONH- heterocycloalkyl, -NHC(O)-C₁-C₁₂-alkyl, -NHC(O)-C₂-C₁₂-alkenyl, -NHC(O)-C₂-C₁₂-alkenyl, -NHC(O)-C₃-C₁₂-cycloalkyl, -NHC(O)-aryl, -NHC(O)-heteroaryl, -NHC(O)-heterocycloalkyl, -NHCO₂-C₁-C₁₂-alkyl, -NHCO₂-C₂-C₁₂-alkenyl, -NHCO₂-C₂-C₁₂-alkenyl, -NHCO₂-C₃-C₁₂-cycloalkyl, -NHCO₂-aryl, -NHCO₂-heteroaryl, -NHCO₂-heterocycloalkyl, -NHC(O)NH₂, NHC(O)NH- C₁-C₁₂-alkyl, -NHC(O)NH-C₂-C₁₂-alkenyl, -NHC(O)NH-C₂-C₁₂-alkenyl, -NHC(O)NH-C₃-C₁₂-cycloalkyl, -NHC(O)NH-aryl, -NHC(O)NH-heteroaryl, -NHC(O)NH-heterocycloalkyl, NHC(S)NH₂, NHC(S)NH- C₁-C₁₂-alkyl, -NHC(S)NH-C₂-C₁₂-alkenyl, -NHC(S)NH-C₂-C₁₂-alkenyl, -NHC(S)NH-C₃-C₁₂-cycloalkyl, -NHC(S)NH-aryl, -NHC(S)NH-heteroaryl, -NHC(S)NH-heterocycloalkyl, -NHC(NH)NH₂, NHC(NH)NH- C₁-C₁₂-alkyl, -NHC(NH)NH-C₂-C₁₂-alkenyl, -NHC(NH)NH-C₂-C₁₂-alkenyl, -NHC(NH)NH-C₃-C₁₂-cycloalkyl, -NHC(NH)NH-aryl, -NHC(NH)NH-heteroaryl, -NHC(NH)NH-heterocycloalkyl, NHC(NH)-C₁-C₁₂-alkyl, -NHC(NH)-C₂-C₁₂-alkenyl, -NHC(NH)-C₂-C₁₂-alkenyl, -NHC(NH)-C₃-C₁₂-cycloalkyl, -NHC(NH)-aryl, NHC(NH)-heteroaryl, -NHC(NH)-heterocycloalkyl, -C(NH)NH-C₁-C₁₂-alkyl, -C(NH)NH-C₂-C₁₂-alkenyl, -C(NH)NH-C₂-C₁₂-alkenyl, -C(NH)NH-C₃-C₁₂-cycloalkyl, -C(NH)NH-aryl, -C(NH)NH-heteroaryl, -C(NH)NH-heterocycloalkyl, -S(O)-C₁-C₁₂-alkyl, - S(O)-C₂-C₁₂-alkenyl, -S(O)-C₂-C₁₂-alkenyl, -S(O)-C₃-C₁₂-cycloalkyl, - S(O)-aryl, - S(O)-heteroaryl, - S(O)-heterocycloalkyl -SO₂NH₂, -SO₂NH-C₁-C₁₂-alkyl, -SO₂NH-C₂-C₁₂-alkenyl, -SO₂NH-C₂-C₁₂-alkenyl, -SO₂NH-C₃-C₁₂-cycloalkyl, -SO₂NH- aryl, -SO₂NH- heteroaryl, -SO₂NH-heterocycloalkyl, -NHSO₂-C₁-C₁₂-alkyl, -NHSO₂-C₂-C₁₂-alkenyl, -NHSO₂-C₂-C₁₂-alkenyl, -NHSO₂-C₃-C₁₂-cycloalkyl, -NHSO₂-aryl, -NHSO₂-heteroaryl, -NHSO₂-heterocycloalkyl, -CH₂NH₂, -CH₂SO₂CH₃, -aryl, -arylalkyl, -heteroaryl, -heteroarylalkyl, -heterocycloalkyl, -C₃-C₁₂-cycloalkyl, -methoxymethoxy, -methoxyethoxy, -SH, -S-C₁-C₁₂-alkyl, -S-C₂-C₁₂-alkenyl, -S-C₂-C₁₂-alkenyl, -S-C₃-C₁₂-cycloalkyl, -S-aryl, -S-heteroaryl, -S-heterocycloalkyl, or methylthiomethyl.

The term "arylalkyl," as used herein, refers to a C₁-C₃ alkyl or C₁-C₆ alkyl residue attached to an aryl ring. Examples include, but are not limited to, benzyl, phenethyl and the like.

The term "substituted arylalkyl," as used herein, refers to an arylalkyl group, as previously defined, substituted by independent replacement or one, two, or three of the hydrogen atoms thereon with substituents including, but not limited to, but not limited to, -F, -Cl, -Br, -I, -OH, protected hydroxy, -NO₂, -CN, -C₁-C₁₂-alkyl optionally substituted with halogen, C₂-C₁₂-alkenyl optionally substituted with halogen, -C₂-C₁₂-alkynyl optionally substituted with halogen, -NH₂, protected amino, -NH -C₁-C₁₂-alkyl, -NH -C₂-C₁₂-alkenyl, -NH -C₂-C₁₂-alkenyl, -NH -C₃-C₁₂-cycloalkyl, -NH -aryl, -NH -heteroaryl, -NH-heterocycloalkyl, -dialkylamino, -diarylamino, -diheteroarylamino, -O-C₁-C₁₂-alkyl, -O-C₂-C₁₂-alkenyl, -O-C₂-C₁₂-alkenyl, -O-C₃-C₁₂-cycloalkyl, -0-aryl, -0-heteroaryl, -O-heterocycloalkyl, -C(O)-C₁-C₁₂-alkyl, -C(O)- C₂-C₁₂-alkenyl, -C(O)- C₂-C₁₂-alkenyl, -C(O)-C₃-C₁₂-cycloalkyl, -C(O)-aryl, -C(O)-heteroaryl, -C(O)-heterocycloalkyl, -CONH₂, -CONH- C₁-C₁₂-alkyl, -CONH- C₂-C₁₂-alkenyl, -CONH- C₂-C₁₂-alkenyl, -CONH-C₃-C₁₂-cycloalkyl, -CONH-aryl, -CONH-heteroaryl, -CONH-heterocycloalkyl, -OCO₂- C₁-C₁₂-alkyl, -OCO₂- C₂-C₁₂-alkenyl, -OCO₂-C₂-C₁₂-alkenyl, -OCO₂-C₃-C₁₂-cycloalkyl, -OCO₂-aryl, -OCO₂-heteroaryl, -OCO₂-heterocycloalkyl, -OCONH₂, -OCONH-C₁-C₁₂-alkyl, -OCONH- C₂-C₁₂-alkenyl, -OCONH- C₂-C₁₂-alkenyl, -OCONH- C₃-C₁₂-cycloalkyl, -OCONH- aryl, -OCONH- heteroaryl, -OCONH- heterocycloalkyl, -NHC(O)- C₁-C₁₂-alkyl, -NHC(O)-C₂-C₁₂-alkenyl, -NHC(O)-C₂-C₁₂-alkenyl, -NHC(O)-C₃-C₁₂-cycloalkyl, -NHC(O)-aryl, -NHC(O)-heteroaryl, -NHC(O)-heterocycloalkyl, -NHCO₂-C₁-C₁₂-alkyl, -NHCO₂-C₂-C₁₂-alkenyl, -NHCO₂-C₂-C₁₂-alkenyl, -NHCO₂-C₃-C₁₂-cycloalkyl, -NHCO₂-aryl, -NHCO₂-heteroaryl, -NHCO₂-heterocycloalkyl, -NHC(O)NH₂, NHC(O)NH-C₁-C₁₂-alkyl, -NHC(O)NH-C₂-C₁₂-alkenyl, -NHC(O)NH-C₂-C₁₂-alkenyl, -NHC(O)NH-C₃-C₁₂-cycloalkyl, -NHC(O)NH-aryl, -NHC(O)NH-heteroaryl, -NHC(O)NH-heterocycloalkyl, NHC(S)NH₂, NHC(S)NH- C₁-C₁₂-alkyl, -NHC(S)NH-C₂-C₁₂-alkenyl, -NHC(S)NH-C₂-C₁₂-alkenyl, -NHC(S)NH-C₃-C₁₂-cycloalkyl, -NHC(S)NH-aryl, -NHC(S)NH-heteroaryl, -NHC(S)NH-heterocycloalkyl, -NHC(NH)NH₂, NHC(NH)NH- C₁-C₁₂-alkyl, -NHC(NH)NH-C₂-C₁₂-alkenyl, -NHC(NH)NH-C₂-C₁₂-alkenyl, -NHC(NH)NH-C₃-C₁₂-cycloalkyl, -NHC(NH)NH-aryl, -NHC(NH)NH-heteroaryl, -NHC(NH)NH-heterocycloalkyl, NHC(NH)-C₁-C₁₂-alkyl, -NHC(NH)-C₂-C₁₂-alkenyl, -NHC(NH)-C₂-C₁₂-alkenyl, -NHC(NH)-C₃-C₁₂-cycloalkyl, -NHC(NH)-aryl, -NHC(NH)-heteroaryl, -NHC(NH)-heterocycloalkyl, -C(NH)NH-C₁-C₁₂-alkyl, -C(NH)NH-C₂-C₁₂-alkenyl, -C(NH)NH-C₂-C₁₂-alkenyl, -C(NH)NH-C₃-C₁₂-cycloalkyl, -C(NH)NH-aryl, -C(NH)NH-heteroaryl, -C(NH)NH-heterocycloalkyl, -S(O)-C₁-C₁₂-alkyl, - S(O)-C₂-C₁₂-alkenyl, -S(O)-C₂-C₁₂-alkenyl, -S(O)-C₃-C₁₂-cycloalkyl, -S(O)-aryl, - S(O)-heteroaryl, - S(O)-heterocycloalkyl -SO₂NH₂, -SO₂NH-C₁-C₁₂-alkyl, -SO₂NH-C₂-C₁₂-alkenyl, -SO₂NH-C₂-C₁₂-alkenyl, -SO₂NH-C₃-C₁₂-cycloalkyl, -SO₂NH-aryl, -SO₂NH-heteroaryl, -SO₂NH-heterocycloalkyl, -NHSO₂-C₁-C₁₂-alkyl, -NHSO₂-C₂-C₁₂-alkenyl, - NHSO₂-C₂-C₁₂-alkenyl, -NHSO₂-C₃-C₁₂-cycloalkyl, -NHSO₂-aryl, -NHSO₂-heteroaryl, -NHSO₂-heterocycloalkyl, -CH₂NH₂, -CH₂SO₂CH₃, -aryl, -arylalkyl, -heteroaryl, -heteroarylalkyl, -heterocycloalkyl, -C₃-C₁₂-cycloalkyl, -methoxymethoxy, -methoxyethoxy, -SH, -S-C₁-C₁₂-alkyl, -S-C₂-C₁₂-alkenyl, -S-C₂-C₁₂-alkenyl, -S-C₃-C₁₂-cycloalkyl, -S-aryl, -S-heteroaryl, -S-heterocycloalkyl, or methylthiomethyl.

The term "heteroaryl," as used herein, refers to a mono-, bi-, or tri-cyclic aromatic radical or ring having from five to ten ring atoms of which one ring atom is selected from S, O and N; zero, one or two ring atoms are additional heteroatoms independently selected from S, O and N; and the remaining ring atoms are carbon, wherein any N or S contained within the ring may be optionally oxidized. Heteroaryl includes, but is not limited to, pyridinyl, pyrazinyl, pyrimidinyl, pyrrolyl, pyrazolyl, imidazolyl, thiazolyl, oxazolyl, isooxazolyl, thiadiazolyl, oxadiazolyl, thiophenyl, furanyl, quinolinyl, isoquinolinyl, benzimidazolyl, benzooxazolyl, quinoxalinyl, and the like.

The term "substituted heteroaryl," as used herein, refers to a heteroaryl group as previously defined, substituted by independent replacement or one, two, or three of the hydrogen atoms thereon with substituents including, but not limited to, -F, -Cl, -Br, -I, -OH, protected hydroxy, -NO₂, -CN, -C₁-C₁₂-alkyl optionally substituted with halogen, C₂-C₁₂-alkenyl optionally substituted with halogen, -C₂-C₁₂-alkynyl optionally substituted with halogen, -NH₂, protected amino, -NH -C₁-C₁₂-alkyl, -NH -C₂-C₁₂-alkenyl, -NH -C₂-C₁₂-alkenyl, -NH -C₃-C₁₂-cycloalkyl, -NH -aryl, -NH -heteroaryl, -NH -heterocycloalkyl, -dialkylamino, -diarylamino, -diheteroarylamino, -O-C₁-C₁₂-alkyl, -O-C₂-C₁₂-alkenyl, -O-C₂-C₁₂-alkenyl, -O-C₃-C₁₂-cycloalkyl, -O-aryl, -O-heteroaryl, -O-heterocycloalkyl, -C(O)-C₁-C₁₂-alkyl, -C(O)- C₂-C₁₂-alkenyl, -C(O)- C₂-C₁₂-alkenyl, -C(O)-C₃-C₁₂-cycloalkyl, -C(O)-aryl, -C(O)-heteroaryl, -C(O)-heterocycloalkyl, -CONH₂, -CONH- C₁-C₁₂-alkyl, -CONH- C₂-C₁₂-alkenyl, -CONH- C₂-C₁₂-alkenyl, -CONH-C₃-C₁₂-cycloalkyl, -CONH-aryl, -CONH-heteroaryl, -CONH-heterocycloalkyl, -OCO₂-C₁-C₁₂-alkyl, -OCO₂-C₂-C₁₂-alkenyl, -OCO₂-C₂-C₁₂-alkenyl, -OCO₂-C₃-C₁₂-cycloalkyl, -OCO₂-aryl, -OCO₂-heteroaryl, -OCO₂-heterocycloalkyl, -OCONH₂, -OCONH- C₁-C₁₂-alkyl, -OCONH- C₂-C₁₂-alkenyl, -OCONH- C₂-C₁₂-alkenyl, -OCONH- C₃-C₁₂-cycloalkyl, -OCONH- aryl, -OCONH-heteroaryl, -OCONH- heterocycloalkyl, -NHC(O)- C₁-C₁₂-alkyl, -NHC(O)-C₂-C₁₂-alkenyl, -NHC(O)-C₂-C₁₂-alkenyl, -NHC(O)-C₃-C₁₂-cycloalkyl, -NHC(O)-aryl, -NHC(O)-heteroaryl, -NHC(O)-heterocycloalkyl, -NHCO₂-C₁-C₁₂-alkyl, -NHCO₂-C₂-C₁₂-alkenyl, -NHCO₂-C₂-C₁₂-alkenyl, -NHCO₂-C₃-C₁₂-_{C}ycloalkyl, -NHCO₂-aryl, -NHCO₂-heteroaryl, -NHCO₂-heterocycloalkyl, -NHC(O)NH₂, NHC(O)NH- C₁-C₁₂-alkyl, -NHC-(O)NH-C₂-C₁₂-alkenyl, -NHC(O)NH-C₂-C₁₂-alkenyl, -NHC(O)NH-C₃-C₁₂-cycloalkyl, -NHC(O)NH-aryl, -NHC(O)NH-heteroaryl, -NHC(O)NH-heterocycloalkyl, NHC(S)NH₂, NHC(S)NH- C₁-C₁₂-alkyl, -NHC(S)NH-C₂-C₁₂-alkenyl, -NHC(S)NH-C₂-C₁₂-alkenyl, -NHC(S)NH-C₃-C₁₂-cycloalkyl, -NHC(S)NH-aryl, -NHC(S)NH-heteroaryl, -NHC(S)NH-heterocycloalkyl, -NHC(NH)NH₂, NHC(NH)NH- C₁-C₁₂-alkyl, -NHC(NH)NH-C₂-C₁₂-alkenyl, -NHC(NH)NH-C₂-C₁₂-alkenyl, -NHC(NH)NH-C₃-C₁₂-cycloalkyl, -NHC(NH)NH-aryl, -NHC(NH)NH-heteroaryl, -NHC(NH)NH-heterocycloalkyl, NHC(NH)-C₁-C₁₂-alkyl, -NHC(NH)-C₂-C₁₂-alkenyl, -NHC(NH)-C₂-C₁₂-alkenyl, -NHC(NH)-C₃-C₁₂-cycloalkyl, -NHC(NH)-aryl, -NHC(NH)-heteroaryl, -NHC(NH)-heterocycloalkyl, -C(NH)NH-C₁-C₁₂-alkyl, -C(NH)NH-C₂-C₁₂-alkenyl, -C(NH)NH-C₂-C₁₂-alkenyl, -C(NH)NH-C₃-C₁₂-cycloalkyl, -C(NH)NH-aryl, -C(NH)NH-heteroaryl, -C(NH)NH-heterocycloalkyl, -S(O)-C₁-C₁₂-alkyl, -S(O)-C₂-C₁₂-alkenyl, S(O)-C₂-C₁₂-alkenyl, -S(O)-C₃-C₁₂-cycloalkyl, -S(O)-aryl, - S(O)-heteroaryl, - S(O)-heterocycloalkyl -SO₂NH₂, -SO₂NH-C₁-C₁₂-alkyl, -SO₂NH-C₂-C₁₂-alkenyl, -SO₂NH-C₂-C₁₂-alkenyl, -SO₂NH- C₃-C₁₂-cycloalkyl, -SO₂NH-aryl, -SO₂NH-heteroaryl, -SO₂NH-heterocycloalkyl, -NHSO₂-C₁-C₁₂-alkyl, -NHSO₂-C₂-C₁₂-alkenyl, -NHSO₂-C₂-C₁₂-alkenyl, -NHSO₂-C₃-C₁₂-cycloalkyl, -NHSO₂-aryl, -NHSO₂-heteroaryl, -NHSO₂-heterocycloalkyl, -CH₂NH₂, -CH₂SO₂CH₃, -aryl, -arylalkyl, -heteroaryl, -heteroarylalkyl, -heterocycloalkyl, -C₃-C₁₂-cycloalkyl, -methoxymethoxy, -methoxyethoxy, -SH, -S-C₁-C₁₂-alkyl, -S-C₂-C₁₂-alkenyl, -S-C₂-C₁₂-alkenyl, -S-C₃-C₁₂-cycloalkyl, -S-aryl, -S-heteroaryl, -S-heterocycloalkyl, or methylthiomethyl.

The term "C₃-C₁₂-cycloalkyl," as used herein, denotes a monovalent group derived from a monocyclic or bicyclic saturated carbocyclic ring compound by the removal of a single hydrogen atom. Examples include, but not limited to, cyclopropyl, cyclobutyl, cyclopentyl, cyclohexyl, bicyclo [2.2.1] heptyl, and bicyclo [2.2.2] octyl.

The term "substituted C₃-C₁₂-cycloalkyl," as used herein, refers to a C₃-C₁₂-cycloalkyl group as previously defined, substituted by independent replacement or one, two, or three of the hydrogen atoms thereon with substituents including, but not limited to, -F, -Cl, -Br, -I, -OH, protected hydroxy, -NO₂, -CN, -C₁-C₁₂-alkyl optionally substituted with halogen, C₂-C₁₂-alkenyl optionally substituted with halogen, -C₂-C₁₂-alkynyl optionally substituted with halogen, -NH₂, protected amino, -NH -C₁-C₁₂-alkyl, -NH -C₂-C₁₂-alkenyl, -NH -C₂-C₁₂-alkenyl, -NH -C₃-C₁₂-cycloalkyl, -NH -aryl, -NH -heteroaryl, -NH-heterocycloalkyl, -dialkylamino, -diarylamino, -diheteroarylamino, -O-C₁-C₁₂-alkyl, -O-C₂-C₁₂-alkenyl, -O-C₂-C₁₂-alkenyl, -O-C₃-C₁₂-cycloalkyl, -O-aryl, -O-heteroaryl, -O-heterocycloalkyl, -C(O)- C₁-C₁₂-alkyl, -C(O)- C₂-C₁₂-alkenyl, -C(O)- C₂-C₁₂-alkenyl, -C(O)-C₃-C₁₂-cycloalkyl, -C(O)-aryl, -C(O)-heteroaryl, -C(O)-heterocycloalkyl, -CONH₂, -CONH- C₁-C₁₂-alkyl, -CONH- C₂-C₁₂-alkenyl, -CONH- C₂-C₁₂-alkenyl, -CONH-C₃-C₁₂-cycloalkyl, -CONH-aryl, -CONH-heteroaryl, -CONH-heterocycloalkyl, -OCO₂-C₁-C₁₂-alkyl, -OCO₂-C₂-C₁₂-alkenyl, -OCO₂-C₂-C₁₂-alkenyl, -OCO₂-C₃-C₁₂-cycloalkyl, -OCO₂-aryl, -OCO₂-heteroaryl, -OCO₂-heterocycloalkyl, -OCONH₂, -OCONH-C₁-C₁₂-alkyl, -OCONH- C₂-C₁₂-alkenyl, -OCONH- C₂-C₁₂-alkenyl, -OCONH- C₃-C₁₂-cycloalkyl, -OCONH- aryl, -OCONH- heteroaryl, -OCONH- heterocycloalkyl, -NHC(O)- C₁-C₁₂-alkyl, -NHC(O)-C₂-C₁₂-alkenyl, -NHC(O)-C₂-C₁₂-alkenyl, -NHC(O)-C₃-C₁₂-cycloalkyl, -NHC(O)-aryl, -NHC(O)-heteroaryl, -NHC(O)-heterocycloalkyl, -NHCO₂-C₁-C₁₂-alkyl, -NHCO₂-C₂-C₁₂-alkenyl, -NHCO₂-C₂-C₁₂-alkenyl, -NHCO₂-C₃-C₁₂-cycloalkyl, -NHCO₂-aryl, -NHCO₂-heteroaryl, -NHCO₂-heterocycloalkyl, -NHC(O)NH₂, NHC(O)NH-C₁-C₁₂-alkyl, -NHC(O)NH-C₂-C₁₂-alkenyl, -NHC(O)NH-C₂-C₁₂-alkenyl, -NHC(O)NH-C₃-C₁₂-cycloalkyl, -NHC(O)NH-aryl, -NHC(O)NH-heteroaryl, -NHC(O)NH-heterocycloalkyl, NHC(S)NH₂, NHC(S)NH- C₁-C₁₂-alkyl, -NHC(S)NH-C₂-C₁₂-alkenyl, -NHC(S)NH-C₂-C₁₂-alkenyl, -NHC(S)NH-C₃-C₁₂-cycloalkyl, -NHC(S)NH-aryl, -NHC(S)NH-heteroaryl, -NHC(S)NH-heterocycloalkyl, -NHC(NH)NH₂, NHC(NH)NH- C₁-C₁₂-alkyl, -NHC(NH)NH-C₂-C₁₂-alkenyl, -NHC(NH)NH-C₂-C₁₂-alkenyl, -NHC(NH)NH-C₃-C₁₂-cycloalkyl, -NHC(NH)NH-aryl, -NHC(NH)NH-heteroaryl, -NHC(NH)NH-heterocycloalkyl, NHC(NH)-C₁-C₁₂-alkyl, -NHC(NH)-C₂-C₁₂-alkenyl, -NHC(NH)-C₂-C₁₂-alkenyl, -NHC(NH)-C₃-C₁₂-cycloalkyl, -NHC(NH)-aryl, -NHC(NH)-heteroaryl, -NHC(NH)-heterocycloalkyl, -C(NH)NH-C₁-C₁₂-alkyl, -C(NH)NH-C₂-C₁₂-alkenyl, -C(NH)NH-C₂-C₁₂-alkenyl, -C(NH)NH-C₃-C₁₂-cycloalkyl, -C(NH)NH-aryl, -C(NH)NH-heteroaryl, -C(NH)NH-heterocycloalkyl, -S(O)-C₁-C₁₂-alkyl, -S(O)-C₂-C₁₂-alkenyl, -S(O)-C₂-C₁₂-alkenyl, -S(O)-C₃-C₁₂-cycloalkyl, -S(O)-aryl, - S(O)-heteroaryl, - S(O)-heterocycloalkyl -SO₂NH₂, -SO₂NH-C₁-C₁₂-alkyl, -SO₂NH-C₂-C₁₂-alkenyl, -SO₂NH-C₂-C₁₂-alkenyl, -SO₂NH-C₃-C₁₂-cycloalkyl, -SO₂NH-aryl, -SO₂NH-heteroaryl, -SO₂NH-heterocycloalkyl, -NHSO₂-C₁-C₁₂-alkyl, -NHSO₂-C₂-C₁₂-alkenyl, -NHSO₂-C₂-C₁₂-alkenyl, -NHSO₂-C₃-C₁₂-cycloalkyl, -NHSO₂-aryl, -NHSO₂-heteroaryl, -NHSO₂-heterocycloalkyl, -CH₂NH₂, -CH₂SO₂CH₃, -aryl, -arylalkyl, -heteroaryl, -heteroarylalkyl, -heterocycloalkyl, -C₃-C₁₂-cycloalkyl, -methoxymethoxy, -methoxyethoxy, -SH, -S-C₁-C₁₂-alkyl, -S-C₂-C₁₂-alkenyl, -S-C₂-C₁₂-alkenyl, -S-C₃-C₁₂-cycloalkyl, -S-aryl, -S-heteroaryl, -S-heterocycloalkyl, or methylthiomethyl.

The term "heterocycloalkyl," as used herein, refers to a non-aromatic 5-, 6- or 7-membered ring or a bi- or tri-cyclic group fused system, where (i) each ring contains between one and three heteroatoms independently selected from oxygen, sulfur and nitrogen, (ii) each 5-membered ring has 0 to 1 double bonds and each 6-membered ring has 0 to 2 double bonds, (iii) the nitrogen and sulfur heteroatoms may optionally be oxidized, (iv) the nitrogen heteroatom may optionally be quaternized, and (iv) any of the above rings may be fused to a benzene ring. Representative heterocycloalkyl groups include, but are not limited to, [1,3]dioxolane, pyrrolidinyl, pyrazolinyl, pyrazolidinyl, imidazolinyl, imidazolidinyl, piperidinyl, piperazinyl, oxazolidinyl, isoxazolidinyl, morpholinyl, thiazolidinyl, isothiazolidinyl, and tetrahydrofuryl.

The term "substituted heterocycloalkyl," as used herein, refers to a heterocycloalkyl group, as previously defined, substituted by independent replacement or one, two, or three of the hydrogen atoms thereon with substituents including, but not limited to, -F, -CI, -Br, -I, -OH, protected hydroxy, -NO₂, -CN, -C₁-C₁₂-alkyl optionally substituted with halogen, C₂-C₁₂-alkenyl optionally substituted with halogen, -C₂-C₁₂-alkynyl optionally substituted with halogen, -NH₂, protected amino, -NH -C₁-C₁₂-alkyl, -NH -C₂-C₁₂-alkenyl, -NH -C₂-C₁₂-alkenyl, -NH -C₃-C₁₂-cycloalkyl, -NH -aryl, -NH -heteroaryl, -NH -heterocycloalkyl, -dialkylamino, -diarylamino, -diheteroarylamino, -O-C₁-C₁₂-alkyl, -O-C₂-C₁₂-alkenyl, -O-C₂-C₁₂-alkenyl, -O-C₃-C₁₂-cycloalkyl, -O-aryl, -O-heteroaryl, -O-heterocycloalkyl, -C(O)-C₁-C₁₂-alkyl, -C(O)- C₂-C₁₂-alkenyl, -C(O)- C₂-C₁₂-alkenyl, -C(O)-C₃-C₁₂-cycloalkyl, -C(O)-aryl, -C(O)-heteroaryl, -C(O)-heterocycloalkyl, -CONH₂, -CONH- C₁C₁₂-alkyl, -CONH- C₂-C₁₂-alkenyl, -CONH- C₂-C₁₂-alkenyl, -CONH-C₃-C₁₂-cycloalkyl, -CONH-aryl, -CONH-heteroaryl, -CONH-heterocycloalkyl, -OCO₂- C₁-C₁₂-alkyl, -OCO₂- C₂-C₁₂-alkenyl, -OCO₂- C₂-C₁₂-alkenyl, -OCO₂-C₃-C₁₂-cycloalkyl, -OCO₂-aryl, -OCO₂-heteroaryl, -OCO₂-heterocycloalkyl, -OCONH₂, -OCONH- C₁-C₁₂-alkyl, -OCONH- C₂-C₁₂-alkenyl, -OCONH- C₂-C₁₂-alkenyl, -OCONH- C₃-C₁₂-cycloalkyl, -OCONH- aryl, -OCONH-heteroaryl, -OCONH- heterocycloalkyl, -NHC(O)-C₁-C₁₂-alkyl, -NHC(O)-C₂-C₁₂-alkenyl, -NHC(O)-C₂-C₁₂-alkenyl, -NHC(O)-C₃-C₁₂-cycloalkyl, -NHC(O)-aryl, -NHC(O)-heteroaryl, -NHC(O)-heterocycloalkyl, -NHCO₂- C₁-C₁₂-alkyl, -NHCO₂- C₂-C₁₂-alkenyl, -NHCO₂- C₂-C₁₂-alkenyl, -NHCO₂- C₃-C₁₂-cycloalkyl, -NHCO₂- aryl, -NHCO₂-heteroaryl, -NHCO₂- heterocycloalkyl, -NHC(O)NH₂, NHC(O)NH- C₁C₁₂-alkyl. -NHC(O)NH-C₂-C₁₂-alkenyl, -NHC(O)NH-C₂-C₁₂-alkenyl, -NHC(O)NH-C₃-C₁₂-cycloalkyl, -NHC(O)NH-aryl, -NHC(O)NH-heteroaryl, -NHC(O)NH-heterocycloalkyl, NHC(S)NH₂, NHC(S)NH- C₁-C₁₂-alkyl, -NHC(S)NH-C₂-C₁₂-alkenyl, -NHC(S)NH-C₂-C₁₂-alkenyl, -NHC(S)NH-C₃-C₁₂-cycloalkyl, -NHC(S)NH-aryl, -NHC(S)NH-heteroaryl, -NHC(S)NH-heterocycloalkyl, -NHC(NH)NH₂, NHC(NH)NH- C₁-C₁₂-alkyl, -NHC(NH)NH-C₂-C₁₂-alkenyl, -NHC(NH)NH-C₂-C₁₂-alkenyl, -NHC(NH)NH-C₃-C₁₂-cycloalkyl, -NHC(NH)NH-aryl, -NHC(NH)NH-heteroaryl, -NHC(NH)NH-heterocycloalkyl, NHC(NH)-C₁-C₁₂-alkyl, -NHC(NH)-C₂-C₁₂-alkenyl, -NHC(NH)-C₂-C₁₂-alkenyl, -NHC(NH)-C₃-C₁₂-cycloalkyl, -NHC(NH)-aryl, -NHC(NH)-heteroaryl, -NHC(NH)-heterocycloalkyl, -C(NH)NH-C₁-C₁₂-alkyl, -C(NH)NH-C₂-C₁₂-alkenyl, -C(NH)NH-C₂-C₁₂-alkenyl, -C(NH)NH-C₃-C₁₂-cycloalkyl, -C(NH)NH-aryl, -C(NH)NH-heteroaryl, -C(NH)NH-heterocycloalkyl, -S(O)-C₁-C₁₂-alkyl, - S(O)-C₂-C₁₂-alkenyl, -S(O)-C₂-C₁₂-alkenyl, - S(O)-C₃-C₁₂-cycloalkyl, - S(O)-aryl, - S(O)-heteroaryl, -S(O)-heterocycloalkyl -SO₂NH₂, -SO₂NH- C₁-C₁₂-alkyl, -SO₂NH- C₂-C₁₂-alkenyl, -SO₂NH- C₂-C₁₂-alkenyl, -SO₂NH- C₃-C₁₂-cycloalkyl, -SO₂NH- aryl, -SO₂NH- heteroaryl, -SO₂NH-heterocycloalkyl, -NHSO₂-C₁-C₁₂-alkyl, -NHSO₂-C₂-C₁₂-alkenyl, - NHSO₂C₂-C₁₂-alkenyl, -NHSO₂-C₃-C-₁₂-cycloalkyl, -NHSO₂-aryl, -NHSO₂-heteroaryl, -NHSO₂-heterocycloalkyl, -CH₂NH₂, -CH₂SO₂CH₃, -aryl, -arylalkyl, -heteroaryl, -heteroarylalkyl, -heterocycloalkyl, -C₃-C₁₂-cycloalkyl, -methoxymethoxy, -methoxyethoxy, -SH, -S-C₁-C₁₂-alkyl, -S-C₂-C₁₂-alkenyl, -S-C₂-C₁₂-alkenyl, -S-C₃-C₁₂-cycloalkyl, -S-aryl, -S-heteroaryl, -S-heterocycloalkyl, or methylthiomethyl.

The term "heteroarylalkyl," as used herein, refers to a C₁-C₃ alkyl or C₁-C₆ alkyl residue residue attached to a heteroaryl ring. Examples include, but are not limited to, pyridinylmethyl, pyrimidinylethyl and the like.

The term "substituted heteroarylalkyl," as used herein, refers to a heteroarylalkyl group, as previously defined, substituted by independent replacement or one, two, or three of the hydrogen atoms thereon with substituents including, but not limited to, -F, -Cl, -Br, -I, -OH, protected hydroxy, -NO₂, -CN, -C₁-C₁₂-alkyl optionally substituted with halogen, C₂-C₁₂-alkenyl optionally substituted with halogen, -C₂-C₁₂-alkynyl optionally substituted with halogen, -NH₂, protected amino, -NH -C₁-C₁₂-alkyl, -NH -C₂-C₁₂-alkenyl, -NH -C₂-C₁₂-alkenyl, -NH -C₃-C₁₂-cycloalkyl, -NH -aryl, -NH -heteroaryl, -NH -heterocycloalkyl, -dialkylamino, -diarylamino, -diheteroarylamino, -O-C₁-C₁₂-alkyl, -O-C₂-C₁₂-alkenyl, -0-C₂-C₁₂-alkenyl, -O-C₃-C₁₂-cycloalkyl, -0-aryl, -O-heteroaryl, -O-heterocycloalkyl, -C(O)-C₁-C₁₂-alkyl, -C(O)- C₂-C₁₂-alkenyl, -C(O)- C₂-C₁₂-alkenyl, -C(O)-C₃-C₁₂-cycloalkyl, -C(O)-aryl, -C(O)-heteroaryl, -C(O)-heterocycloalkyl, -CONH₂, -CONH- C₁-C₁₂-alkyl, -CONH- C₂-C₁₂-alkenyl, -CONH- C₂-C₁₂-alkenyl, -CONH-C₃-C₁₂-cycloalkyl, -CONH-aryl, -CONH-heteroaryl, -CONH-heterocycloalkyl, -OCO₂- C₁-C₁₂-alkyl, -OCC₂-C₂-C₁₂-alkenyl, -OCO₂- C₂-C₁₂-alkenyl, -OCO₂-C₃-C₁₂-cycloalkyl, -OCO₂-aryl, -OCO₂-heteroaryl, -OCO₂-heterocycloalkyl, -OCONH₂, -OCONH- C₁-C₁₂-alkyl, -OCONH- C₂-C₁₂-alkenyl, -OCONH- C₂-C₁₂-alkenyl, -OCONH- C₃-C₁₂-cycloalkyl, -OCONH- aryl, -OCONH-heteroaryl, -OCONH- heterocycloalkyl, -NHC(O)- C₁-C₁₂-alkyl, -NHC(O)-C₂-C₁₂-alkenyl, -NHC(O)-C₂-C₁₂-alkenyl, -NHC(O)-C₃-C₁₂-cycloalkyl, -NHC(O)-aryl, -NHC(O)-heteroaryl, -NHC(O)-heterocycloalkyl, -NHCO₂- C₁-C₁₂-alkyl, -NHCO₂- C₂-C₁₂-alkenyl, -NHCO₂- C₂-C₁₂-alkenyl, -NHCO₂- C₃-C₁₂-cycloalkyl, -NHCO₂- aryl, -NHCO₂-heteroaryl, -NHCO₂- heterocycloalkyl, -NHC(O)NH₂, NHC(O)NH- C₁-C₁₂-alkyl, -NHC(O)NH-C₂-C₁₂-alkenyl, -NHC(O)NH-C₂-C₁₂-alkenyl, -NHC(O)NH-C₃-C₁₂-cycloalkyl, -NHC(O)NH-aryl, -NHC(O)NH-heteroaryl, -NHC(O)NH-heterocycloalkyl, NHC(S)NH₂, NHC(S)NH- C₁-C₁₂-alkyl, -NHC(S)NH-C₂-C₁₂-alkenyl, -NHC(S)NH-C₂-C₁₂-alkenyl", -NHC(S)NH-C₁-C₁₂-cycloalkyl, -NHC(S)NH-aryl, -NHC(S)NH-heteroaryl, -NHC(S)NH-heterocycloalkyl, -NHC(NH)NH₂, NHC(NH)NH- C₁-C₁₂-alkyl, -NHC(NH)NH-C₂-C₁₂-alkenyl, -NHC(NH)NH-C₂-C₁₂-alkenyl, -NHC(NH)NH-C₃-C₁₂-cycloalkyl, -NHC(NH)NH-aryl, -NHC(NH)NH-heteroaryl, -NHC(NH)NH-heterocycloalkyl, NHC(NH)-C₁-C₁₂-alkyl, -NHC(NH)-C₂-C₁₂-alkenyl, -NHC(NH)-C₂-C₁₂-alkenyl, -NHC(NH)-C₃-C₁₂-cycloalkyl, -NHC(NH)-aryl, -NHC(NH)-heteroaryl, -NHC(NH)-heterocycloalkyl, -C(NH)NH-C₁-C₁₂-alkyl, -C(NH)NH-C₂-C₁₂-alkenyl, -C(NH)NH-C₂-C₁₂-alkenyl,-C(NH)NH-C₃-C₁₂-cycloalkyl, -C(NH)NH-aryl, -C(NH)NH-heteroaryl, -C(NH)NH-heterocycloalkyl, -S(O)-C₁-C₁₂-alkyl, - S(O)-C₂-C₁₂-alkenyl, -S(O)-C₂-C₁₂-alkenyl, - S(O)-C₃-C₁₂-cycloalkyl, - S(O)-aryl, - S(O)-heteroaryl, - S(O)-heterocycloalkyl -SO₂NH₂, -SO₂NH- C₁C₁₂-alkyl, -SO₂NH- C₂-C₁₂-alkenyl, -SO₂NH- C₂-C₁₂-alkenyl, -SO₂NH- C₃-C₁₂-cycloalkyl, -SO₂NH- aryl, -SO₂NH- heteroaryl, -SO₂NH-heterocycloalkyl, -NHSO₂-C₁-C₁₂-alkyl, -NHSO₂-C₂-C₁₂-alkenyl, - NHSO₂-C₂-C₁₂-alkenyl, -NHSO₂-C₃-C₁₂-cycloalkyl, -NHSO₂-aryl, -NHSO₂-heteroaryl, -NHSO₂-heterocycloalkyl, -CH₂NH₂, -CH₂SO₂CH₃, -aryl, -arylalkyl, -heteroaryl, -heteroarylalkyl, -heterocycloalkyl, -C₃-C₁₂-cycloalkyl, -methoxymethoxy, -methoxyethoxy, -SH, -S-C₁-C₁₂-alkyl, -S-C₂-C₁₂-alkenyl, -S-C₂C₁₂-alkenyl, -S-C₃-C₁₂-cycloalkyl, -S-aryl, -S-heteroaryl, -S-heterocycloalkyl, or methylthiomethyl.

The term "C₁-C₆ alkoxy," as used herein, refers to a C₁-C₆ alkyl group, as previously defined, attached to the parent molecular moiety through an oxygen atom. Examples of C₁-C₆-alkoxy include, but are not limited to, methoxy, ethoxy, propoxy, isopropoxy, *n*-butoxy, *tert*-butoxy, neopentoxy and n-hexoxy.

The term "C₁-C₃-alkyl-amino," as used herein, refers to one or two C₁-C₃-alkyl groups, as previously defined, attached to the parent molecular moiety through a nitrogen atom. Examples of C₁-C₃-alkyl-amino include, but are not limited to, methylamino, dimethylamino, ethylamino, diethylamino, and propylamino.

The term "alkylamino" refers to a group having the structure -NH(C₁-C₁₂ alkyl) where C₁-C₁₂ alkyl is as previously defined.

The term "dialkylamino" refers to a group having the structure -N(C₁C₁₂ alkyl) (C₁-C₁₂ alkyl), where C₁-C₁₂ alkyl is as previously defined. Examples of dialkylamino are, but not limited to, dimethylamino, diethylamino, methylethylamino, piperidino, and the like.

The term "alkoxycarbonyl" represents an ester group, i.e., an alkoxy group, attached to the parent molecular moiety through a carbonyl group such as methoxycarbonyl, ethoxycarbonyl, and the like.

The term "carboxaldehyde," as used herein, refers to a group of formula -CHO.

The term "carboxy," as used herein, refers to a group of formula -COOH.

The term "carboxamide," as used herein, refers to a group of formula -C(O)NH(C₁-C₁₂ alkyl) or - C(O)N(C₁-C₁₂ alkyl) (C₁-C₁₂ alkyl), -C(O)NH₂, and the like.

The term "hydroxy protecting group," as used herein, refers to a labile chemical moiety which is known in the art to protect a hydroxyl group against undesired reactions during synthetic procedures. After said synthetic procedure(s) the hydroxy protecting group as described herein may be selectively removed. Hydroxy protecting groups as known in the are described generally in T.H. Greene and P.G. M. Wuts, Protective Groups in Organic Synthesis, 3rd edition, John Wiley & Sons, New York (1999). Examples of hydroxy protecting groups include, but are not limited to, methylthiomethyl, *tert*-butyl-dimethylsilyl, *tert*-butyldiphenylsilyl, acyl substituted with an aromatic group and the like.

The term "protected hydroxy," as used herein, refers to a hydroxy group protected with a hydroxy protecting group, as defined above, including benzoyl, acetyl, trimethylsilyl, triethylsilyl, methoxymethyl groups, for example.

The term "amino protecting group," as used herein, refers to a labile chemical moiety which is known in the art to protect an amino group against undesired reactions during synthetic procedures. After said synthetic procedure(s) the amino protecting group as described herein may be selectively removed. Amino protecting groups as known in the are described generally in T.H. Greene and P.G. M. Wuts, Protective Groups in Organic Svnthesis, 3rd edition, John Wiley & Sons, New York (1999). Examples of amino protecting groups include, but are not limited to, t-butoxycarbonyl, 9-fluorenylmethoxycarbonyl, benzyloxycarbonyl, and the like.

The term "protected amino," as used herein, refers to an amino group protected with an amino protecting group as defined above.

The term "aprotic solvent," as used herein, refers to a solvent that is relatively inert to proton activity, i.e., not acting as a proton-donor. Examples include, but are not limited to, hydrocarbons, such as hexane and toluene, for example, halogenated hydrocarbons, such as, for example, methylene chloride, ethylene chloride, chloroform, and the like, heterocyclic compounds, such as, for example, tetrahydrofuran and N-methylpyrrolidinone, and ethers such as diethyl ether, bis-methoxymethyl ether. Such compounds are well known to those skilled in the art, and it will be obvious to those skilled in the art that individual solvents or mixtures thereof may be preferred for specific compounds and reaction conditions, depending upon such factors as the solubility of reagents, reactivity of reagents and preferred temperature ranges, for example. Further discussions of aprotic solvents may be found in organic chemistry textbooks or in specialized monographs, for example: Organic Solvents Physical Properties and Methods of Purification, 4th ed., edited by John A. Riddick et al., Vol. II, in the Techniques of Chemistry Series, John Wiley & Sons, NY, 1986.

The term "protogenic organic solvent," as used herein, refers to a solvent that tends to provide protons, such as an alcohol, for example, methanol, ethanol, propanol, isopropanol, butanol, t-butanol, and the like. Such solvents are well known to those skilled in the art, and it will be obvious to those skilled in the art that individual solvents or mixtures thereof may be preferred for specific compounds and reaction conditions, depending upon such factors as the solubility of reagents, reactivity of reagents and preferred temperature ranges, for example. Further discussions of protogenic solvents may be found in organic chemistry textbooks or in specialized monographs, for example: Organic Solvents Physical Properties and Methods of Purification, 4th ed., edited by John A. Riddick et al., Vol. II, in the Techniques of Chemistry Series, John Wiley & Sons, NY, 1986.

"An effective amount," as used herein, refers to an amount of a compound which confers a therapeutic effect on the treated subject. The therapeutic effect may be objective (i.e., measurable by some test or marker) or subjective (i.e., subject gives an indication of or feels an effect). An effective amount of the compound described above may range from about 0.1 mg/Kg to about 500 mg/Kg, preferably from about 1 to about 50 mg/Kg. Effective doses will also vary depending on route of administration, as well as the possibility of co-usage with other agents.

Combinations of substituents and variables envisioned by this invention are only those that result in the formation of stable compounds. The term "stable", as used herein, refers to compounds which possess stability sufficient to allow manufacture and which maintains the integrity of the compound for a sufficient period of time to be useful for the purposes detailed herein (e.g., therapeutic or prophylactic administration to a subject).

The synthesized compounds can be separated from a reaction mixture and further purified by a method such as column chromatography, high pressure liquid chromatography, or recrystallization. As can be appreciated by the skilled artisan, further methods of synthesizing the compounds of the formulae herein will be evident to those of ordinary skill in the art. Additionally, the various synthetic steps may be performed in an alternate sequence or order to give the desired compounds. Synthetic chemistry transformations and protecting group methodologies (protection and deprotection) useful in synthesizing the compounds described herein are known in the art and include, for example, those such as described in R. Larock, Comprehensive Organic Transformations, VCH Publishers (1989); T.W. Greene and P.G.M. Wuts, Protective Groups in Organic Synthesis, 2d. Ed., John Wiley and Sons (1991); L. Fieser and M. Fieser, Fieser and Fieser's Reagents for Organic Synthesis, John Wiley and Sons (1994); and L. Paquette, ed., Encyclopedia of Reagents for Organic Synthesis, John Wiley and Sons (1995), and subsequent editions thereof.

The term "subject" as used herein refers to an animal. Preferably the animal is a mammal. More preferably the mammal is a human. A subject also refers to, for example, dogs, cats, horses, cows, pigs, guinea pigs, fish, birds and the like.

The compounds of this invention may be modified by appending appropriate functionalities to enhance selective biological properties. Such modifications are known in the art and may include those which increase biological penetration into a given biological system (e.g., blood, lymphatic system, central nervous system), increase oral availability, increase solubility to allow administration by injection, alter metabolism and alter rate of excretion.

The compounds described herein contain two or more asymmetric centers and thus give rise to enantiomers, diastereomers, and other stereoisomeric forms that may be defined, in terms of absolute stereochemistry, as (R)- or (S)- , or as (D)- or (L)- for amino acids. The present invention is meant to include all such possible isomers, as well as their racemic and optically pure forms. Optical isomers may be prepared from their respective optically active precursors by the procedures described above, or by resolving the racemic mixtures. The resolution can be carried out in the presence of a resolving agent, by chromatography or by repeated crystallization or by some combination of these techniques which are known to those skilled in the art. Further details regarding resolutions can be found in Jacques, et al., Enantiomers, Racemates, and Resolutions (John Wiley & Sons, 1981). When the compounds described herein contain olefinic double bonds or other centers of geometric asymmetry, and unless specified otherwise, it is intended that the compounds include both E and Z geometric isomers. Likewise, all tautomeric forms are also intended to be included. The configuration of any carbon-carbon double bond appearing herein is selected for convenience only and is not intended to designate a particular configuration unless the text so states; thus a carbon-carbon double bond depicted arbitrarily herein as *trans* may be *cis, trans*, or a mixture of the two in any proportion.

As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, S. M. Berge, et al. describes pharmaceutically acceptable salts in detail in J. Pharmaceutical Sciences, 66: 1-19 (1977). The salts can be prepared in situ during the final isolation and purification of the compounds of the invention, or separately by reacting the free base function with a suitable organic acid. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, alkyl having from 1 to 6 carbon atoms, sulfonate and aryl sulfonate.

When the compositions of this invention comprise a combination of a compound of the formulae described herein and one or more additional therapeutic or prophylactic agents, both the compound and the additional agent should be present at dosage levels of between about 1 to 100%, and more preferably between about 5 to 95% of the dosage normally administered in a monotherapy regimen. The additional agents may be administered separately, as part of a multiple dose regimen, from the compounds of this invention. Alternatively, those agents may be part of a single dosage form, mixed together with the compounds of this invention in a single composition.

As used herein, unless otherwise indicated, the term "bacterial infection(s)" or "protozoa infections"; includes, but is not limited to, bacterial infections and protozoa infections that occur in mammals, fish and birds as well as disorders related to bacterial infections and protozoa infections that may be treated or prevented by administering antibiotics such as the compounds of the present invention. Such bacterial infections and protozoa infections and disorders related to such infections include, but are not limited to, the following: pneumonia, otitis media, sinusitus, bronchitis, tonsillitis, and mastoiditis related to infection by Streptococcus pneumoniae, Haemophilus influenzae, Moraxella catarrhalis, Staphylococcus aureus, or Peptostreptococcus spp. Pseudomonas spp.; pharynigitis, rheumatic fever, and glomerulonephritis related to infection by Streptococcus pyogenes, Groups C and G streptococci, Clostridium diptheriae, or Actinobacillus haemolyticum; respiratory tract infections related to infection by Mycoplasma pneumoniae, Legionella pneumophila, Streptococcus pneumoniae, Haemophilus influenzae, or Chlamydia pneumoniae; uncomplicated skin and soft tissue infections, abscesses and osteomyelitis, and puerperal fever related to infection by Staphylococcus aureus, coagulase-positive staphylococci (i.e., S. epidermidis, S. hemolyticus, etc.), S. pyogenes, S. agalactiae, Streptococcal groups C-F (minute-colony streptococci), viridans streptococci, Corynebacterium spp., Clostridium spp., or Bartonella henselae; uncomplicated acute urinary tract infections related to infection by S. saprophyticus or Enterococcus spp.; urethritis and cervicitis; and sexually transmitted diseases related to infection by Chlamydia trachomatis, Haemophilus ducreyi, Treponema pallidum, Ureaplasma urealyticum, or Nesseria gonorrheae; toxin diseases related to infection by S. aureus (food poisoning and Toxic shock syndrome), or Groups A, S. and C streptococci; ulcers related to infection by Helicobacter pylori; systemic febrile syndromes related to infection by Borrelia recurrentis; Lyme disease related to infection by Borrelia burgdorferi; conjunctivitis, keratitis, and dacrocystitis related to infection by C. trachomatis, N. gonorrhoeae, S. aureus, S. pneumoniae, S. pyogenes, H. influenzae, or Listeria spp.; disseminated Mycobacterium avium complex (MAC) disease related to infection by Mycobacterium avium, or Mycobacterium intracellulare; gastroenteritis related to infection by Campylobacter jejuni; intestinal protozoa related to infection by Cryptosporidium spp. odontogenic infection related to infection by viridans streptococci; persistent cough related to infection by Bordetella pertussis; gas gangrene related to infection by Clostridium perfringens or Bacteroides spp.; Skin infection by S. aureus, Propionibacterium acne; atherosclerosis related to infection by Helicobacter pylori or Chlamydia pneumoniae; or the like.

Bacterial infections and protozoa infections and disorders related to such infections that may be treated or prevented in animals include, but are not limited to, the following: bovine respiratory disease related to infection by P. haemolytica., P. multocida, Mycoplasma bovis, or Bordetella spp.; cow enteric disease related to infection by E. coli or protozoa (i.e., coccidia, cryptosporidia, etc.), dairy cow mastitis related to infection by S. aureus, S. uberis, S. agalactiae, S. dysgalactiae, Klebsiella spp., Corynebacterium, or Enterococcus spp.; swine respiratory disease related to infection by A. pleuropneumoniae., P. multocida, or Mycoplasma spp.; swine enteric disease related to infection by E. coli, Lawsonia intracellularis, Salmonella spp., or Serpulina hyodyisinteriae; cow footrot related to infection by Fusobacterium spp.; cow metritis related to infection by E. coli; cow hairy warts related to Infection by Fusobacterium necrophorum or Bacteroides nodosus; cow pink-eye related to infection by Moraxella bovis, cow premature abortion related to infection by protozoa (i.e. neosporium); urinary tract infection in dogs and cats related to infection by E. coli; skin and soft tissue infections in dogs and cats related to infection by S. epidermidis, S. intermedius, coagulase neg. Staphylococcus or P. multocida; and dental or mouth infections in dogs and oats related to infection by Alcaligenes spp., Bacteroides spp., Clostridium spp., Enterobacter spp., Eubacterium spp., Peptostreptococcus spp., Porphfyromonas spp., Campylobacter spp., Actinomyces spp., Erysipelothrix spp., Rhodococcus spp., Trypanosoina spp., Plas,odium spp., Babesia spp., Toxoplasma spp., Pneumocystis spp., Leishmania spp., and Trichomonas spp. or Prevotella spp. Other bacterial infections and protozoa infections and disorders related to such infections that may be treated or prevented in accord with the method of the present invention are referred to in J. P. Sanford at al.,"The Sanford Guide To Antimicrobial Therapy," 26th Edition, (Antimicrobial Therapy, Inc., 1996).

### Antibacterial Activity

Susceptibility tests can be used to quantitatively measure the *in vitro* activity of an antimicrobial agent against a given bacterial isolate. Compounds were tested for *in vitro* antibacterial activity by a micro-dilution method. Minimal Inhibitory Concentration (MIC) was determined in 96 well microtiter plates utilizing the appropriate Mueller Hinton Broth medium (CAMHB) for the observed bacterial isolates. Antimicrobial agents were serially diluted (2-fold) in DMSO to produce a concentration range from about 64 µg/ml to about 0.03 µg/ml. The diluted compounds (2 µl/well) were then transferred into sterile, uninoculated CAMHB (0.2 mL) by use of a 96 fixed tip-pipetting station. The inoculum for each bacterial strain was standardized to 5 x 10⁵ CFU/mL by optical comparison to a 0.5 McFarland turbidity standard. The plates were inoculated with 10 µl/well of adjusted bacterial inoculum. The 96 well plates were covered and incubated at 35 +/- 2°C for 24 hours in ambient air environment. Following incubation, plate wells were visually examined by Optical Density measurement for the presence of growth (turbidity). The lowest concentration of an antimicrobial agent at which no visible growth occurs was defined as the MIC. The compounds of the invention generally demonstrated an MIC in the range from about 64 µg/ml to about 0.03 µg/ml.

All *in vitro* testing follows the guidelines described in the Approved Standards M7-A4 protocol, published by the National Committee for Clinical Laboratory Standards (NCCLS).

### Pharmaceutical Compositions.

The pharmaceutical compositions of the present invention comprise a therapeutically effective amount of a compound of the present invention formulated together with one or more pharmaceutically acceptable carriers or excipients.

As used herein, the term "pharmaceutically acceptable carrier or excipient" means a non-toxic, inert solid, semi-solid or liquid filler, diluent, encapsulating material or formulation auxiliary of any type. Some examples of materials which can serve as pharmaceutically acceptable carriers are sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil, safflower oil, sesame oil, olive oil, corn oil and soybean oil; glycols such as propylene glycol; esters such as ethyl oleate and ethyl laurate; agar; buffering agents such as magnesium hydroxide and aluminun hydroxide; alginic acid; pyrogen-free water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator.

The pharmaceutical compositions of this invention may be administered orally, parenterally, by inhalation spray, topically, rectally, nasally, buccally, vaginally or via an implanted reservoir, preferably by oral administration or administration by injection. The pharmaceutical compositions of this invention may contain any conventional non-toxic pharmaceutically-acceptable carriers, adjuvants or vehicles. In some cases, the pH of the formulation may be adjusted with pharmaceutically acceptable acids, bases or buffers to enhance the stability of the formulated compound or its delivery form. The term parenteral as used herein includes subcutaneous, intracutaneous, intravenous, intramuscular, intraarticular, intraarterial, intrasynovial, intrasternal, intrathecal, intralesional and intracranial injection or infusion techniques.

Liquid dosage forms for oral administration include pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable preparations, for example, sterile injectable aqueous or oleaginous suspensions, may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a drug, it is often desirable to slow the absorption of the drug from subcutaneous or intramuscular injection. This may be accomplished by the use of a liquid suspension of crystalline or amorphous material with poor water solubility. The rate of absorption of the drug then depends upon its rate of dissolution, which, in turn, may depend upon crystal size and crystalline form. Alternatively, delayed absorption of a parenterally administered drug form is accomplished by dissolving or suspending the drug in an oil vehicle. Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions that are compatible with body tissues.

Compositions for rectal or vaginal administration are preferably suppositories which can be prepared by mixing the compounds of this invention with suitable non-irritating excipients or carriers such as cocoa butter, polyethylene glycol or a suppository wax which are solid at ambient temperature but liquid at body temperature and therefore melt in the rectum or vaginal cavity and release the active compound.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or: a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like.

The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes.

Dosage forms for topical or transdermal administration of a compound of this invention include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, ear drops, eye ointments, powders and solutions are also contemplated as being within the scope of this invention.

The ointments, pastes, creams and gels may contain, in addition to an active compound of this invention, excipients such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to the compounds of this invention, excipients such as lactose, talc, silicic acid, aluminum hydroxide, calcium silicates and polyamide powder, or mixtures of these substances. Sprays can additionally contain customary propellants such as chlorofluorohydrocarbons.

Transdermal patches have the added advantage of providing controlled delivery of a compound to the body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

According to the methods of treatment of the present invention, bacterial infections are treated or prevented in a patient such as a human or other animals by administering to the patient a therapeutically effective amount of a compound of the invention, in such amounts and for such time as is necessary to achieve the desired result.

By a "therapeutically effective amount" of a compound of the invention is meant a sufficient amount of the compound to treat bacterial infections, at a reasonable benefit/risk ratio applicable to any medical treatment. It will be understood, however, that the total daily usage of the compounds and compositions of the present invention will be decided by the attending physician within the scope of sound medical judgment. The specific therapeutically effective dose level for any particular patient will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combination or contemporaneously with the specific compound employed; and like factors well known in the medical arts.

The total daily dose of the compounds of this invention administered to a human or other animal in single or in divided doses can be in amounts, for example, from 0.01 to 50 mg/kg body weight or more usually from 0.1 to 25 mg/kg body weight. Single dose compositions may contain such amounts or submultiples thereof to make up the daily dose. In general, treatment regimens according to the present invention comprise administration to a patient in need of such treatment from about 10 mg to about 1000 mg of the compound(s) of this invention per day in single or multiple doses. ,

The compounds of the formulae described herein can, for example, be administered by injection, intravenously, intraarterially, subdermally, intraperitoneally, intramuscularly, or subcutaneously; or orally, buccally, nasally, transmucosally, topically, in an ophthalmic preparation, or by inhalation, with a dosage ranging from about 0.5 to about 100 mg/kg of body weight, alternatively dosages between 1 mg and 1000 mg/dose, every 4 to 120 hours, or according to the requirements of the particular drug. The methods herein contemplate administration of an effective amount of compound or compound composition to achieve the desired or stated effect. Typically, the pharmaceutical compositions of this invention will be administered from about 1 to about 6 times per day or alternatively, as a continuous infusion. Such administration can be used as a chronic or acute therapy. The amount of active ingredient that may be combined with the carrier materials to produce a single dosage form will vary depending upon the host treated and the particular mode of administration. A typical preparation will contain from about 5% to about 95% active compound (w/w). Alternatively, such preparations may contain from about 20% to about 80% active compound.

Lower or higher doses than those recited above may be required. Specific dosage and treatment regimens for any particular patient will depend upon a variety of factors, including the activity of the specific compound employed, the age, body weight, general health status, sex, diet, time of administration, rate of excretion, drug combination, the severity and course of the disease, condition or symptoms, the patient's disposition to the disease, condition or symptoms, and the judgment of the treating physician.

Upon improvement of a patient's condition, a maintenance dose of a compound, composition or combination of this invention may be administered, if necessary. Subsequently, the dosage or frequency of administration, or both, may be reduced, as a function of the symptoms, to a level at which the improved condition is retained when the symptoms have been alleviated to the desired level. Patients may, however, require intermittent treatment on a long-term basis upon any recurrence of disease symptoms.

The pharmaceutical compositions of this invention can be administered orally to fish by blending said pharmaceutical compositions into fish feed or said pharmaceutical compositions may be dissolved in water in which infected fish are placed, a method commonly referred to as a medicated bath. The dosage for the treatment of fish differs depending upon the purpose of administration (prevention or cure of disease) and type of administration, size and extent of infection of the fish to be treated. Generally, a dosage of 5
- 1000 mg, preferably 20 - 100 mg, per kg of body weight of fish may be administered per day, either at one time or divided into several times. It will be recognized that the above-specified dosage is only a general range which may be reduced or increased depending upon the age, body weight, condition of disease, etc. of the fish.

Unless otherwise defined, all technical and scientific terms used herein are accorded the meaning commonly known to one with ordinary skill in the art. All publications, patents, published patent applications, and other references mentioned herein are hereby incorporated by reference in their entirety.

### Abbreviations

Abbreviations which have been used in the descriptions of the scheme and the examples that follow are:
Ac for acetyl;
AIBN for azobisisobutyronitrile;
Bu₃SnH for tributyltin hydride;
CDI for carbonyldiimidazole;
dba for dibenzylidene acetone;
dppb for diphenylphosphino butane;
DBU for 1,8-diazabicyclo[5.4.0]undec-7-ene;
DEAD for diethylazodicarboxylate;
DMAP for dimethylaminopyridine;
DMF for dimethyl formamide;
DPPA for diphenylphosphoryl azide;
EtOAc for ethyl acetate;
MeOH for methanol;
NaN(TMS)₂ for sodium bis(trimethylsilyl)amide;
NMMO for N-methylmorpholine N-oxide;
TEA for triethylamine;
THF for tetrahydrofuran;
TPP or PPh₃ for triphenylphosphine; ,
MOM for methoxymethyl;
Boc for t - butoxycarbonyl;
Bz for benzyl;
Ph for phenyl;
POPd for dihydrogen dichlorobis(di-tert-butylphosphinito-κP)palladate(II);
TBS for *tert*-butyl dimethylsilyl; or
TMS for trimethylsilyl.

### Synthetic Methods

The compounds and processes of the present invention will be better understood in connection with the following synthetic schemes that illustrate the methods by which the compounds of the invention may be prepared.

A preferred intermediate for the preparation of compounds represented by formula I is a compound represented by formula IX as illustrated below 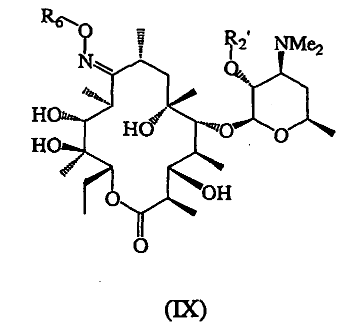 wherein R₆ and R₂' are as previously defined.

A second preferred intermediate for the preparation of compounds represented by formula I is a compound represented by formula X as illustrated below 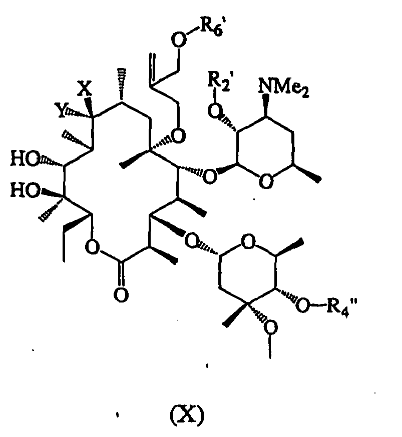 where X, Y, R2^{'}, R₄^{"} and R₆^{'} are as previously defined.

Schemes 1 - 4 describe processes for the preparation of intermediates which are useful in the preparation of compounds according to the invention.

Compounds of formula (**1**-2), which are useful as the starting materials for the preparation of compounds of the present invention, may be synthesized as detailed in Schemes 1 and 2 below. An erythromycin derivative (**1**-2) is prepared from erythromycin using the procedures described in U.S. Patents 4,990,602; 4,331,803; 4,680,386; 4,670,549; and European Patent Application EP 260,938.

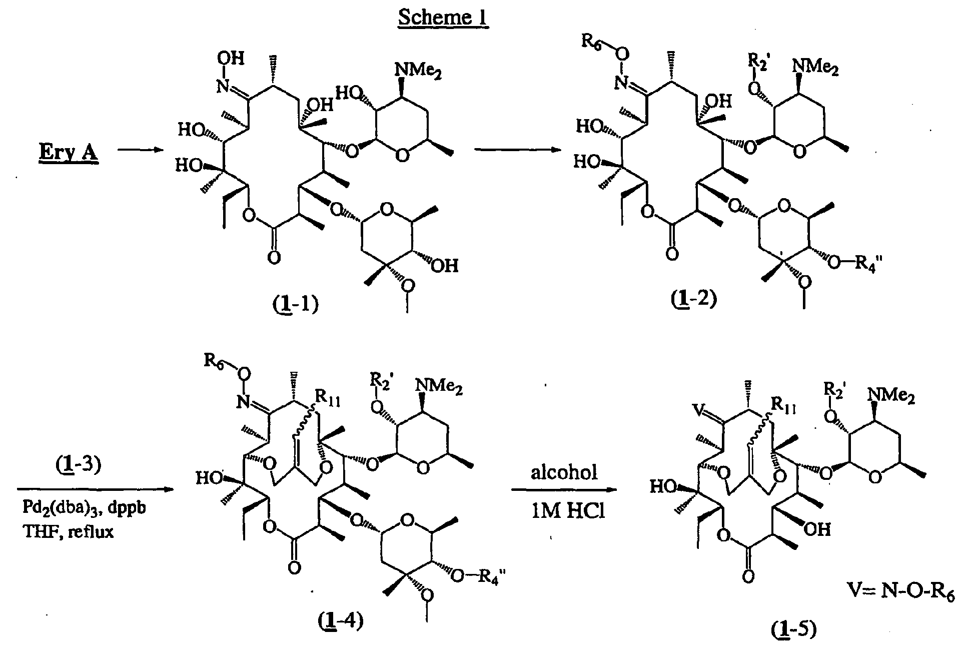

The erythromycin derivative of formula (**1**-2), is then reacted with an alkylating agent of the formula:

R₁₃-OC(O)O-CH₂[C=CH R₁₁]CH₂-OC(O)-OR₁₃ (**1**-3)

where R₁₃ is C₁-C₁₂-alkyl and R₁₁ is as previously defined.

Most palladium (0) catalysts are expected to work in this process. Some palladium (II) catalysts, such as palladium (II) acetate, which is converted into a palladium (0) species *in-situ* by the actions of a phosphine, will work as well. See, for example, Beller et al. Angew. Chem. Int. Ed. Engl., 1995, 34 (17), 1848. The palladium catalyst can be selected from, but not limited to, the group consisting of palladium (II) acetate, tetrakis(triphenylphospine)palladium (0), tris(dibenzylideneacetone)dipalladium, tetradi(benzylideneacetone)dipalladium and the like. Palladium on carbon and palladium (II) halide catalysts are less preferred than other palladium catalysts for this process.

Suitable phosphines include, but are not limited to, triphenylphosphine, bis(diphenylphosphino)methane, bis(diphenylphosphino)ethane, bis(diphenylphosphino)propane, 1,4-bis(diphenylphosphino)butane, bis(diphenylphosphino)pentane, and tri(o-tolyl)phosphine, and the like.

The reaction is carried out in an aprotic solvent, preferably at elevated temperature, for example, at or above 50 °C. Suitable aprotic solvents include, but are not limited to, tetrahydrofuran, N,N-dimethylformamide, dimethyl sulfoxide, N-methyl-2-pyrrolidone, hexamethylphosphoric triamide, 1,2-dimethoxyethane, methyl-*tert*-butyl ether, heptane, acetonitrile, isopropyl acetate and ethyl acetate. The most preferred solvents are tetrahydrofuran or toluene.

The alkylating agents useful in the processes of the invention are di-carbonates (**1**-3). Generally, the alkylating agents have the formula (**1**-3), previously described. The preferred alkylating agents are those wherein R₁₃ is a tert-butyl, isopropyl or isobutyl group. The alkylating reagents are prepared by reaction of a di-ol with a wide variety of compounds for incorporating the di-carbonate moiety. The compounds include, but are not limited to, *tert-*butyl chloroformate, di-*tert*-butyl dicarbonate, and 1-(*tert*-butoxycarbonyl)imidazole and the reaction is carried out in the presence of an organic or an inorganic base. The temperature of the reaction varies from about -30 °C to about 30°C. Preferably, the alkylating reagent is di-*tert*-butyl dicarbonate.

An alternative method of converting the alcohol into the carbonate involves treating the alcohol with phosgene or triphosgene to prepare the chloroformate derivative of the di-ol. The di-chloroformate derivative is then converted into the di-carbonate by the methods described in Cotarca, L., Delogu, P., Nardelli, A., Sunijic, V, Synthesis, 1996, 553. The reaction can be carried out in a variety of organic solvents such as dichloromethane, toluene, diethyl ether, ethyl acetate and chloroform in the presence of a base. Examples of suitable bases include, but are not limited to, sodium hydroxide, potassium hydroxide, ammonium hydroxide, sodium carbonate, potassium carbonate, ammonium carbonate, dimethylaminopyridine, pyridine, triethylamine and the like. The temperature conditions can vary from 0 °C to about 60 °C. The reaction typically takes about 3 to 5 hours to run to completion.

The cladinose moiety of macrolide (**1**-4) is removed either by mild acid hydrolysis or by enzymatic hydrolysis to give compounds of formula (**1**-5). Representative acids include dilute hydrochloric acid, sulfuric acid, perchloric acid, chloroacetic acid, dichloroacetic acid or trifluoroacetic acid. Suitable solvents for the reaction include methanol, ethanol, isopropanol, butanol and the like. Reaction times are typically 0.5 to 24 hours. The reaction temperature is preferably -10°C to 80°C

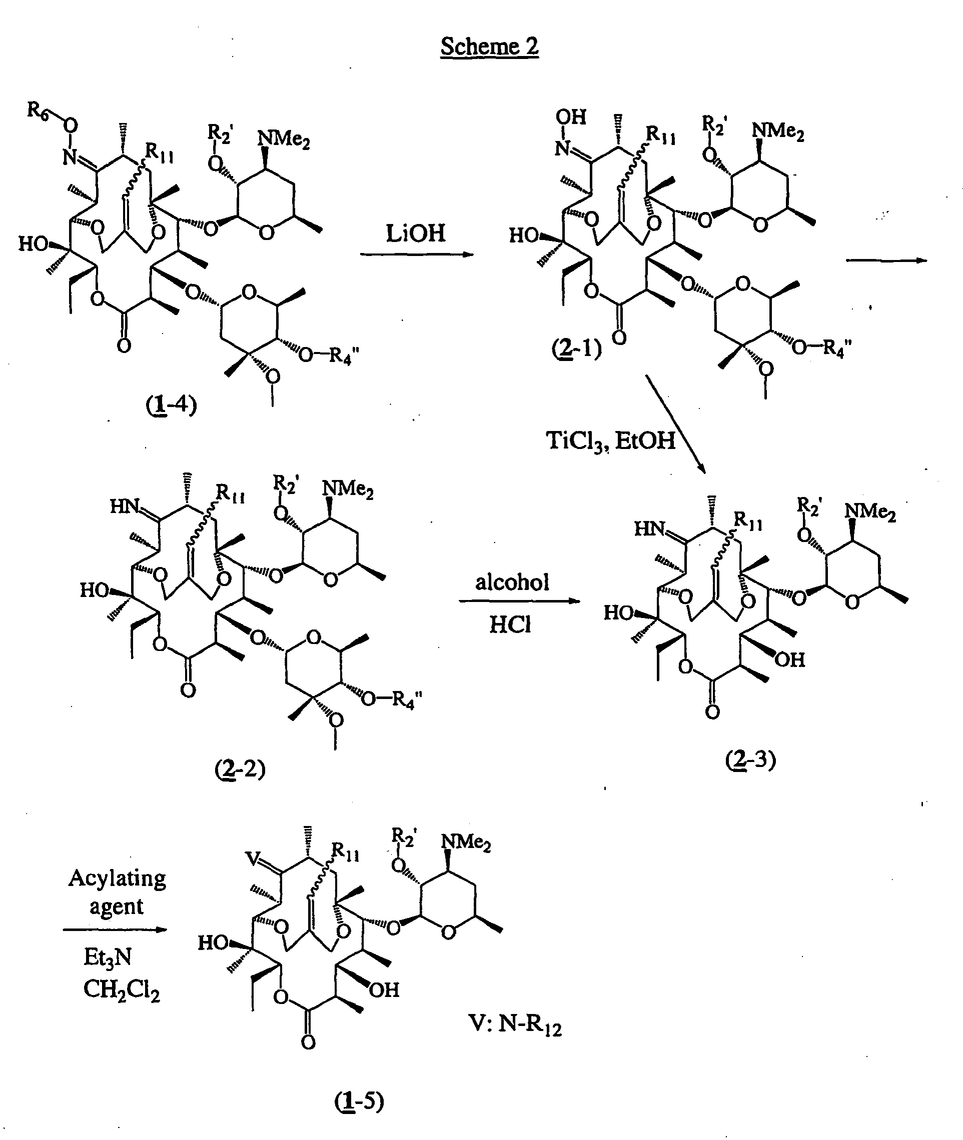

Compounds of formula (**1**-4), where R₆ is an acetyl group, can be converted into the corresponding imine as outlined in Scheme 2. Selective deprotection of the oxime is typically accomplished via alkaline hydrolysis in protic solvents. Representative alkali compounds include lithium hydroxide, sodium hydroxide, potassium hydroxide, and the like. Suitable solvents include, but are not limited to, tetrahydrofuran, 1,4-dioxane, 1,2-dimethoxyethane, isopropanol, ethanol, butanol and mixtures there of. The reaction temperature is preferably 0° to 35°C and reaction time is preferably 0.5 to 8 hours.

In a like fashion, simultaneous deprotection of both the oxime and the 2' hydroxyl can be accomplished under a variety of conditions. Conditions for deprotection include, but not limited to, treating with an alcoholic solvent from room temperature to reflux, or treatment with a primary amine, such as butylamine. Alcoholic solvents preferred for the deprotection are methanol and ethanol. A more thorough discussion of the procedures, reagents and conditions for removing protecting groups is described in literature, for example, by T.W. Greene and P.G.M. Wuts in "Protective Groups in Organic Synthesis" 3rd ed., John Wiley & Son, Inc, 1999.

Deoxygenation of compounds of formula (**2**-1) under reducing conditions gives the macrolide imine of formula (**2**-2). Many reducing agents can be used to effect this transformation including, but not limited to, lithium aluminum hydride, titanium trichloride, borane, and various sulfides such as sodium hydrogen sulfide and sodium nitrite. For a more detailed account of oxime reduction see J. March in "Advanced Organic Chemistry" 4th ed., Wiley & Son, Inc, 1992.

A particularly useful method for the reduction of oximes to the corresponding imine uses a sulfite reducing agent, such as sodium hydrogensulfite, under acidic conditions, typically in protic solvents. Representative acids include, but are not limited to, acetic acid, formic acid, dilute hydrochloric acid, dilute phosphoric acid, dilute sulfuric acid, and the like. Suitable protic solvents include, but are not limited to, mixtures of water and methanol, ethanol, isopropanol, or butanol. The reaction is typically carried out at 50° to 110 °C, preferably for between 1 and 10 hours.

Hydrolysis of the cladinose moiety can be accomplished as previously described in scheme 1 to give compounds of formula (**2-**3)

Alternatively compounds of formula (**2-**3) can be formed directly from compounds of formula (**2**-1) via treatment with TiCl₃ in alcoholic solvents, preferably methanol or ethanol

Imines of formula (**2**-3) can be acylated under basic conditions using a suitable acylating agent in an aprotic solvent. Typical acylating agents include, but are not limited to, acetyl chloride, acetic anhydride, benzoyl chloride, benzoic anhydride, and benzyl chloroformate.

Examples of aprotic solvents are dichloromethane, chloroform, tetrahydrofuran, N-methylpyrrolidinone, dimethylsulfoxide, N,N-dimethylformamide, N,N -dimethylacetamide, hexamethylphosphoric triamide, a mixture thereof or a mixture of one of these solvents with ether, tetrahydrofuran, 1,2-dimethoxyethane, 1,2-dichloroethane, acetonitrile, ethyl acetate, acetone and the like. Aprotic solvents do not adversely affect the reaction. Preferably, the solvent is selected from dichloromethane, chloroform, N,N -dimethylformamide, tetrahydrofuran, N-methylpyrrolidinone or mixtures thereof.

Typical bases include, but are not limited to, pyridine, triethylamine, diisopropyl ethylamine, N-methyl morpholine, N-methyl pyrrolidine, 2,6-lutidine, 1,8-diazabicyclo[5.4.0]undec-7-ene. For a more extensive discussion of acylating conditions see, for example, T.W. Greene and P.G.M. Wuts in "Protective Groups in Organic Synthesis" 3rd ed., John Wiley & Son, Inc, 1999.

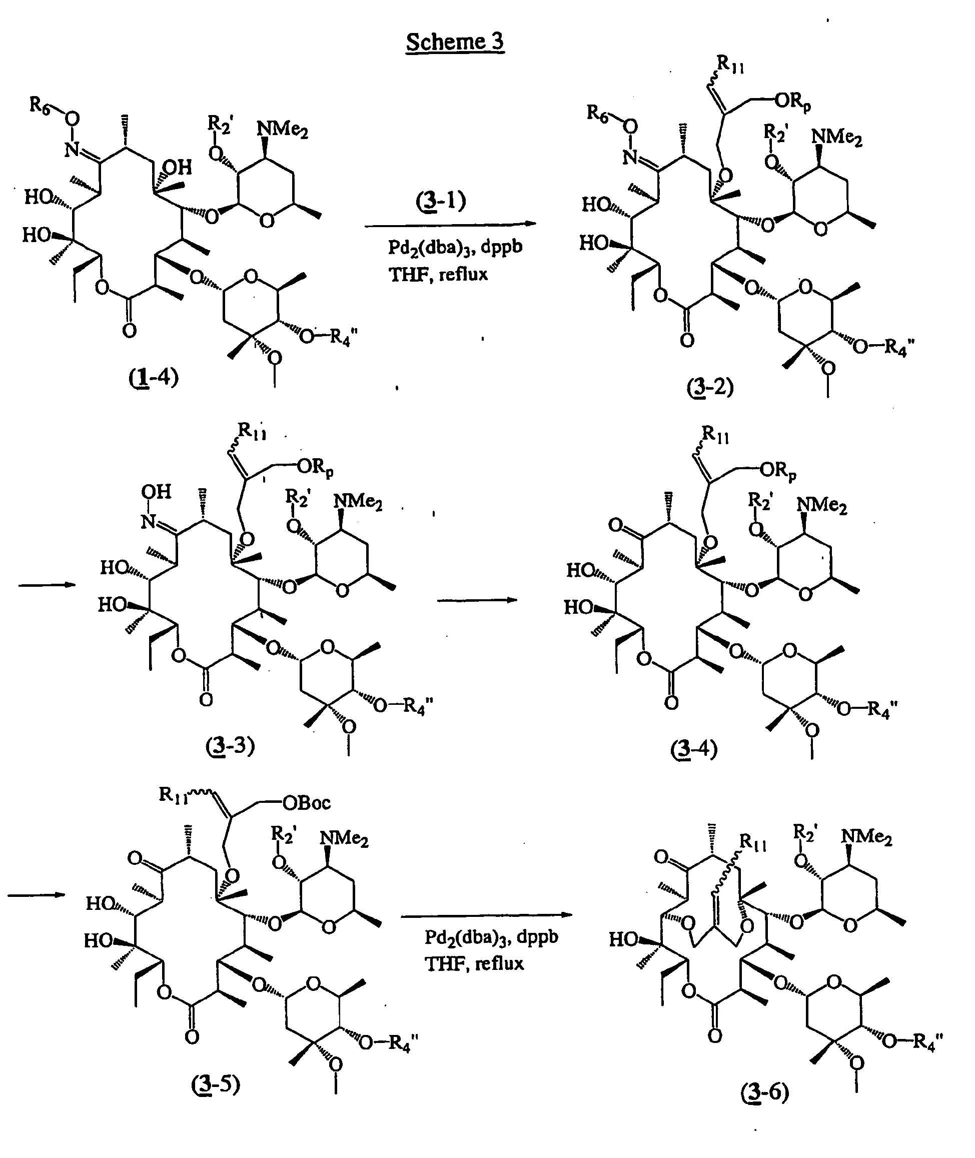

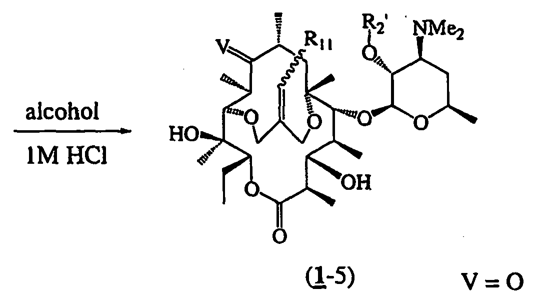

Stepwise formation of the 6-11 bridged macrolides is also possible as outlined in Scheme 3. In a similar manner as previously described, the procedure involves reacting a compound of formula (**1**-4) with a suitable alkylating agent. As before, the erythromycin derivative of formula (**1**-4) is reacted with an alkylating agent of the formula:

R₁₃-OC(O)O-CH₂[C=CHR₁₁]CH₂-O-Rₚ (**3**-1)

where R₁₃ is C₁-C₁₂-alkyl, and R₁₁ and Rₚ are as previously defined.

As discussed previously, most palladium (0) catalysts are expected to work in this process and the reaction is carried out in an aprotic solvent, preferably at elevated temperature, preferably at or above 50 °C. The most preferred solvents are tetrahydrofuran and toluene.

The alkylating agents useful in the process of the invention are the mixed silyl ether carbonates (**3**-1). Generally, the alkylating agents have the formula (**3**-1), previously described. The preferred alkylating agents are those wherein R₁₃ is *tert*-butyl, isopropyl or isobutyl and Rp is *tert*-butyl dimethyl silyl, triisopropyl silyl, *tert*-butyl diphenyl silyl or the like.

The alkylating reagents of formula (**3**-1) are prepared by reaction of a diol sequentially with a wide variety of compounds for incorporating the carbonate moiety, followed by a wide variety of compounds for incorporating the silyl moiety. Alkylating reagents include, but not limited to, *tert*-butyl chloroformate, di-*tert*-butyl dicarbonate, and 1-(*tert*-butoxycarbonyl)imidazole; where as silylating reagents include, but are not limited to *tert*-butyl dimethyl silyl chloride, *tert*-butyl dimethyl silyl triflate, *tert*-butyl dimethyl silyl cyanide, and *tert*-butyl dimethyl silyl imidazole. Both reactions are carried out in the presence of an organic or an inorganic base. The temperature of the reactions vary from about -30°C to about 30°C. Preferably, the alkylating reagent is di-*tert*-butyl dicarbonate and the silylating reagent is *tert*-butyl dimethyl silyl chloride.

The free oxime (**3**-3) is prepared using essentially the same procedure as for the deprotection of oxime (**1**-4) where R₆ is Ac in Scheme 2.

Compounds of formula (**3**-4) can be formed directly from compounds of formula (**3-**3) by the application of the previously described procedure for the reduction of oximes of formula (**2**-1) to the corresponding imine of formula (**2**-2).

The protecting group (Rp) is then removed from the hydroxyl of the compound of formula (**3**-4) using the appropriate conditions as outlined in T.W. Greene and P.G.M. Wuts in "Protective Groups in Organic Synthesis" 3rd ed., John Wiley & Son, Inc, 1999. For example, when the protecting group is TBS, tetra-*n*-butyl ammonium fluoride, hydrofluoric acid or trifluoro acetic acid may be used'. Using standard conditions, the primary hydroxyl is converted to the *tert*-butyl carbonate, and subsequently the 11-hydroxyl group is alkylated by means of a palladium (0) catalyst as described previously. In this way compounds of formula (**3**-6) can be prepared readily.

Removal of the cladinose sugar is accomplished as previously described in Scheme 1 to give a compound of formula (**1**-5).

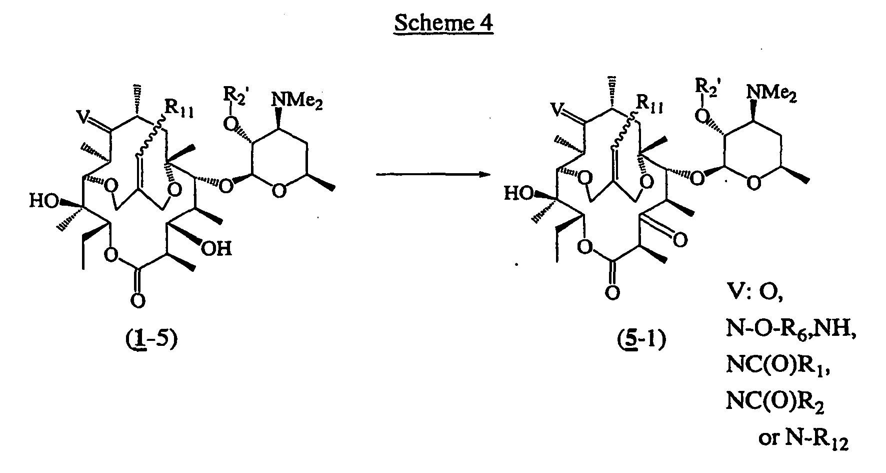

Compounds according to the invention (**5**-1) may be prepared by oxidation of the secondary alcohol using Dess Martin periodinane as the oxidant. The reaction is typically run in an aprotic solvent at 0° to 25°C. The reaction time is typically between 1 and 12 hours.

Alternatively the oxidation can be accomplished using pyridinium chlorochromate, sulfur trioxide pyridine complex in dimethyl sulfoxide, tetra-n-propyl ammonium perruthenate and N-methyl morpholine N-oxide, Swern oxidation or the like. A more thorough discussion of the oxidation of secondary alcohols can be found in J. March in "Advanced Organic Chemistry" 4th ed., Wiley & Son, Inc, 1992.

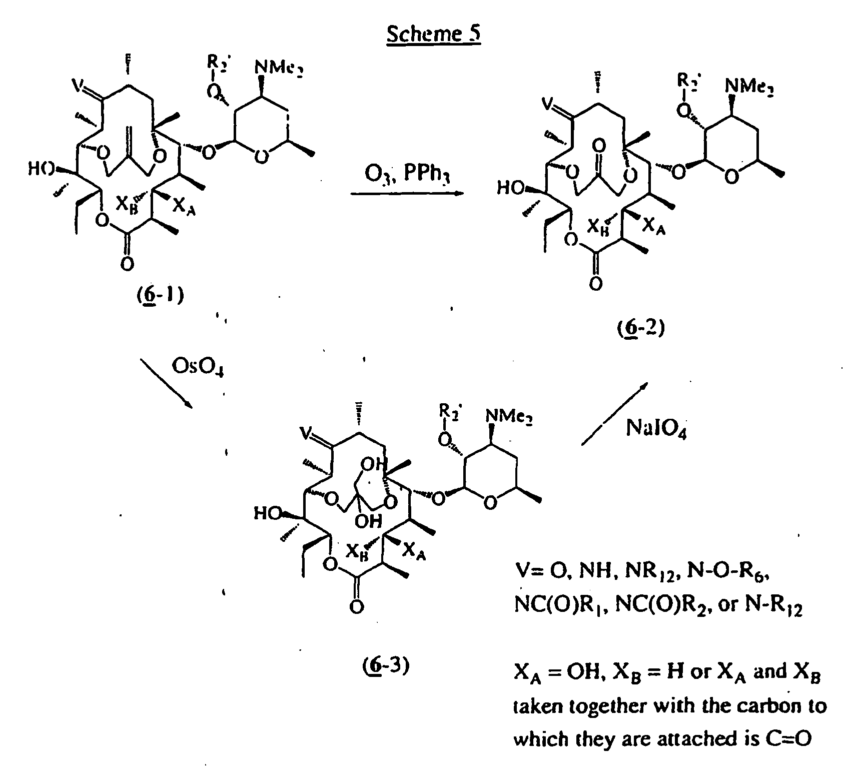

Scheme 5 illustrates another process of the invention for the preparation of compounds according to the invention. Conversion of alkene (**6**-1) into ketone (**6**-2) can be accomplished by exposure of the alkene to ozone followed by decomposition of the ozonide with the appropriate reducing agent, as outlined in Scheme 3. The reaction is typically carried out in an inert solvent such as, but not limited to, methanol, ethanol, ethyl acetate, glacial acetic acid, chloroform, methylene chloride or hexanes or mixtures thereof, preferably methanol, preferably at -78° to -20°C. Representative reducing agents are , for example, triphenylphosphine, trimethyl phosphite, , thiourea, and dimethyl sulfide, preferably triphenylphosphine. A more thorough discussion of ozonolysis and the conditions there for can be found in J. March "Advanced Organic Chemistry" 4th ed., Wiley & Son, Inc, 1992.

An alternative method for the preparation of ketone (**6**-2) involves dihydroxylation of the alkene followed by diol cleavage. The glycol (**6**-3) is first prepared by reacting alkene (**6-**1) with osmium tetroxide. This reaction can be carried out with stochiometric amounts of osmium tetroxide, or, if an oxidant such as hydrogen peroxide, tert-butyl hydroperoxide, or N-methylmorpholine-N-oxide is present, with catalytic amounts of osmium tetroxide. These reactions can be carried out in a variety of solvents including: 1,4-dioxane, tetrahydrofuran, tert-butanol and diethyl ether, preferably at 0 °C

The glycol can be cleaved by a variety of reagents including, but not limited to, periodic acid, lead tetraacetate, manganesedioxide, potassium permanganate, sodium metaperiodate, and N-iodosuccinamide. Depending on the cleavage reagent, a variety of solvents can be used. Preferably the cleavage reagent is sodium metaperiodate, the solvent is preferably a mixture of ethanol, methanol, acetone, or 1,4-dioxane and water and the reaction temperature is 0 ° to 25 °C.

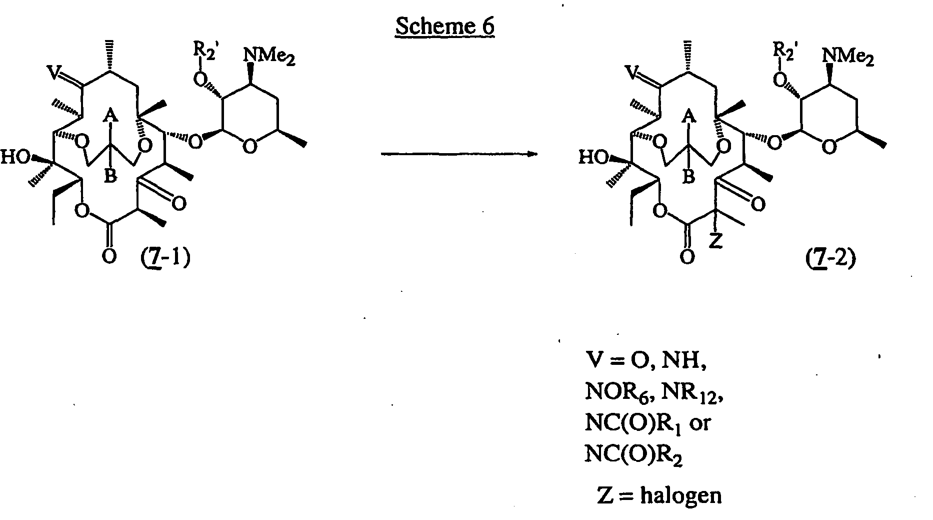

Scheme 6 illustrates the procedure by which compounds of formula (**7**-1) may be converted to compounds of formula (**7**-2) by treatment with a halogenating reagent. This reagent acts to replace a hydrogen atom with a halogen atom at the C-2 position of the ketolide. Various halogenating reagents may be suitable for this procedure.

Fluorinating reagents include, but are not limited to, *N*-fluorobenzenesulfonimide in the presence of base, 10% F₂ in formic acid, 3,5-dichloro-1-fluoropyridinium tetrafluoroborate, 3,5-dichloro-1-fluoropyridinium triflate, (CF₃SO₂)₂NF, N-fluoro-N-methyl-p-toluenesulfonamide in the presence of base, N-fluoropyridinium triflate, N-fluoroperfluoropiperidine in the presence of base.

Chlorinating reagents include, but are not limited to, hexachloroethane in the presence of base, CF₃CF₂CH₂ICl₂, SO₂Cl₂, SOCl₂, CF₃SO₂Cl in the presence of base, Cl₂, NaOCl in the presence of acetic acid.

Brominating reagents include, but are not limited to, Br₂•pyridine•HBr, Br₂/acetic acid, *N*-bromosuccinimide in the presence of base, LDA/BrCH₂CH₂Br, or LDA/CBr₄

A suitable iodinating reagent is N-Iodosuccinimide in the presence of base, or I₂, for example

Suitable bases for the halogenating reactions requiring them are compounds such as alkali metal hydrides, such as NaH and KH, or amine bases, such as LDA or triethylamine, for example. Different reagents may require different type of base, but this is well known within the art.

A preferred halogenating reagent is N-fluorobenzenesulfonimide in the presence of sodium hydride.

Suitable solvents are dimethylformamide, dimethyl sulfoxide, pyrrolidinone and the like.

It will be appreciated by one skilled in the art that compounds of formula (**7**-1) or (**7-**2) can be substituted for compounds of formula (**6**-1) or (**6**-2) in the preceding examples if the corresponding C-2 halogenated product is desired.

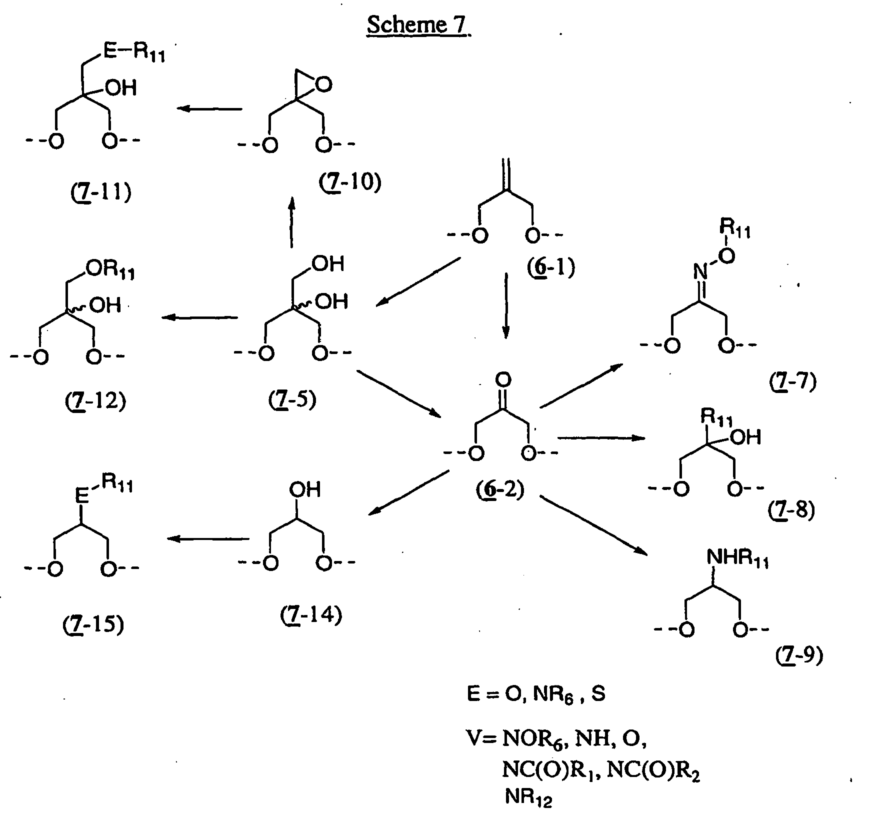

For ease of illustration, in Scheme 7 only the 6-11 bridged moiety of each particular compound according to formula I will be shown, it being understood that it is intended to illustrate a compound according to formula I with the specified bridged moiety. Compounds according to the invention of the formulas (**6**-1) and (**6**-2) can be further functionalized in a variety of ways. Scheme 7 details a procedure for the conversion of the ketone (**6**-2) into an oxime of formula (**7**-7). Oxime formation can be accomplished, using the appropriate substituted hydroxylamine, under either acidic or basic conditions in a variety of solvents. Representative acids include, but are not limited to, hydrochloric, phosphoric, sulfuric, p-toluenesulfonic, and pyridinium p-toluene sulfonate. Likewise, bases that are useful are, for example, triethylamine, pyridine, diisopropylethyl amine, 1,5-lutidine, and the like. Appropriate solvents include, but are not limited to, methanol, ethanol, water, tetrahydrofuran, 1,2-dimethoxyethane, and ethyl acetate. Preferably the reaction is carried out in ethanol using triethylamine as the base. Reaction temperature is generally 25° C and reaction time is 1 to 12 hours.

Ketone (**6**-2) can also be further utilized by conversion into the amine (**7**-9) via a reductive amination protocol. Thus, the ketone is treated with an amine in the presence of a reducing agent to obtain the product amine (**7**-9). The reaction can be carried out either with or without added acid. Examples of acids that are commonly used include, hydrochloric, phosphoric, sulfuric, acetic, and the like. Reducing agents that effect reductive amination include, but are not limited to, hydrogen and a catalyst, zinc and hydrochloric acid, sodium cyanoborohydride, sodium borohydride, iron petnatcarbonyl, and alcoholic potassium hydroxide. Generally alcoholic solvents are used. The preferred conditions use sodium cyanoborohydride in methanol with added acetic acid.

A still further way to functionalize ketone (**6**-2) is via addition of Grignard reagents to form alcohols of formula (**7**-8). Scheme 8 depicts this protocol. The requisite Grignard reagents are readily available via the reaction of a variety of alkyl or aryl halides with magnesium under standard conditions (see B.S. Furniss, A.J. Hannaford, P.W.G. Smith, A.R. Tatchell "Vogel's Textbook of Practical Organic Chemistry" 5th ed., Longman, 1989). The addition is performed in an inert solvent, generally at low temperature. Suitable solvents include, but are not limited to tetrahydrofuran, diethylether, 1,4-dioxane, 1,2-dimethoxyethane, and hexanes. Preferably the solvent is tetrahydrofuran or diethylether. Preferably the reaction is run at -78° to 0° C.

In a similar way, reaction with other organometallic reagents gives rise to alcohols of type (**7**-8). Examples of organometallic reagents that may be used include, but are not limited to, organo-aluminum, organo-lithium, organo-cerium, organo-zinc, organo-thallium, and organo-boron reagents. A more thorough discussion of organometallic reagents can be found in.B.S. Furniss, A.J. Hannaford, P.W.G. Smith, A.R. Tatchell "Vogel's Textbook of Practical Organic Chemistry" 5th ed., Longman, 1989.

Furthermore, alcohols of type (**7**-14) can be prepared by reduction of the corresponding ketone (**6**-2) under a variety of conditions. (see Hudlicky, M. Reductions in Organic Chemistry, Ellis Horwood Limited: Chichester, 1984). The alcohols thus derived can be further modified to give compounds of type (**7**-15). Processes to generate compounds of formula (**7**-15) include, but are not limited to: alkylation of the alcohol with an electrophile or conversion of the alcohol into a leaving group such as a triflate, tosylate, phosponate, halide, or the like followed by displacement with a heteroatom nucleophile (e.g. an amine, alkoxide, sulfide or the like).

It will be appreciated by one skilled in the art, that the unsaturated compounds represented by compounds (**6**-1) and (**6**-2) can be reduced to form the corresponding saturated compound ( see Hudlicky, M. Reductions in Organic Chemistry, Ellis Horwood Limited: Chichester, 1984).

The glycol (**7**-5) can be prepared by reacting alkene (**6**-1) with osmium tetroxide.
This reaction can be carried out either with stochiometric amounts of osmium tetroxide, or, if a oxidant such as hydrogen peroxide, tert-butyl hydroperoxide, or N-methylmorpholine-N-oxide are present, with catalytic amounts of osmium tetroxide. These reactions can be run in a variety of solvents including: 1,4-dioxane, tetrahydrofuran, tert-butanol and diethyl ether, preferably at 0 °C. The glycols thus derived can be further modified to give compounds of type (**7**-12) by, for instance, selective alkylation of the primary alcohol with an electrophile (see B.S. Furniss, A.J. Hannaford, P.W.G. Smith, A.R. Tatchell "Vogel's Textbook of Practical Organic Chemistry" 5th ed., Longman, 1989).

Epoxides of type (**7**-10) can be prepared by the conversion of the primary alcohol into a leaving group such as a triflate, tosylate, phosponate, halide, or the like, followed by intramolecular nucleophillic displacement (see Tetrahedron Lett., 1983, 661-664). Epoxides of formula (**7**-10) can be further funtionalized via ring opening with a variety of nucleophiles. Representative nucleophiles include, but are not limited to, amines, alkoxides, sulfides, organometallic reagents and the like. Reactions can be carried out in the presence or absence of Lewis acid activators such as silver carbonate, silver triflate, boron trifluoride etherate, aluminum trichloride and the like. (see (a) J. Med Chem., 1997, 2762-2769, (b) J. Amer. Chem Soc.,1999, 10251-10263, (c) Tetrahedron Lett., 2000, 4229-4234)

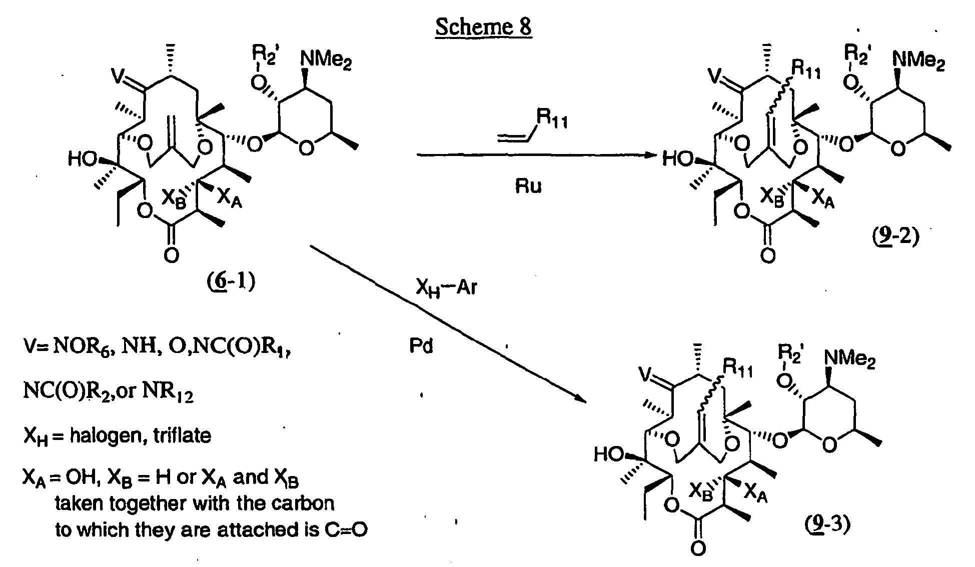

Compounds of the invention according to formula (**6**-1) are also capable of further functionalization to generate compounds of the present invention. Alkene (**6**-1) can be treated with an aryl halide or aryl triflate in the presence of a palladium catalyst [Pd(0) or Pd(II)] to provide compound (**9**-3): (See (a) Heck, Palladium Reagents in Organic Synthesis, Academic Press: New York, 1985, Chapter 1; (b) Sonogashira, Comprehensive Organic Synthesis, Volume 3, Chapters 2,4; (c) Sonogashira, Synthesis 1977, 777.). Under the Heck coupling conditions, regioisomers and stereoisomers of the double bond are possible. Alternatively, compound (**6**-1) can undergo a cross metathesis reaction with vinylaromatic derivatives using ruthenium catalysts to give compounds of formula (**9**-2) (see (a) J. Org. Chem. 2000, 65, 2204-2207; (b) Reviews: Synlett. 1999, 2, 267; (c) Reviews: Ivin, K. J.; Mol, J. C. Olefin Metathesis and Metathesis Polymerization, 2nd ed.; Academic Press: New York, 1997; (d) J. Org. Chem. 1999, 64, 4798-4816; (e) Angew. Chem., Int. Ed. Engl. 1997, 36, 2036-2056; (f) Tetrahedron 1998, 54, 4413-4450).

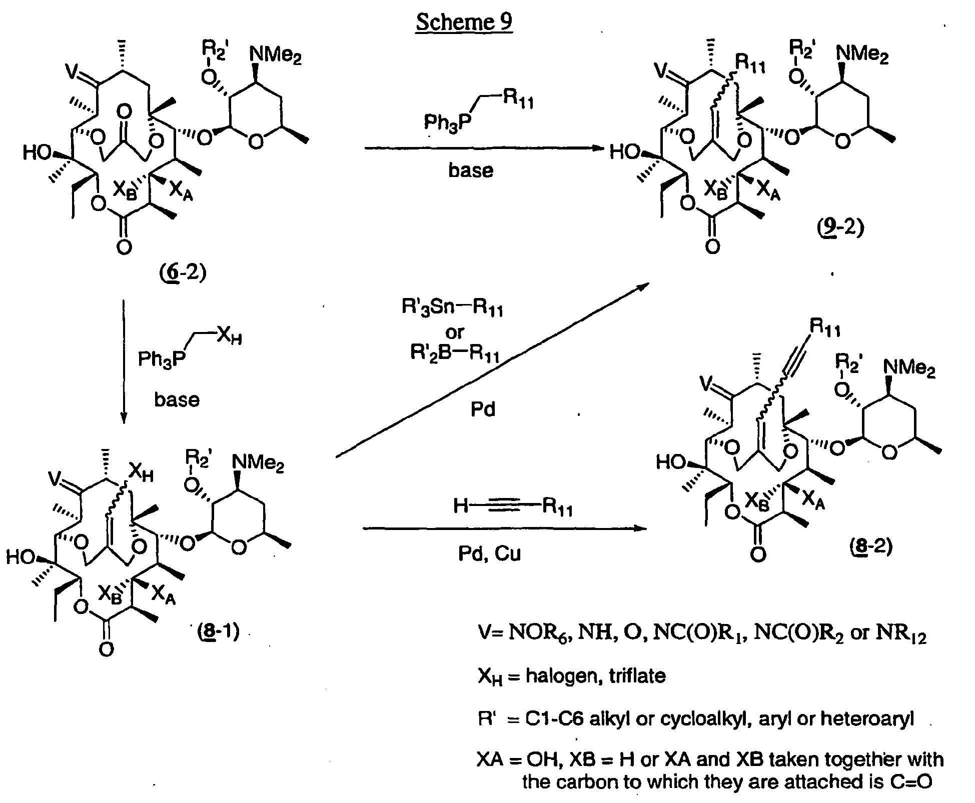

It will be appreciated that ketones of formula (**6**-2) can be transformed into alkenes of formula (**9**-2) and (**8**-1) via Wittig reaction with the appropriate phosphonium salt in the presence of a base. (see (a) Burke, Tetrahedron Lett., 1987, 4143-4146, (b) Rathke and Nowak, J. Org. Chem., 1985, 2624-2626, (c) Maryanoff and Reitz, Chem. Rev., 1989, 863-927. Furthermore, vinyl halides of formula (**8**-1) can be functionalized by Sonogashira coupling with alkynes in the presence of a palladium catalyst, a copper halide and an amine base to give compounds of formula (**8**-2) (see (a) Sonogashira, Comprehensive Organic Synthesis, Volume 3, Chapters 2,4; (b) Sonogashira, Synthesis 1977, 777.). In a similar manner, alkenes of formula (**9**-2) can be obtained from vinyl halides (**8**-1) via Suzuki cross coupling with organoboron reagents in the presence of a palladium catalyst and a base, or via Stille cross coupling with organostananes in the presence of a palladium catalyst. ( see (a) Suzuki, J. Organomet. Chem. 1999, 576,147-168, (b) Stille, Angew. Chem. Int. Ed. Engl., 1986, 508-524 (c) Farina, J. Am. Chem. Soc., 1991, 9585-9595).

It will be appreciated by one skilled in the art, that the unsaturated compounds represented by compounds (**9**-2) and (**8**-2) can be reduced to form the corresponding saturated compound (see Hudlicky, M. Reductions in Organic Chemistry, Ellis Horwood Limited: Chichester, 1984).

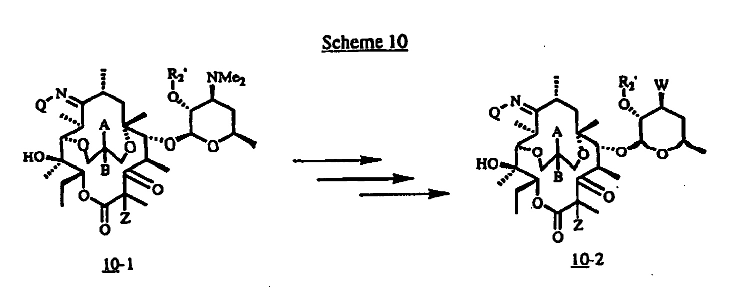

It will be appreciated that compounds of the present invention include modification of the 3' N of compounds of the formula (**10**-1). Compounds of formula (**10**-2) can be made via the methods delineated in U. S. Patents 6,034, 069 and 6,387, 885.

### Examples

The compounds and processes of the present invention will be better understood in connection with the following examples, which are intended as an illustration only and not limiting of the scope of the invention.

### Example 1

### Compound of formula I: A and B taken together with the carbon atom to which they are attached are C=CH₂, X and Y taken together with the carbon atom to which they are attached are C=N-Ac, L = CH₂CH₃, Z = H, and R₂' = Ac

### Step 1a: Compound of formula 1-2: R₆ = Ac, R₂' = Ac and R₄" = Ac

To a solution of erythromycin A oxime (74.9 g, 0.1 mol) in 400 ml THF was added acetic anhydride (35.9 ml, 0.38 mol), triethylamine (55.7 ml, 0.4 mol) and DMAP (3.7 g, 0.03 mol) at room temperature. The mixture was stirred at room temperature for 16 hours and was condensed to ∼200 ml, diluted with 300 ml of ethyl acetate and the resulting mixture washed with NaHCO₃ (Sat.) (500 ml X 4) and brine (500 ml) and dried on anhydrous sodium sulfate. The solvent was evaporated and the residue was recrystallized from ethyl acetate to give title compound (78g).
MS (ESI) m/z 875 (M+H)⁺.
¹³C NMR(CDCl₃): δ 178.5, 175.4, 170.6, 170.2, 168.2, 100.2, 96.1, 83.3, 79.3, 78.7, 75.2, 74.5, 72.9, 70.0, 67.6, 63.4, 63.2, 60.6, 49.5, 44.7, 40.9, 35.4, 31.8, 28.5, 22.8, 21.7,21.6, 21.5, 21.3, 21.2, 21.1, 19.9, 18.6, 18.4, 16.7, 14.9, 14.4, 14.3, 10.8, 9.2

### Step 1b: Compound of formula 1-3: R₁₁ = H and R₁₃ = t-Bu:

To a solution of 2-methylene-1,3-propane diol (5.28g, 0.06 mmol) and di-*tert*-butyl dicarbonate (35 g, 0.16 mol) in 150 ml of dichloromethane was added 6N NaOH (70 ml) and tetrabutylammoniahydrogensulfate ( 3.4 g, 10 mmol). The mixture was stirred at room temperature overnight. The organic layer was separated, washed with NaHCO₃ (200 ml x 3) and brine(200 ml), dried over anhydrous MgSO₄, concentrated and dried over vacuum to give the title compound.
¹H NMR (CDCl₃): δ 5.20(s, 2H); 4.44(s, 4H); 1.18(s, 18H).
¹³C NMR(CDCl₃): δ 153.3, 138.5, 117.3, 82.3, 66.9, 27.8.

### Step 1c: Compound of formula 1-4: R₆ = Ac, R₁₁ = H, R₂' = Ac and R₄" = Ac

To a solution of erythromycin oxime 2', 4", 9-triacetate from Step 1a (112g, 128 mmol), the compound from step 1b (44.3 g, 154 mmol) and dppb (1.71 g, 4 mmol) in THF (500 ml), was added Pd₂(dba)₃ (1.83g, 2 mmol) under nitrogen. The mixture was refluxed for 5 hours and concentrated. The residue was purified by flash chromatography (SiO₂ hexane:acetone/2: 1) to give the title compound.(110g).
MS (ESI) m/z 927.64 (M+H)⁺
¹³C NMR(CDCl₃): δ 176.5, 175.9, 170.7, 170.1, 169.9, 141.6, 124.7, 100.4, 96.0, 79,1, 78.7, 78.2, 78.0, 77.4, 76.5, 73.5, 73.0, 72.4, 72.1, 67.8, 66.1, 63.4, 63.3, 49.6, 44.1, 41.2, 40.9, 37.3, 35.4, 35.1, 31.3, 29.5, 28.5, 27.1, 23.4, 21.7, 21.3, 21.1, 20.9, 20.3, 18.8, 18.3, 17.4, 15.7, 13.4, 12.7, 8.6.

### Step 1d: Compound of formula 2-1: R₁₁ = H, R₂' = Hand R₄" = Ac

A solution of the compound of Step 1c (32g) in 400 ml methanol was refluxed for 48 hours and then concentrated. The residue was purified by flash chromatography (SiO₂, CH₂Cl₂:2M ammonia in methanol = 95:5) to give the title compound (28.5 g).
MS (ESI) m/z 843 (M+H)⁺
¹³C NMR(CDCl₃): δ 176.2, 170.8, 168.8, 142.0, 124.2, 102.5, 95.9, 79.4, 78.7, 78.1, 78.0, 76.6, 73.0, 71.8, 71.1, 68.2, 65.6, 63.2, 49.7, 44.2, 41.7, 40.5, 37.7, 35.0, 34.4, 29.3, 25.8, 23.5, 21.9, 21.3, 21.1, 19.0, 18.1, 17.5, 15.3, 13.2, 12.7, 8.7.

### Step 1e: Compound of formula 2-2: R₁₁ = H and R₂' = H

Titanium trichloride (40 ml, 20% in 3% hydrochloric acid) was added dropwise over 10 minutes to a stirred solution of the compound from Step 1d (9.5 g, 11.3 mmol) and ammonium acetate (17.4 g, 226 mmol) in 120 ml of methanol at 0°C. The reaction mixture was allowed to warm up to room temperature and stirred over night. The pH of the reaction mixture was adjusted to pH=10 by slow addition of 3N aqueous sodium hydroxide. The aqueous solution was extracted with ethyl acetate (200 ml) and the organic phase was washed once with saturated sodium bicarbonate (200ml), dried over sodium sulfate and solvent was removed *in vacuo.* The residue was purified by flash chromatography (Si02, CH₂Cl₂: 2M ammonia in methanol/95:5) to give the title compound (3.0 g).
MS (ESI) m/z: 627 (M+H).
¹³C-NMR(100 MHz, CDCl₃): δ 188.5, 176.0, 143.9, 118.9, 106.9, 90.8, 79.8, 79.6, 79.2, 77.4, 75.9, 75.3, 70.8, 70.4, 65.8, 65.3, 44.6, 42.1, 40.4, 38.6, 36.4, 35.3, 28.2, 22.9, 21.5, 20.0, 19.7, 16.8, 15.1, 14.9, 11.5, 8.3.

### Step 1f: Compound of formula 1-5: V = N-O-Ac, R₁₁ = H and R₂' = Ac

To a solution of the compound from Step 1e (3 g, 4.8 mmol) in 40 ml of dichloromethane was added acetic anhydride (1.36 ml, 14.4 mmol) and triethyl amine(2.8 ml, 20 mmol). The mixture was stirred at room temperature for 4 hours. The solvent was removed under vacuum and the residue was purified by flash chromatography (Si02, hexane:acetone/1:1) to give the title compound (2.9 g).
MS (ESI) m/z 711.50 (M+H)⁺
¹³C NMR(CDCl₃): δ 184.7, 176.9, 174.9, 170.1, 141.9, 122.2, 99.4, 81.2, 79.0, 77.8, 77.7, 76.1, 73.5, 71.7, 68.8, 65.7, 63.2, 43.7, 40.8, 39.9, 38.2, 36.2, 35.6, 31.0, 25.5, 23.2, 21.6, 21.2, 19.9, 19.5, 17.1, 15.8, 14.7, 11.8, 7.9.

### Step 1g: Compound of formula I: A and B taken together with the carbon atom to which they are attached are C=CH₂, X and Y taken together with the carbon atom to which they are attached are C=N-Ac, L = CH₂CH₃, Z = H and R₂' = Ac

To a solution of the compound from Step If (2.9 g, 4.08 mmol) in 40 ml , dichloromethane was added Dess-Martin reagent (1.9 g, 4.5 mmol) at room temperature. The mixture was stirred at room temperature for 2 hours. The reaction was quenched with sodium bicarbonate (50 ml) and Na₂S₂O₃ (2g). The organic phase was separated and washed with brine (50 ml). The solvent was removed under vacuum and the residue was purified on chromatography (hexane:acetone/1:1) to give the title compound (2.0 g).
MS (ESI) m/z 709.28 (M+H)⁺
¹³C NMR(CDCl₃): 205.8, 184.5, 177.4, 170.0, 168.0, 141.2, 124.3, 100.5, 79.2, 78.1, 77.5, 76.2, 74.5, 73.4, 72.1, 71.4, 69.0, 65.7, 63.1, 50.5, 45.5, 40.3, 38.5, 30.7, 25.3, 23.4, 21.5, 21.1, 20.0, 19.4, 17.4, 15.4, 13.8, 13.3, 12.5.

### Example 2

### Compound of formula I: A and B taken together with the carbon atom to which they are attached are C=CH₂, X and Y taken together with the carbon atom to which they are attached are C=N-Ac, L = CH₂CH₃, Z = H and R₂' = H

A solution of the compound of Example 1 (2.0 g, 2.82 mmol) in 40 ml methanol was refluxed for 5 hours. The solvent was evaporated to give the crude title compound.
MS (ESI) m/z 667.40 (M+H)⁺
¹³C NMR(CDCl₃): 205.9, 184.5, 177.5, 168.1, 141.3, 124.4, 103.1, 79.1, 78.2, 77.5, 76.2, 75.6, 73.4, 72.1, 70.4, 69.6, 66.0, 65.7, 50.5, 46.2, 40.4, 38.7, 28.5, 25.4, 23.4, 21.4, 20.0, 19.6, 17.5, 15.4, 13.9, 13.5, 12.6.

### Example 3

### Compound of formula I: A and B taken together with the carbon atom to which they are attached are C=O, X and Y taken together with the carbon atom to which they attached are C=N-Ac, L = CH₂CH₃, Z = H and R₂' =H

A solution of the crude compound from Example 2 in methanol (10 ml) and dichloromethane (30 ml) was cooled to -78°C and ozone was bubbled through the reaction until the solution became light blue. Then nitrogen was bubbled through the reaction mixture to remove excess ozone and triphenyl phosphine (5.64 mmol) was added. The solution was allowed to warm to room temperature over 1 hour. The solvent was evaporated and the residue was dissolved in 40 ml of THF. Triphenyl phosphine (5.64 mmol) was added and the mixture was refluxed overnight. The solvent was removed under vacuum and the residue was purified by flash chromatography (SiO₂, CH₂Cl₂:2M ammonia in methanol = 95 : 5) to give the title compound (1.5g).
MS (ESI) m/z 669.38 (M+H)⁺
¹³C NMR(CDCl₃): δ 205.6, 205.1, 184.4, 175.8, 169.7, 102.5, 80.2, 79.0, 78.8, 77.5, 76.1, 75.8, 75.5, 70.4, 69.7, 68.6, 66.0, 50.9, 45.9, 40.4, 39.7, 38.8, 36.6, 28.4, 25.5, 23.1, 21.4, 19.9, 19.7, 17.2, 15.4, 14.2, 13.1, 11.6.

### Example 4

### Compound of formula I: A and B taken together with the carbon atom to which they are attached are C=N-O-CH₂-Ph, X and Y taken together with the carbon atom to which they are attached are C=N-Ac, L = CH₂CH₃, Z = H and R₂' = H

A solution of the crude compound from Example 3 ( 34 mg, 0.05 mmol), benzyl hydroxylamine (16 mg, 0.1 mmol) and pyridine (0.2 mmol) in 4 ml ethanol was stirred at room temperature for 1 hour. The reaction mixture was concentrated and purified by flash chromatography (SiO₂, CH₂Cl₂:2M ammonia in methanol = 95 : 5) to give the title compound (35 mg, 3:1 cis and trans mixture).
MS (ESI) m/z 774.48 (M+H)⁺.

### Example 5

### Compound of formula I: A and B taken together with the carbon atom to which they are attached are C=N-O-CH₂-(3-pyridyl), X and Y taken together with the carbon atom to which they are attached are C=N-Ac, L = CH₂CH₃, Z = H and R₂' = H

### Step 5a: N-phthaloyl-O-pyridin-3-ylmethyl-hydroxylamine

To a solution ofN-hydroxyphthalimide (653 mg, 4 mmol) and sodium carbonate (848 mg, 8 mmol) in DMF-CH₃CN-H₂O(5 ml/l ml/5 ml) was added 3-(bromomethyl)pyridine hydrobromide (1.01 g, 4 mmol), portion by portion, at room temperature. The mixture was stirred at room temperature for 2 hours, diluted with ethyl acetate (30 ml), washed with 5% of Trisamine and dried over Na₂SO₄. The solvent was removed under vacuum and the residue was purified by silica gel chromatography (hexane:ethyl acetate/3:2) to give the title compound (0.8g).

### Step 5b: Compound of formula I: A and B taken together with the carbon atom to which they are attached are C=N-O-CH₂-(3-pyridyl), X and Y taken together with the carbon atom to which they are attached are C=N-Ac, L = CH₂CH₃, Z = Hand R₂' = H

To a solution of N-phthaloyl-O-pyridin-3-ylmethyl-hydroxylamine (81.3 mg, 0.32 mmol) in 5 ml of ethanol was added hydrazine hydrate (12 mg, 0.24 mmol) at room temperature. The mixture was stirred at 40°C for 2 hours and then cooled to room temperature. Acetic acid (7 µl, 0.12 mmol) was added followed by the compound of Example 3 (27 mg, 0.04 mmol). The mixture was stirred at 60° C for 12 hours. The solvent was removed under vacuum and the residue was purified by flash chromatography (SiO₂, CH₂Cl₂: 2M ammonia in CH₃OH = 95:5) to give the title compound (24 mg).
MS (ESI) m/z 774.48 (M+H)⁺.

### Example 6

### Compound of formula I: A and B taken together with the carbon atom to which they are attached are C=N-O-CH₂-(2-pyridyl), X and Y taken together with the carbon atom to which they are attached are C=N-Ac, L = CH₂CH₃, Z = H and R₂' = H

Using essentially the same procedure as for the preparation of the compound of Example 5, 81.3 mg (0.32 mmol) of N-phthaloyl-O-pyridin-2-ylmethyl-hydroxylamine was reacted with 12 mg (0.24 mmol) of hydrazine hydrate. Then 7µl of glacial acetic acid was added followed by 27 mg (0.04 mmol) of the compound of Example 3. After isolation, 23 mg of the desired product were obtained.
MS: (ESI) m/z 774.48 (M+H)⁺.

### Example 7

### Compound of Formula I: A and B taken together with the carbon atom to which they are attached are C=N-O-CH₂-(3-quinolyl), X and Y taken together with the carbon atom to which they are attached are C=N-Ac, L = CH₂CH₃, Z = H and R₂' =.H

Using essentially the same procedure as for the preparation of the compound of Example 5, 81.3 mg (0.32 mmol)of N-phthaloyl-O-quinolin-3-ylmethyl-hydroxylamine was reacted with 12 mg (0.24 mmol) of hydrazine hydrate. Then 7 µl of glacial acetic acid was added followed by 27 mg (0.04 mmol) of the compound of Example 3. After isolation, 24 mg of the desired product were obtained.
MS (ESI) m/z 846.98 (M+H)⁺.

### Example 8

### Compound of formula I: A and B taken together with the carbon atom to which they are attached are C=N-O-CH₂-(2-quinolyl), X and Y taken together with the carbon atom to which they are attached are C=N-Ac, L = CH₂CH₃, Z = H and R₂' = H

Using essentially the same procedure as for the preparation of the compound of Example 5, 81.3 mg (0.32 mmol)of N-phthaloyl-O-quinolin-2-ylmethyl-hydroxylamine was reacted with 12 mg (0.24 mmol) of hydrazine hydrate. Then 7µl of glacial acetic acid was added followed by 27 mg (0.04 mmol) of the compound of Example 3. After isolation, 24 mg of the desired product were obtained.
MS (ESI) m/z 846.96 (M+H)⁺.

### Example 9

### Compound of formula I: A and B taken together with the carbon atom to which they are attached are C=N-O-5-pyridin-2-ylthiophen-2yl-methyl, X and Y taken together with the carbon atom to which they are attached are C=N-Ac, L = CH₂CH₃, Z = H and R₂' = H

Using essentially the same procedure as for the preparation of the compound of Example 4, 27 mg (0.04 mmol) of the compound of Example 3, 17 mg (0.08 mmol) of O-(5-pyridin-2-yl-thiophen-2-ylmethyl)-hydroxyl amine and 2.3 µl (0.04 mmol) of glacial acetic acid were combined in 2 ml of ethanol. After isolation, 22 mg of the desired product were obtained.
MS (ESI) m/z 774.48 (M+H)⁺.

### Example 10

### Compound of formula I: A and B taken together with the carbon atom to which they are attached are C=N-O-3-pyrimidin-2-ylprop-2-ynyl , X and Y taken together with the carbon atom to which they are attached are C=N-Ac, L = CH₂CH₃, Z = H and R₂' = H

Using essentially the same procedure as for the preparation of the compound of Example 4, 30 mg (0.04 mmol) of the compound of Example 3, 20 mg (0.1 mmol) of O-(3-pyrimidin-2-yl-prop-2-ynyl)-hydroxyl amine and 2.3 µl (0.12 mmol) of triethyl amine were combined in 5 ml of ethanol. After isolation, 23 mg of the desired product were obtained.
MS (ESI) m/z 800.44 (M+H)⁺.

### Example 11

### Compound of formula I: A and B taken together with the carbon atom to which they are attached are C=N-O-Ph, X and Y taken together with the carbon atom to which they are attached are C=N-Ac, L = CH₂CH₃, Z = H and R₂' = H

Using essentially the same procedure as for the preparation of the compound of Example 5, 81.3 mg (0.32 mmol)of N-phthaloyl-O-phenyl-hydroxylamine were reacted with 12 mg (0.24 mmol) of hydrazine hydrate. Then 7 µl of glacial acetic acid was added followed by 27 mg (0.04 mmol) of the compound of Example 3. After isolation, 24 mg of the desired product were obtained.
MS (ESI) m/z 760.12 (M+H)⁺.

### Example 12

### Compound of Formula I: A = NHCH₂Ph, B = H, X and Y taken together with the carbon atom to which they are attached are C=N-Ac, L = CH₂CH₃, Z = H and R₂' = H

To a solution of the compound of Example 3 (34 mg, 0.05 mmol), acetic acid (5.7 µl, 0.1 mmol) and benzyl amine (16.4 µl, 0.15 mmol) in 3 ml of methanol was added NaCNBH₄ (6.6 mg, 0.1 mmol) at room temperature. The reaction mixture was stirred at room temperature for 5 hours and quenched with 5% Trisamine, extracted with ethyl acetate (15 ml), washed with brine (15 ml), and dried over Na₂SO₄. The solvent was removed under vacuum and the residue was purified by flash chromatography (SiO₂, CH₂Cl₂:2M ammonia in methanol = 95:5) to give the title compound (25mg)
MS (ESI) m/z 760.26 (M+H)⁺.

### Example 13

### Compound of formula I: A = NHCH₂CH₂Ph, B = H, X and Y taken together with the carbon atom to which they are attached are C=N-Ac, L = CH₂CH₃, Z = Hand R₂' = H

Using essentially the same procedure as for the preparation of the compound of Example 12, 53.5 mg (0.08 mmol) of the compound of Example 3, 30 µl (0.24 mmol) of phenethyl amine, 9.2 µl of glacial acetic acid and 10 mg (0.16 mmol) of sodium cyanoborohydride were combined in 5 ml of methanol. After isolation, 40 mg of the desired product were obtained.
MS (ESI) m/z 774.25 (M+H)⁺.

### Example 14

### Compound of Formula I: A and B taken together with the carbon atom to which they are attached are C=CH₂, X and Y taken together with the carbon atom to which they are attached are C=N-O-CH₂-O-CH₃, L = CH₂CH₃, Z = H, and R₂' = Ac

### Step 14a: Compound of formula 1-5: V = N-O-H. R₁₁ = H and R₂' = Ac

To a solution of the compound from Step 1b (4.2 g, 4.5 mmol) in 50 ml methanol was added 2M HCl (10 ml). The mixture was refluxed for 1.5 hours, condensed to 30 ml, diluted with saturated NaHCO₃(30 ml), extracted with ethyl acetate (50 ml) and dried over Na₂SO₄. The solvent was evaporated and the residue was purified by silia gel chromatography (hexane:acetone/1:1) to give the title compound (2.5g).
MS (ESI) m/z 685 (M+H)⁺.
¹³C-NMR (100 MHz, CDCl₃): δ 175.2, 170.2, 166.3, 143.6, 119.3, 99.6, 82.2, 79.5, 78.1, 77.5, 76.0, 73.7, 71.7, 68.9, 65.5, 63.3, 43.8, 40.8, 37.4, 35.9, 34.3, 31.1, 25.6, 23.3, 21.7, 21.3, 19.9, 19.6, 17.1, 15.7, 14.7, 11.9, 7.9.

### Step 14b: Compound of formula 1-5: V = N-O-CH₂OCH₃, R₁₁ = H and R₂' = Ac

To a solution of the compound from Step 14a (6.85g, 10 mmol) in 40 ml DMF was added NaH (303 mg, 1.3 mmol) at 0°C, portion by portion. After 10 minutes, MOM-Cl (900 ul, 1.15 mmol) was added at 0°C during 15 minutes. The mixture was stirred at room temperature for 16 hours and quenched with saturated NaHCO₃ (60 ml), extracted with ethyl acetate (60 ml) and dried over Na₂SO₄. The solvent was evaporated and the residue was purified by silica gel chromatography (hexane: acetone/1:1) to give the title compound (4.5 g) MS (ESI) m/z 729 (M+H)⁺.

### Step 14c. Compound of formula I: A and B taken with together with the carbon atom to which they are attached are C=CH₂, X and Y taken together with the carbon atom to which they are attached are C=N-O-CH₂-O-CH₃, L = CH₂CH₃, Z = H, and R₂' = Ac

To a solution of the compound from Step14b (4.4 g, 6 mmol) in 50 ml dichloromethane was added a solution of Dess-Martin reagent (3.05 g, 7.2 mmol) in 20 ml of dichloromethane. The mixture was stirred at room temperature for 2 hours. The reaction was quenched by addition of saturated NaHCO₃(50 ml) and Na₂S₂O₃ (10.4g, 42 mmol). The organic layer was separated and dried over Na₂SO₄. The solvent was removed and the residue was purified by flash chromatography (SiO₂, hexane:acetone/1:1) to give the title compound (3.0 g)
MS (ESI) m/z 727.32 (M+H)⁺
¹³C NMR(CDCl₃): δ 205.8, 169.8, 168.3, 168.1, 142.0, 123.5, 100.9, 98.4, 79.1, 78.5, 76.0, 73.4, 71.7, 69.1, 65.5, 63.5, 56.5, 50.8, 46.5, 40.7, 37.8, 34.4, 30.8, 26.9, 23.4, 21.5, 21.2, 20.1, 19.3, 17.4, 15.0, 14.0, 13.9, 12.5.

### Example 15

### Compound of formula I: A and B taken together with the carbon atom to which they are attached are C=CH₂, X and Y taken together with the carbon atom to which they are, attached are C=N-O-CH₂-O-CH₃, L = CH₂CH₃, Z = H and R₂' = H

A solution of the compound of Example 14 (440 mg, 0.6 mmol) in 5 ml methanol was refluxed for 4 hours and concentrated to give the desired compound without purification.
MS (ESI) m/z 685.18 (M+H)⁺

### Example 16

### Compound of formula I: A and B taken together with the carbon atom to which they are attached are C=O, X and Y taken together with the carbon atom to which they are attached are C=N-O-CH₂-O-CH₃, L = CH₂CH₃, Z = H and R₂' = H

A solution of the compound of Example 15 (420 mg, 0.6 mmol) in 7 ml methanol and 20 ml CH₂Cl₂ was purged with O₃ at -78°C until the solution became light blue. Nitrogen was bubbled through the solution to remove excess O₃ and then PPh₃ (2 eq) was added. The mixture was warmed to room temperature and stirred at room temperature for 2 hours, concentrated and the residue dissolved in 10 ml THF and another 2 eq. of PPh₃ was added. The resulting mixture was refluxed overnight and concentrated. The residue was purified by flash chromatography (SiO₂, CH₂Cl₂: 2M ammonia in CH₃OH = 95: 5) to give the title compound (280 mg).
MS (ESI) m/z 687.25 (M+H)⁺
¹³C NMR(CDCl₃): δ 205.8, 205.8, 170.2, 166.7, 102.4, 98.8, 80.6, 80.5, 78.8, 76.7, 76.0, 75.6, 70.5, 69.7, 69.5, 66.1, 60.6, 57.1, 50.8, 45.8, 40.5, 38.2, 34.3, 28.6, 26.9, 23.1, 21.5, 21.3, 19.9, 19.4, 17.0, 15.4, 14.5, 14.4, 13.0, **11.7.**

### Example 17

### Compound of formula I: A and B taken together with the carbon atom to which they are attached are C=NOCH₂Ph, X and Y taken together with the carbon atom to which they are attached are C=N-O-CH₂-O-CH₃, L = CH₂CH₃, Z = H and R₂' = H

Using essentially the same procedure as for the preparation of the compound of Example 4, 87 mg (0.13 mmol) of the compound of Example 16, 41.5 mg (0.26 mmol) of O-benzyl hydroxylamine and 21 µl (0.26 mmol) of pyridine were combined in 10 ml of ethanol. After isolation, 75 mg of the desired product was obtained.
ESMS: 774.35 (M+H)⁺

### Example 18

### Compound of formula I: A and B taken together with the carbon atom to which they are attached are C=O, X and Y taken together with the carbon atom to which they are attached are C=O, L = CH₂CH₃, Z = H, and R₂' = Ac

### Step 18a. Compound of formula 3-1: R₁₁ = H, R₁₃ = H and Rₚ = tert-butyl dimethyl silyl

To a suspension of NaH (1.26g, 50 mmol) in 40 ml of THF was added a solution of 2-methylene-1,3-propane diol (4.4g, 50 mmol) in 30 ml of THF. The mixture was stirred at room temperature for 45 minutes and then a solution of *tert*-butyl dimethylsilyl chloride (7.54g, 50 mmol) in 30 ml of THF added. The mixture was stirred at room temperature for 1 hour and then quenched with saturated NaHCO₃ (200 ml), extracted with diethylether (150 ml x 2) and the combined organic layers were dried over MgSO₄. The solvent was removed and the resulting oil was purified by flash chromatography (SiO₂, hexane:ethyl acetate/10:1) to give the title compound (8.4g).

### Step 18b: Compound of formula 3-1: R₁₁ = H, R₁₃ = t-butoxycarbonyl and Rₚ = tert-butyl dimethyl silyl

To a solution of the compound from Step 18a (8.1g, 40 mmol) in 100 ml of methylenechloride was added di-tertbutyl dicarbonate (13.1g, 60 mmol), tetrabutylammoniahydrogensulfate (1.2g, 3.5 mmol) and 30 ml of 6N NaOH. The mixture was stirred at room temperature for 16 hours, diluted with 100 ml of methylene chloride and washed with saturated NaHCO₃ (200 ml x 3). The organic layer was dried over Na₂SO₄ and the solvent was removed under vacuum. The residue was purified by flash chromatography (SiO₂, hexane:ethylacetate/96:4) to give the title compound (6.8g).

### Step 18c. Compound of formula 3-2: Rₚ = tert-butyl dimethyl silyl, R₆ = Ac, R₁₁ = H, R₂' = Ac and R₄" = Ac

To a solution of erythromycin A oxime 9,2',4"-triacetate from Step 1a (22 g, 25 mmol), the compound from Step 18b (9.1 g, , 30 mmol) and dppb (853 mg, 1 mmol) in 250 ml THF was added Pd₂(dba)₃ (916 mg, 1 mmol). The mixture was refluxed overnight, concentrated and purified by flash chromatography( SiO₂, acetone:hexane/1:3) to give the title compound (25 g).
MS (ESI) m/z 1059.65 (M+H)⁺
¹³C NMR(CDCl₃): δ 181.2, 179.3, 175.9, 175.5, 173.5, 148.5, 116.5, 104.8, 102.0, 85.2, 84.3, 83.9, 83.6, 82.8, 82.2,79.7, 78.1, 77.6, 75.6, 72.4, 70.4, 69.0, 68.6, 54.6, 49.9, 46.2, 43.2, 40.8, 36.5, 33.6, 31.4, 27.1, 27.0, 26.6, 26.3, 25.2, 25.1, 24.0, 23.7, 22.0, 20.4, 16.0, 15.2, 0.5, 0.0.

### Step 18d: Compound of formula (3-2) Rp = tert-butyl dimethyl silyl, R₆ = H, R₁₁ = H, R₂' = H, R₄" = Ac

A solution of the compound of Step 18c (3.18g, 3 mmol) in 80 ml methanol was refluxed for 8 hours. The solution was concentrated and purified by flash chromatography (SiO₂, 2M ammonia in methanol:dichloromethane/3:97) to give the title compound (2.6 g, 89%).
MS (ESI) m/z 975.47 (M+H)⁺
¹³C NMR(CDCl₃): δ 179.5, 178.9, 175.7, 150.6, 121.5, 106.8, 101.4, 85.3, 83.9, 83.7, 82.4, 82.0, 79.3, 77.8, 76.7, 76.5, 72.7, 70.4, 70.1, 69.3, 68.3, 54.6, 49.8, 45.5, 43.0, 42.9, 40.6, 38.1, 34.0, 31.1, 30.5, 27.1, 26.3, 26.1, 26.0, 24.3, 23.7, 23.5, 21.5, 19.9, 15.7, 14.8, 0.5, 0.0.

### Step 18e. Compound of formula 3-4: Rₚ = H, R₁₁ = H, R₂' = H and R₄'' = Ac

To an emulsion of the compound of Step 18d (2.44g, 2.5 mmol) in 25 ml of isopropanol and 30 ml of water was added formic acid (380 µl, 10 mmol) and Na₂S₂O₄ (1.39, 8 mmol). The reaction mixture was heated to 90°C and stirred for 8 hours. The solution was diluted with ethyl acetate(60 ml) and washed with saturated NaHCO₃ (60 ml x 3). The organic layer was separated and dried over anhydrous Na₂SO₄(∼5g), concentrated and purified by flash chromatography (SiO₂, 2M ammonia in methanol:dichloromethane/3:97) to give the title compound (1.7g).
MS (ESI) m/z 846.54 (M+H)⁺.
¹³C NMR(CDCl₃): δ 221.3, 175.3, 170.6, 147.0, 114.1, 101.8, 96.6, 79.9, 79.2, 78.8, 78.7, 77.4, 75.0, 72.8, 71.4, 68.8, 67.8, 65.4, 65.3, 63.7, 63.4, 60.6, 49.6, 45.5, 44.8, 40.4, 38.2, 38.0, 35.6, 22.0, 21.2, 21.1, 19.6, 18.6, 16.5, 14.4, 12.2, 10.6, 9.8.

### Step 18f. Compound of formula 3-4: Rₚ = H, R₁₁ = H, R₂' = Ac and R₄" = Ac

Acetic anhydride (94ul, 1 mmol) was added to a solution of the compound of Step 18e (338.4mg, 0.4 mmol) in dichloromethane (5 ml). The mixture was stirred at room temperature for 16 hours. Solvent was removed under vacuum and the product was purified by flash chromatography (SiO₂, acetone:hexane/4:6 vl) to give the title compound (330 mg).
MS (ESI) m/z 888.58 (M+H)⁺
¹³C NMR(CDCl₃): δ 221.3, 175.1, 70.6, 170.3, 146.8, 14.2, 99.6, 96.5, 79.9, 79.1, 78.5, 78.4, 77.1, 74.9, 72.8, 72.1, 68.9, 67.1, 65.1, 63.7, 63.5, 63.1, 49.3, 45.5, 44.8, 40.6, 38.0, 37.7, 37.6, 35.5, 29.4, 21.8, 21.3, 21.1, 21.0, 19.4, 18.6, 16.6, 12.2, 10.6, 9.6.

### Step 18g. Compound of formula 3-4: Rₚ = tert-butoxycarbonyl, R₁₁ = H, R₂' = Ac and R₄'' = Ac

Di-tert-butyl-dicarbonate (69 µl, 0.3 mmol) was added to a solution of the compound of Step 18f (178 mg, 0.2 mmol) and triethylamine (56 µl, 0.4 mmol) in dichloromethane (8 ml) at room temperature. After 10 minutes, DMAP (12.2 mg, 0.1 mmol) was added. The resulting solution was stirred at room temperature for 2 hours. Solvent was removed under vacuum and the product was purified by flash chromatography (SiO₂, acetone:hexane/1:3 vl) to give the title compound (180 mg).
MS (ESI) m/z 988.41 (M+H)⁺
¹³C NMR(CDCl₃): δ 219.6, 174.6, 170.6, 170.3, 153.8, 141.3, 116.8, 99.6, 96.5, 82.0, 80.2, 79.4, 78.7, 78.6, 76.8, 74.9, 72.9, 72.4, 69.1, 67.9, 67.2, 64.8, 63.6, 63.4, 49.4, 45.2, 44.8, 41.0, 37.9, 37.7, 37.6, 35.6, 31.8, 31.3, 31.2, 28.2, 28.1, 22.9, 21.8, 21.5, 21.4, 21.1, 19.4, 18.7,16.7,16.6,14.4,12.5,10.7,9.7

### Step 18h. Compound of formula 3-6: R₁₁ = H, R₂' = Ac and R₄'' = Ac

1,4-Bis(diphenylphosphino)butane (8.5 mg, 0.02 mmol) and Pd₂(dba)3 (9.2 mg, 0.01 mmol) were added to a solution of the compound of Step 18g (98.8 mg, 0.1 mmol) in 2 ml anhydrous THF at room temperature. The resulting mixture was refluxed for 30 minutes. Solvent was removed under vacuum to give the title compound (85 mg).
MS (ESI) m/z 870.49 (M+H)⁺

### Step 18i. Compound of formula 1-5: V = O, R₁₁ = H and R₂' = Ac

To a solution of the compound of Step 18h (700 mg, 0.8 mmol) in 10 ml ethanol was added 25 ml of 1M HCl. The mixture was refluxed for 2 hours and then cooled to room temperature. The pH of the mixture was adjusted to 10 by addition of 2M NaOH and then extracted with ethyl acetate. The extract was dried over Na₂SO₄, concentrated and the residue purified by flash chromatography (SiO₂, hexane:acetone/1:1) to give the title compound (480 mg).
MS (ESI) m/z 670.23 (M+H)⁺
¹³C NMR(CDCl₃): 8 216.3, 175.0, 170.1, 141.8, 122.1, 99.4, 81.1, 79.0, 77.7, 77.5, 76.2, 75.6, 72.1, 71.7, 68.8, 65.6, 63.2, 60.5, 46.5, 43.7, 40.8, 39.1, 38.6, 35.9, 31.1, 23.0, 21.6, 21.3, 21.2, 19.8, 18.5, 17.3, 14.8, 14.3, 13.0, 11.7, 7.9.

### Step 181. Compound of formula I: A and B taken together with the carbon atom to which they are attached are C=O, X and Y taken together with the carbon atom to which they are attached are C=O, L = CH₂CH₃, Z = Hand R₂' = Ac

To a solution of the compound of Step 18i (480 mg, 0.7 mmol) in 10 ml of dichloromethane was added Dess-Martin reagent (385 mg, 0.9 mmol) at room temperature. The reaction mixture was stirred at room temperature for 2 hours and quenched with saturated NaHCO₃ (15 ml) and sodium thiosulfate (0.4g). The organic phase was separated, washed with brine (15 ml), dried over Na₂SO₄ and concentrated. The residue was purified by flash chromatography (SiO₂, hexanes:acetone/2:1) to give the title compound (400 mg).
MS (ESI) m/z 668.02 (M+H)⁺
¹³C NMR(CDCl₃): δ 218.2, 205.8, 170.0, 168.1, 140.9, 125.1, 101.0, 78.9, 78.3, 76.5, 75.0, 72.7, 70.4, 69.3, 65.7, 63.6, 50.8, 46.6, 46.3, 40.9, 39.3, 39.1, 30.8, 23.4, 21.6, 21.3, 19.9, 18.5, 17.8, 14.2, 14.0, 12.5, 12.4.

### Example 19

### Compound of formula I: A and B taken together with the carbon atom to which they are attached are C=CH₂, X and Y taken together with the carbon atom to which they are attached are C=O, L = CH₂CH₃, Z = H, and R₂' = H

A solution of the compound of Example 18 (300mg, 0.45 mmol) in 10 ml of methanol was refluxed for 8 hours. Solvent was removed under vacuum and the residue was purified by silica gel chromatography (CH₂Cl₂:2M ammonia in methanol/97:3 vl) to give the title compound (270 mg).
MS (ESI) m/z 626.10 (M+H)⁺.
¹³C NMR(CDCl₃): δ 218.4, 205.9, 168.1, 141.0, 125.2, 103.5, 78.7, 78.3, 76.6, 76.1, 72.6, 70.6, 70.3, 69.8, 66.1, 65.7, 50.9, 47.3, 46.4, 40.5, 39.4, 39.3, 28.5, 23.4, 21.5, 20.0, 18.5, 17.9, 14.6, 14.1, 12.5, 12.4.

### Example 20

### Compound of formula I: A and B taken together with the carbon atom to which they are attached are C=O, X and Y taken together with the carbon atom to which they are attached are C=O, L = CH₂CH₃, Z = H, and R₂' = H

A solution of the compound of Example 19 (94mg, 0.15 mmol) in 2 ml of methanol and 4 ml of CH₂Cl₂ was purged with O₃ at -78°C until the solution became light blue. Nitrogen was bubbled through the solution to remove excess O₃ and then PPh₃ (2 eq) was added. The mixture was warmed to room temperature and stirred at room temperature for 2 hours. The mixture was concentrated and the residue was dissolved in 5 ml of THF and another 2 eq of PPh₃ was added. The resulting mixture was refluxed overnight and concentrated. The residue was purified by flash chromatography (SiO₂, CH₂Cl₂:2M ammonia in methanol/95:5) to give the title compound (80 mg).
MS (ESI) m/z 628.10 (M+H)⁺
¹³C-NMR(CDCl₃): δ 215.2, 205.6, 205.3, 169.9, 102.5, 80.5, 79.4, 78.6, 77.5, 76.3, 76.1, 75.3, 70.5, 69.8, 68.5, 66.1, 51.0, 46.3, 46.2, 40.5, 39.8, 39.0, 28.5, 22.9, 21.5, 19.8, 18.3, 17.3, 14.4, 13.6, 12.4, 11.6

### Example 21

### Compound of formula I: A and B taken together with the carbon atom to which they are attached are C=N-O-CH₂Ph, X and Y taken together with the carbon atom to which they are attached are C=O, L = CH₂CH₃, Z = H, and R₂' = H

Using essentially the same procedure as for the preparation of the compound of Example 4, 19 mg (0.03 mmol) of the compound of Example 20, 10 mg (0.06 mmol) of O-benzyl hydroxyl amine and 5 µl (6 mmol) of pyridine were combined in 5 ml of ethanol. After isolation, 20 mg of the desired product was obtained.
MS (ESI) m/z 733.24 (M+H)⁺

### Example 22

### Compound of formula I: A and B taken together with the carbon atom to which they are attached are C=CH₂, X and Y taken together with the carbon atom to which they are attached are C=NH, L = CH₂CH₃, Z = H, and R₂' = H

Potassium carbonate (50 mg) was added to a solution of the compound of Example 2 in methanol (6ml). The mixture was stirred at room temperature for 3 days. The solvent was removed *in vacuo* and the residue was purified by flash chromatography (SiO₂, CH₂Cl₂:2M ammonia in methanol/95:5) to give the title compound (70mg).
MS (ESI) m/z: 625.36(M+H)⁺

### Example 23

### Compound of formula I: A and B taken together with the carbon atom to which they are attached are C=CH-O-CH₂-p-NO₂Ph, X and Y taken together with the carbon atom to which they are attached are C=N-Ac, L = CH₂CH₃, Z = H, and R₂' = H

A solution of the compound of Example 3 (53.5 mg, 0.08 mmol), benzyl hydroxylamine HCl salt (33 mg, 0.16 mmol) and pyridine (0.16 mmol) in 4 ml ethanol was stirred at room temperature for 1 hour. The solvent was removed *in vacuo* and the residue was purified by flash chromatography (SiO₂, CH₂Cl₂:2M ammonia in methanol/95:5) to give the title compound (52 mg) as a 3:1 mixture of cis and trans.
MS (ESI) m/z: 819.22(M+H)⁺

### Example 24

### Compound of formula I: A and B taken together with the carbon atom to which they are attached are C=N-O-(CH₂)₂-Ph, X and Y taken together with the carbon atom to which they are attached are C=N-Ac. L = CH₂CH₃, Z = H, and R₂' = H

Using essentially the same procedure as for the preparation of the compound of Example 5, 50 mg (0.074 mmol) of the compound of Example 3, and 100 mg (0.37 mmol) of N-phthaloyl-O-phenethyl-hydroxylamine were reacted to give the title compound.
MS (ESI) m/z: 788(M+H)⁺

### Example 25

### Compound of formula I: A and B taken together with the carbon atom to which they are attached are C=N-O-(CH₂)₃-Ph, X and Y taken together with the carbon atom to which they are attached are C=N-Ac, L = CH₂CH₃, Z = H, and R₂' = H

Using essentially the same procedure as for the preparation of the compound of Example 5, 50 mg (0.074 mmol) of the compound of Example 3, and 105 mg (0.37 mmol) of N-phthaloyl-O-1-(3-phenyl)propyl-hydroxylamine were reacted to give the title compound.
MS (ESI) m/z: 802(M+H)⁺

### Example 26

### Compound of formula I: A and B taken together with the carbon atom to which they are attached are C=N-O-CH₂-CH=CH-Ph, X and Y taken together with the carbon atom to which they are attached are C=N-Ac, L = CH₂CH₃, Z = H, and R₂' = H

Using essentially the same procedure as for the preparation of the compound of Example 5, 50 mg (0.074 mmol) of the compound of Example 3, and 105 mg (0.37 mmol) of N-phthaloyl-O-1-(3-phenyl)prop-2-enyl-hydroxylamine were reacted to give the title compound.
MS (ESI) m/z: 800(M+H)⁺

### Example 27

### Compound of formula I: A is NH-(CH₂)₃-Ph, B is H, X and Y taken together with the carbon atom to which they are attached are C=N-Ac, L = CH₂CH₃, Z = H, and R₂' = H

Using essentially the same procedure as for the preparation of the compound of Example 12, 33.5 mg (0.05 mmol) of the compound of Example 3, and 21.8 µl (0.1 mmol) of 3-phenyl propylamine were reacted to give 18 mg of the title compound.
MS (ESI) m/z: 788(M+H)⁺

### Example 28

### Compound of formula I: A is NH-(CH₂)₄-Ph, B is H, X and Y taken together with the carbon atom to which they are attached are C=N-Ac, L = CH₂CH₃, Z=H, and R₂' = H

Using essentially the same procedure as for the preparation of the compound of Example 12, 24 mg (0.05 mmol) of the compound of Example 3, and 33.5 µL (0.15 mmol) of 4-phenyl butylamine were reacted to give 12 mg of the the title compound.
MS (ESI) m/z: 802(M+H)⁺

### Example 29

### Compound of formula I: A is CH₂-CH=CH₂, B is OH, X and Y taken together with the carbon atom to which they are attached are C=N-Ac, L = CH₂CH₃, Z = H, and R₂' = H

A solution of the compound of Example 3 (0.23 g, 0.34 mmol) in 20 mL anhydrous THF was cooled to -78°C. Allylmagnesium bromide in THF (1.0 M, 1.5 mL, 1.5 mmol) was added via syringe. The reaction mixture was stirred for 1 hour at -78° C and then was quenched with aqueous NaHCO₃. The mixture was warmed to room temperature slowly and then extracted with CH₂Cl₂ (3x30 mL). The combined organic layers were dried over anhydrous Na₂SO₄. The solvent was evaporated and the residue was purified by flash chromatography (SiO₂, CH₂Cl₂ containing 5% 2M ammonia solution in methanol) to provide the title compound (0.21 g, 86 %).
MS (ESI) m/z 711 (M+H)⁺
¹³C-NMR (100 MHz, CDCl₃): δ 205.5, 186.1, 179.9, 169.7, 132.9, 118.3, 102.3, 80.0, 79.4, 76.1, 73.7, 72.7, 70.4, 70.4, 69.7, 66.2, 50.8, 44.9, 41.2, 40.5, 39.8, 39.4, 37.1, 28.6, 25.5, 23.6, 21.5, 20.2, 20.0, 17.3, 15.9, 14.3, 12.6, 12.0.

### Example 30

### Compound of formula I: A = CH₂-Ph, B is OH, X and Y taken together with the carbon atom to which they are attached are C=N-Ac, L = CH₂CH₃, Z = H, and R₂' = H

The compound of Example 3 (70 mg, 0.1 mmol) was treated with benzylmagnesium bromide (0.85 M in THF, 0.6 mL, 0.5 mmol) as described in Example 29. After the same work up, the crude mixture was purified by flash chromatography (SiO₂, CH₂Cl₂ containing 5% 2M ammonia solution in methanol) to provide the title compound (12 mg, 18 %).
MS (ESI) m/z 761 (M+H)⁺
¹³C-NMR (100 MHz, CDCl₃): δ 205.7, 186.3, 179.5, 169.8, 136.3, 131.0, 128.1, 126.5, 101.8, 80.1, 79.3, 76.0, 73.5, 73.1, 70.6, 70.1, 69.2, 66.6, 50.9, 44.8, 42.4, 40.5, 39.8, 39.3, 37.1, 29.9, 25.6, 23.6, 21.3, 20.2, 20.1, 17.3, 15.9, 14.4, 12.7, 12.0.

### Example 31

### Compound of formula I: A = (CH₂)₂-Ph, B = OH, X and Y taken together with the carbon atom to which they are attached are C=N-Ac, L = CH₂CH₃, Z = H, and R₂' = H

The compound of Example 3 (70 mg, 0.1 mmol) was treated with phenethylmagnesium bromide (1.0 M in THF, 0.5 mL, 0.5 mmol) as described in Example 29. After the same work up, the crude mixture was purified by flash chromatography (SiO₂, CH₂Cl₂ containing 5% 2M ammonia solution in methanol) to provide the title compound (12 mg, 16 %).
MS (ESI) m/z 775 (M+H)⁺

### Example 32

### Compound of formula I: A = Ph, B = OH, X and Y taken together with the carbon atom to which they are attached are C=N-Ac, L = CH₂CH₃, Z = H, and R₂' = H

The compound of Example 3 (70 mg, 0.1 mmol) was treated with phenylmagnesium bromide (1.0 M in THF, 0.5 mL, 0.5 mmol) as described in Example 29. After the same work up, the crude mixture was purified by flash chromatography (SiO₂, CH₂Cl₂ containing 5% 2M ammonia solution in methanol) followed by a semi-prep. HPLC to provide the title compound (5 mg).
MS (ESI) m/z 747 (M+H)⁺

### Example 33

### Compound of formula I: A is CH₂-CH=CH-Ph, B is OH, X and Y taken together with the carbon atom to which they are attached are C=N-Ac, L = CH₂CH₃, Z = H, and R₂' = H

The compound from Example 29 (50 mg, 0.07 mmol), iodobenzene (32 mg, 0.15 mmol), Pd(OAc)₂ (2.5 mg), (o-Tolyl)₃P (10 mg) and triethyl amine (0.1 mL, excess) were dissolved in 3 ml CH₃CN and the solution was degassed at -40°C. The reaction mixture was warmed to room temperature under nitrogen, heated at 50°C for 1 hour, and then left at 80°C for 12 hours. The reaction mixture was diluted with ethyl acetate, washed with saturated aqueous NaHCO₃, and dried over anhydrous Na₂SO₄. The solvent was evaporated and the residue was purified by flash chromatography (SiO₂, CH₂Cl₂ containing 5% 2M ammonia solution in methanol) to provide the title compound (40 mg, 76 %).
MS (ESI) m/z 787 (M+H)t
Selected ¹³C-NMR (100 MHz, CDCl₃): δ 205.5, 186.3, 179.7, 169.9, 137.7, 133.2, 128.6, 127.2, 126.5, 124.8, 101.9, 80.0, 79.4, 76.1, 73.9, 73.2, 70.5, 70.1, 69.3, 66.3, 50.9; 46.5, 44.8, 40.7, 40.2, 39.8, 39.4, 37.0, 29.4, 25.6, 23.6, 21.3, 20.2, 20.1, 17.3, 16.0, 14.5, 12.6, 11.9, 8.9.

### Example 34

### Compound of formula I: A is (CH₂)₃-Ph, B is OH, X and Y taken together with the carbon atom to which they are attached are C=N-Ac. L = CH₂CH₃, Z = H, and R₂' = H

The compound from Example 24 (15 mg, 0.02 mmol) was hydrogenated under 1 atm H₂ over Pd-C in ethanol at room temperature for 24 hours. The solvent was evaporated under vacuum. Purification by flash chromatography (SiO₂, CH₂Cl₂ containing 5% 2M ammonia solution in methanol) gave the title compound (13.2 mg, 88 %).
MS (ESI) m/z 789 (M+H)⁺

### Example 35

### Compound of formula I: A and B taken together with the carbon atom to which they are attached are C=CH-CH=CH-Ph, X and Y taken together with the carbon atom to which they are attached are C=N-Ac, L = CH₂CH₃, Z = H, and R₂' = H

### Step 35a: Compound of formula (1-5): V is N-Ac, R₁₁ is CH-CH-Ph, R₂' = Ac

To a solution of the compound of formula (**1**-5), wherein V = N-Ac, R₁₁ = H, and R₂' = Ac (0.5 g, 0.7 mmol) in 8 ml anhydrous DMF, β-bromostyrene (0.15 ml, 1.2 mmol) and K₂CO₃ (200 mg, 1.5 mmol) were added at room temperature. The mixture was degassed briefly and a catalytic amount of dihydrogen dichlorobis(di-tert-butylphosphinito-κP)palladate(II) (POPd from Combiphos catalysts, Inc.) was added. The reaction mixture was heated to 100°C in a sealed tube for 48 hours. Ethyl acetate (50 mL) was added and the solution was washed 3 times with aqueous NaHCO₃. The organic layer was dried over anhydrous Na₂SO₄. The solvent was evaporated under vacuum and the residue was purified by flash chromatography (SiO₂, acetone:hexanes/1:1) to provide the title compound.
MS (ESI) m/z 813 (M+H)⁺ ,

### Step 35b: Compound of formula I: A and B taken together with the carbon atom to which they are attached are C=CH-CH=CH-Ph, X and Y taken together with the carbon atom to which they are attached are C=N-Ac, L = CH₂CH₃, Z = H, and R₂' = Ac

The compound from Step 35a was dissolved in CH₂Cl₂ and Dess-Martin periodinane (1,1,1-triacetoxy-1,1-dihydro-1,2-benziodoxol-3(1H)-one) (0.3 g, 0.7 mmol) was added. The mixture was stirred for 1 hour and then aqueous NaHCO₃ was added. The mixture was extracted 3 times with CH₂Cl₂ and the combined organic layers were dried over anhydrous Na₂SO₄. The solvent was evaporated under vacuum and the residue was purified by flash chromatography (SiO₂, acetone:hexanes/2:3) to provide the title compound (0.24 g, 42%). MS (ESI) m/z 811 (M+H)⁺

### Step 35c: Compound of formula I: A and B taken together with the carbon atom to which they are attached are C=CH-CH=CH-Ph, X and Y taken together with the carbon atom to which they are attached are C=N-Ac, L = CH₂CH₃, Z = H. and R₂' = H

A solution of the compound from Step 35b (0.16g, 0.05 mmol) in 10 mL of methanol was stirred for 48 hours at room temperature. The solvent was evaporated under vacuum. Purification by flash chromatography (SiO₂, CH₂Cl₂ containing 3% 2M ammonia solution in methanol) gave the title compound (0.12 g, 79 %).
MS (ESI) m/z 769 (M+H)⁺
¹³C-NMR (100 MHz, CDCl₃): δ 206,4, 184,7, 177.9, 167.8, 137.6, 136,4, 136,2, 134.2, 128,7, 128.0, 127.0, 124.0, 103.4, 79.8, 76.4, 72.5, 70.5, 69.7, 66.8, 66.6, 66.2, 51.1, 47.2, 40.5, 38.8, 28.6, 25.4, 23.9, 21.5, 20.0, 17.7, 15.2, 14.1, 13.1.

### Example 36

### Compound of formula I: A is (CH₂)₃-Ph, B is H, X and Y taken together with the carbon atom to which they are attached are C=N-Ac, L = CH₂CH₃, Z = H, and R₂' = H

The compound of Example 35 (15 mg, 0.02 mmol) was hydrogenated under H₂ (30 psi) over Pd-C in ethanol at room temperature for 12 hours. The solvent was evaporated under vacuum. Purification by flash chromatography (SiO₂, CH₂Cl₂ containing 5% 2M ammonia solution in methanol) gave the title compound (7.0 mg, 50 %).
MS (ESI) m/z 773 (M+H)⁺

### Example 37

### Compound of formula I: A and B taken together with the carbon atom to which they are attached are C=CH-CH=CH-3-pyridyl, X and Y taken together with the carbon atom to which they are attached are C=N-Ac, L = CH₂CH₃, Z = H, and R₂' = H

Using essentially the same procedure as for the preparation of the compound of Example 35, 190 mg (0.2 mmol) of the compound of Example 1, and 35 mg (0.2 mmol) of 1-bromo-2-(3-pyridyl)ethylene were reacted to give the the title compound.
MS (ESI) m/z 770 (M+H)⁺

### Example 38

### Compound of formula I: A and B taken together with the carbon atom to which they are attached are C=CH-CH=CH-3-quinolyl, X and Y taken together with the carbon atom to which they are attached are C=N-Ac, L = CH₂CH₃, Z = H, and R₂' = H

Using essentially the same procedure as for the preparation of the compound of Example 35, 240 mg (0.35 mmol) of the compound of Example 1, and 100 mg (0.43 mmol) of 1-bromo-2-(3-quinolyl)ethylene were reacted to give the the title compound.
MS (ESI) m/z 794 (M+H)⁺

### Example 39

### Compound of formula I: A and B taken together with the carbon atom to which they are attached are C=CH-2-quinolyl, X and Y taken together with the carbon atom to which they are attached are C=N-Ac, L = CH₂CH₃, Z = H, and R₂' = H

Using essentially the same procedure as for the preparation of the compound of Example 35, 500 mg (0.7 mmol) of the compound of Example 1, and 25 mg (1.2 mmol) of 3-bromoquinoline were reacted to give the the title compound.
MS (ESI) m/z 820 (M+H)⁺

### Example 40

### Compound of formula I: A and B taken together with the carbon atom to which they are attached are C=CH-2-quinolyl, X and Y taken together with the carbon atom to which they are attached are C=N-H, L = CH₂CH₃, Z = H, and R₂' = H

Using essentially the same procedure as for the preparation of the compound of Example 35, 63 mg (0.1 mmol) of the compound of Example 22, and 42 mg (0.2 mmol) of 3-bromoquinoline were reacted to give 7.5 mg of the title compound.
MS (ESI) m/z 742 (M+H)⁺

### Example 41

### Compound of formula I: A and B taken together with the carbon atom to which they are attached are C=CH-4-biphenyl, X and Y taken together with the carbon atom to which they are attached are C=N-Ac, L = CH₂CH₃, Z = H, and R₂' = H

Using essentially the same procedure as for the preparation of the compound of Example 35, 213 mg (0.3 mmol) of the compound of Example 1, and 142 mg (0.6 mmol) of 4-bromobiphenyl were reacted to give the the title compound.
MS (ESI) m/z 819 (M+H)⁺

### Example 42

### Compound of formula I: A and B taken together with the carbon atom to which they are attached are C=CH-3-biphenyl, X and Y taken together with the carbon atom to which they are attached are C=N-Ac, L = CH₂CH₃, Z = H, and R₂' = H

Using essentially the same procedure as for the preparation of the compound of Example 35, 213 mg (0.3 mmol) of the compound of Example 1, and 103 µL (0.6 mmol) of 3-bromobiphenyl were reacted to give the the title compound.
MS (ESI) m/z 819 (M+H)⁺

### Example 43

### Compound of formula I: A and B taken together with the carbon atom to which they are attached are C=CH-4-phenoxynhenyl, X and Y taken together with the carbon atom to which they are attached are C=N-Ac, L = CH₂CH₃, Z = H, and R₂' = H

Using essentially the same procedure as for the preparation of the compound of Example 35, 142 mg (0.2 mmol) of the compound of Example 1, and 71 µL (0.4 mmol) of 4-bromodiphenyl ether were reacted to give the the title compound.
MS (ESI) m/z 835 (M+H)⁺

### Example 44

### Compound of formula I: A and B taken together with the carbon atom to which they are attached are C=CH-Ph, X and Y taken together with the carbon atom to which they are attached are C=N-Ac, L = CH₂CH₃, Z = H, and R₂' = H

### Step 44a: 3-propanone-1,3-di-t-butyldicarbonate

To a solution of 1,3-dihydroxyacetone dimer (36.03 g, 0.20 mol) and DMAP (1.22 g, 10.0 mmol) in dichloromethane (80 mL) and pyridine (97.0 mL, 1.20 mol) was added a solution of di-*tert*-butyl dicarbonate (200.0 g, 0.92 mol) in dichloromethane (40 mL) via a dropping funnel over 3 hours at room temperature. After stirring at room temperature for 15 hours, the reaction mixture was condensed *in vacuo*. The residue was diluted with a 1:1 mixture of hexanes and diethyl ether, washed with saturated aqueous CuSO₄, water and brine. The organic phase was dried over Na₂SO₄ and the solvent was removed *in vacuo.* The residue was purified by flash chromatography (SiO₂, hexane:ethyl acetate gradient from 95:5 to 85:15) to give the title compound (45.0 g, 39%).
¹³C-NMR (125 MHz, CDCl₃): δ 198.5, 152.6, 83.5, 68.5, 27.6.

### Step 44b: Compound of formula 1-3: R₁₁ = Ph and R₁₃ = t-Bu

A suspension of benzyltriphenylphosphonium bromide (520 mg, 1.20 mmol) in THF (5.0 mL) was treated with *n*-butyl lithium (1.6 M in hexanes, 0.81 mL, 1.30 mmol) at -78°C under nitrogen. The mixture was warmed to -15°C over 1 hour before a solution of the compound from Step 44a (290 mg, 1.0 mmol) in THF (2.5 mL) was added at -70°C. The reaction mixture was warmed to room temperature over 1 hour and stirred for a further 14 hours at room temperature. The reaction mixture was diluted with ethyl acetate and the organic phase was washed with water and brine, and dried over Na₂SO₄. The solvent was evaporated and the residue was purified by flash chromatography (SiO₂, hexanes:CH₂Cl₂/1:1) to give the title compound (253 mg, 70% yield).
¹³C-NMR (125 MHz, CDCl₃): δ 153.1, 153.0, 135.1, 134.6, 130.4, 128.6, 128.2, 127.6, 82.0, 81.9, 68.4, 62.7, 27.6, 27.5.

### Step 44c: Compound of formula 1-4: R₆ = Ac, R₁₁ = Ph, R₂' = Ac, R₄" = Ac

A mixture of Erythromycin A oxime triacetate (525 mg, 0.60 mmol), the compound from Step 44b (250 mg, 0.69 mmol), 1,4-bis(diphenylphosphino)butane (51.2 mg, 0.12 mmol), and tris(dibenzylideneacetone)dipalladium (54.9 mg, 0.06 mmol) in THF (5.0 mL) was degassed and heated to 75°C for 15 hours. The solvent was removed under vacuum and the resulting residue was purified by flash chromatography (SiO₂, hexanes:acetone/4:1∼1.5:1) to give the title compounds as a 2.6:1 mixture of double bond isomers (330 mg, 55% yield).
MS (ESI) m/z: 1003 (M+H)⁺

### Step 44d: Compound of formula 1-4: R₆ = Ac, R₁₁ = Ph, R₂' = H, R₄" = Ac

The title compound was prepared by refluxing the compound from Step 44c in methanol according to the procedure described in Step 1 of Example 1.
MS (ESI) m/z: 919 (M+H)⁺

### Step 44e: Compound of formula 2-2: R₁₁ = Ph, R₂' = H

A solution of the compound from Step 44d (0.30 mmol) in methanol (5.0 mL) was treated with titanium (III) chloride (20% in 3% HCl, 0.77 mL) for 2 hours at room temperature, then for 1 hour at 50°C. The mixture was then partitioned between CH₂Cl₂ and aqueous saturated NaHCO₃. The aqueous solution was extracted with CH₂Cl₂ and the combined extracts were washed with brine and dried over Na₂SO₄. After evaporation, the residue was purified by flash chromatography (SiO₂, CH₂Cl₂:2 M NH₃ in MeOH/98:2∼93:7) to give the title compounds as a 4:1 mixture of double bond isomers (105 mg, 50% yield).
MS (ESI) m/z: 703 (M+H)⁺

### Step 44f: Compound of formula 1-5: V = N-Ac, R₁₁ = Ph, R₂' = Ac

A solution of the compound from Step 44e (105 mg, 0.15 mmol) in CH₂Cl₂ (3.0 mL) was treated with triethylamine (104 µL, 0.74 mmol) and acetic anhydride (42 µL, 0.45 mmol) at room temperature for 19 hours before evaporation and drying in vacuo to give the title compound.
MS (ESI) m/z 787 (M+H)⁺

### Step 44 g: Compound of formula I: A and B taken together with the carbon atom to which they are attached are C=CH-Ph, X and Y taken together with the carbon atom to which they are attached are C=N-Ac, L = CH₂CH₃, Z = H, and R₂' = Ac

A solution of the compound from Step 44f (0.15 mmol at most) in CH₂Cl₂ (3.0 mL) was treated with Dess-Martin periodinane (108 mg, 0.25 mL) for 4.5 hours at room temperature. The resulting mixture was partitioned between ethyl aceate and aqueous saturated NaHCO₃:Na₂S₂O₃/3:1. The organic phase was washed with water and brine. After drying (Na₂SO₄) and evaporation, the residue was purified by flash chromatography (SiO₂, hexanes:acetone/4:1∼1.5:1) to give the title compound as a 5:1 mixture of double bomd isomers (62.7 mg, 54% yield).
MS (ESI) m/z 785 (M+H)⁺

### Step 44h: Compound of formula I: A and B taken together with the carbon atom to which they are attached are C=CH-Ph, X and Y taken together with the carbon atom to which they are attached are C=N-Ac, L = CH₂CH₃, Z = H, and R₂' = H

A solution of the compound from Step 44g (62.7 mg, 0.08 mmol) in methanol (3.0 mL) was stirred at room temperature for 5 days before evaporation. Purification by flash chromatography (SiO₂, CH₂Cl₂:2 M NH₃ in MeOH/99:1-96:4) gave the title compound as a 5:1 mixture of double bond isomers (49.6 mg, 84%).
MS (ESI) m/z 743 (M+H)⁺

### Example 45

### Compound of formula I: A and B taken together with the carbon atom to which they are attached are C=CH-2-(2pyridyl)-thiophen-5-yl, X and Y taken together with the carbon atom to which they are attached, are C=N-Ac, L = CH₂CH₃, Z = H, and R₂' = H

### Step 45a: Compound of formula 6-3: V is N-Ac, X_{A} = OH, X_{B} = H, R₂' = Ac

To a solution of the compound from Step 1f of Example 1 (8.70 g, 12.25 mmol) in *t-*BuOH (18 mL) was added 4-methylmorpholine *N*-oxide (2.07 g, 14.7 mmol) and OsO₄ (4% in water, 0.78 ml). The mixture was stirred at room temperature for 1 hour and then partitioned between CH₂Cl₂ and aqueous saturated NaHCO₃. The aqueous layer was extracted with CH₂Cl₂ and the combined organic extracts were dried over NaSO₄ and condensed in vacuo. Purification by flash chromatography (SiO2, hexanes:acetone/1:1∼1:3) afforded the title compound (6.90 g, 76% yield) as a 1:1 mixture of diastereoisomers.
MS (ESI) m/z 745 (M+H)⁺

### Step 45b: Compound of formula 6-2: V is N-Ac, X_{A} = OH, X_{B} = H, R₂' = Ac

A solution of the compound from Step 45a (2.00 g, 2.69 mmol) in acetone and water (1:1, 20.0 mL) was treated with sodium periodate (1.15 g, 5.37 mmol). The mixture was stirred at room temperature for 3.5 hours and then partitioned between CH₂Cl₂ and aqueous saturated NaHCO₃. The aqueous layer was extracted with CH₂Cl₂ and the combined organic extracts were dried over NaSO₄ and condensed in vacuo. Purification by flash chromatography (SiO₂, hexanes:acetone/1:1) afforded the title compound (1.50 g, 78% yield).
MS (ESI) m/z 713 (M+H)⁺
¹³C NMR (CDCl₃): δ 205.4, 184.4, 175.8, 175.4, 169.9, 99.2, 81.8, 80.6, 79.4, 78.2, 77.4, 76.5, 75.7, 71.4, 69.0, 68.8, 63.0, 43.8, 50.6, 39.1, 38.3, 36.1, 36.0, 30.8, 25.3, 22.6, 21.4, 21.0,19.6,19.2,16.7,15.5,14.6,11.1,7.6.

### Step 45c: Compound of formula 8-1: V is N-Ac, X_{A} = OH, X_{B} = H, X_{H} = Br, R₂' = Ac

A suspension of bromomethyltriphenylphosphonium bromide (443 mg, 1.01 mmol) in THF (4.0 mL) was treated with sodium bis(trimethylsilyl)amide (1.0 M in THF, 1.00 mL, 1.00 mmol) at -78° C under nitrogen. The mixture was stirred at -70∼-60°C for 1 hour before a solution of the compound from Step 45b (127 mg, 0.18 mmol) in THF (5.0 mL) was added. The reaction mixture was warmed to room temperature over 1 hour and stirred at that temperature for 5 hours. The mixture was then partitioned between ethyl acetate and water. The organic extract was washed with water and brine, dried over Na₂SO₄, and condensed in vacuo. Purification by flash chromatography (SiO₂, hexanes:acetone/9:1∼1.5:1) gave the title compound (87 mg, 62% yield) as a 2.5-4:1 mixture of double bond isomers.
MS (ESI) m/z 789/791 (M+H)⁺

### Step 45d: 2-(2-pyridine)thiophenyl-5-bromide

A solution of 2-(2-thiophenyl)pyridine (3.00 g, 18.6 mmol) in CH₂Cl₂ (90 mL) was added dropwise to a solution of bromine (0.95 mL) in CH₂Cl₂ (5 mL) at 0° C. The mixture was warmed to room temperature with vigorous stirring over 1.5 hours before dilution with CH₂Cl₂ (200 mL). The mixture was washed with saturated NaHCO₃, Na₂SO₃, brine and dried over Na₂SO₄. Evaporation gave the title compound (4.40 g, 100%).
MS (ESI) m/z 240, 242 (M+H)⁺
¹³C NMR (CDCl₃): δ 152.0, 149.8, 146.5, 136.9, 131.1, 124.6, 122.4, 118.3, 115.3.

### Step 45e: 2-(2-pyridine)thiophenyl-5-tributylstannane

A solution of the compound from Step 45d (600 mg, 2.50 mmol) in dry THF (8.0 mL) was treated with n-butyl lithium (1.6 M in hexanes, 1.56 mL, 2.50 mL) at -78 °C with stirring for 30 minutes before tri(n-butyl)tin chloride (0.80 mL, 2.95 mmol) was added. The mixture was warmed to room temperature over 1.5 hours and then partitioned between ethyl acetate and aqueous saturated NaHCO₃. The organic layer was washed with water and brine, dried over Na₂SO₄, and condensed in vacuo. Purification by flash chromatography (SiO₂, hexanes:ethyl acetate/98:2) gave the title compound (998 mg, 89% yield).
MS (ESI) m/z 448/449/450/451/452 (M+H)⁺
¹³C NMR (CDCl₃): δ 152.7, 150.1, 149.6, 140.4, 136.6, 136.3, 125.6, 121.5, 118.8, 28.9, 27.2, 13.6, 10.8.

### Step 45f: Compound of formula 9-2: V is N-Ac, X_{A} = OH, X_{B} = H, R₁₁ = 2-(2pyridyl)-thiophen-5-yl, R₂' = Ac

A solution of the compound from Step 42c (210 mg, 0.26 mmol), the compound from Step 45e (150 mg, 0.33 mmol), and tetrakis(triphenylphosphine)palladium (61 mg, 0.05 mmol) in toluene (8.0 mL) was degassed and then stirred under nitrogen for 14 hours at 100°C. The resulting material was purified by flash chromatography (SiO₂, hexanes:acetone/9:1∼-1:1.5) to give the title compounds as a 1:3.8 mixture of double bond isomers (140 mg, 61% yield).
MS (ESI) m/z 870 (M+H)⁺

### Step 45g: Compound of formula I: A and B taken together with the carbon atom to which they are attached are C=CH-2-(2pyridyl)-thiophen-5-yl, X and Y taken together with the carbon atom to which they are attached are C=N-Ac, L = CH₂CH₃, Z = H, and R₂' = Ac

A solution of the compound from Step 45f (140 mg, 0.16 mmol) in CH₂Cl₂ (5.0 mL) was treated with Dess-Martin periodinane (122 mg, 0.29 mmol) for 1 hour at room temperature and then partitioned between ethyl acetate and aqueous saturated NaHCO₃:Na₂S₂O₃/3:1. The organic layer was washed with water and brine. After drying over Na₂SO₄ and evaporation the crude title compound was obtained as a 1:3.8 mixture of double bond isomers( 140 mg, 100% yield).
MS (ESI) m/z 868 (M+H)⁺

### Step 45h: Compound of formula I: A and B taken together with the carbon atom to which they are attached are C=CH-2-(2pyridyl)-thiophen-5-yl, X and Y taken together with the carbon atom to which they are attached are C=N-Ac, L = CH₂CH₃, Z = H, and R₂' = H

A solution of the compound from Step 45g (140 mg, 0.16 mmol) in methanol (4.0 mL) was stirred at room temperature for 64 hours and then condensed in vacuo. Purification by flash chromatography (SiO₂, CH₂Cl₂:2 M NH₃ in MeOH/99:1∼96:4) gave the title compound as a 1:3 mixture of double bond isomers (97 mg, 73%).
MS (ESI) m/z 826 (M+H)⁺

### Example 46

### Compound of formula I: A and B taken together with the carbon atom to which they are attached are C=O, X and Y taken together with the carbon atom to which they are attached are C=N-Ac, L = CH₂CH₃, Z= F, and R₂' = Ac.

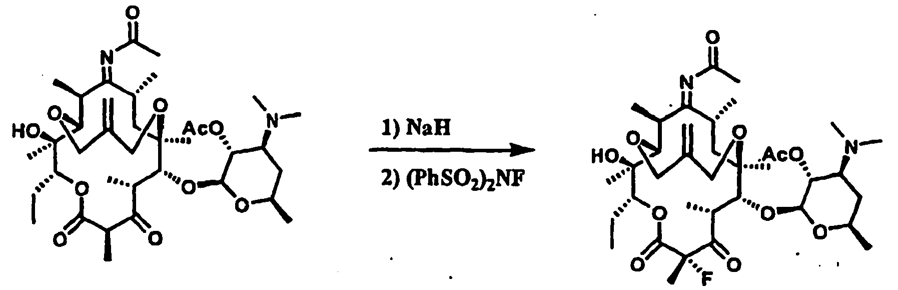

### Step 46a: Fluorination of the 3 position.

To a solution of the 3-keto compound of Example 3 (12.04 g, 17.0 mmol) in anhydrous DMF (70 mL) at 0°C, sodium hydride (60% in mineral oil, 1.50 g, 37.5 mmol) was added in one portion. Then the cooling bath was removed following the addition of sodium hydride. The reaction mixture was allowed to stir at room temperature for 30 minutes, during which period the reaction mixture turned into greenish color and then to light yellow. Upon recooling to 0°C, *N*-fluorobenzenesulfonimide (5.90 g, 18.7 mmol) was added as solid and stirred at 0°C for 2 hours. Then it was diluted with isopropyl acetate (600 mL), washed with water (200 mL X2), dried (Na₂SO₄), and evaporated. The residue was purified by flash column chromatography (silica gel, acetone/hexanes, 40:60) to give 7.77 g (63%) of the title compound as a white solid.
¹³C-NMR (125 MHz, CDCl₃): δ 205.1, 204.9, 184.1, 177.1, 170.0, 165.1, 164.9, 141.9, 125.3, 101.6, 99.8, 98.2, 79.8, 79.3, 78.5, 76.1, 71.9, 71.0, 69.4, 65.5, 63.4, 41.2, 40.8, 38.8, 31.1, 30.8, 25.3, 24.4, 24.3, 23.0, 21.6, 21.3, 20.9, 17.3,14.5, 12.5.
MS (ESI) m/z = 727 (M+H)⁺.

### Step 46b: Deprotection of 2'-hydroxy

The fluorinated compound from Step 46a is refluxed in MeOH according to the procedure set forth in Example 2 to yield the 2' hydroxy compound.

### Step 46c: Ozonolysis

The compound prepared in Step 46b is converted to the title compound via ozonolysis according to the procedure elucidated in Example 3.

Example compounds 47-114 of the formula A: 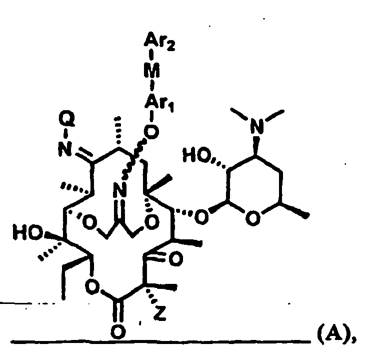 wherein Ar₁, Ar₂, M, Q, and Z are as delineated for each example in Table **A**.

Example compounds 47-114, where Z = H, are made from the title compound of Example 3 and the appropriate hydroxylamine of formula Ar₂-M-Ar₁-O-NH₂ via the method delineated in Example 4.

Example compounds 47-114, where Z = F, are made from the title compound of Example 46 and the appropriate hydroxylamine of formula Ar₂-M-Ar₁-O-NH₂ via the method delineated in Example 4.

In all of the following examples a mixture of E and Z isomers are present which may be separated by HPLC.

The substituted hydroxylamines used in the following examples are either commercially available or can be made from readily-available starting materials via synthetic methods well known by one of ordinary skill in the art.

**TABLE A.**

| **Example** | **Q** | **-Ar₁-M-Ar₂** | **Z** | **MS (ESI):** **m/z** **(M+H)⁺** | **¹³C NMR (125 MHz, CDCl₃): δ** |
|---|---|---|---|---|---|
| Example 47. | Ac | 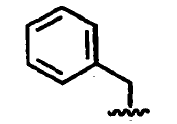 | H | 774 | N/A |
| Example 48. | Ac | 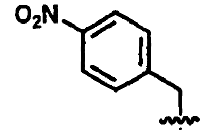 | H | 819 | N/A |
| Example 49. | Ac | 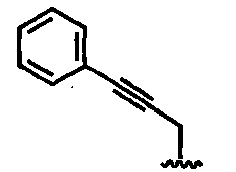 | H | 800 | N/A |
| Example 50. | Ac | 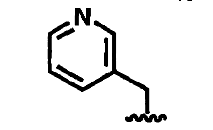 | H | 775 | N/A |
| Example 51. | Ac | 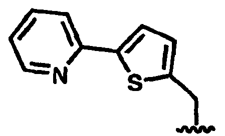 | H | 857 | N/A |
| Example 52. | Ac | 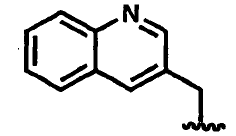 | H | 825 | N/A |
| Example 53. | Ac | 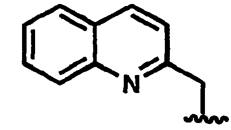 | H | 825 | N/A |
| Example 54. | Ac | 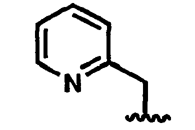 | H | 775 | N/A |
| Example 55. | MOM | 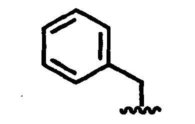 | H | 792 | N/A |
| Example 56. | Ac | 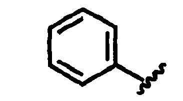 | H | 760 | N/A |
| Example 57. | Ac | 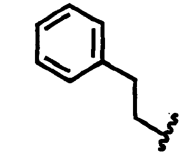 | H | 787 | N/A |
| Example 58. | Ac | 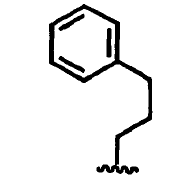 | H | 802 | N/A |
| Example 59. | Ac | 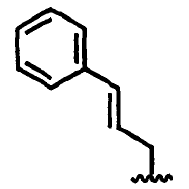 | H | 800 | N/A |
| Example 60. | Ac | 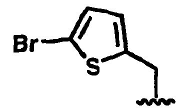 | H | 858 | N/A |
| Example 61. | Ac | 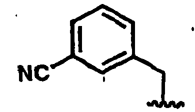 | H | 799 | N/A |
| Example 62. | Ac | 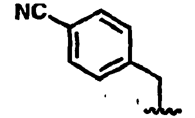 | H | 799 | N/A |
| Example 63. | Ac | 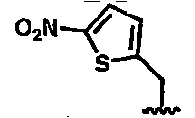 | H | 809 | N/A |
| Example 64. | Ac | 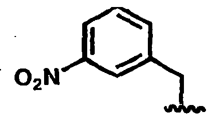 | H | 819 | N/A |
| Example 65. | Ac | 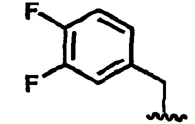 | H | 810 | N/A |
| Example 66. | Ac | 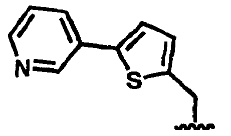 | H | 857 | N/A |
| Example 67. | Ac | 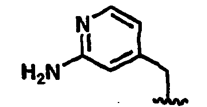 | H | 790 | N/A |
| Example 68. | Ac | 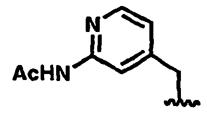 | H | 832 | N/A |
| Example 69. | Ac | 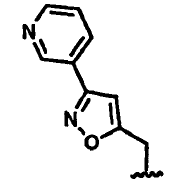 | H | 842 | N/A |
| Example 70. | Ac | 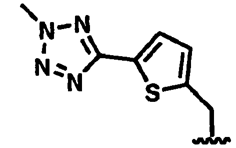 | H | 862 | N/A |
| Example 71. | Ac | 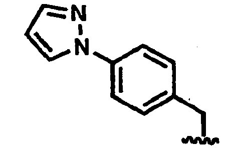 | H | 840 | N/A |
| Example 72. | Ac | 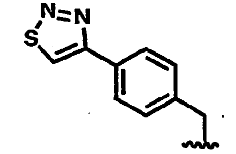 | H | 858 | N/A |
| Example 73. | Ac | 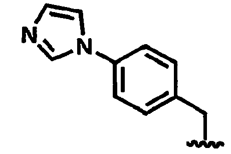 | H | 840 | N/A |
| Example 74. | Ac | 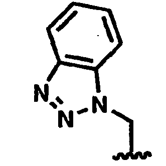 | H | 815 | N/A |
| Example 75. | Ac | 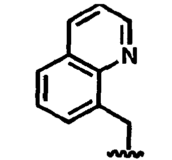 | H | 825 | N/A |
| Example 76. | Ac | 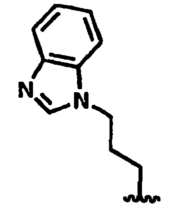 | H | 844 | N/A |
| Example 77. | Ac | 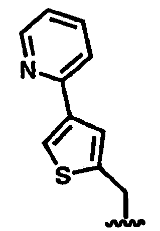 | H | 857 | N/A |
| Example 78. | Ac | 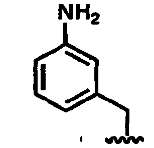 | H | 789 | N/A |
| Example 79. | Ac | 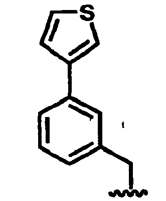 | H | 856 | N/A |
| Example 80. | Ac | 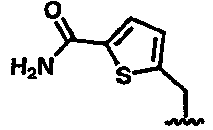 | F | 841 | N/A |
| Example 81. | H | 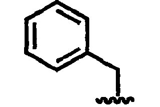 | H | 732 | N/A |
| Example 82. | Ac | 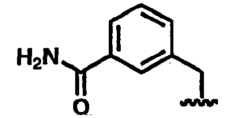 | H | 817 | N/A |
| Example 83. | Ac | 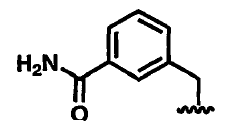 | F | 835 | N/A |
| Example 84. | Ac | 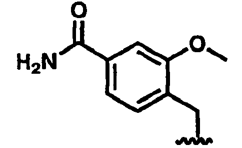 | H | 847 | N/A |
| Example 85. | Ac | 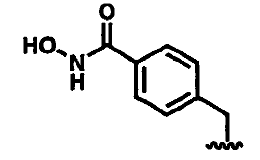 | H | 831 | N/A |
| Example 86. | Ac | 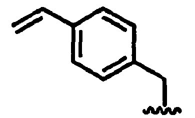 | H | 800 | N/A |
| Example 87. | Ac | 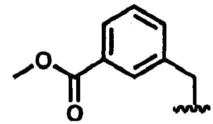 | H | 832 | N/A |
| Example 88. | Ac | 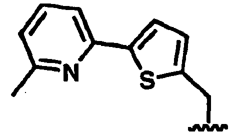 | H | 871 | N/A |
| Example 89. | Ac | 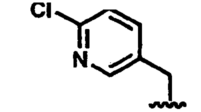 | H | 809 | N/A |
| Example 90. | H | 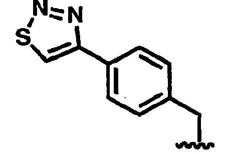 | H | 816 | N/A |
| Example 91. | H | 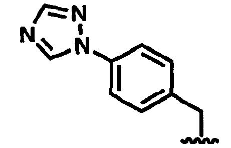 | H | 799 | N/A |
| Example 92. | OMe | 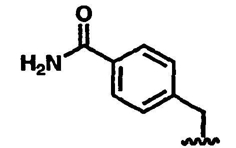 | H | 805 | N/A |
| Example 93. | MOM | 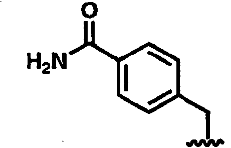 | H | 835 | N/A |
| Example 94. | -OCH₂C N | 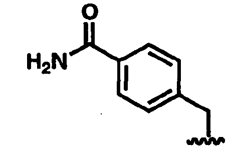 | H | 830 | N/A |
| Example 95. | -OCH₂C H₂OH | 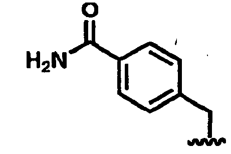 | H | 837 | N/A |
| Example 96. | H | 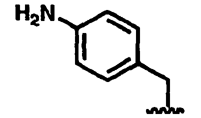 | H | 747 | N/A |
| Example 97. | Ac | 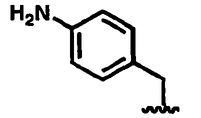 | H | 789 | N/A |
| Example 98. | Ac | 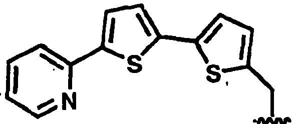 | H | 939 | N/A |
| Example 99. | Ac | 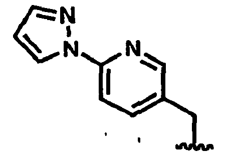 | H | 841 | N/A |
| Example 100. | Ac | 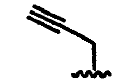 | H | 722 | N/A |
| Example 101. | Ac | 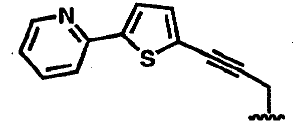 | H | 881 | N/A |
| Example 102. | Ac | 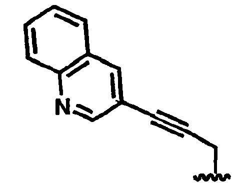 | H | 849 | N/A |
| Example 103. | Ac | 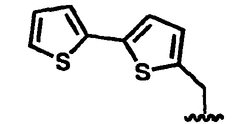 | H | 862 | N/A |
| Example 104. | Ac | 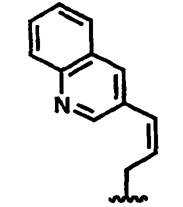 | H | 851 | N/A |
| Example 105. | Ac | 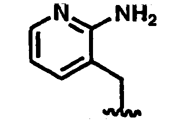 | H | 790.55 | N/A |
| Example 106. | Ac | 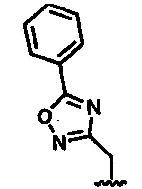 | H | 842 | N/A |
| Example 107. | Ac | 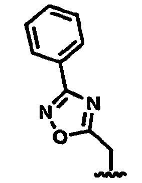 | H | 843 | N/A |
| Example 108. | Ac | 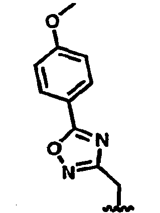 | H | 872 | N/A |
| Example 109. | Ac | 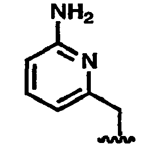 | H | 790 | N/A |
| Example 110. | Ac | 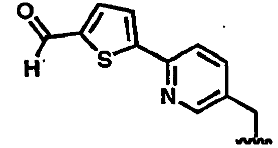 | H | 885 | N/A |
| Example 111. | Ac | 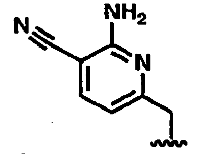 | H | 815 | N/A |
| Example 112. | Ac | 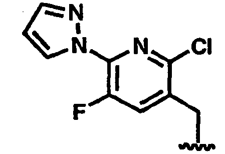 | H | 893 | N/A |
| Example 113. | Ac | 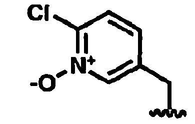 | H | 825 | N/A |
| Example 114. | Ac | 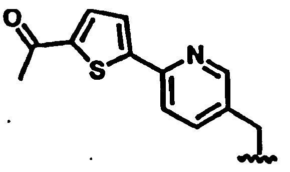 | H | 900 | N/A |

Example compounds 115-263 of formula A1: 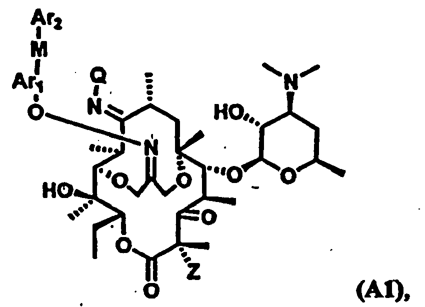 wherein Ar₁, M₂, M, Q, and Z are as delineated for each example in Table **A1**.

Example compounds 115-262, where Z = H, are made from the title compound of Example 3 and the appropriate hydroxylamine of formula Ar₂-M-Ar₁-O-NH₂ via the method delineated in Example 4.

Example compounds 115-262, where Z = F, are made from the title compound of Example 46 and the appropriate hydroxylamine of formula Ar₂-M-Ar₁-O-NH₂ via the method delineated in Example 4.

The Examples described in Table A1 are single isomers of the E designation, which are separated from the E/Z mixture via silica chromatography or HPLC.

The substituted hydroxylamines used in the following examples are either commercially available or can be made from readily-available starting materials via synthetic methods well known by one of ordinary skill in the art.

**TABLE A1.**

| **Example** | **Q** | **-Ar₁-M-Ar₂** | **Z** | **MS (ESI):** **m/z** **(M+H)⁺** | **¹³C NMR (125 MHz, CDCl₃): δ** |
|---|---|---|---|---|---|
| Example 115. | Ac | 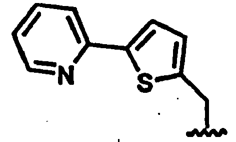 | H | 857 | 205.8, 184.7, 178.1, 169.3, 156.2, 153.9, 149.7, 145.3, 142.9, 136.8, 128.3, 124.5, 122.0, 119.0, 103.0. |
| Example 116. | Ac | 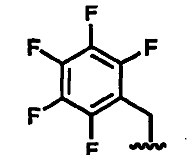 | H | 864 | Selected data: 205.7, 184.7, 178.0, 167.7, 154.5, 147.1, 145.1, 138.6, 136.6, 103.1, 79.1, |
| Example 117. | Ac | 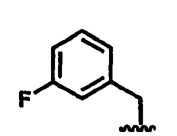 | H | 792 | Selected: 205.8, 184.8, 178.0, 167.8, 153.8, 140.9, 134.5, 130.0, 123.5, 114.9, 114.7(2), 114.5, 110.0, 103.0, 79.4, 76.7, 75.3, 74.7, 70.5, 69.7, 66.1, 63.1, 62.9, 50.7, 46.2, 405, 38.8, 32.0, 29.9, 28.5, 25.4, 23.8, 21.5, 20.3, 19.5, 17.8, 15.1, 14.1, 13.7, 12.8, |
| Example 118. | Ac | 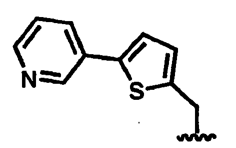 | H | 857 | 205.8, 184.7, 178.0, 167.7, 154.0, 148.6, 147.0, 141.5, 141.0, 133.0, 130.7, 128.2, 124.0, 123.8, 103.1, 79.3, 79.1, 76.7, 75.5, 74.5, 70.7, 70.5, 69.7, 66.0, 63.1, 62.8, 50.7, 46.2, 40.4, 38.7, 37.2, 31.1, 29.5, 28.5, 25.3, 23.8, 21.5, 20.3, 19.5, 17.8, 15.0, 14.1, 13.7, 13.0, |
| Example 119. | Ac | 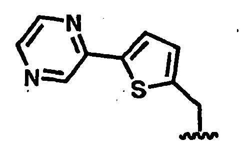 | H | 858 | 205.8,184.7, 178.1, 167.8, 154.2, 148.8, 144.8, 144.2, 142.5, 141.6, 140.7, 128.0, 125.7, 103.1, 79.5, 79.2, 76.8, 75.6, 74.5, 70.9, 70.5, 69.8, 66.1, 63.1, 62.9, 50.7, 46.2, 40.5, 38.8, 37.3, 29.5, 28.5, 25.4, 23.9, 21.5, 20.3, 19.5, 17.8, 15.1, 14.1, 13.6, 13.0, |
| Example 120. | Ac | 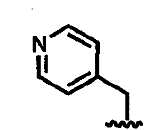 | <H | 775 | Selected: 84.5, 177.6, 167.7, 154.1, 149.7, 147.3, 121.9, 102.8, 79.1, 76.5, 75.3, 73.9, 70.3, 69.5, 65.8, 62.8, 62.7, 50.5, 46.1, 40.2, 38.5, 28.2, 25.1, 23.6, 21.2, 20.0, 19.3, 17.5, 14.9, 13.8, 13.4, 12.7, |
| Example 121. | Ac | 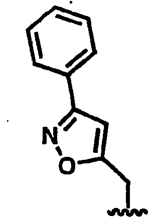 | H | 842 | 205.8, 184.7, 178.0, 170.0, 167.8, 163.3, 154.8, 150.0, 148.8, 137.0, 124.6, 124.6, 121.9, 103.0, 102.5, 7935, 79.2, 76.8, 74.6, 70.4, 69.6, 66.9, 66.2, 63.0, 62.7, 50.7, 46.1, 40.5, 38.8, 28.7, 25.4, 23.9, 21.5, 20.2, 19.5, 17.8, 15.1, 14.1, 13.6, 13.0, |
| Example 122. | Ac | 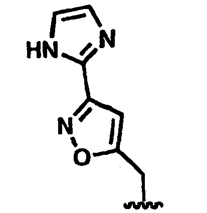 | H | 831 | 205.7, 184.8, 178.0. 169.8, 167.9,155.4,154.9,139.9, 137.4, 103.1, 102.2, 79.5, 79.3, 76.7, 74.7, 70.5, 69.7, 66.7, 66.0, 62.9, 62.7, 50.7, 46.1, 40.4, 38.7, 28.5, 25.3, 23.9, 21.5, 20.2, 19.5, 17.8, 15.1, 14.0,13.6, 13.0, 10.9. |
| Example 123. | Ac | 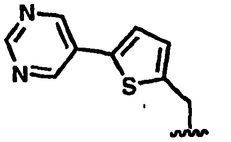 | H | 858 | N/A |
| Example 124. | Ac | 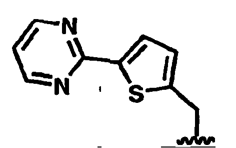 | H | 858 | 205.8, 184.7, 178.1, 167.8, 161.8, 157.4, 154.1, 145.6, 143.3, 128.9, 127.8, 118.6, 103.1, 79.5, 79.2, 76.8, 75.5, 74.5, 71.1, 70.5, 69.7, 66.1, 63.1, 62.9, 50.7, 46.2, 40.5, 38.8, 37.3, 31.2, 28.5, 25.3, 23.9, 21.5, 20.3, 19.5, 17.8, 15.1, 14.1, 13.6, 13.0. |
| Example 125. | Ac | 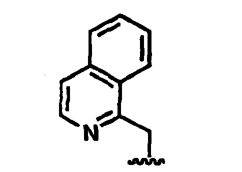 | H | 825 | Selected: 205.8, 184.6, 177.9, 167.9, 156.8, 153.7, 142.0, 136.6, 130.2, 127.4, 127.2, 126.4, 121.3, 103.0, 79.2, 76.6, 76.3, 74.9, 70.5, 69.7, 66.0, 63.1, 62.8, 50.8, 46.2, 40.4, 38.7, 28.4, 25.3, 23.7, 21.4, 20.3, 19.6, 17.7, 15.1, 14.1, 13.7, 12.7. |
| Example 126. | Ac | 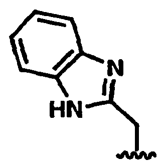 | H | 831 | Selected: 205.8, 184.8, 178.0, 169.5, 167.7, 155.2, 153.3, 135.3, 126.1, 125.2, 123.3, 121.9, 103.1, 79.3, 76.7, 74.7, 73.5, 70.5, 69.8, 66.1, 62.9, 50.7, 40.5, 38.8, 28.5, 25.4, 23.8, 21.5, 20.3, 19.5, 17.7, 15.1, 14.1, 12.9, |
| Example 127. | Ac | 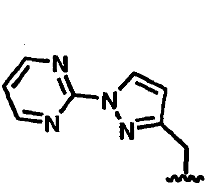 | H | 843 | 205.8, 184.8, 177.7, 167.9, 159.3, 155.8, 154.6, 136.6, 134.5, 120.7, 102.9, 79.3, 76.7, 75.0, 70.4, 69.7, 67.9, 66.2, 62.9, 62.8, 50.8, 46.3, 40.5, 38.8, 28.7, 25.4, 23.8, 25.4, 23.8, 21.5, 20.2, 19.5, 17.7, 15.1, 14.1, 13.7, 12.9, |
| Example 128. | Ac | 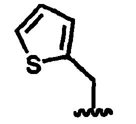 | H | 780 | N/A |
| Example 129. | Ac | 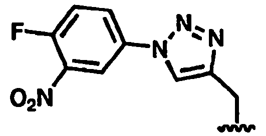 | H | 904 | N/A |
| Example 130. | Ac | 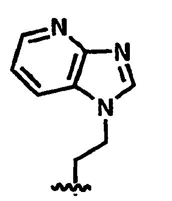 | H | 829 | Selected: 205.8, 184.6, 177.5, 168.2, 154.8, 147.2, 145.6, 144.2, 135.6, 128.0, 118.3, 103.0, 79.4, 79.1, 76.5, 75.5, 72.0, 70.5, 69.7, 66.1, 63.0, 62.9, 50.8, 46.4, 43.0, 40.5, 38.7, 28.5, 25.4, 23.5, 21.5, 20.2, 19.7, 17.7, 15.2, 14.1, 13.8, 12.6. |
| Example 131. | Ac | 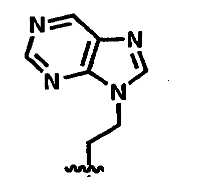 | H | 830 | Selected: 205.7, 184.6, 177.3, 168.4, 155.2, 152.6, 151.6, 148.6, 147.1, 134.2, 103.1, 79.5, 79.1, 765, 75.9, 71.8, 70.5, 69.8, 66.1, 63.0, 50.8, 43.1, 40.5, 38.7, 28.5, 25.5, 23.5, 21.5, 19.8, 17.7,15.2, 14.0, 12.6. |
| Example 132. | Ac | 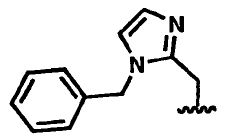 | H | 854 | E oxime isomer: Selected: 205.6, 184.6, 177.9, 167.9, 153.8, 145.0, 137.5, 128.9 128.6, 127.9, 127.3, 121.3, 103.2, 9.2, 76.6, 75.9, 74.6, 70.5, 69.7, 67.2, 66.0. 63.1, 62.4, 50.7, 50.1, 46.3, 40.6, 38.7, 28.6, 25.3, 23.6, 21.5, 20.3, 19.6, 17.7, 15.0, 14.1, 12.8. |
| Example 133. | Ac | 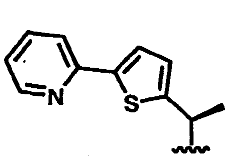 | H | 871 | 206.0, 184.6, 178.0, 167.8, 153.6, 153.0, 149.7, 148.1, 143.9, 136.7, 125.6, 124.2, 121.9, 118.8, 102.9, 78.7, 76.7, 74.8, 70.4, 69.6, 66.2, 63.3, 51.0, 40.5, 38.9, 29.9, 28.9, 25.4, 23.6, 23.3, 22.7, 22.2, 21.5, 20.4, 19.7, 17.7, 15.1,14.2,12.6,11.7. |
| Example 134. | Ac | 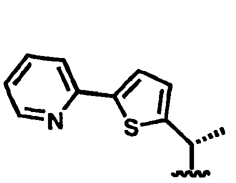 | H | 871 | 205.9, 184.7, 178.2, 167.7, 153.4, 153.0, 150.2, 149.7, 143.8, 136.7, 125.6, 124.2, 121.9, 118.8, 102.6, 78.9, 76.9, 74.4, 70.3, 69.2, 66.5, 63.2, 62.7, 50.7, 46.0, 40.5, 38.9, 29.6, 29.5, 25.3, 23.6, 22.2, 21.4, 20.3, 19.4, 17.9, 15.1, 14.3, 13.5, 12.6. |
| Example 135. | Ac | 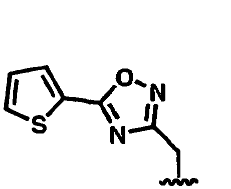 | H | 848 | 205.8, 184.7, 178.1, 171.8, 168.1, 167.8, 154.8, 132.3, 132.1, 128.7, 125.9, 102.9. 79.4, 76.7, 74.6, 705, 69.7, 67.0, 66.1, 62.9, 62.7, 50.7, 46.1, 405, 38.8, 28.5, 25.3, 23.9, 21.5, 20.2, 19.5, 17.8, 15.1, 14.1, 12.9. |
| Example 136. | Ac | 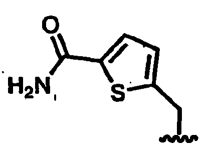 | H | 823 | 205.6, 184.5, 177.8, 167.6, 163.3, 154.2, 146.5, 137.3, 129.3, 126.8, 102.9, 79.2, 76.5, 74.4, 70.4, 70.3, 69.6, 69.5, 65.8, 62.8, 62.6, 53.8, 50.8, 50.5, 46.1, 40.2, 38.5, 31.7, 29.3, 28.3, 25.1, 23.6, 21.3, 20.1, 19.3, 17.5, 14.8, 13.9, 12.8. |
| Example 137. | Ac | 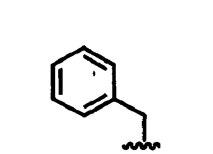 | H | 774 | 205.9, 184.8, 178.0, 167.8, 153.3, 138.1, 128.4, 128.2, 127.8, 102.9, 79.4, 76.8, 76.2, 74.6, 70.5, 69.6, 66.2, 63.2, 62.9, 50.8. 40.5, 38.8, 28.7, 25.3, 23.8, 21.5, 20.3, 19.5, 17.8, 15.1, 14.1, 12.8. |
| Example 138. | Ac | 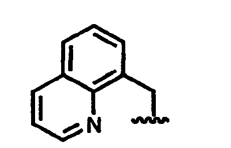 | F | 843 | 205.3, 205.1, 184.2, 153.6, 149.8, 146.3, 136.5, 136.3, 128.4, 128.2, 127.5, 126.4, 121.2, 104.1, 99.7, 98.1, 79.6, 76.6, 73.7, 72.8, 70.7, 69.4, 66.0, 63.3, 62.7, 41.2, 40.5, 39.1, 28.4, 25.3, 24.7, 24.5, 23.2, 21.4, 21.1, 17.4, 15.1, 14.3, 12.5. |
| Example 139. | Ac | 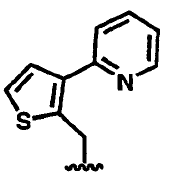 | H | 857 | 205.8, 184.8, 178.0, 167.7, 154.5, 153.9, 149.7, 139.4, 138.6, 136.8, 128.6, 124.9, 123.3, 121.8, 103.1, 79.2, 76.7, 74.7, 70.5, 70.2, 69.7, 66.1, 63.1, 63.0, 50.9, 46.4, 40.5, 38.8, 28.5, 25.4, 23.7, 21.5, 20.4, 19.6, 17.7, 15.1, 14.1, 13.8, 12.7. |
| Example 140. | Ac | 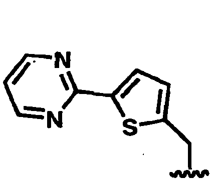 | F | 876 | 205.2(d), 184.1, 177.4, 165.1(d), 161.7, 157.4, 154.3, 145.3, 143.4, 128.9, 127.8, 118.6, 104.0, 99.8, 98.1, 79.8, 76.6, 73.6, 71.2, 70.6, 69.7, 66.1, 63.2, 62.7, 41.3, 40.5, 39.0, 29.9, 28.7, 25.3, 24.6, 24.4, 23.2, 21.4, 21.0, 17.4, 15.0, 14.3, 12.8. |
| Example 141. | Ac | 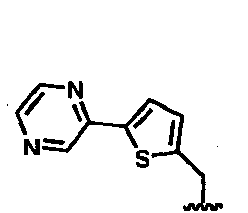 | F | 876 | (205.1, 204.9), 183.9, 176.5, (164.9, 164.7), 154.1, 148.6, 144.2, 144.0, 142.3, 141.4, 140.5, 127.7, 125.5, 103.9, (99.5, 97.9), 795, 76.3, 73.4, 70.7, 70.4, 69.6, 65.8, 62.9, 62.4, 53.8, 41.1, 40.2, 38.7, 29.7, 293, 28.2, 25.1, 24.4, 24.2, 23.0, 21.2, 20.8, 17.1, 14.8, 14.1, 12.6. |
| Example 142. | Ac | 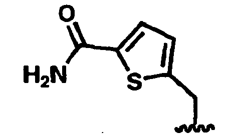 | F | 841 | N/A |
| Example 143. | Ac | 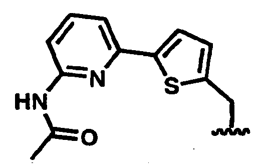 | H | 914 | 205.9, 184.8, 178.0, 169.0, 167.8, 154.0, 151.1, 151.0, 144.4, 143.2, 139.2, 127.3, 124.5, 114.6, 111.6, 102.8, 79.4, 79.3, 76.8, 74.6, 71.1, 70.4, 69.4, 66.4, 63.0, 62.8, 50.8, 46.1, 40.5, 38.8, 29.9, 29.1, 25.4, 25.0, 23.8, 21.4, 20.3, 19.5, 17.8, 15.1, 14.2, 13.8, 12.9. |
| Example 144. | Ac | 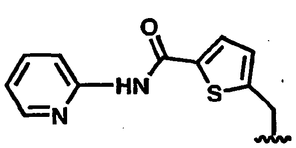 | H | 900 | 205.8, 184.7, 178.1, 167.8, 160.2, 154.5, 151.5, 148.1, 147.6, 138.7, 138.6, 129.0, 127.2, 120.1, 114.4, 103.1, 79.5, 79.2, 76.8, 74.6, 70.7, 70.5, 69.8, 66.1, 63.1, 62.8, 50.7, 46.3, 40.5, 38.8, 28.5, 25.3, 23.9, 21.5, 20.3, 19.5, 17.8, 15.1, 14.1, 13.6, 13.0 |
| Example 145. | Ac | 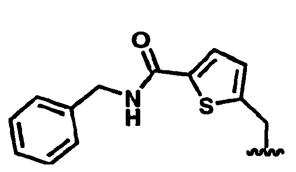 | H | 913 | 205.6, 194.5, 177.8, 167.6, 161.8, 154.1, 145.5, 138.2, 138.1, 128.8, 128.2, 127.9, 127.6, 126.8, 102.8, 79.2, 76.5, 74.3, 70.4, 70.3, 69.5, 65.9, 62.8, 62.6, 50.5, 46.0, 44.0, 40.2, 38.5, 29.3, 28.3, 25.1, 23.6, 21.3, 20.0, 19.3, 17.5, 14.8, 13.9, 13.4, 12.8. |
| Example 146. | Ac | 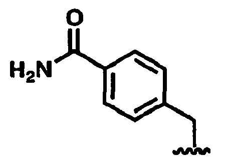 | H | 817.28 | 205.9, 184.8, 169.4, 167.9, 153.9, 142.6, 132.7, 103.1, 79.4, 75.3, 74.8, 70.5, 69.7, 66.1, 63.1, 63.0, 50.8, 38.8, 28.5, 25.3, 23.8, 21.5, 20.3, 19.6, 17.8, 15.1, 14.1, 12.9 |
| Example 147. | Ac | 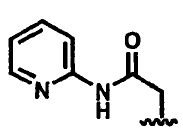 | H | 818 | Selected: 205.6, 184.4, 168.4, 168.3, 157.0, 150.8, 148.1, 138.8, 120.3, 114.2, 110.0, 103.1, 78.3, 765, 72.7, 705, 69.8, 66.1, 625, 62.1, 50.9, 40.4, 31.2, 28.5, 25.4, 23.3, 21.5, 19.8, 18.4, 15.1, 14.2, 11.9, |
| Example 148. | Ac | 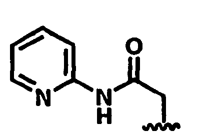 | F | 836 | Selected: 203.1, 202.8, 181.8, 175.1, 165.9, 163.2, 163.0, 154.8, 148.5, 146.0, 136.7, 118.3, 112.0, 102.0, 76.8, 72.6, 70.6, 68.5, 67.8, 63.9, 38.3, 26.3, 19.3, 19.3, 16.3, 12.9, 9.7. |
| Example 149. | Ac | 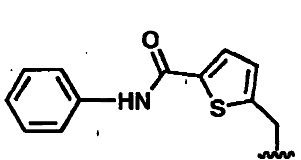 | H | 899 | 205.8, 194.8, 178.0, 167.8, 160.1, 154.4, 146.6, 138.8, 137.9, 129.3, 129.3, 128.9, 127.2, 124.7, 120.4, 103.1, 79.5, 76.8, 74.6, 70.7, 70.5, 69.8, 69.7, 66.1, 63.1, 62.9, 54.0, 50.7, 40.5, 38.8, 32.0, 29.9, 29.5, 28.6, 25.4, 23.9, 21.5, 20.3, 19.5, 17.8, 15.1, 14.1, 13.0. |
| Example 150. | Ac | 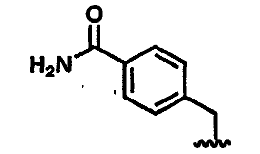 | F | 835.39 | 205.4, 184.3, 169.3, 154.1 142.4, 132.7, 128.4, 127.9 127.6, 104.1, 79.7, 76.6, 75.4, 73.7, 70.6, 69.8, 66.1, 62.6, 41.3. 40.5, 28.5, 25.3, 23.2, 21.4, 21.0, 17.4, 12.7 |
| Example 151. | Ac | 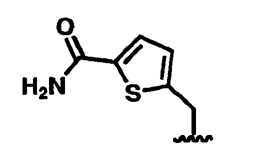 | H | 808 | 205.5, 184.5, 182.9, 177.7, 167.6, 154.4, 151.6, 143.5, 136.2, 126.9, 102.8, 79.2, 79.0, 76.5, 74.3, 70.5, 70.2, 69.4, 65.9, 62.7, 50.6, 45.9, 40.2, 38.5, 28.4, 25.1, 23.6, 21.2, 20.0, 192, 17.5, 14.8, 13.8, 13.3, 12.8 |
| Example 152. | Ac | 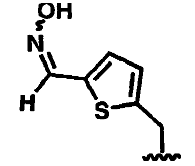 | H | 823 | N/A |
| Example 153. | Ac | 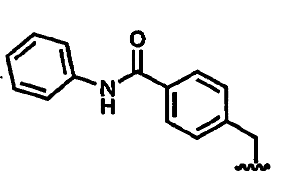 | H | 893 | 205.9, 184.9, 177.8, 167.9, 165.9, 153.9, 142.4, 138.3, 134.4, 129.3, 128.2, 127.3, 124.7, 120.4, 102.8, 79.4, 79.2, 76.7, 75.3, 74.9, 70.4, 69.5, 66.3, 63.1, 63.0, 50.7, 46.2, 40.5, 38.8, 32.0, 28.9, 25.3, 23.8, 21.4, 20.3, 19.5, 17.8, 15.1, 14.1, 13.6, 12.9. |
| Example 154. | Ac | 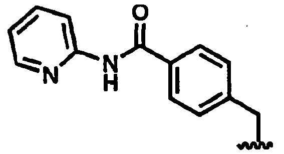 | H | 894 | N/A |
| Example 155. | Ac | 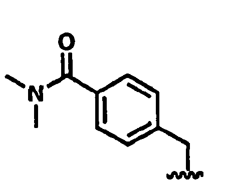 | H | 845 | 205.9, 184.8, 178.0, 171.8, 167.8, 153.7, 139.8, 135.7, 129.9, 127.9, 127.3, 103.0, 79.4, 79.2, 76.8, 75.6, 74.7, 70.5, 69.7, 66.1, 63.1, 62.9, 50.7, 46.2, 40.5, 38.8, 28.5, 25.4, 23.8, 21.5, 20.3, 19.5, 17.8, 15.1, 14.1, 13.7, 12.9. |
| Example 156. | Ac | 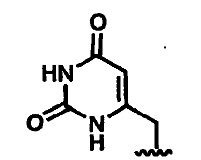 | H | 808 | 205.5, 184.8, 177.7, 168.4, 164.3, 156.3, 151.8, 103.2, 99.9, 79.4, 79.2, 76.8, 75.2, 71.1, 70.5, 69.6, 66.0, 62.9, 62.7, 50.7, 46.4, 40.4, 38.7, 29.9, 28.6, 25.4, 235, 21.5, 20.2, 19.6, 17.9, 15.0, 14.0, 13.7, 12.7. |
| Example 157. | Ac | 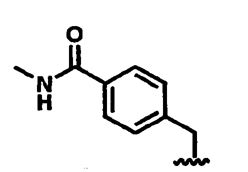 | H | 831.60 | 205.8, 184.8, 177.9, 168.3, 167.9, 153.8, 141.8, 134.0, 128.0, 127.0, 103.0, 79.4, 79.2, 76.7, 75.4, 74.8, 70.5, 69.8, 66.1, 63.1, 63.0, 50.7, 46.2, 40.5, 38.8, 28.5, 27.1, 25.3, 23.8, 21.5, 20.3, 19.5, 17.8, 15.1, 14.1, 13.7, 12.9 |
| Example 158. | Ac | 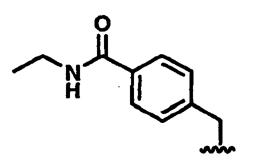 | H | 845.61 | 205.8, 184.8, 177.9, 167.8, 167.5, 153.8, 141.7, 134.2, 128.0, 127.0, 103.0, 79.4, 79.2, 76.7, 75.4, 74.7, 70.5, 69.7, 66.1, 63.1, 63.0, 50.7, 46.2, 40.5, 38.8. 35.1, 28.5, 25.3, 23.8, 21.5, 20.3, 19.5, 17.8, 15.1. 14.1, 13.7, 12.9 |
| Example 159. | Ac | 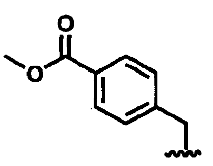 | H | 832 | 204.6, 183.5, 176.7, 166.6, 166.0, 152.6, 142.3, 128.5, 128.2, 126.4, 101.8, 78.1, 77.9, 75.5, 74.2, 73.4, 69.2, 68.5, 64.8, 61.8, 61.7,51.0,49.5,45.0, 39.2, 37.5, 27.3, 24.1, 22.6, 20.2, 19.0, 18.3, 16.5, 13.9, 12.8, 12.4, 11.6 |
| Example 160. | Ac | 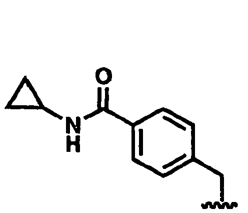 | H | 857 | 205.8, 184.8, 177.9, 168.9, 167.8, 153.8, 142.0, 133.8, 128.0, 127.0, 103.0, 79.4, 76.7, 75.4, 74.8, 70.5, 69.7, 66.1, 63.1, 63.0, 50.7, 46.2, 40.5, 38.8, 28.5, 25.3, 23.8, 23.3, 21.5, 20.3, 19.5, 17.8, 15.1, 14.1, 13.7, 12.9, 7.0 |
| Example 161. | Ac | 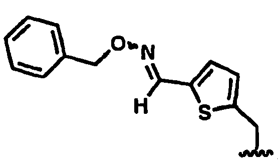 | H | 913 | N/A |
| Example 162. | Ac | 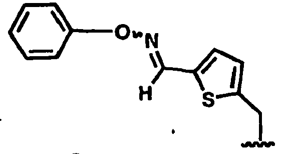 | H | 899 | N/A |
| Example 163. | Ac | 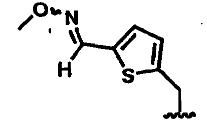 | H | 837 | N/A |
| Example 164. | Ac | 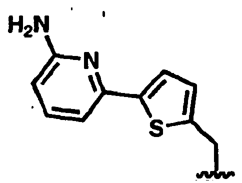 | H | 872 | 205.9, 184.7, 178.1, 167.7, 158.2, 153.7, 151.2, 145.6, 142.0, 138.5, 127.7, 124.0, 109.3, 107.1, 102.7,79.5, 79.1, 76.8, 74.6, 71.1, 70.3, 69.3, 66.5, 63.1, 62.9, 50.8, 46.0, 40.6, 38.8, 29.9, 29.3, 25.4, 23.8, 21.4, 20.3, 19.5, 17.8, 15.1, 14.2, 13.5, 13.0. |
| Example 165. | Ac | 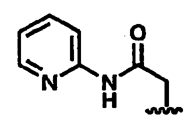 | H | 817 | 205.5, 184.7, 177.4, 168.7, 168.2, 157.0, 137.4, 128.9, 124.8, 121.4, 103.0, 79.7, 78.9, 76.6, 75.9, 73.6, 70.5, 69.7, 63.0, 62.9, 51.0, 46.4, 40.5, 38.7, 36.9, 28.6, 25.3, 23.4, 215, 20.3, 19.7, 17.7, 15.2, 14.2, 12.3. |
| Example 166. | Ac | 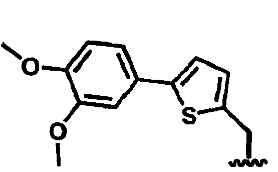 | H | 916 | 205.8, 184.6, 176.6, 169.3, 156.2, 149.3, 148.9, 145.5, 138.8, 128.4, 128.0, 122.1, 118.7, 111.7, 109.7, 102.4, 79.6, 79.0, 76.2, 75.4, 71.0, 70.6, 70.4, 69.5, 66.3, 58.4, 56.22, 56.20, 50.9, 45.2, 40.5, 39.6, 39.0, 36.8, 29.9, 28.9, 25.5, 23.4, 21.5, 20.2, 19.7, 17.2, 15.7, 14.5, 12.8, 12.0. |
| Example 167. | Ac | 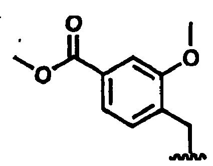 | H | 862 | 205.8, 184.8, 177.9, 167.8, 156.9, 153.6, 132.3, 130.3, 128.2, 122.0, 110.9, 103.0, 79.3, 79.2, 76.7, 74.8, 71.0, 70.5, 69.7, 66.0, 63.1, 55.7, 52.3, 50.8, 46.2, 40.4, 38.8, 28.5, 25.3, 23.8, 21.4, 20.3, 19.6, 17.7, 15.1, 14.1, 13.7, 12.8 |
| Example 168. | Ac | 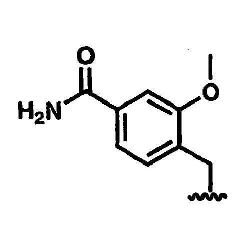 | H | 847 | 205.8, 184.9, 177.8, 169.4, 167.8, 157.3, 153.6, 133.7, 131.3, 128.4, 118.7, 109.7, 103.0, 79.3, 79.2, 76.7, 75.0, 70.9, 70.4, 69.7, 66.0, 63.0, 55.7, 50.8, 46.2, 40.4, 38.7, 28.4, 25.3, 23.8, 21.4, 20.3, 19.6, 17.7, 15.0, 14.0, 13.7, 12.8. |
| Example 169. | Ac | 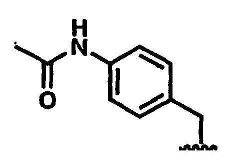 | H | 831 | 204.7, 183.5, 176.8, 167.2, 166.5, 152.0, 136.3, 132.8, 127.9, 118.6, 101.5, 78.1, 77.8, 75.5, 74.5, 73.4, 69.1, 68.1, 65.2, 61.9, 61.6, 52.4, 49.5, 44.8, 39.3, 37.6, 30.8, 28.7, 28.0, 24.1, 23.6, 22.5, 20.1, 19.0, 18.2, 16.5, 13.9, 12.9, 12.3, 11.6. |
| Example 170. | Ac | 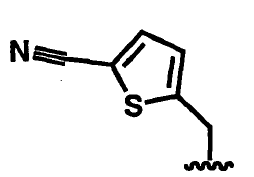 | H | 805 | 205.5, 184.4, 177.7, 167.6, 154.6, 148.7, 137.2, 126.4, 114.3, 109.6, 102.9, 79.1, 76.4, 74.3, 70.2, 69.8, 69.5, 65.8, 62.7, 62.5, 50.4, 46.1, 40.2, 38.5, 28.2, 25.1, 23.5, 21.2, 20.0, 19.2, 17.5, 14.8, 13.8, 12.7 |
| Example 171. | Ac | 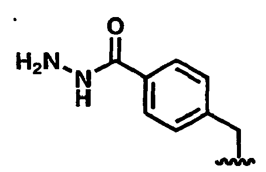 | H | 832 | 205.8, 184.8, 177.9, 168.7, 167.9, 153.9, 142.4, 132.0, 128.1, 127.1, 79.3, 76.7, 75.3, 74.8, 70.5, 69.7, 66.0, 63.0, 50.8, 46.3, 40.4, 38.7, 28.5, 25.3, 23.8, 21.5, 20.3, 19.5, 17.8, 15.1, 14.1, 13.7, 12.9 |
| Example 172. | Ac | 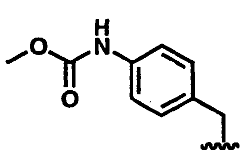 | H | 847 | 203.7, 182.6, 175.9, 165.6, 151.1, 135.4, 131.0, 127.2, 116.5, 100.8, 77.2, 77.1, 74.6, 73.7, 72.5, 68.3, 67.6, 64.0, 61.0, 60.8, 50.4, 48.6, 44.0, 38.3, 36.7, 27.8, 26.4, 23.2, 21.6, 19.3, 18.2, 17.4, 15.6, 13.0, 12.0, 11.5, 10.7. |
| Example 173. | Ac | 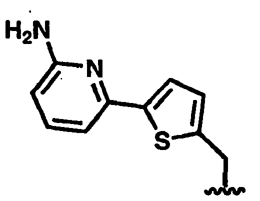 | F | 890 | (205.1, 204.9), 183.9, 164.4, 158.0, 153.8, 151.0, 145.5, 141.5, 138.2, 127.4, 123.7, 109.1, 106.8, 103.9, 99.5, 97.9, 79.5, 76.3, 73.3, 71.0, 70.4, 69.6, 65.8, 62.9, 62.4, 41.0, 40.2, 29.7, 28.2, 25.1, 24.4, 24.2, 23.0, 21.2, 20.8, 17.1, 14.8, 14.0, 12.5. |
| Example 174. | Ac | 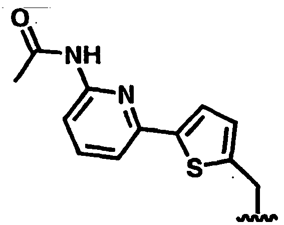 | F | 932 | Selected data: (205.1, 204.9), 184.0, 168.7, 154.1, 150.8, 144.2, 139.0, 124.1, 111.6, 103.9, 79.3, 73.4, 71.1, 70.4, 69.6, 65.8, 62.3, 41.0, 40.2, 29.7, 28.2, 25.1, 22.9, 21.2, 17.2, 14.9, 14.0, 12.3. |
| Example 175. | Ac | 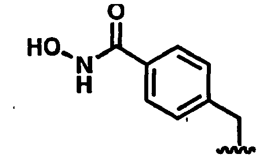 | H | 831 | N/A |
| Example 176. | Ac | 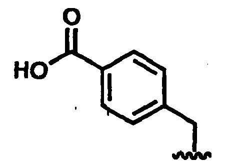 | H | 818 | 205.9, 184.8, 178.1, 173.2, 167.8, 153.6, 141.9, 129.9, 127.4, 102.5, 79.4, 79.1, 76.8, 75.7, 74.7, 70.1, 68.8, 66.3, 63.1, 62.9, 50.7, 46.1, 39.9, 38.8, 29.9, 29.6, 25.4, 23.8, 21.4, 20.3, 19.5, 17.8, 15.1, 14.2, 13.6, 12.9. |
| Example 177. | Ac | 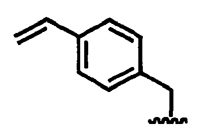 | H | 800 | 205.8, 184.7, 178.0, 167.8, 153.4, 137.8, 136.9, 128.5, 126.4, 113.9, 103.0, 79.4, 76.7, 75.9, 74.6, 70.5, 69.7,66.0,63.1,50.8, 40.5, 38.8, 28.5, 25.3, 23.8,21.5,20.3,19.5, 17.8,15.1,14.1,13.7, 12.9 |
| Example 178. | Ac | 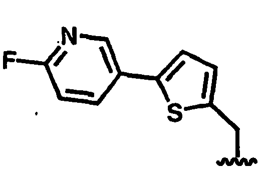 | H | 876 | 205.8, 184.7, 178.0, 167.8, 164.0, 162.1, 154.1, 144.7, 144.6,141.6,139.7, 138.7, 138.6, 128.2, 124.1, 110.0, 109.7, 103.1, 79.4, 79.3, 76.8, 75.9, 74.5, 70.7, 70.5, 69.8, 66.1, 63.1, 62.9, 50.7, 46.3, 40.5, 38.8, 28.5, 25.4, 23.8, 21.5, 20.3, 19.5, 17.8, 15.1, 14.1, 13.7, 13.0. |
| Example 179. | Ac | 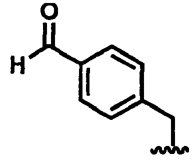 | H | 801 | N/A |
| Example 180. | H | 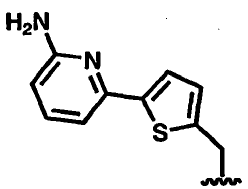 | H | 830 | 205.2, 170.0, 169.2, 158.3, 154.1, 151.2, 145.9, 141.7, 138.4, 127.9, 124.0, 109.3, 107.1, 103.6, 80.5, 79.0, 78.8, 75.8, 71.1, 70.6, 69.8, 66.1, 64.7, 63.0, 51.7, 48.1, 40.5, 38.3, 28.4, 23.0, 21.5, 21.1, 20.2, 17.4, 16.0, 14.4, 14.0, 11.9. |
| Example 181. | Ac | 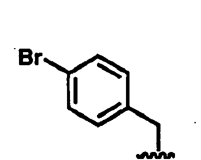 | H | 852, 854 | 205.4, 184.5, 177.6, 169.8, 167.3,153.4, 136.9, 131.3, 129.6, 121.5, 100.4, 79.2, 76.4, 75.1, 74.3, 71.5, 69.0, 63.3, 62.8, 50.4, 40.6, 31.6, 30.6, 25.1, 23.5, 22.6, 21.0, 175, 14.8, 14.1, 13.7, 12.6. |
| Example 182. | Ac | 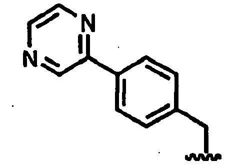 | H | 852 | N/A |
| Example 183. | Ac | 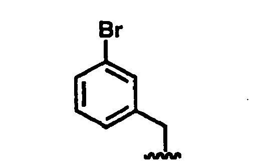 | H | 895, 897 | 205.5, 184.5, 177.7, 169.9, 168.5, 167.5, 153.5, 140.3, 130.8, 130.7, 130.6, 130.0, 129.8, 126.4, 123.2, 102.8, 101.8, 79.0, 76.5, 75.0, 74.9, 74.4, 70.2, 69.5, 65.8, 62.8, 50.5, 47.4, 45.9, 44.2, 40.3, 40.2, 38.5, 34.9. 33.3, 30.8, 28.2, 25.1, 23.5, 21.2, 17.5, 14.8, 14.5, 13.8, 12.5. |
| Example 184. | Ac | 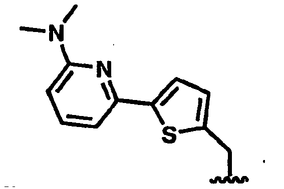 | F | 918 | N/A |
| Example 185. | Ac | 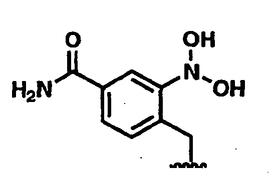 | H | 862 | 205.9, 184.9, 177.5, 168.1, 167.0, 155.1, 147.4, 139.1, 133.6, 132.4, 129.9, 123.9, 102.8, 795, 79.2, 76.7, 75.7, 72.6, 70.4, 69.5, 66.2, 63.2, 62.9, 60.7, 50.8, 46.3, 40.5, 38.7, 29.9, 28.9, 25.4, 23.8, 21.4, 20.3, 19.7, 17.7, 15.2, 14.1, 12.8. |
| Example 186. | Ac | 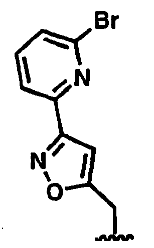 | H | 921 | 205.53, 184.45, 177.74, 169.96, 167.57, 162.00, 154.51, 149.41, 141.94, 139.01, 128.79, 120.30, 102.58, 102.5, 79.19, 78.94, 76.49, 74.40, 70.08, 69.15, 66.52, 66.12, 62.72, 62.41, 50.42, 45.81, 40.26, 38.50, 28.87, 25.10, 23.62, 21.15, 19.95, 19.18, 17.52, 14.85, 13.86, 12.75 |
| Example 187. | Ac | 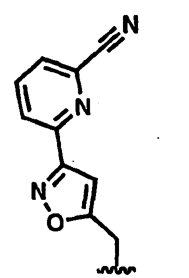 | H | 867 | 205.48, 184.43, 177.67, 170.41, 167.61, 61.75, 154.70, 150.29, 137.87, 133.77, 128.81, 124.82, 102.70, 102.48, 79.13, 79.00, 76.47, 74.41, 70.15, 69.35, 66.43, 65.91, 62.69, 62.42, 50.40, 45.90, 40.21, 38.46, 30.89, 29.23, 28.44, 25.08, 23.59, 21.18, 19.93, 19.20, 17.49, 14.81, 13.80, 13.31, 12.74 |
| Example 188. | Ac | 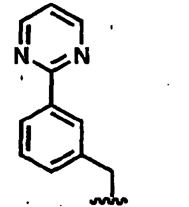 | H | 851 | N/A |
| Example 189. | Ac | 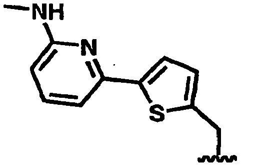 | F | 904 | N/A |
| Example 190. | Ac | 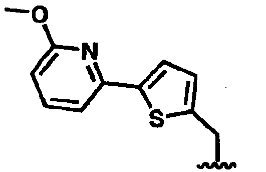 | H | 887 | 205.9, 184.7, 178.1, 167.8, 163.7, 153.8, 150.3, 145.6, 142.2, 139.2, 127.9, 124.1, 111.3, 109.1, 103.0, 79.4, 76.8, 74.5, 71.1, 70.4, 69.6, 66.3, 63.1, 62.9, 53.6, 50.8, 46.1, 40.5, 38.8, 30.0, 28.8, 25.4, 23.8, 21.5, 20.3, 19.5, 17.8, 15.1, 14.2, 13.6, 12.9. |
| Example 191. | H | 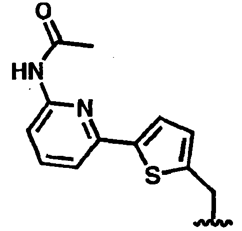 | H | 872 | 205.2, 187.9, 169.3, 169.0, 154.5, 151.0, 144.7, 142.9, 139.2, 127.5, 124.5, 114.7, 112.0, 103.6, 80.6, 78.8, 77.8, 76.0,71.1,70.6,69.8,66.1, 64.7, 63.0, 51.6, 48.1, 41.7, 40.5, 38.3, 35.3, 30.0, 28.5, 25.0, 23.0, 21.5, 21.2, 20.2, 17.3, 16.0, 14.4, 14.1, 11.9. |
| Example 192. | Ac | 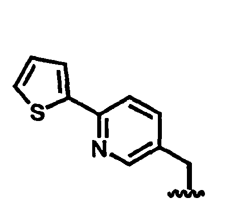 | H | 857 | 205.8, 184.7, 178.0, 167.8, 154.0, 152.2, 149.7, 145.0, 137.0, 131.8, 128.2, 127.7, 124.7, 118.6, 103.1, 79.4, 74.6, 73.5, 70.5, 69.8, 66.1, 63.1, 62.8, 50.7, 40.5, 28.5, 25.4, 23.8, 21.5, 19.5, 17.8, 15.1, 14.1, 13.0 |
| Example 193. | Ac | 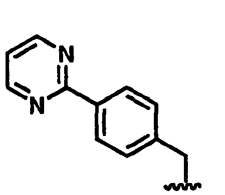 | H | 852 | 205.8, 184.7, 178.1, 167.8, 164.9, 157.5, 153.6, 141.0, 137.1, 128.3, 119.2, 103.0, 79.4, 76.8, 75.8, 74.6, 70.5, 69.8, 66.1, 63.1, 63.0, 50.8, 40.5, 38.8, 285, 25.4, 23.8, 21.5, 19.5, 17.8, 15.1, 14.1, 12.9 |
| Example 194. | H | 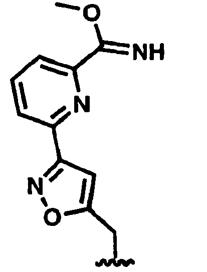 | H | 857 | 204.82, 169.43, 168.98, 166.28, 162.73, 148.03, 147.32, 138.23, 123.11, 121.60, 103.20, 102.74, 80.34, 78.59, 75.61, 70.22, 69.43, 66.51, 65.98, 62.66, 53.92, 51.34, 47.70, 40.25, 28.40, 22.74, 21.18, 20.88, 19.81, 17.10, 14.09, 13.75, 11.63 |
| Example 195. | Ac | 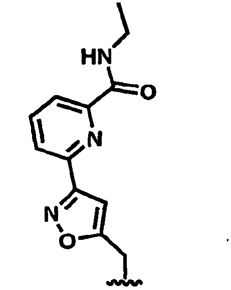 | H | 913 | 205.66, 184.49, 177.58, 169.88, 167.64, 162.38, 154.66, 150.06, 147.03, 138.27, 124.01, 122.92, 102.37, 79.06, 76.46, 74.36, 70.13, 69.34, 66.40, 65.94, 62.63, 62.44, 50.47, 46.02, 40.22, 38.45, 35.21, 34.34, 28.51, 25.06, 23.49, 21.15, 19.94, 19.21, 17.46, 15.47, 14.95, 14.80, 13.79, 12.62 |
| Example 196. | H | 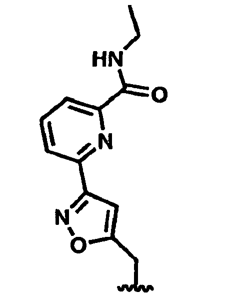 | H | 871 | 204.90, 169.61, 168.96, 163.60, 162.46, 154.94, 150.06, 146.95, 138.29, 123.97, 122.96, 103.31,102.46, 80.35, 78.54, 75.61, 70.24, 69.52, 66.45, 65.86, 62.51, 51 35, 47.82, 40.21, 34.33, 28.17, 22.72, 21.16, 20.82, 19.84, 17.09, 15.72, 14.96, 14.06, 13.63, 11.63 |
| Example 197. | H | 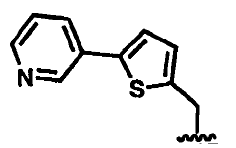 | H | 816 | 205.1, 169.3, 154.3, 148.8, 148.7, 147.1, 141.4, 141.3, 133.1, 130.7, 128.5, 128.4, 124.1, 123.9, 123.8, 103.5, 80.6, 78.8, 75.9, 70.8, 70.6, 69.8, 66.2, 63.0, 51.7, 48.1, 40.5, 38.3, 28.5, 23.0, 21.5, 21.2, 20.1, 17.4, 16.0, 14.4, 14.0, 11.9. |
| Example 198. | H | 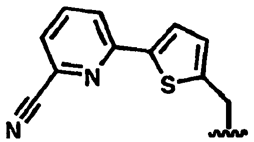 | H | | N/A |
| Example 199. | Ac | 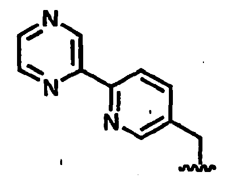 | H | 853 | 205.8, 184.7, 178.0, 167.9, 154.2, 153.7, 151.3, 149.5, 144.6, 143.8, 143.6, 137.1, 134.6, 121.3, 103.1, 79.4, 76.8, 74.7, 73.4, 70.5, 69.8, 66.1, 63.1, 62.8, 50.7, 463, 40.5, 38.8, 28.5, 25.4, 23.8, 21.5, 20.3, 19.5, 17.8, 15.1, 14.1, 13.7, 13.0. |
| Example 200. | Ac | 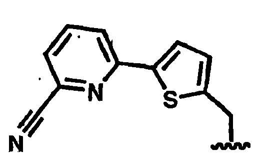 | H | 882 | 205.9, 184.6, 176.5, 169.4, 156.6, 154.6, 144.7, 142.9, 137.7, 133.8, 128.4, 126.5, 126.4, 122.2, 117.5, 102.5, 79.7, 79.1, 76.2, 75.5, 70.9, 70.4, 69.6, 66.4, 58.5, 50.9, 45.4, 40.6, 40.0, 39.1, 36.8, 30.0, 29.0, 25.6, 23.4, 21.6, 20.2, 19.8, 17.3, 15.7, 14.5, 12.9, 12.0. |
| Example 201. | Ac | 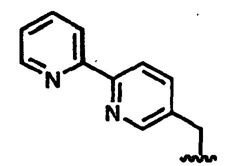 | H | 852 | 205.8, 184.7, 178.0, 167.8 156.3, 155.8, 154.0, 149.4, 149.2, 137.1, 133.7, 123.9, 121.3, 120.9, 103.1, 79.4, 76.8, 74.6, 73.6, 70.5, 69.8, 66.1, 63.1, 50.7, 40.5, 28.5, 25.4, 23.8, 21.5, 19.5, 17.8, 15.1, 14.1, 3.0 |
| Example 202. | H | 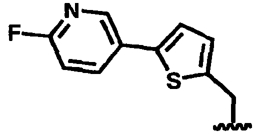 | H | 833 | N/A |
| Example 203. | H | 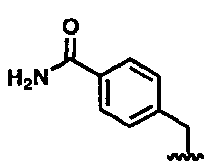 | H | 775 | 205.2, 188.5, 169.5, 169.4, 154.2, 142.1, 133.0, 128.2, 127.8, 103.5, 805, 78.8, 78.6, 77.9, 75.9, 75.6, 70.6, 69.8, 66.1, 64.7, 63.0, 51.6, 48.0, 40.5, 31.2, 285, 23.0, 21.4, 21.1, 20.0, 17.4, 15.9, 14.3, 14.0, 11.8 |
| Example 204. | H | 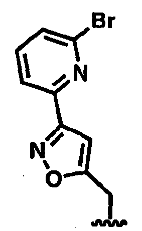 | H | 880 | 204.84, 169.57, 169.01, 162.08, 154.68, 149.35, 141.96, 139.04, 128.85, 120.37, 103.17, 102.78, 80.34, 78.60, 75.60, 70.23, 69.44, 66.54, 65.99, 64.25, 62.67, 51.34, 47.68, 40.25, 38.08, 35.06, 28.41, 22.73, 21.18, 20.87, 19.80, 17.09, 1557, 14.09, 13.78, 12.63, 11.62 |
| Example 205. | Ac | 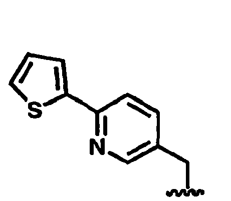 | H | 858 | 205.8, 184.7, 178.0, 169.5, 167.9, 154.2, 151.0, 149.5, 144.3, 137.2, 134.7, 121.5, 119.5, 103.1, 79.4, 79.2, 76.8, 74.7, 73.4, 70.5, 69.8, 66.1, 63.1, 62.8, 50.7, 46.3, 40.5, 38.8, 28.5, 25.4, 23.9, 21.5, 20.3, 19.5, 17.8, 15.1, 14.1, 13.7, 13.0. |
| Example 206. | H | 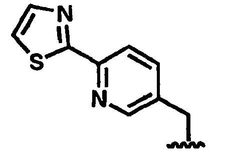 | H | 816 | N/A |
| Example 207. | Ac | 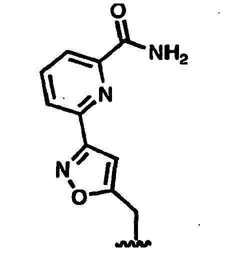 | H | 885 | 205.68, 184.54, 177.56, 170.00, 167.59, 162.28, 154.63, 149.44, 147.27, 138.27, 124.27, 123.10, 102.25, 101.99, 79.11, 78.92, 76.41, 74.36, 69.71, 68.49, 66.43, 66.06, 62.61, 62.36, 50.41, 40.29, 38.37, 30.01, 25.05, 23.48, 20.94, 19.89, 19.14, 17.44, 14.76, 13.86, 12.63 |
| Example 208. | Ac | 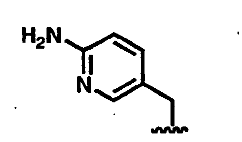 | H | 790 | 203.7, 182.6, 175.9, 165.6, 156.3, 151.3, 146.7, 137.0, 121.1, 106.3, 101.0, 77.2, 77.0, 74.6, 73.5, 72.5, 71.7, 68.4, 67.6, 63.9, 61.0, 60.6, 48.6, 44.1, 38.3, 36.7, 35.2, 26.4, 23.2, 21.6, 19.4, 18.2, 17.4, 15.7, 12.9, 12.0, 11.5, 10.7. |
| Example 209. | Ac | 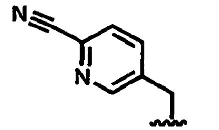 | H | 800 | 205.7, 184.7, 177.8, 168.0, 154.9, 150.8, 137.9, 136.5, 133.0, 128.3, 117.5, 103.1, 79.3, 76.7, 72.6, 50.6, 46.4, 40.5,38.7. |
| Example 210. | Ac | 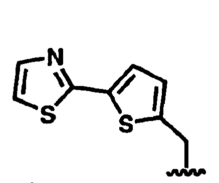 | H | 863 | 205.5, 184.4, 177.8, 167.5, 162.0, 153.8, 143.2, 143.0, 137.4, 127.1, 126.1, 117.9, 102.5, 79.2, 78.9, 76.5, 74.3, 70.4, 70.1, 69.2, 66.0, 62.8, 62.6, 50.4, 45.8, 40.2, 38.5, 28.8, 25.1, 23.6, 21.1, 20.0, 19.2, 17.5, 14.8, 13.9, 13.3, 12.7. |
| Example 211. | Ac | 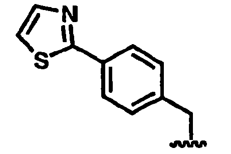 | F | 875 | Selected data: (205.1, 204.9), 184.0, 168.2, 164.7(d), 153.6, 143.7, 139.8, 132.9, 128.7, 128.3, 126.5, 118.7, 103.9, 995, 97.9. |
| Example 212. | H | 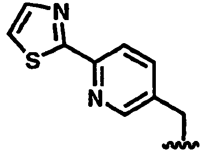 | F | 833 | Selected data: (2043, 204.0), 187.4, 165.8, 153.5, 143.7, 139.6, 133.1, 128.5, 126.6, 118.8, 104.1, 99.0, 97.3. |
| Example 213. | H | 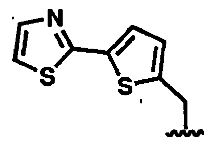 | H | 821 | 204.9, 187.4, 169.0, 162.0, 154.3, 143.3, 142.7, 137.7, 127.4, 126.1, 118.1, 103.4, 80.3, 78.7, 78.6, 75.6, 70.5, 70.3, 69.6, 65.9, 64.4, 62.8, 51.4, 7.8, 40.3, 38.1, 28.2, 22.8, 21.2, 20.9, 19.9, 17.1, 15.7, 14.1, 13.7, 11.7. |
| Example 214. | Ac | 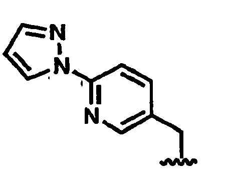 | F | 859 | 205.1, 204.9, 183.9, 176.7, 164.9, 164.7, 154.0, 151.1, 147.8, 142.0, 138.9, 131.0, 127.0, 112.0, 107.1, 103.9, 99.5, 97.9, 79.4, 76.3, 73.4, 73.0, 70.4, 69.6, 65.8, 62.9, 62.4, 41.1, 40.2 38.8, 37.2, 30.9, 28.2, 25.1, 24.4, 24.2, 23.0, 21.2, 20.7, 17.2, 14.8, 14.7, 14.1, 12.5 |
| Example 215. | Ac | 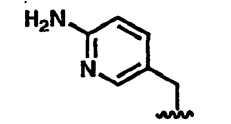 | F | 808 | Selected: 205.3, 205.1, 184.1, 158.4, 153.5, 148.7, 139.0, 123.2, 108.4, 104.2, 99.7, 98.1, 79.6, 76.5, 74.0, 73.6, 70.6, 69.9, 66.0, 63.1, 62.7, 41.3, 40.5, 28.4, 25.3, 24.6, 24.4, 23.2, 21.4, 21.0, 17.4, 15.0, 14.3, 12.7 |
| Example 216. | Ac | 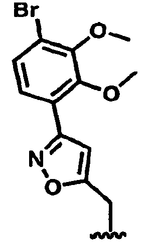 | H | 979 | 205.5, 184.4, 177.7, 168.3, 167.5, 159.0, 154.3, 152.3, 148.8, 128.0, 126.0, 114.5, 113.6, 105.0, 102.7, 79.1, 78.9, 76.4, 74.2, 70.1, 69.4, 66.8, 65.8, 62.6, 62.4, 61.7, 56.0, 50.4, 45.9, 40.1, 38.4, 28.3, 25.0, 23.5, 21.1, 19.9, 19.1, 17.4, 14.7, 13.7, 13.3, 12.8, |
| Example 217. | Ac | 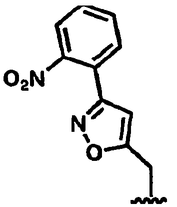 | H | 886 | N/A |
| Example 218. | Ac | 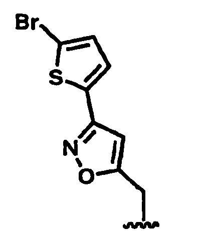 | H | 927 | 205.61, 184.49, 177.64, 169.80, 167.64, 156.83, 154.72, 30.37, 127.62, 114.93, 102.60, 101.03, 79.17, 76.47, 74.47, 70.11, 69.26, 66.47, 66.10, 62.68, 62.43, 53.41, 50.44, 45.90, 40.26, 38.48, 28.70, 25.11, 23.59, 21.16, 19.94, 19.20, 17.50, 14.84, 13.84, 13.31, 12.74 |
| Example 219. | Ac | 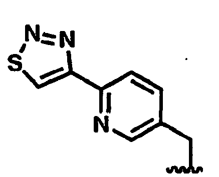 | F | 876 | 204.1, 203.9, 183.0, 161.7, 152.6, 138.2, 129.1, 128.9, 127.5, 126.3, 102.9, 98.5, 96.9, 78.4, 72.4, 69.4, 68.6, 64.8, 61.4, 40.0, 39.2,27.1,24.1,23.4, 23.2, 21.9, 20.2, 19.8, 16.1, 13.7, 13.1, 11.4, |
| Example 220. | Ac | 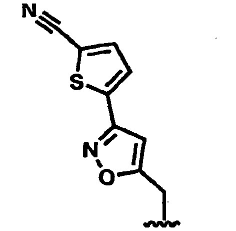 | H | 872 | 205.63, 184.51, 177.58, 170.82, 167.69, 156.01, 155.00, 131.90, 127.11, 113.71, 111.05, 102.55, 101.54, 79.18, 76.46, 74.51, 70.06, 69.18, 66.34, 69.18, 66.34, 62.41, 50.44, 45.93, 40.28, 38.47, 25.12, 23.59, 21.15, 19.94, 19.21, 17.51, 14.84, 13.84, 12.76 |
| Example 221. | Ac | 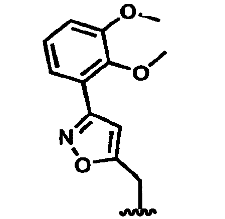 | H | 901 | 205.7, 184.5, 177.8, 168.4, 167.5, 159.8, 154.2, 153.2, 147.5, 124.4, 123.4, 121.0, 113.7, 104.6, 101.9, 79.3, 76.5, 74.4, 69.8, 68.4, 66.6, 60.9, 55.9, 50.5, 45.6, 40.1, 38.5, 34.6, 30.1, 29.7, 25.1, 23.6, 21.0, 19.9, 19.1, 17.5, 14.9, 13.9, 12.8, 6.6, 5.8, |
| Example 222. | Ac | 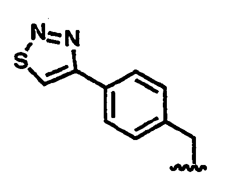 | H | 858 | 204.6, 183.5, 176.7, 166.6, 161.7, 152.4, 138.4, 129.0, 128.9, 127.6, 126.3, 101.8, 78.1, 75.5, 74.4, 73.4, 69.2, 68.5, 64.8, 61.9, 61.7, 49.5, 45.0, 39.2, 37.5, 27.2, 24.1, 22.6, 20.2, 19.0, 18.3, 16.5, 13.9, 12.8, 12.4, 11.7, |
| Example 223. | Ac | 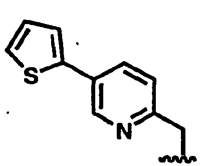 | H | 857 | 205.4, 184.4, 177.6, 169.7, 167.3, 153.1, 144.2, 137.1, 133.7, 132.0, 128.4, 127.9, 125.8, 124.6, 123.0, 100.3, 79.2, 76.4, 75.5, 74.3, 71.5, 69.0, 63.3, 62.8, 50.4, 40.6, 38.3, 30.5, 25.0, 23.5, 22.6, 21.3, 21.0, 17.4, 14.8, 14.1, 13.7, 12.6. |
| Example 224. | Ac | 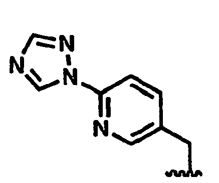 | H | 842 | 205.8, 184.7, 177.9, 167.9, 054.3, 153.0, 149.1, 148.5, 141.8, 139.6, 133.3, 112.8, 103.1, 79.3, 79.2, 76.8, 74.7, 72,9, 70.5, 69.8, 66.1, 63.1, 62.8, 50.7, 46.3, 40.5, 38.7, 28.5, 25.4, 23.8, 21.5, 20.2, 19.5, 17.8, 15.1, 14.1, 13.7, 13.0 |
| Example 225. | Ac | 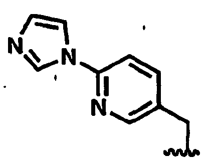 | H | 841 | 205.8, 184.7, 177.9, 167.9, 154.3, 149.3, 148.8, 139.5, 135.2, 132.2, 130.9, 116.4, 112.1, 103.1, 79.3, 79.2, 76.8, 75.6, 74.7, 72.8. 70.5, 69.8, 66.1, 63.1, 62.8, 50.7, 46.3, 40.5, 38.7, 37.2, 28.5, 25.4, 23.8, 21.5, 20.2, 19.5, 17.8, 15.1, 14.1, 13.7, 13.0. |
| Example 226. | Ac | 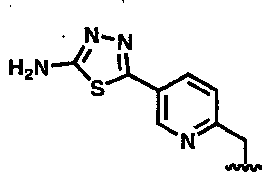 | H | 873 | 205.61, 184.52, 177.70, 167.61, 159.73, 153.56, 140.14, 129.94, 128.25, 126.88, 102.69, 79.11, 78.95, 75.21, 74.47, 70.18, 69.35, 65.88, 62.80, 62.67, 50.80, 50.52, 46.03, 40.21, 38.61, 28.47, 25.10, 23.54, 21.17, 20.04, 19.28, 17.52, 14.84, 13.83, 13.47, 12.62 |
| Example 227. | Ac | 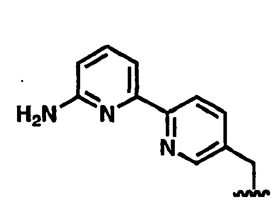 | H | 867 | 205.8, 184.7, 178.0, 167.8, 158.2, 156.1, 154.8, 153.9, 149.2, 138.8, 136.9, 133.2, 120.8, 111.9, 109.0, 103.1, 79.4, 79.2, 76.8, 75.9, 74.6, 73.6, 70.5, 69.8, 66.1, 63.1, 62.8, 50.7, 46.2, 40.5, 38.8, 28.5, 25.4, 23.8, 21.5, 20.3, 19.5, 17.8, 15.1, 14.1, 13.7, 13.0. |
| Example 228. | Ac | 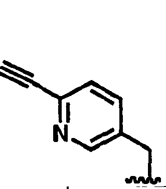 | H | 799 | 205.8, 184.7, 177.9, 167.8, 154.2, 150.0, 141.7, 136.2, 133.6, 127.2, 103.1, 83.0, 79.3, 76.7, 74.7, 73.2, 70.5, 69.8, 66.1, 63.0, 62.8, 50.7, 46.3, 40.5, 38.8, 28.5, 25.4, 23.8, 21.5, 20.2, 19.5, 17.8, 15.1, 14.1, 13.7, 13.0. |
| Example 229. | H | 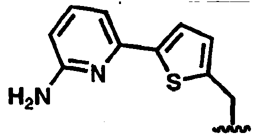 | F | 849 | N/A |
| Example 230. | H | 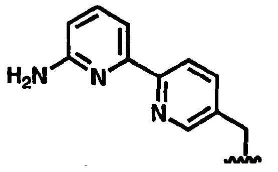 | H | 825 | N/A |
| Example 231. | Ac | 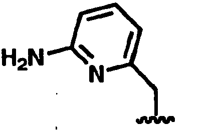 | H | 790 | N/A |
| Example 232. | Ac | 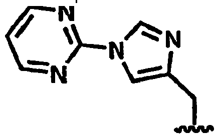 | H | 842 | N/A |
| Example 233. | Ac | 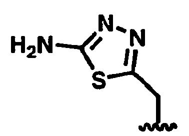 | H | 797 | 205.60, 184.52, 177.70, 169.21, 167.48, 157.86, 154.78, 102.77, 79.04, 76.44, 74.37, 70.21, 70.17, 69.36, 65.87, 62.65, 62.46, 50.47, 46.05, 40.20, 38.26, 29.66, 28.47, 25.10, 23.52, 21.20, 20.00, 19.26, 17.49, 14.81, 13.83, 13.44, 12.70 |
| Example 234. | Ac | 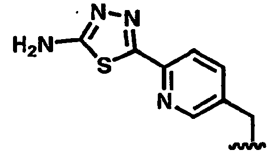 | H | 874 | 205.64, 184.49, 177.63, 167.60, 153.94, 149.06, 148.90, 136.74, 134.40, 119.56, 109.74, 102.27, 79.18, 76.50, 74.48, 72.98, 69.95, 68.88, 66.44, 62.81, 62.52, 50.45, 45.77, 40.33, 38.53, 25.13, 23.58, 21.08, 19.96, 19.20, 17.55, 14.86, 13.90, 13.28, 12.71 |
| Example 235. | Ac | 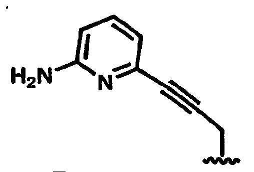 | H | 814 | 205.8, 184.8, 178.1, 167.7, 158.4, 154.1, 140.9, 137.9, 117.9, 108.9, 103.0, 85.8, 84.4, 79.6, 79.2, 76.8, 74.5, 70.5, 69.7, 66.1, 63.0, 62.8, 62.5, 50.7, 46.1, 40.5, 38.8, 28.5, 25.4, 24.0, 21.5, 20.3, 19.5, 7.8, 15.1, 14.1, 13.6, 13.1. |
| Example 236. | Ac | 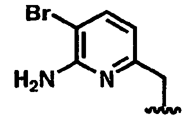 | H | 868 | N/A |
| Example 237. | Ac | 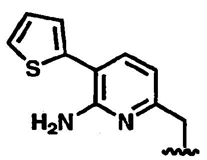 | H | 872 | 205.8, 184.8, 178.0, 167.7, 156.7, 155.6, 153.8, 140.1, 138.9, 128.0, 126.1, 125.8, 113.6, 112.4, 102.9, 79.3, 79.2, 76.7, 76.5, 75.0, 70.5, 69.7, 66.1, 63.1, 50.8, 46.1, 40.5, 38.8, 28.5, 25.4, 23.8, 21.5, 20.3, 19.6, 17.7, 15.2, 14.2, 13.6, 12.8. |
| Example 238. | Ac | 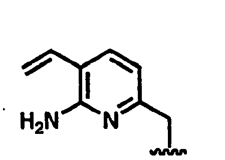 | H | 816 | 205.9, 184.8, 178.0, 167.7, 156.3, 155.4, 153.7, 135.6, 32.0, 17.4, 116.9, 112.9, 110.0, 102.9, 79.3, 79.2, 76.7, 76.6, 74.9, 70.5, 69.7, 66.1, 63.1, 50.8, 46.1, 40.5, 38.8, 28.5, 25.4, 23.8, 21.5, 20.3, 19.6,17.7, 15.2, 14.2, 13.6, 12.8. |
| Example 239. | Ac | 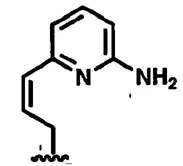 | H | 816 | N/A |
| Example 240. | Ac | 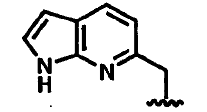 | H | 814 | N/A |
| Example 241. | Ac | 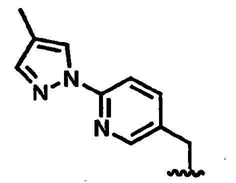 | H | 855 | 205.8, 184.7, 178.0, 167.8, 153.9, 151.4, 148.1, 143.2,, 139.2, 130.8, 125.8, 118.6, 11.8, 103.1, 79.3, 79.2, 77.6, 77.5, 77.3, 77.0, 76.8, 74.6, 73.2, 70.5, 69.7, 66.1, 63.1, 62.8, 50.7, 46.2, 40.5, 38.8, 28.5, 25.4, 23.8, 21.5, 20.3, 19.5, 17.8, 15.1, 14.1, 13.6, 13.0,9.3. |
| Example 242. | Ac | 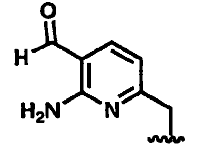 | H | 818 | N/A |
| Example 243. | Ac | 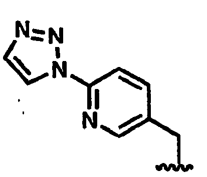 | H | 842 | 205.6, 184.4, 177.6, 167.6, 154.1, 148.6, 148.2, 139.2, 134.1, 133.7, 121.0, 113.4, 102.7, 79.1, 76.5, 74.4, 72.6, 70.1, 69.3, 66.0, 62.8, 62.5, 50.4, 46.0, 40.2, 38.5, 31.5, 28.6, 25.1, 23.5, 22.6, 21.2, 19.9, 19.2, 17.5, 14.8, 14.1, 13.8, 13.4, 12.7. |
| Example 244. | Ac | 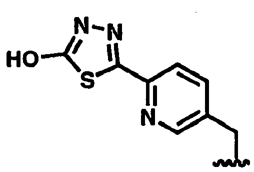 | H | 874 | 205.60, 184.57, 177.70, 171.97, 167.62, 153.60, 153.04, 141.16, 129.92, 128.23, 125.88, 102.60,79.15, 78.91, 76.49, 75.09, 7455, 70.17, 69.28, 65.94, 62.82, 62.68, 50.48, 45.92, 40.21, 38.51, 28.63, 25.12, 2355, 21.17, 20.00, 19.26, 1752, 14.88, 13.86, 13.37, 12.65 |
| Example 245. | Ac | 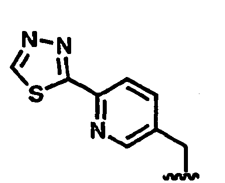 | H | 858 | 205.58, 184.50, 177.67, 168.20, 167.57, 153.61, 151.03, 141.67, 128.46, 128.08, 102.60, 79.14, 78.90, 76.48, 75.11, 74.45, 70.14, 69.29, 66.02, 62.82, 62.65, 50.47, 40.25, 38.52, 28.60, 25.11, 23.56, 21.17, 19.99, 19.24, 17.51, 14.86, 13.85, 13.35, 12.67 |
| Example 246. | Ac | 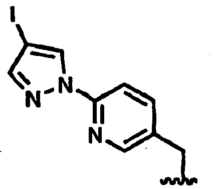 | H | 967 | 205.6, 184.5, 177.7, 167.6, 153.8, 150.2, 147.9, 146.6, 139.1, 131.7, 131.6, 111.5, 102.6, 79.1, 76.5, 74.4, 72.8, 70.1, 69.2, 66.1, 62.8, 625, 59.6, 50.4, 45.9, 40.3, 38.5, 31.6, 28.7, 25.1, 23.5, 22.6, 21.2, 19.9, 19.2, 17.5, 14.9, 14.1, 13.8, 13.4, 12.7, |
| Example 247. | Ac | 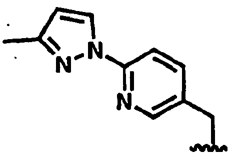 | H | 855 | 205.4, 184.3, 176.7, 167.5, 153.6, 151.5, 151.0, 147.8, 138.8, 130.3, 127.6, 111.5, 107.8, 102.7, 79.0, 76.4, 74.3, 72.9, 70.1, 69.4, 65.8, 62.8, 62.4, 50.3, 45.9, 40.1, 38.4, 31.5, 282, 25.1, 23.5, 22.5, 21.1, 19.9, 19.2, 17.5, 14.8, 14.0, 13.8, 13.7, 13.3, 12.6, |
| Example 248. | Ac | 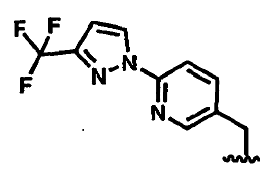 | H | 909 | 205.6, 184.5, 177.6, 167.6, 153.9, 150.2, 147.9, 139.1, 132.5, 128.4, 112.4, 105.8, 102.6, 79.1, 78.9, 76.5, 75.4, 74.4, 72.7, 70.1, 69.2, 66.1, 62.8, 625, 50.4, 45.9, 40.2, 38.5, 31.6, 28.8, 25.1, 23.5, 22.6, 21.2, 20.0, 19.2, 17.5, 14.8, 14.1, 13.9, 13.3, 12.7, |
| Example 249. | Ac | 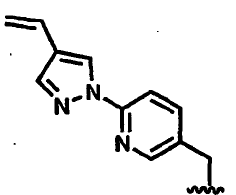 | H | 867 | 205.6, 184.5, 177.7, 167.5, 153.7, 150.8, 147.9, 139.9, 139.0, 131.0, 126.4, 124.3, 122.8, 113.4, 111.8, 102.3, 79.1, 76.5, 74.4, 72.9, 69.9, 68.9, 66.3, 62.8, 62.5, 50.4, 45.8, 40.3, 38.5, 31.6, 29.3, 25.1, 235, 22.6, 21.1, 20.0, 19.2, 17.5, 14.8, 14.1, 13.9, 13.3,12.7, |
| Example 250. | Ac | 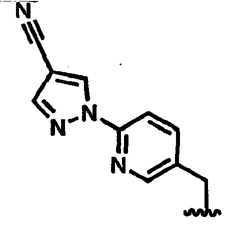 | H | 866 | 205.6, 184.5, 177.6, 167.6, 154.0, 149.5, 148.2, 1435, 139.3, 133.1, 132.1, 113.1, 112.5, 102.3, 94.4, 79,1, 76.5, 74.5, 72.6, 69.9, 68.9, 66.3, 62.8, 62.5, 50.4, 45.9, 40.3, 38.5, 31.6, 29.3, 25.1, 23.5, 22.6, 21.1, 19.9, 19.2, 17.5, 14.8, 13.9, 13.3, 12.7, |
| Example 251. | Ac | 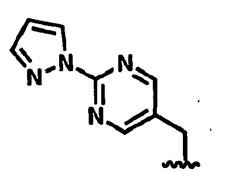 | H | 842 | 205.7, 184.7, 177.9, 167.9, 159.4, 155.8, 154.6, 143.8, 129.4, 128.5, 108.8, 103.1, 79.3, 79.2, 76.8, 74.7, 70.7, 70.5, 69.7, 66.2, 63.0, 62.7, 60.6, 50.7, 46.3, 40.5, 38.7, 28.7, 25.4, 23.8, 21.5, 21.3, 20.2,19.5, 17.8, 15.1, 14.5, 14.1, 13.7, 13.1. |
| Example 252. | Ac | 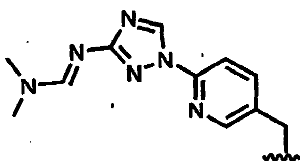 | H | 912 | 205.8, 184.7, 178.0, 168.3, 167.9, 157.5, 154.1, 149.3, 148.2, 140.9, 139.6, 132.0, 112.3, 103.1, 79.3, 76.8, 74,7, 73.1, 70.5, 69.7, 66.1, 63,1, 62.8, 54.0, 50.7, 46.3, 41.0, 40.5, 38.8, 34.9, 29.5, 28.5, 25.4, 23.8, 21.5, 20.3, 19.5, 17.8,15.1, 14.1, 13.7, 13.0. |
| Example 253. | Ac | 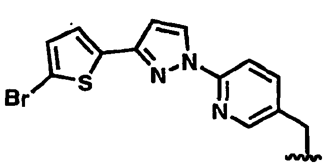 | H | 1003 | 205.85, 184.53, 177.72, 167.64, 153.73, 150.67, 148.09, 147.87, 138.99, 138.83,137.85,131.16, 130.27, 128.38, 12457, 112.18, 111.95, 104.81, 102.08, 79.16, 78.7876.48, 76.24, 74.51, 72.90, 71.97, 67.71, 62.76, 62.48, 59.22, 52.00, 50.40, 45.82, 38.50, 34.91, 25.12, 23.55, 20.97; 20.69, 19.89, 19.15, 17.56, 14.87, 13.74, 13.71, 13.21, 12.69 |
| Example 254. | Ac | 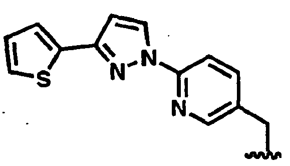 | H | 923 | 205.84, 184.52, 177.73, 167.64, 153.70, 150.81, 148.92, 147.86, 138.93, 136.20, 130.95, 128.25, 127.46, 125.20, 124.60, 111.98, 105.28, 102 08, 79.15, 78.77, 76.48, 76.22, 74.50, 72.93, 71.98, 67.71, 62.76, 62.47, 59.30, 51.91, 5039, 45.81, 38.50, 34.93, 25.12, 23.54, 20.97, 19.88, 19.15, 17.56, 14.86, 13.73, 13.21, 12.68 |
| Example 255. | Ac | 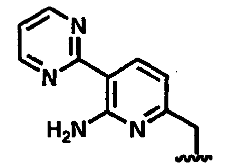 | H | 868 | Selected data: 206.1, 184.9, 177.9, 167.7, 153.9, 143.5, 142.1, 141.7, 137.0, 111.3, 102.0. |
| Example 256. | H | 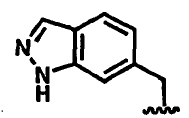 | H | 772 | 205.2, 169.5, 154.0, 140.4, 136.5, 134.3, 123.0, 121.1, 120.9, 109.9, 103.4, 80.4, 78.8, 78.3, 76.6, 75.9, 70.5, 69.7, 66.1, 64.9, 63.1, 51.6, 47.8, 41.2, 40.5, 38.3, 35.3, 28.5, 23.0, 21.4, 21.2, 19.9, 17.4, 15.7, 14.4, 14.1, 11.9. |
| Example 257. | Ac | 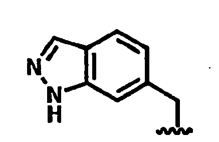 | H | 814 | 205.9, 184.8, 178.0, 167.9, 153.7, 140.4, 137.3, 134.8, 122.9, 121.5, 120.8, 109.1, 102.9, 79.4, 79.2, 76.8, 76.3, 74.9, 70.4, 69.6, 66.2, 63.1, 63.0, 50.8, 46.2, 40.5, 38.8, 28.9, 25.4, 23.8, 21.4, 20.3, 19.6, 17.9, 15.2, 14.2, 13.7, 12.9. |
| Example 258. | Ac | 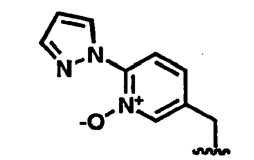 | H | 857 | 205.7, 184.7, 178.0, 167.9, 154.9, 142.4, 140.1, 133.6, 133.1, 127.2, 119.7, 107.5, 103.1, 79.3, 76.8, 74.7, 72.0. 705, 69.8, 66.1, 63.0, 62.7, 53.7, 50.7, 46.3, 40.5, 38.7, 28.5, 25.4, 23.8, 21.5, 20.2, 19.5, 17.8, 15.1, 14.0, 13.7, 13.0. |
| Example 259. | Ac | 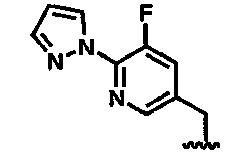 | H | 859 | N/A |
| Example 260. | -COCH₂C H₃ | 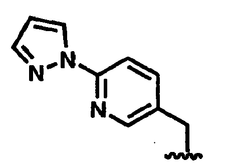 | H | 855 | 205.5, 187.7, 178.1, 167.5, 153.8, 151.1, 147.9, 141.9, 139.0, 131.1, 127.0, 111.9, 107.6, 102.9, 79.1, 76.5, 74.3, 72.9, 70.2, 69.5, 65.8, 62.8, 62.5, 50.4, 46.0, 40.2, 38.6, 31.6, 31.0, 28.2, 23.5, 22.6, 21.2, 20.1, 19.2, 17.5, 14.9, 14.1, 13.8, 13.4, 12.7, 8.6 |
| Example 261. | Ac | 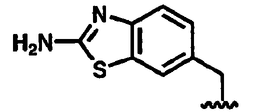 | H | 846 | N/A |
| Example 262. | Ac | 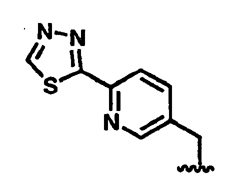 | H | 859 | 205.50, 184.42, 177.59, 170.17, 167.61, 154.15, 153.29, 149.42, 147.88, 135.95, 135.78, 120.77, 102.71, 79.07, 78.93, 76.47, 74.43, 72.79, 70.14, 69.34, 65.87, 62.73, 62.50, 50.39, 45.98, 40.17, 38.45, 29.64, 28.43, 25.08, 23.53, 21.16, 19.93, 19.21, 17.49, 14.79, 13.78, 13.37, 12.71 |

Example compounds 263-337 of formula **A2**: 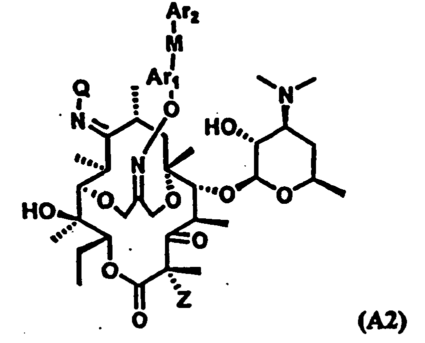 wherein Ar₁, Ar₂, M, Q, and Z are as delineated for each example in Table **A2.**

Example compounds 263-337, where Z = H, are made from the title compound of Example 3 and the appropriate hydroxylamine of formula Ar₂-M-Ar₁-O-NH₂ via the method delineated in Example 4.

Example compounds 263-337, where Z = F, are made from the title compound of Example 46 and the appropriate hydroxylamine of formula Ar₂-M-Ar₁-O-NH₂ via the method delineated in Example 4.

The Examples described in Table A2 are single isomers of the Z designation, which are separated from the E/Z mixture via silica chromatography or HPLC.

The substituted hydroxylamines used in the following examples are either commercially available or can be made from readily-available starting materials via synthetic methods well known by one of ordinary skill in the art.

**TABLE A2.**

| **Example** | **Q** | **-Ar₁-M-Ar₂** | **Z** | **MS (ESI):** **m/z (M+H)⁺** | **¹³C NMR (125 MHz, CDCl₃): δ** |
|---|---|---|---|---|---|
| Example 263. | Ac | 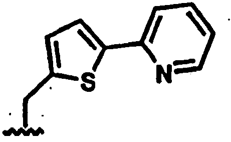 | H | 857 | 205.7, 184.6. 176.6, 167.7, 153.9, 152.8, 149.7, 145.2, 1423, 136.8, 127.8, 124.4, 122.0, 118.5, 102.5, |
| Example 264. | Ac | 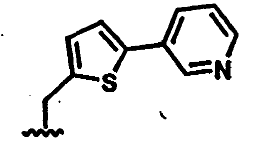 | H | 857 | N/A |
| Example 265. | Ac | 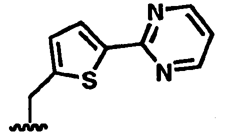 | H | 858 | N/A |
| Example 266. | Ac | 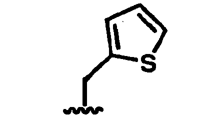 | H | 780 | N/A |
| Example 267. | Ac | 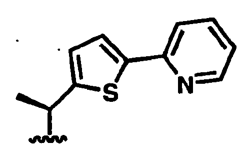 | H | 871 | 205.7, 184.5, 176.2, 169.5, 155.7, 153.0, 149.7, 148.6, 144.0, 136.7, 126.1, 124.4, 121.9, 118.9, 102.2, 79.7, 79.1, 78.7, 76.2, 75.8, 71.5, 70.2, 69.2, 66.7, 59.0, 50.8, 45.3, 40.6, 39.1, 29.9, 25.6, 23.4, 21.5, 20.3, 19.8, 17.1, 15.8, 14.6, 12.7, 11.9. |
| Example 268. | Ac | 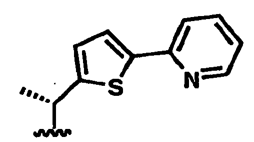 | H | 871 | 205.9, 184.7, 177.2, 169.0, 155.5, 153.0, 149.7, 149.0, 143.9, 136.8, 125.9, 124.4, 121.9, 118.8, 102.4, 79.4, 79.1, 77.7, 76.3, 70.3, 70.0, 69.4, 66.4, 58.0, 50.8, 45.1, 40.6, 39.0, 29.9, 25.4, 23.4, 21.9, 21.5, 20.4, 19.7, 17.3, 15.5, 14.5, 12.8, 12.1. |
| Example 269. | Ac | 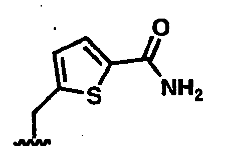 | H | 823 | 206.1, 184.6, 176.5, 169.3, 164.0, 157.0, 146.2, 137.4, 129.9, 127.3, 102.5, 79.7, 79.0, 76.2, 75.2, 70.7, 70.5, 70.3, 69.8, 66.1, 58.4, 54.0, 50.9, 45.4, 40.5, 39.0, 36.8, 32.0, 29.5, 28.5, 25.5, 23.3, 21.6, 20.1, 19.7, 17.2, 15.7, 14.4, 12.9, 12.0 |
| Example 270. | Ac | 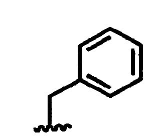 | H | 774 | 205.9, 184.6, 176.7, 169.3, 155.9, 137.9, 128.6, 128.5, 128.0, 102.5, 79.6, 79.0, 76.6, 76.2, 75.4. 70.4, 66.2, 58.4, 50.9, 45.3, 40.5, 39.0, 28.7, 25.5, 23.4, 21.5, 20.2, 19.7, 17.2, 15.6, 14.4, 12.8, 12.0. |
| Example 271. | Ac | 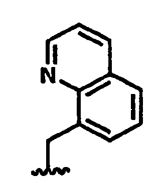 | F | 843 | 204.6, 184.0, 165.8, 157.1, 149.6, 146.2, 136.5, 136.3, 128.4, 128.2, 127.1, 126.8, 121.1, 104.0, 80.0, 79.2, 79.1, 74.4, 72.8, 70.6, 69.9, 68.2, 66.1, 57.0,412, 40.5, 39.5, 28.4, 25.3, 24.4, 22.8, 21.8, 21.5, 21.0, 173, 15.2, 14.1,11.8. |
| Example 272. | Ac | 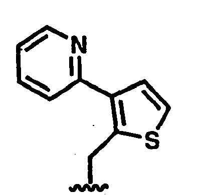 | H | 857 | 205.8, 201.1, 184.6, 176.5, 169.4, 156.4, 1545, 150.1, 149.7, 139.8, 138.1, 136.8, 128.5, 125.3, 123.4, 121.8, 102.6, 79.7, 79.0, 77.8, 76.2, 75.4, 70.8, 705, 70.2, 69.7, 66.1, 58.6, 50.9, 45.4, 40.5, 39.5, 39.0, 36.8, 28.5, 25.5, 23.4, 21.6, 20.2, 19.8, 17.2, 15.7, 14.5, 12.9, 12.0. |
| Example 273. | Ac | 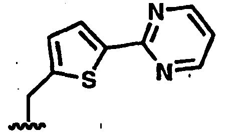 | F | 876 | Selected data: 204.4(d), 184.0, 165.6, 161.8, 157.4, 157.1, 145.1, 143.5, 128.9, 128.4, 103.7. |
| Example 274. | Ac | 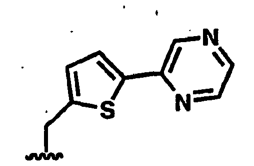 | F | 876 | (204.4, 204.2), 183.8, 176.4, (165.6, 165.4), 157.1, 148.7, 144.0, 143.9, 142.2, 141.4, 140.5, 128.3, 125.7, 103.7, (99.4, 97.8), 80.3, 79.6, 79.0, 75.9, 74.1, 70.6, 70.4, 69.6, 67.9, 65.8, 56.2, 53.8, 41.0, 40.2, 39.0, 29.7,29.3,28.2,25.1, (24.1, 23.9), 22.6, 21.3, 21.2, 20.7, 17.0, 14.9, 14.0, 11.6. |
| Example 275. | Ac | 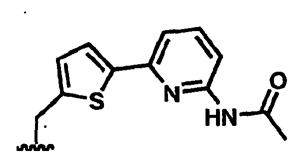 | H | 914 | 206.1, 184.7, 176.5, 169.3, 156.3, 151.1, 151.0, 144.8, 142.6, 139.2, 128.2, 124.6, 114.8, 111.9, 102.0, 79.6, 79.1, 76.2, 75.6, 71.1, 70.6, 70.1, 69.0, 66.9, 58.5, 50.9, 45.2, 40.6, 39.1, 29.9, 25.5, 25.1, 23.4, 21.4, 20.2, 19.7, 17.2, 15.7, 14.6, 12.8, 12.0. |
| Example 276. | Ac | 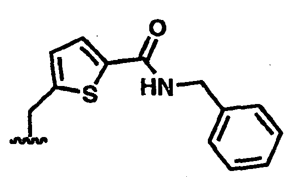 | H | 913 | 205.7, 184.3, 176.2, 169.1, 161.8, 156.6, 145.0, 138.1, 128.7, 128.66, 127.9, 127.6, 127.1, 102.3, 79.4, 78.8, 76.0, 75.0, 70.5, 702, 70.1, 69.4, 65.9, 58.1, 50.7, 45.1, 44.0, 40.2, 39.4, 38.8, 36.6, 29.7, 28.4, 25.2, 23.1, 21.3, 19.9, 19.4, 17.0, 15.4, 14.2, 12.6,11.7 |
| Example 277. | Ac | 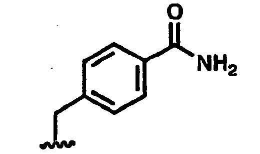 | H | 817 | N/A |
| Example 278. | Ac | 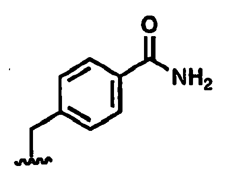 | F | 835 | 204.8, 184.1, 169.5, 165.8 165.7, 157.4, 142,3, 132.7, 128.4, 128.3, 127.6, 104.0, 79.3, 75.7, 70.6, 69.8, 66.1, 56.4, 41.2, 40.5, 28.5, 25.4, 22.8, 21.5, 20.9,17.3, 15.2,14.2, 11.9 |
| Example 279. | Ac | 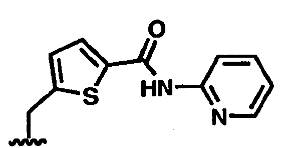 | H | 900 | 205.8, 184.5, 176.4, 169.3, 160.2, 156.9, 151.6, 148.1, 147.1, 138.7, 138.6, 129.2, 127.7, 120.1, 114.1, 102.6, 79.7, 79.0, 762, 75.3, 70.6, 70.5, 70.4, 69.7, 66.1, 58.3, 50.9, 45.4, 41.2, 40.5, 39.6, 39.0, 36.7, 28.5, 25.5, 21.6, 20.1, 9.7, 17.2, 15.7, 14.4, 12.9, 12.0. |
| Example 280. | Ac | 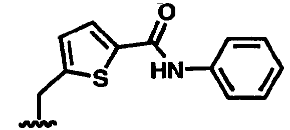 | H | 899 | N/A |
| Example 281. | Ac | 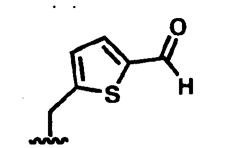 | H | 808 | 205.6, 184.3, 183.0, 176.0, 169.1, 156.9, 151.2, 143.6, 136.2, 127.5, 102.3, 79.5, 78.7, 75.9, 75.1, 70.5, 70.2, 69.5, 65.9, 58.1, 50.6, 45.2, 40.2, 38.8, 28.3, 25.3, 23.1, 21.3, 19.8, 19.4, 16.9, 15.4, 14.2, 12.7, 11.7 |
| Example 282. | Ac | 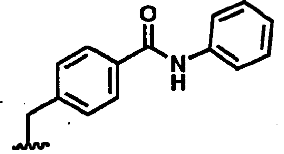 | H | 893 | Selected data: 206.0, 184.6, 176.3, 169.4, 165.9, 156.6, 142.2, 138.3, 134.5, 129.3, 128.5, 127.3, 124.7, 120.4, 102.5, 79.7, 79.1. |
| Example 283. | Ac | 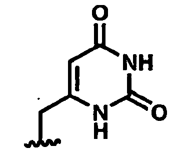 | H | 808 | Selected data: 205.9, 185.9, 174.7, 170.0, 158.7, 151.0, 145.7, 102.5, 98.8. |
| Example 284. | Ac | 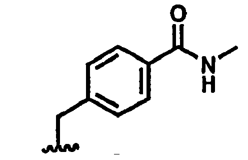 | H | 831 | 205.9, 184.6, 177.5, 169.3, 168.4, 156.5, 141.6, 134.1, 128.3, 127.0, 102.7, 79.7, 76.2, 75.8, 70.5, 69.8, 66.1, 58.4, 50.9, 45.4, 40.5, 39.7, 39.0, 285, 28.5, 27.1, 25.5, 23.4, 21.6, 20.2, 19.7, 17.2, 15.6,14.4, 13.0, 12.0, |
| Example 285. | Ac | 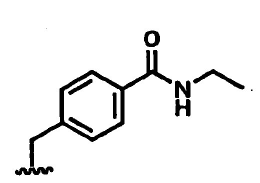 | H | 845 | 205.9, 184.6, 1765, 169.3, 167.6, 156.4, 141.5, 134.3, 128.3, 127.0, 102.6, 79.7, 79.0, 76.2, 75.8, 75.4, 70.5, 69.7, 66.1, 58.4, 50.9, 45.4, 40.5, 39.6, 39.0, 36.8, 35.1, 28.5, 25.5, 23.4, 21.6, 20.2, 19.7, 17.2, 15.6, 15.2, 14.4, 13.0, 12.0 |
| Example 286. | Ac | 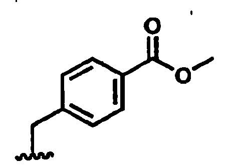 | H | 832 | 204.6, 183.5, 176.7, 166.6, 166.0, 152.6, 142.3, 128.5, 128.2, 126.4, 101.8, 78.1, 78.0, 755, 74.2, 73.4, 69.2, 685, 64.8, 61.8, 61.7,51.0,49.5,45.0, 39.2,37.5,27.3,24.1, 22.6, 20.2, 19.0, 18.3, 16.5, 13.9, 12.8, 12.4, 11.6, |
| Example 287. | Ac | 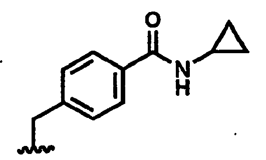 | H | 857 | 205.9, 184.6, 176.5, 169.3, 169.0. 156.5, 141.7, 133.9, 128.3, 127.0, 102.6, 79.7, 79.0,76.2,75.7,75.4, 70.5, 69.7, 66.1, 58.4, 50.9, 45.4, 40.5, 39.0, 28.5, 25.5, 23.4, 21.6, 20.2, 19.7, 17.2, 15.6, 14.4, 13.0, 12.0, 7.0 |
| Example 288. | Ac | 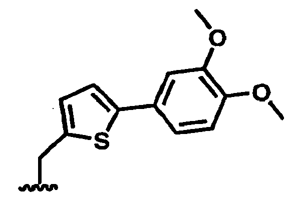 | H | 916 | 205.9, 184.8, 178.1, 167.7, 153.7, 149.3, 148.9, 145.3, 139.2, 128.03, 127.95, 122.0, 118.6, 111.7, 109.6, 102.8, 79.4, 79.1, 76.8, 74.5, 71.0, 70.3, 69.4, 66.4, 63.1, 62.9, 56.21, 56.19, 50.8, 46.1, 40.5, 38.8, 29.9, 29.2, 25.4, 23.8, 21.4, 20.3, 19.5, 17.8, 15.1, 14.2, 13.6, 13.0, |
| Example 289. | Ac | 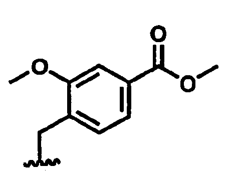 | H | 862 | 205.9, 184.5, 176.5, 169.3, 167.4, 156.7, 156.3, 132.1, 130.3, 128.8, 122.2, 110.8, 102.5, 79.6, 78.9, 77.6, 76.2, 75.3, 71.0, 70.6. 70.4, 69.7, 66.1, 58.6, 55.7, 52.3, 50.8, 45.4, 40.4, 39.6, 39.0, 29.5, 28.5, 25.4, 22.3, 21.5, 20.2, 19.7, 17.2, 15.6, 14.4, 12.9, 11.9 |
| Example 290. | Ac | 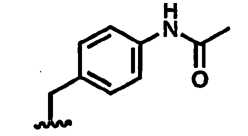 | H | 831 | Selected data: 205.9, 184.6, 176.6, 169.3, 168.4, 155.9, 137.7, 133.8, 129.5, 119.8, 102.4. |
| Example 291. | Ac | 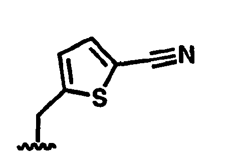 | H | 805 | 205.6, 184.3, 175.9, 169.1, 157.2, 148.4, 137.1, 126.7, 114.4, 109.7, 102.3,79.5, 78.7, 75.9, 75.0, 70.2, 69.9, 69.5, 58.1, 50.6, 45.2, 40.2, 38.8, 28.2, 25.3, 23.1, 21.3, 19.8, 19.4, 16.9, 15.4, 14.1, 12.7, 11.7 |
| Example 292. | Ac | 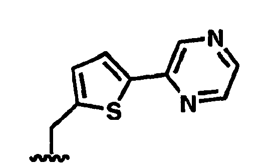 | H | 858 | 205.8, 184.6, 176.5, 169.3, 156.5, 148.9, 144.3, 144.2, 142.5, 141.7, 140.8, 128.5, 125.9, 102.6, 79.7, 79.0, 77.6, 76.2, 75.4, 70.8, 70.6, 70.5, 69.7, 66.1, 58.4, 50.8, 45.3, 40.5, 39.6, 39.0, 36.8, 28.5, 25.5, 23.4, 21.6, 20.2, 19.7, 17.2, 15.7, 14.4, 12.9, 12.0. |
| Example 293. | Ac | 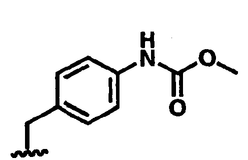 | H | 847 | 205.8, 184.6, 176.7, 169.3, 155.9, 137.7, 133.0, 129.6, 118.7, 102.6, 79.6, 79.0, 76.2, 76.1, 75.4, 70.5(2), 69.6, 66.2, 58.4, 52.6, 50.9, 45.3, 40.5, 39.6, 39.0, 36.9, 29.9, 28.7, 25.5, 23.4, 21.6, 20.3, 19.7, 17.2, 15.6, 14.5, 12.9, 12.0. |
| Example 294. | Ac | 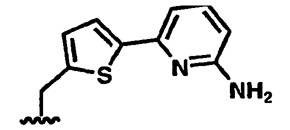 | F | 890 | N/A |
| Example 295. | Ac | 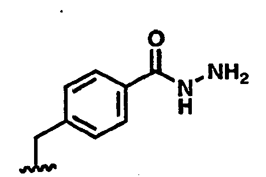 | H | 832 | 205.9, 184.6, 176.4, 169.3, 168.8, 167.9, 156.6, 153.9, 142.3, 132.0, 128.4, 128.1, 127.1, 102.6, 79.7, 79.0, 76.2, 75.6, 70.5, 69.7, 66.1, 58.5, 50.9, 45.4, 40.5, 39.0, 28.5, 25.5, 23.4, 21.6, 20.1, 19.7, 17.2, 15.7, 14.4, 13.0, 12.0 |
| Example 296. | Ac | 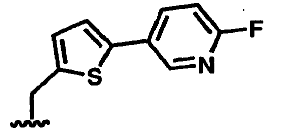 | H | 876 | N/A |
| Example 297. | Ac | 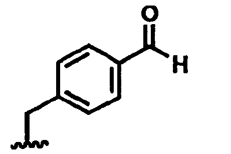 | H | 801 | N/A |
| Example 298. | Ac | 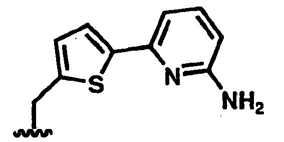 | H | 872 | N/A |
| Example 299. | H | 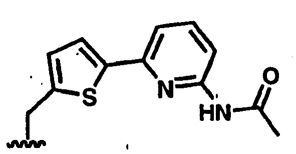 | H | 872 | 205.6, 189.1, 170.0, 169.2, 157.5, 151.1, 151.0, 144.8, 142.8, 139.2, 1 27.9, 124.5, 114.7, 112.1, 102.3, 80.6, 80.3, 78.8, 76.9, 76.0, 71.9, 71.2, 70.4, 69.5, 66.1, 60.2, 50.7, 45.7, 41.6, 40.4, 39.5, 35.3, 28.9, 25.0, 23.2, 21.6, 20.0, 19.7, 16.9, 14.8, 14.6, 12.9, 11.9. |
| Example 300. | Ac | 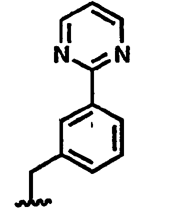 | H | 851 | N/A |
| Example 301. | Ac | 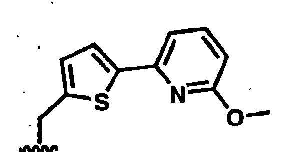 | H | 887 | 205.9, 184.6, 176.6, 169.3, 163.7, 156.1, 150.3, 145.8, 141.8, 139.2, 128.4, 124.2, 111.4, 109.1, 102.3, 79.6, 79.1, 76.2, 75.5, 71.1, 70.6, 70.3, 69.3, 66.5, 58.4, 53.6, 50.9, 45.2, 40.6, 39.0, 30.0, 25.5, 23.4, 21.5, 20.3, 19.7, 17.2, 15.7, 14.5, 12.8, 12.0, |
| Example 302. | H | 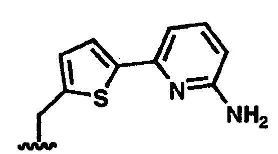 | H | 830 | 205.5, 188.1, 170.0, 158.3, 157.5, 151.2, 145.9, 141.7, 138.4, 127.9, 124.0, 109.4, 107.1, 102.4, 80.6, 80.3, 78.7, 77.1, 76.0, 71.9, 71.2, 70.5, 69.7, 66.1, 60.4, 50.7, 45.7, 42.0, 40.5, 39.5, 35.3, 29.9, 28.6, 23.2, 21.6, 20.1, 19.7, 16.9, 14.8, 14.6, 12.9, 11.9. |
| Example 303. | H | 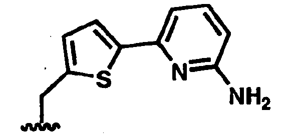 | F | 848 | N/A |
| Example 304. | Ac | 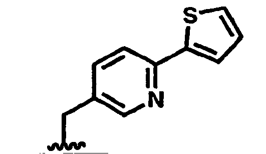 | H | 857 | N/A |
| Example 305. | Ac | 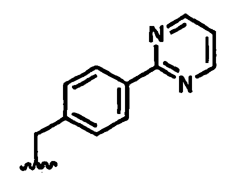 | H | 852 | 205.8, 184.6, 176.7, 169.3, 164.9, 157.4, 156.1, 140.7, 1372, 128,6, 128.4, 119.2, 102.6, 79.6, 79.0, 76.2, 75.4, 70.5, 69.7, 66.1, 58.4, 50.9, 45.4, 40.5, 39.0, 28.5, 25.5, 25.5, 23.4, 21.6, 20.3, 19.7, 15.6, 14.5, 12.9, 12.0 |
| Example 306. | Ac | 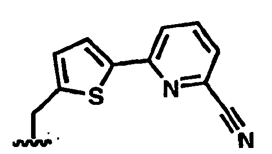 | H | 882 | 205.8, 184.7, 178.1, 167.8, 154.5, 154.2, 145.0, 142.6, 137.7, 133.8, 127.9, 126.41, 126.37, 122.1, 117.4, 103.0, 79.4, 79.2, 76.8, 74.6, 70.9, 70.5, 69.7, 66.2, 63.1. 62.9, 50.7, 46.2, 40.5, 38.8, 28.7, 25.4, 23.9, 21.5, 20.3, 19.5, 17.8, 15.1, 14.1, 13.7, 13.0. |
| Example 307. | Ac | 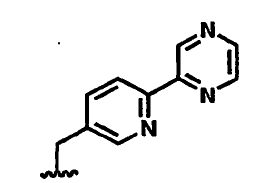 | H | 853 | 205.6, 184.3, 176.1, 169.1, 156.6, 153.5, 151.2, 149.3, 144.3, 143.5, 143.4, 137.2, 134.3, 121.1, 102.4, 79.5, 79.7, 76.0, 75.2, 73.4, 70.2, 695, 65.9. 58.2, 50.6, 452, 40.2, 38.8, 28.3, 253, 23.1, 21.3, 19.9, 195, 17.0, 15.4, 14.2, 12.8, 11.7 |
| Example 308. | Ac | 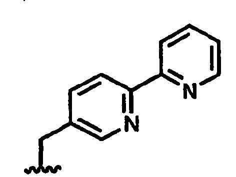 | H | 852 | 205.8, 184.5, 1765, 169.3, 156.6, 156.4, 155.9, 149.3, 137.5, 137.1, 1335, 123.8, 121.4, 121.0, 102.7, 79.7, 79.0, 76.2, 75.4, 3.8, 70.5, 69.8, 66.1, 58.4, 50.9, 45.4, 40.5. 39.0, 28.5,25.5,23.4,21.6, 20.2, 19.7, 17.2, 15.7, 14.4, 13.0, 12.0 |
| Example 309. | Ac | 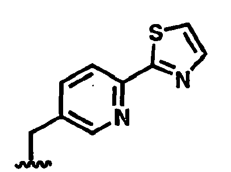 | H | 858 | 205.8, 184.6, 176.4, 169.4, 156.9, 151.0, 149.4, 144.2, 137.6, 134.7, 121.5, 119.6, 102.7, 79.7, 79.0, 76.2, 75.5, 73.6, 705, 70.4, 69.7, 66.1, 58.4, 50.9, 45.5, 40.5, 39.0, 36.8, 30.0, 28.5, 25.6, 23.4, 21.6, 20.2, 19.7, 17.2, 15.7, 14.4, 13.0, 12.0. |
| Example 310. | Ac | 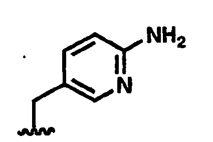 | H | 790 | 205.8, 184.5, 176.6, 169.3, 158.5, 155.9, 148.8, 139.4, 123.2, 108.6, 102.7, 79.6, 79.0, 76.2, 75.4, 74.0, 70.5, 69.7, 66.1, 58.4, 50.8, 45.4, 40.5, 39.6, 39.0, 36.8, 28.5, 25.5, 23.4, 21.6, 203, 19.7, 17.2, 15.6, 14.4, 12.9, 12.0. |
| Example 311. | Ac | 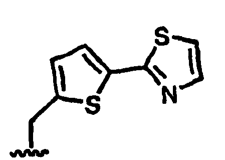 | H | 863 | 205.6, 184.3, 176.1, 169.0, 162.0, 156.1, 143.2, 142.6, 137.6, 127.7, 126.1, 118.0, 101.8, 79.3, 78.8, 75.9, 75.4, 70.4, 70.4, 69.9, 68.8, 66.5, 58.2, 50.6, 44.9, 40.3, 38.8, 365, 29.6, 25.2, 23.1, 21.1, 19.9, 19.4, 16.9, 15.4, 14.3, 12.5, 11.7. |
| Example 312. | Ac | 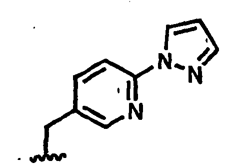 | F | 859 | 204.8, 2048, 184.0, 176.3, 165.8, 165.6, 157.4, 151.4, 148.2, 142.1, 139.8, 131.1, 127.3, 112.3, 107.8, 104.0, 99.6, 98.0, 79.9, 79.3, 76.1, 73.9, 73.4, 70.6, 69.9, 67.9, 66.0, 56.2, 41.2, 40.5, 39.2, 37.7, 31.2, 29.9, 28.4, 25.3, 24.5, 24.4, 22.8, 21.6, 21.5, 21.0, 17.3, 15.2, 14.2, 11.9 |
| Example 313. | Ac | 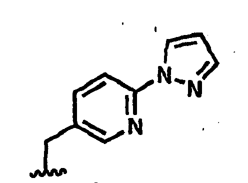 | H | 841 | 205.8, 184,5, 176.4, 169.3, 156.7, 151.4, 148.1, 142.1, 139.6, 131.3, 127.3, 112.3, 107.9, 102.6, 79.7, 79.0, 76.2, 75.4, 73.4, 705, 70.3, 69.7, 66.1, 58.3, 50.9, 45.4, 40.5, 39.7, 39.0, 36.8, 28.5, 25.5, 23.3, 21.6, 20.2, 19.7, 17.2, 15.6, 14.4, 13.0, 12.0. |
| Example 314. | Ac | 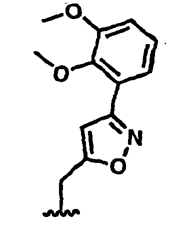 | H | 901 | N/A |
| Example 315. | Ac | 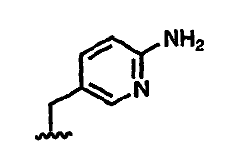 | F | 808 | 204.6, 204.4, 184.0, 176.7, 165.8, 165.6, 158.4, 156.8, 148.9, 139.5, 123.1, 108.5, 104.1, 99.6, 98.0, 79.9, 79.3, 76.1, 74.0, 70.6, 69.9, 68.0, 66.0, 56.3, 41.2, 40.5, 39.2, 37.8, 31.2, 29.9, 28.4, 25.3, 245, 24.3, 22.8, 21.6, 21.5, 20.9, 17.3, 15.2, 14.2, 11.9. |
| Example 316. | Ac | 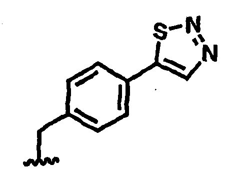 | H | 858 | 205.9, 184.6, 175.5, 169.3, 163.1, 156.3, 139.5, 130.4, 130.1, 129.2,127.6, 102.7, 79.7, 76.2, 76.0, 70.5, 69.7, 66.1, 58.4, 50.9, 45.4, 40.5, 39.0; 28.5, 25.5, 23.4, 21.6, 20.2, 19.7, 17.2, 15.7, 14.4, 12.0 |
| Example 317. | Ac | 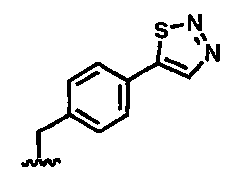 | F | 876 | 203.5, 203.3, 182-8, 164.6, 164.4, 161.9, 156.0, 138.1, 129.1, 128.7, 128.0, 126.3, 102.8, 98.4, 96.7, 78.0, 74.7, 72.8, 69.3, 68.6, 66.7, 64.8, 55.2, 39.9, 39.2, 27.1, 24.1, 23.2, 23.0, 21.5, 20.4, 20.2, 19.7, 16.0, 14.0, 12.9, 10.6 |
| Example 318. | Ac | 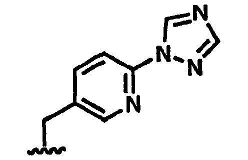 | H | 842 | N/A |
| Example 319. | Ac | 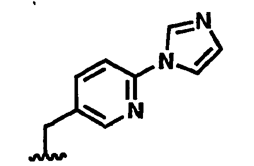 | H | 841 | 205.9, 184.5, 176.3, 169.4, 157.0, 149.1, 148.8, 139.7, 135.2, 132.1, 130.8, 116.5, 112.2, 102.7, 79.7, 79.0, 77.4, 76.2, 75.4, 73.0, 70.4, 70.3, 69.7, 66.1, 58.4, 50.9, 45.5, 40.5, 39.7, 39.0, 36.7, 28.5, 255, 23.3, 21.6, 20.1, 19.7, 17.2, 15.6, 14.4, 13.0, 12.0. |
| Example 320. | Ac | 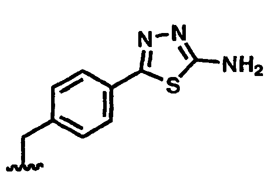 | H | 873 | 205.62, 184.33, 176.23, 169.06, 167.60, 159.80, 156.14, 139.99, 130.03, 128.60, 126.92, 102.26, 79.37, 78.75, 75.93, 75.57, 75.15, 70.14, 69.34, 67.94, 65.91, 58.17, 53.40, 50.61, 45.12, 40.20, 39.39, 38.76, 36.53, 29.66, 28.47, 25.58, 25.26, 23.08, 21.27, 19.89, 19.41, 16.95, 15.40, 14.16, 12.68, 11.72 |
| Example 321. | Ac | 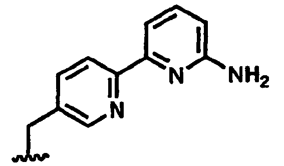 | H | 867 | N/A |
| Example 322. | Ac | 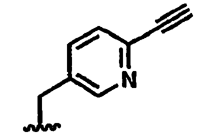 | H | 799 | N/A |
| Example 323. | Ac | 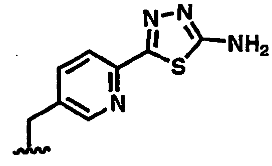 | H | 874 | Partial ¹³C NMR 205.63, 184.32, 176.04 |
| Example 324. | Ac | 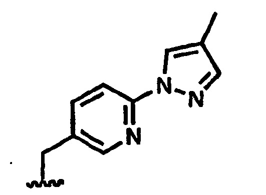 | H | 855 | 205.8, 1845, 176.4, 169.3, 156.6, 1515, 148.1, 143.1, 139.5, 130.7, 125.8, 118.5, 111.9, 102.6, 79.7, 79.0, 76.2, 75.5, 73.5, 70.4, 70.3, 69.7, 66.2, 58.3, 50.9, 45.4, 40.5, 39.7, 39.0, 36.8, 29.5, 28.6, 25.5, 23.3, 21.6, 20.2, 19.7, 17.2, 15.6, 14.4, 13.0, 12.0, 9.3. |
| Example 325. | Ac | 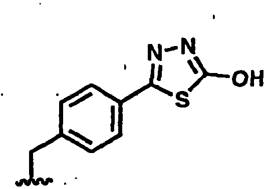 | H | 874 | 205.71, 184.39, 176.16, 171.88, 169.10, 156.24, 153.13, 141.02, 129.99, 128.53, 125.93, 102.14, 79.40, 78.82, 75.92, 75.42, 75.20, 70.27, 70.09, 69.19, 66.09, 58.26, 53.41, 50.63, 45.13, 40.24, 39.37, 38.80, 36.50, 28.79, 25.31, 23.09, 21.24, 19.87, 19.41, 16.94, 15.48, 14.20, 12.68, 11.73 |
| Example 326. | Ac | 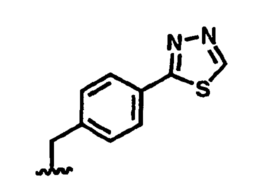 | H | 858 | 205.67, 184.31, 176.14, 169.08, 168.29, 156.29, 151.02, 141.50, 128.73, 128.13, 102.22, 79.39. 78.76, 75.93, 75.42, 5.21, 70.11, 69.27, 66.07, 58.17, 50.64, 45.11, 40.24, 39.39, 38.78, 36.55, 28.62, 25.28, 23.08, 21.26, 19.90, 19.39, 16.95, 15.40, 14.18, 12.68, 11.72 |
| Example 327. | Ac | 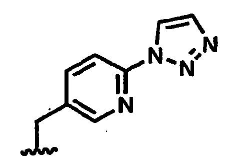 | H | 842 | 205.8, 184.4, 169.1, 156.8, 148.1, 139.6, 134.1, 121.1, 113.5, 109.7, 101.8, 79.4, 78.8, 75.9, 75.3, 72.8, 69.8, 68.7, 66.4, 58.1, 50.7, 45.1, 40.3, 38.8, 36.4, 31.6, 29.5, 25.3, 23.0, 22.6, 21.1, 19.8, 19.3, 16.9, 15.4, 14.2, 14.1, 12.7,11.7. |
| Example 328. | Ac | 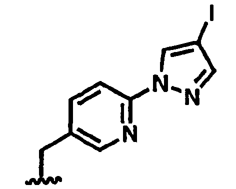 | H | 967 | 205.6, 184.3, 176.1, 169.1, 156.5, 150.3, 147.9, 146.5, 139.4, 131.7(2), 111.6, 102.2, 79.4, 78.8, 75.9, 73.0, 70.1, 69.2, 66.2, 59.6, 58.1, 50.7, 45.1, 40.3, 39.4, 38.8; 36.5, 31.6, 28.7, 25.3, 23.1, 21.3, 19.9, 19.4, 16.9, 15.4, 14.2, 14.1, 12.7, 11.7, |
| Example 329. | Ac | 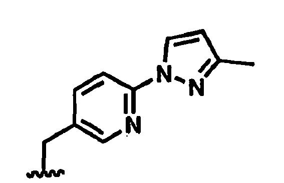 | H | 855 | 205.5, 184.2, 176.2, 169.0, 156.3, 151.5, 151.1, 147.9, 139.2, 130.3, 127.7, 111.6, 107.8, 102.3, 79.4, 78.7, 75.9, 75.2, 73.2, 70.0, 69.3, 66.0, 58.0, 50.6, 45.1, 40.2, 38.7, 31.5, 28.5, 252, 23.1, 22.6, 21.3, 19.9, 19.4, 16.9, 15.4, 14.2, 14.0, 13.9, 12.7, 11.7, |
| Example 330. | Ac | 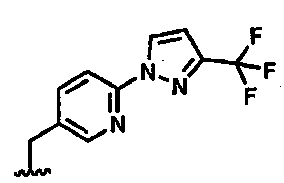 | H | 909 | 205.6, 184.3, 176.1, 169.1, 156.7, 150.2, 147.9, 139.5, 132.4, 128.4, 112.5, 105.7, 102.3, 79.4, 78.7, 75.9, 75.2, 72.9, 70.2, 70.0, 69.4, 65.9, 58.1, 50.6, 45.2, 40.2, 39.4, 38.7, 36.5, 31.6, 28.3, 25.3, 23.0, 22.6, 21.3, 19.9, 19.4, 16.9, 15.4, 14.1, 12.8, 11.7, |
| Example 331. | Ac | 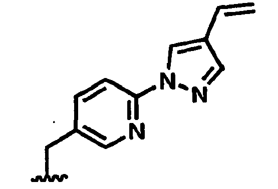 | H | 867 | N/A |
| Example 332. | Ac | 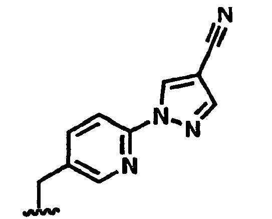 | H | 866 | N/A |
| Example 333. | Ac | 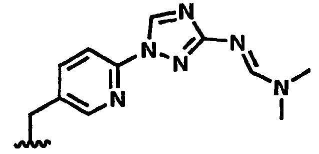 | H | 912 | N/A |
| Example 334. | Ac | 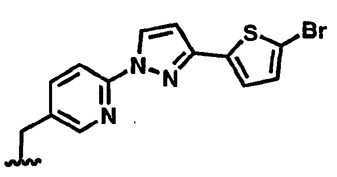 | H | 1003 | 205.76, 184.34, 176.04, 169.17, 156.45, 150.73, 148.04, 147.87, 139.38, 137.91, 131.05, 130.28, 128.41, 124.55, 112.12, 112.06, 104.75, 101.59, 79.31, 78.76, 76.26, 75.91, 75.02, 73.11, 71.95, 70.20, 67.70, 59.27, 58.12, 51.91, 58.12, 51.92, 50.57, 45.18, 39.41, 38.77, 36.45, 34.93, 25.28, 23.06, 21.06, 19.80, 19.35, 16.95, 15.43, 13.98. 12.65, 11.696 |
| Example 335. | Ac | 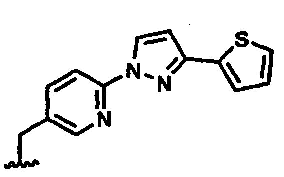 | H | 923 | 205.75, 184.34, 176.07, 169.17, 156.40, 50.90, 148.88, 147.90, 13936, 136.28, 130.84, 128.30, 127.49, 125.17, 124.59, 112.10, 105.24, 101.61, 79.31, 78.77, 7626, 75.92, 75.06, 73.19, 71.96, 70.21, 67.71, 59.27, 58.13, 51.94, 50.59, 45.18, 39.41, 38.78, 36.46, 34.95, 25.30, 23.08, 21.07, 19.82,19.36, 16.96, 15.44, 14.00, 12.66, 11.70 |
| Example 336. | H | 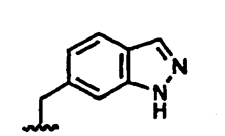 | H | 772 | 205.7, 169.9, 157.5, 140.7, 137.3, 134.5, 122.8, 120.8, 120.5, 108.7,102.5, 80.7, 80.1, 78.9, 76.7, 762, 76.0, 71.9, 70.5, 69.7, 66.1, 602, 50.7, 45.9, 41.8, 40.5, 39.5, 35.5, 28.6, 23.2, 21.6, 20.0, 17.0, 14.8, 14.5, 13.1, 11.9. |
| Example 337. | Ac | 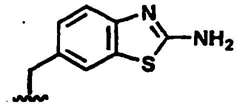 | H | 846 | N/A |

Example compounds 338-352 of formula **B:** 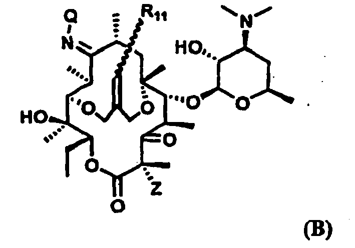 wherein R₁₁, Q, and Z are as delineated for each example in Table B.

Example compounds 338-352, where Z = H, are made from the title compound of formula (**1**-5), wherein V = N-Ac, R₁₁ = H, and R₂' = H, and the appropriate bromo compound of formula Br-R₁₁, via essentially the same synthetic route described in Example 35.

Example compounds 338-352, where Z = F, are made from the title compound of Example 46a and the appropriate precursor via the method delineated in Example 35.

The Examples described in Table B comprise mixtures ofE and Z isomers, which can be separated via silica chromatography or HPLC.

The bromo compounds used to form the following examples are commercially available or can be made from readily-available starting materials via synthetic methods well known by one of ordinary skill in the art.

**Table B**

| **Examples** | **Q** | **-Ar₁-M=Ar₂** | **Z** | **MS (ESI):** **m/z (M+H)⁺** | **13C NMR (125 MHz, CDCl₃): δ** |
|---|---|---|---|---|---|
| Example 338. | H | H | H | 625 | N/A |
| Example 339. | MOM | H | H | 685 | N/A |
| Example 340. | MOM | 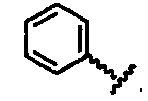 | H | 761 | N/A |
| Example 341. | Ac | 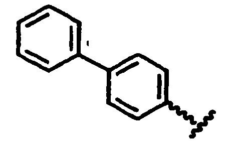 | H | 819 | N/A |
| Example 342. | Ac | 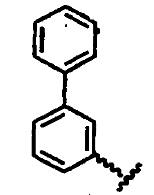 | H | 819 | N/A |
| Example 343. | Ac | 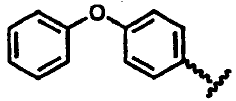 | H | 835 | N/A |
| Example 344. | Ac | 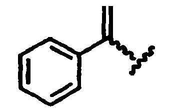 | H | 769 | N/A |
| Example 345. | Propionyl | 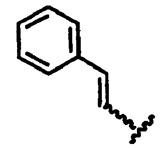 | H | 783.36 | N/A |
| Example 346. | Ac | 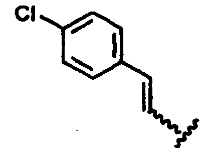 | H | 803 | N/A |
| Example 347. | C(O)OMe | 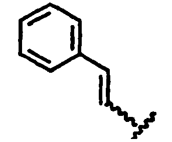 | H | 785 | N/A |
| Example 348. | C(O)NH₂ | H | H | 668 | N/A |
| Example 349. | Me | H | H | 639 | N/A |
| Example 351. | Ac | 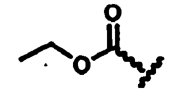 | H | 739 | N/A |
| Example 352. | Ac | 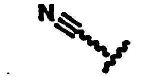 | H | 692 | N/A |

Example compounds 353-374 of formula **B1:** 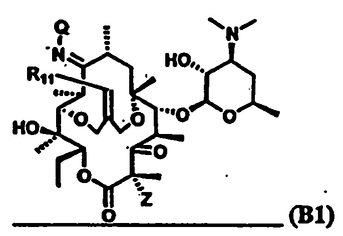 wherein R₁₁, Q, and Z are as delineated for each example in Table B1.

Example compounds 353-374, where Z = H, are made from the title compound of formula (**1**-5), wherein V = N-Ac, R₁₁ = H, and R₂' = H, and the appropriate bromo compound of formula Br-R₁₁, wherein R₁₁ is as previously defined, via essentially the same synthetic route described in Example 35.

Example compounds 353-374, where Z = F, are made from the title compound of Example 46a and the appropriate precursor via the method delineated in Example 35.

The Examples described in Table B1 are single isomers of the E designation, which are separated from the E/Z mixture via silica chromatography or HPLC.

The bromo compounds used to form the following examples are commercially available or can be made from readily-available starting materials via synthetic methods well known by one of ordinary skill in the art.

**Table B1**

| **Examples** | **Q** | **-Ar₁-M-Ar₂** | **Z** | **MS (ESI):** **m/z** **(M+H)⁺** | **¹³C NMR (125 MHz, CCCl₃: δ** |
|---|---|---|---|---|---|
| Example 353. | Ac | 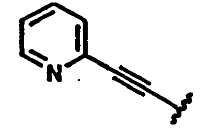 | H | 768 | N/A |
| Example 354 | Ac | 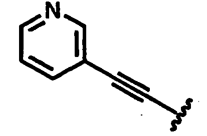 | H | 768 | N/A |
| Example 355 | Ac | 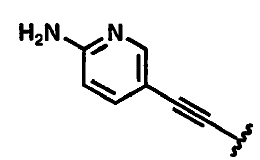 | H | 783 | 206.2, 184.4, 177.7, 167.8, 157.4, 151.5, 144.3, 140.4, 117.9, 110.1, 107.9, 103.0, 93.3, 86.9, 79.5, 78.4, 76.2, 72.7, 70.2, 69.3, 68.2, 66.1, 65.8, 50.8, 46.6, 40.3, 38.5, 25.1, 23.8, 21.2, 19.5, 17.3, 15.1, 13.8, 12.9, |
| Example 356 | Ac | 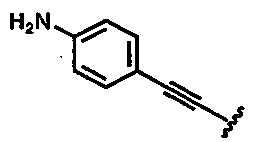 | H | 782 | 206.2, 184.4, 177.8, 167.7, 146.6, 143.4, 133.0, 118.4, 114.6, 112.9, 103.0, 96.8, 84.4, 79.6, 78.3, 76.2, 72.6, 70.2, 69.3, 68.3, 66.1, 65.8, 50.8, 46.6, 40.3, 38.5, 25.1, 23.7, 21.2, 19.5, 17.3, 15.1, 13.8, 12.7, |
| Example 357 | Ac | 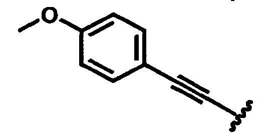 | H | 797 | N/A |
| Example 358 | Ac | 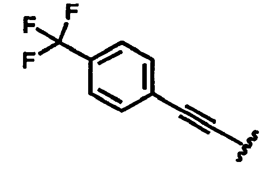 | H | 835 | N/A |
| Example 359 | Ac | 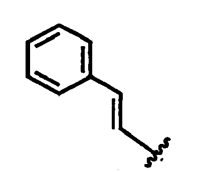 | F | 787 | 205.36 (d), 183.93, 164.92 (d), 137.25, 136.54, 136.08, 134.26, 12852, 128.17, 126.87, 123.95, 103.95, 80.17, 79.96, 75.93, 71.71, 70.37, 69.51, 66.33, 65.99, 65.82, 40.65, 40.20, 38.90, 37.24, 28.27, 25.08, 24.68, 24.50, 23.07, 21.18, 21.06, 17.11, 15.05, 14.14, 12.57 |
| Example 360 | Ac | 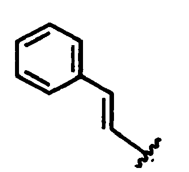 | H | 771 | 205.72, 169.32, 169.60, 137.16, 135.90, 128.53, 128.25, 127.86, 126.71, 123.91, 103.75, 80.62, 78.92, 76.88, 70.41, 70.30, 69.30, 66.16, 65.56, 59.83, 53.76, 51.63, 48.93, 40.27, 29.23, 28.69, 23.57, 23.14, 21.45, 21.16, 16.62, 14.34, 12.61 |
| Example 361 | Ac | 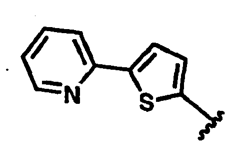 | H | 827 | 206.2, 184.4, 177.6, 167.6, 152.5, 149.5, 145.6, 140.7, 1365, 132.2, 131.1, 129.4, 124.6, 121.8, 118.8, 103.1, 79.3, 78.6, 76.2, 75.0, 73.0, 70.3, 69.4, 67.7, 67.1, 66.2, 65.9, 50.9, 46.9, 40.2, 38.6, 29.7, 28.4, 25.1, 23.8, 21.2, 20.5, 19.7, 17.4, 14.9, 14.2, 13.8, 12.1. |
| Example 362 | Ac | 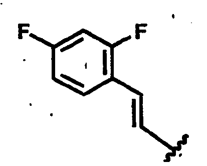 | H | 805 | Selected: 206.3, 184.7, 177.9, 167.8, 163.3, 161.4, 159.6, 136.2, 135.2. 128.7, 127.7, 126.3, 111.8, 111.6, 103.4, 79.7, 78.7, 76.4, 72.5, 70.5, 69.7, 66.1, 51.1, 47.2, 40.5, 38.8, 29.5, 28.6, 25.4, 23.9, 21.5, 19.9, 17.7, 15.1, 14.1, 13.1. |
| Example 363 | 2-methoxyacetyl | 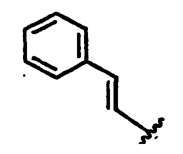 | H | 799 | 206.4, 183.9, 182.0, 167.8, 137.6, 136.3, 134.2, 128.7, 128.6, 128.0, 127.1, 127.0, 124.1, 103.4, 79.8, 78.7, 77.5, 76.5, 73.5, 72.7, 70.5, 69.7, 66.7, 66.1, 59.7, 51.1, 40.5, 39.0, 28.5, 23.9, 21.5, 20.9, 19.9, 17.7, 15.2, 14.2, 13.1. |
| Example 364 | 2-O-acyl-acetyl | 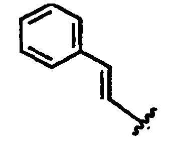 | H | 827 | N/A |
| Example 365 | 2-Fmoc-acetyl | 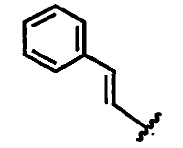 | H | 1006 | N/A |
| Example 366 | Ac | 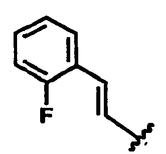 | H | 787 | 206.3, 184.7, 178.0, 167.8, (161.7, 159.7), 136.4, 135.1, (129.1, 129.06), 128.7, 128.0, 126.7, 126.65, (125.5, 125.4), 124.2, (116.1, 116.0), 103.5, 79.7, 78.7, 76.4, 72.5, 70.6, 69.8, 66.7, 66.5, 66.1, 51.1, 47.2, 40.5, 38.8, 31.2, 28.5, 25.4, 23.9, 21.5, 20.8, 20.0, 17.7, 15.1, 14.5, 14.1, 13.0. |
| Example 367 | Ac | 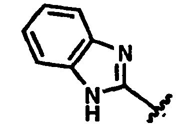 | H | 783 | N/A |
| Example 368. | 2-hydroxy acetyl | 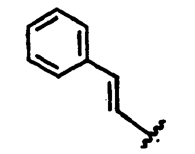 | H | 785 | N/A |
| Example 369. | aminoacetyl | 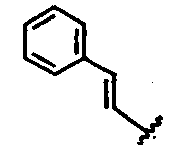 | H | 784 | N/A |
| Example 370. | Ac | 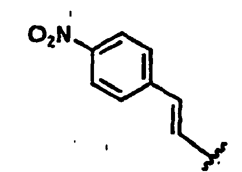 | H | 814 | 206.1, 184.4, 177.5, 167.7, 146.8, 143.8, 137.6, 135.0, 133.4, 130.1, 128.1, 127.0, 124.0, 123.4, 103.2, 79.4, 78.6, 76.2, 72.3, 70.3, 69.5, 66.4, 66.0, 65.8, 50.8, 47.0, 40.2, 38.5, 28.2, 25.1, 23.7, 21.2, 19.7, 17.5, 14.8, 13.8, 13.0 |
| Example 371. | Ac | 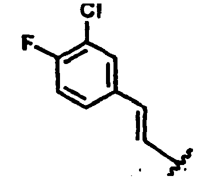 | H | 821 | Partial ¹³C NMR: 206.09, 184.41, 167.60, 158.43 |
| Example 372. | Ac | 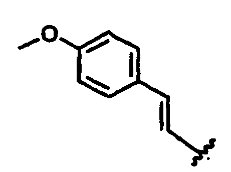 | H | 799 | 206.10, 184.38, 167.47, 159.30, 136.23, 135.69, 132.65, 130.18, 127.96, 121.79, 113.88, 103.16, 79.58, 78.31, 76.16, 72.09, 70.27, 69.46, 66:53, 66.43, 65.80, 55.19, 50.82, 46.87, 40.19, 38.57, 31.63, 28.20, 25.22, 25.08, 23.68, 22.60, 21.22, 20.51, 19.67, 17.43, 14.87, 14.08, 13.80, 12.89 |
| Example 373. | Ac | 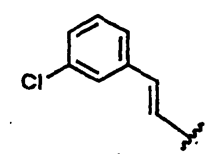 | H | 803 | Selected: 206.4, 184.7, 177.9, 167.8, 139.5, 135.7, 135.5, 134.8, 134.7, 129.9, 127.8, 126.8, 125.4, 125.3, 103.4, 79.8, 78.7, 76.5, 72.5, 70.5, 69.7, 66.8, 66.4, 66.1, 54.0, 51.1, 47.2, 40.5, 38.8, 31.2, 29.5, 28.5, 25.4, 23.9, 21.5, 19.9, 17.8, 15.1, 14.1, 13.2. |
| Example 374. | Ac | 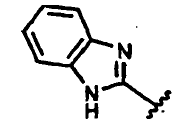 | H | 800 | N/A |

Example compounds 375-383 of formula **B2:** 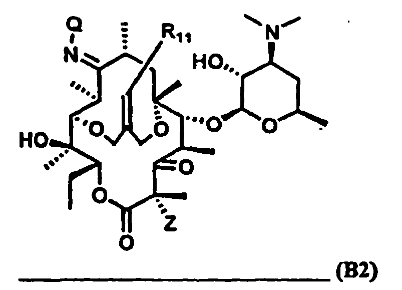 wherein R₁₁, Q, and Z are as delineated for each example in Table B2.

Example compounds 375-383, where Z = H, are made from the title compound of formula (**1**-5), wherein V = N-Ac, R₁₁ = H, and R₂' = H, and the appropriate bromo compound of formula Br-R₁₁, wherein R₁₁ is as previously defined, via essentially the same synthetic route described in Example 35.

Example compounds 375-383, where Z = F, are made from the title compound of Example 46a and the appropriate precursor via the method delineated in Example 35.

The Examples described in Table B2 are single isomers of the Z designation, which are separated from the E/Z mixture via silica chromatography or HPLC.

The bromo compounds used to form the following examples are commercially available or can be made from readily-available starting materials via synthetic methods well known by one of ordinary skill in the art.

**Table B2**

| Examples | Q | **-Ar₁-M-Ar₂** | **Z** | **MS**(**ESI):** **m/z** **(M+H)⁺** | ¹**³C NMR (125 MHz, CDCl₃): δ** |
|---|---|---|---|---|---|
| Example 375. | Ac | 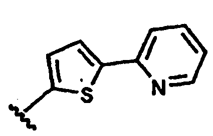 | H | 827 | 205.3, 184.5, 176.0, 168.8, 152.4, 149.4, 145.1, 141.0, 136.4, 135.2, 129.5, 126.1, 124.8, 121.7, 118.7, 102.4, 79.0, 78.6, 77.6, 76.3, 76.0, 74.8, 70.2, 69.4, 65.8, 61.6, 50.3, 45.1, 40.1, 39.2, 36.4, 28.2, 25.0, 23.2, 21.2, 20.0, 19.9, 16.9, 15.5, 14.0, 12.5, 11.9. |
| Example 376. | Ac | 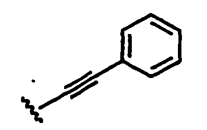 | H | 767 | 205.33, 184.75, 177.42, 168.32, 146.16, 131.61, 128.18, 128.12, 123.39, 115.83, 102.24, 96.78, 85.24, 78,87, 78.59,76.18, 75.22, 72.63, 70.19, 69.37, 65.94, 61.88, 50.45, 44.87, 40.25, 38.71, 25.29, 23.31, 21.22, 20.00, 19.56, 1730, 15.34, 14.13, 12.65, 12.11 |
| Example 377. | Ac | 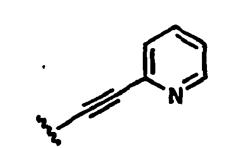 | H | 768 | 205.36, 184.66, 177.23, 168.24, 149.87, 148.14, 135.89, 127.32, 122.58, 114.98, 102.54, 95.65, 84.94, 78.86, 78.77, 76.12, 75.33, 74.94, 72.49, 70.24, 69.94, 69.49, 65.83, 63.03, 50.46, 45.13, 40.22, 38.67, 28.25, 25.29, 23.30,21.25, 20.03, 19.59, 17.27, 15.29, 14.09, 12.82, 12.11 |
| Example 378. | Ac | 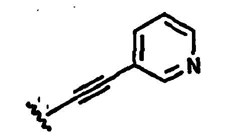 | H | 768 | 205.23, 184.50, 177.45, 168.08, 152.35, 148.40, 147.32, 138.48, 122.79, 115.62, 102.36, 93.03, 88.40, 78.74, 78.57, 76.18, 74.60, 74.51, 71.82, 70.19, 69.46, 65.83, 61.75, 50.33, 44.98, 40.20, 39.90, 38.71, 28.27, 25.22, 23.32, 21.23, 19.85, 19.36, 17.38, 15.20, 13.98, 12.74, 12.20 |
| Example 379. | Ac | 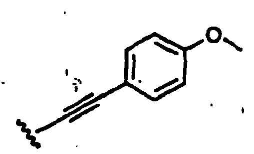 | H | 797 | 205.33, 194.80, 177.48, 168.27, 159.56, 145.23, 133.06, 132.98, 116.10, 115.59, 113.93, 113.77, 102,30, 96,86, 84.07, 78.88, 78.53, 76.16, 75.16, 72.70, 70.21, 69.43, 65.84, 61.87, 55.25, 50.42, 44.84, 40.22, 38.69, 30.91, 28.33, 25.28, 23.31, 21.26, 20.00, 19.57, 17.30, 15.33, 14.13, 12.63, 12.12 |
| Example 380. | Ac | 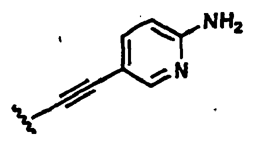 | H | 784 | In CD₃OD 211.7, 207.1, 187.1, 180.0, 170.0, 160.1, 151.4, 147.1, 141.7, 116.6, 109.6, 103.5, 95.4, 86.6, 80.1, 77.6, 76.4, 72.5, 72.0, 70.6, 70.2, 65.9, 62.5, 56.0, 46.1, 40.8, 40.2, 32.2, 32.1, 29.5, 25.4, 24.5, 21.5, 20.3, 20.2, 18.4, 15.6, 14.5, 12.6 |
| Example 381. | Ac | 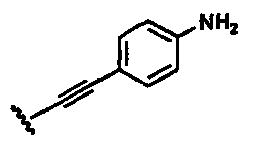 | H | 782 | 205.3, 184.9, 177.5, 168.4, 144.5, 133.0, 130.9, 128.8, 116.2, 114.6, 102.3, 97.8, 83.4, 78.9, 78.5, 76.2, 73.0, 70.2, 69.4, 65.9, 61.9, 50.4, 44.8, 40.2, 38.7, 31.6, 28.4, 25.3, 23.3, 21.3, 20.0, 19.6, 17.3, 15.4, 14.1, 12.6, 12.1 |
| Example 382. | Ac | 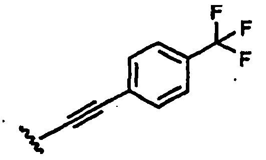 | H | 835 | 205.44, 184.55, 177.45, 168.12, 147.55, 131.84, 125.02, 115.58, 102.35, 95.04, 87.57, 78.77, 78.60, 76.18, 74.59, 71.89, 70.19, 69.48, 65.85, 61.76, 50.41, 44.98, 40.21, 39.88, 38.71, 28.27, 25.24, 23.31, 21.21, 19.88, 19.39, 17.37, 15.22, 14.04, 12.74, 12.18 |
| Example 383. | Ac | 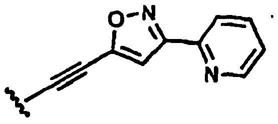 | H | 835 | N/A |

Example compounds 384-390 of formula **C:** 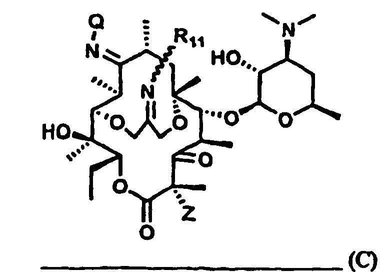 wherein R₁₁, Q, and Z are as delineated for each example in Table B2.

Example compounds 384-390, where Z = H, are made from the title compound of formula (**1**-5), wherein V = N-Ac, R₁₁ = H, and R₂' = H, and the appropriate compounds of formula NH₂-R₁₁, NH₂NH₂-SO₂-R₁₁, NH₂NH₂- R₁₁, NH₂NH₂-N=CH-R₁₁, NH₂NH₂-C(O)-R₁₁, where R₁₁ is as previously defined via essentially the same synthetic route described in Example 4.

Example compounds 384-390, where Z = F, are made from the title compound of Example 46 and the appropriate precursor via the method delineated in Example 4.

The Examples described in Table C comprise mixtures of E and Z isomers, which can be separated via silica chromatography or HPLC.

The amino compounds used to form the following examples are commercially available or can be made from readily-available starting materials via synthetic methods well known by one of ordinary skill in the art.

| **Examples** | Q | **-Ar₁-M-Ar₂** | **Z** | **MS (ESI):** **m/z (M+H)⁺** | **¹³C NMR (125 MHz, CDCl₃): δ** |
|---|---|---|---|---|---|
| Example 384. | Ac | 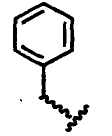 | H | 758 | N/A |
| Example 385. | Ac | 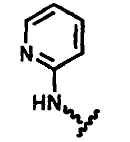 | H | 760 | N/A |
| Example 386. | Ac | 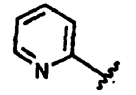 | H | 772 | N/A |
| Example 387. | Ac | 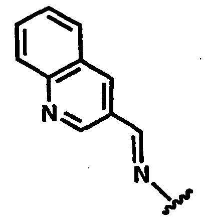 | H | 822 | N/A |
| Example 388. | Ac | 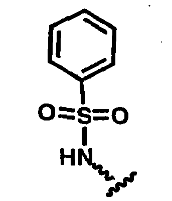 | H | 823 | 205.6, 185.1, 174.3, 169.8, 156.9, 138.6, 132.9, 129.0, 128.6, 102.3, 80.4, 78.9, 78.6, 76.1, 75.3, 73.9, 70.4, 69.8, 66.1, 59.9, 50.8, 45.6, 40.5, 38.9, 36.0, 29.9, 28.5, 25.5, 23.1, 21.6, 21.5, 19.9, 19.7, 17.1, 15.3, 14.3, 12.9, 11.8. |
| Example 389. | Ac | 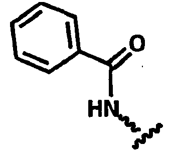 | H | 788 | N/A |
| Example 390. | Ac | 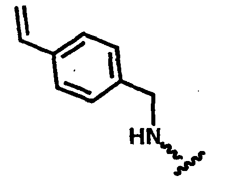 | H | 799 | N/A |

Although the invention has been described with respect to various preferred embodiments, it is not intended to be limited thereto, but rather those skilled in the art will recognize that variations and modifications may be made therein which are within the spirit of the invention and the scope of the appended claims.

## Claims

1. A compound represented by the formula: 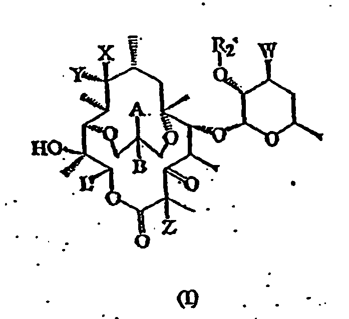 or its pharmaceutically acceptable salt or ester
wherein
A is selected from:
a) -OH;
b) -ORₚ, where Rₚ is a hydroxy protecting group;
c) -R_{1,} where R₁ is independently selected from:
(1) aryl;
(2) substituted aryl;
(3) heteroaryl; and
(4) substituted heteroaryl;
d) -OR₁, where R₁ is as previously defined;
e) -R₂, where R₂ is selected from:
(1) hydrogen;
(2) halogen;
(3) C₁-C₁₂ alkyl optionally containing 0, 1, 2, or 3 heteroatoms selected from O, S or N, optionally substituted with one or more substituents selected from halogen, aryl, substituted aryl, heteroaryl, and substituted heteroaryl;
(4) C₂-C₁₂ alkenyl optionally containing 0, 1, 2, or 3 heteroatoms selected from O S and N, optionally substituted with one or more substituents selected from halogen, aryl; substituted aryl, heteroaryl, and substituted heteroaryl; and
(5) C₂-C₁₂ alkynyl optionally containing 0, 1, 2 or 3 heteroatoms selected from O, S and N, optionally substituted with one or more substituents selected from halogen, aryl, substituted aryl, heteroaryl, and substituted heteroaryl;
f) -OR₂, where R₂ is independently previously defined;
g) -S(O)ₙR₁₁, where n = 0, 1 or 2, and R₁₁ is independently hydrogen, R₁ or R₂, where R₁ and R₂ are as previously defined;
h) -NHC(O)R₁₁, where R₁₁ is as previously defined;
i) -NHC(O)NHR₁₁, where R₁₁ is as previously defined;
j) -NHS(O)₂R₁₁, where R₁₁ is as previously defined;
k) -NR₁₄R₁₅, where R₁₄ and R₁₅ are each independently R₁₁, where R₁₁ is as previously defined; and
l) -NHR₃, where R₃ is an amino protecting group;
B is selected from:
a) hydrogen;
b) deuterium;
c) halogen;
d) -OH;
e) R₁, where R₁ is as previously defined;
f) R₂, where R₂ is as previously defined; and
g) -ORₚ, where Rₚ is as previously defined,
provided that when B is halogen, -OH, or -ORₚ, A is R₁ or R₂;
or alternatively, A and B taken together with the carbon atom to which they are attached are selected from:
a) C=O;
b) C(OR₂)₂, where R₂ is as previously defined;
c) C(SR₂)₂, where R₂ is as previously defined;
d) C[-O(CH₂)ₘ]₂, where m = 2 or 3;
e) C[-S(CH₂)ₘ]₂, where m is as previously defined;
f) C=CHR₁₁, where R₁₁ is as previously defined;
g) C=N-O-R₁₁, where R₁₁ is as previously defined;
h) C=N-O-Ar₁-M-Ar₂, wherein
(1) -Ar₁-is R₃₁, where R₃₁ is independently selected from:
(a) R₁, where R₁ is as previously defined;
(b) C₁-C₁₂ alkyl optionally containing 0, 1, 2, or 3 heteroatoms selected from O, S or N, optionally substituted with one or more substituents selected from halogen, aryl, substituted aryl, heteroaryl, and substituted heteroaryl;
(c) C₂-C₁₂ alkenyl optionally containing 0, 1, 2, or 3 heteroatoms selected from O, S and N, optionally substituted with one or more substituents selected from halogen, aryl, substituted aryl, heteroaryl, and substituted heteroaryl; or
(d) C₂-C₁₂ alkynyl optionally containing 0, 1, 2, or 3 heteroatoms selected from O, S and N, optionally substituted with one or more substituents selected from halogen, aryl, substituted aryl, heteroaryl, and substituted heteroaryl;
(2) -M- is absent or selected from:
(a) -C₁-C₁₂ alkyl optionally containing:
0-3 heteroatoms selected from O, S or N; and
0-3 groups selected from -C=N-, -N=N, -C(O)-;
(b) -C₂-C₁₂ alkenyl optionally containing:
0-3 heteroatoms selected from O, S or N; and
0-3 groups selected from -C=N-, -N=N, -C(O)-;
(c) -C₂-C₁₂ alkynyl optionally containing;
0-3 heteroatoms selected from O, S or N;
0-3 groups selected from -C=N-, -N=N-, -C(O)-;
(d) substituted aryl;
(e) substituted heteroaryl; or
(f) substituted heterocyclo alkyl; and
(3) -Ar₂ is selected from:
(a) aryl;
(b) substituted aryl;
(c) heteroaryl; or
(d) substituted heteroaryl;
i) C=NNHR₁₁, where R₁₁ is as previously defined;
j) C=NNHC(O)R₁₁, where R₁₁ iis as previously defined;
k) C=NNHC(O)NHR₁₁, where R₁₁ is as previously defined;
l) C=NNHS(O)₂R₁₁, where R₁₁ is as previously defined;
m) C=NNHR₃, where R₃ is as previously defined;
n) C=NR₁₁, where R₁₁ is as previously defined; or
o) C=N-N=CHR₁₁, where R₁₁ is as previously defined;
one of X and Y is hydrogen and the other is selected:
a) hydrogen;
b) deuterium;
c) -OH;
d) -ORₚ, where Rₚ is as previously defined;
e) -NR₄R₅, where R₄ and R₅ are each independently selected from:
(1) hydrogen;
(2) C₁-C₁₂ alkyl, optionally substituted with one or more substituents selected from halogen, aryl, substituted aryl, heteroaryl and substituted heteroaryl; or
(3) R₄ and R₅, taken together with the nitrogen atom to which they are attached from a 3-10 membered heteroalkyl ring containing 0-2 additional hetero atoms selected from O, S and N; or
alternatively, X and Y taken together with the carbon atom to which they are attached are selected from:
a) C=O;
b) C=N-Q, where Q is selected from:
(1) -R₁₁, where R₁₁ is as previously defined;
(2) amino protecting group;
(5) -C(O)R₁₁, where R₁₁ is as previously defined;
(6)-OR₆, where R₆ is independently selected from:
(a) hydrogen;
(b) -CH₂O(CH₂)₂OCH₃.
(c) -CH₂O(CH₂O)₂CH₃, where n is as previously defined;
(d) -C₁-C₁₂ alkyl, optionally substituted with one or more substituents selected from aryl, substituted aryl, heteroaryl and substituted heteroaryl;
(e) -C₃-C₁₂ cycloalkyl;
(f) -C(O)-C₁-C₁₂ alkyl;
(g) -C(O)-C₃-C₁₂ cycloalkyl;
(h) -C(O)-R₁, where R₁ is as previously defined; or
(i) -Si(Rₐ)(R_{b})(R_{c}), wherein Rₐ, R_{b} and R_{c} are each independently selected from C₁-C₁₂ alkyl, aryl and substituted aryl; or
(5) O-C(R₇)(R₈)-O-R₆, where R₆ is as previously defined, provided that R₆ is not C(O)-C₁-C₁₂ alkyl, C(O)-C₃-C₁₂ cycloalkyl, or C(O)-R₁, and R₇ and R₈ taken together with the carbon atom to which they are attached form a C₃-C₁₂ cycloalkyl group or each independently is selected from:
(1) hydrogen; or
(2) C₁C₁₂ alkyl;
L is selected from:
a) -CH₃;
b) -CH₂CH₃;
c) -CH(OH)CH₃;
d) C₁-C₆ alkyl, optionally substituted with one or more substituents selected from aryl, substituted aryl, heteroaryl, and substituted heteroaryl;
e) C₂-C₆ alkenyl, optionally substituted with one or more substituents selected from aryl, substituted aryl, heteroaryl, and substituted heteroaryl; or
f) C₂-C₆ alkynyl, optionally substituted with one or more substituents selected from aryl, substituted aryl, heteroaryl, and substituted heteroaryl;
W is -NR₂₀R₂₁, where R₂₀ and R₂₁ are each independently selected from:
a) hydrogen;
b) C₁-C₁₂ alkyl, optionally substituted with one or more substituents selected from halogen, aryl, substituted aryl, heteroaryl and substituted heteroaryl;
c) C₂-C₁₂ alkenyl, optionally substituted with one or more substituents selected from halogen, aryl, substituted aryl, heteroaryl and substituted heteroaryl;
d) C₂-C₁₂ alkynyl, optionally substituted with one or more substituents selected from halogen, aryl, substituted aryl, heteroaryl and substituted heteroaryl;or
e) R₂₀ and R₂₁, taken together with the nitrogen atom to which they are attached form a heterocycloalkyl moiety; or
Z is selected from:
a) hydrogen;
b) methyl; or
c) halogen; and
R₂' is hydrogen or Rₚ, where Rₚ, is as previously defined.

2. A compound according to claim 1 which is represented by the formula: 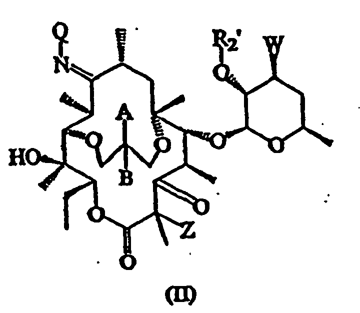 wherein A, B, R₂', Q, W, and Z are as defined in claim 1.

3. A compound according to claim 1 which is represented by the formula: 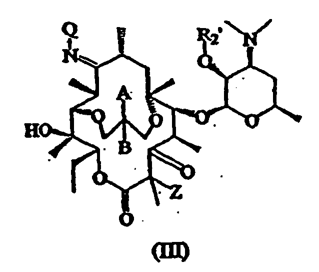 wherein A, B, R₂', Q, and Z are as defined in claim 1.

4. A compound according to claim 1 which is represented by the formula: 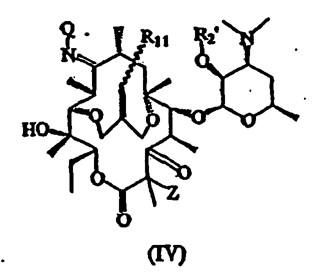 wherein R₁₁, R₂', Q, and Z are as defined in claim 1.

5. A compound according to claim 1 which is represented by the formula: 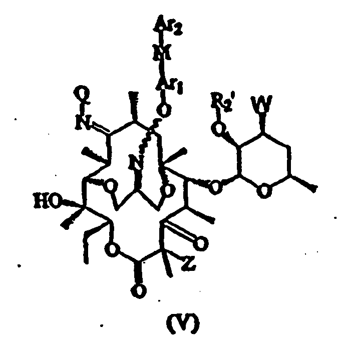 wherein Ar₁, Ar₂, R₂', M, Q, W, and Z are as defined in claim 1.

6. A compound according to claim 1 which is represented by the formula: 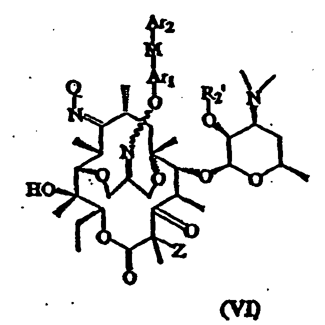 wherein Ar₁, Ar₂, R₂', M, Q, and Z are as defined in claim 1.

7. A compound according to claim 1 which is represented by the formula: 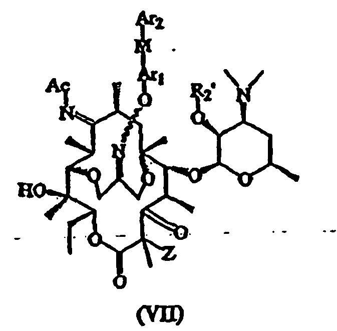 wherein Ar₁, Ar₂, R₂', M, and Z are as defined in claim 1.

8. A compound according to claim 1 which is represented by the formula: 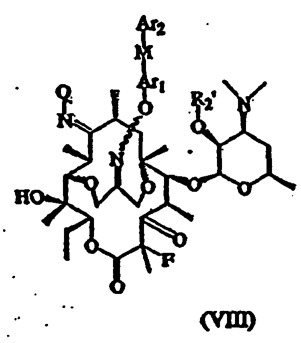 wherein Ar₁, Ar₂, R_{2'}, M, and Q are as defined in claim 1.

9. A compound according to claim 1 which is selected from:
(1) compound of formula I: A and B taken together with the carbon atom to which they attached are C=CH₂, X and Y taken together with the carbon atom to which they are attached are C=N=Ac, L = CH₂CH₃, Z = H, and R₂' = Ac;
(2) Compound of formula I: A and B taken together with the carbon atom to which they are attached are C=CH₂, X and Y taken together with the carbon atom to which they are attached are C=N=Ac, L = CH₂CH₃, Z = H and R₂' = H;
(3) Compound of formula I: A and B taken together with the carbon atom to which they are attached are C=O, X and Y taken together with the carbon atom to which they attached are C=N-Ac, L = CH₂CH₃, Z = H and R₂' = H;
(4) Compound of formula I: A and B taken together with the carbon atom to which they are attached are C=N-O-CH₂-Ph, X and Y taken together with the carbon atom to which they are attached are C=N-Ac, L = CH₂CH₃, Z = H and R₂' = H;
(5) Compound of formula I: A and B taken together with the carbon atom to which they are attached are C=N-O-CH₂-(3-pyridyl), X and Y taken together with the carbon atom to which they are attached are C=N-Ac, L = CH₂CH₃, Z = H and R₂' = H;
(6) Compound of formula I: A and B taken together with the carbon atom to which they are attached are C=N-O-CH₂-(2-pyridyl), X and Y taken together with the carbon atom to which they are attached are C=N-Ac, L = CH₂CH₃, Z = H and R₂' = H;
(7) Compound of formula I: A and B taken together with the carbon atom to which they are attached are C=N-O-CH₂-(3-quinolyl), X and Y taken together with the carbon atom to which they are attached are C=N-Ac, L = CH₂CH₃, Z = H and R₂' = H;
(8) Compound of formula I: A and B taken together with the carbon atom to which they are attached are C=N-O-CH₂-(2-quinolyl), X and Y taken together with the carbon atom to which they are attached are C=N-Ac, L = CH₂CH₃, Z = H and R₂' = H;
(9) Compound or formula I: A and B taken together with the carbon atom to which they are attached are C=N-O'-(5-pyridin-2-ylthiophen-2yl)-methyl, X and Y taken together with the carbon atom to which they are attached are C=N-Ac, L = CH₂CH₃, Z = H and R₂' = H;
(10) Compound of formula I: A and B taken together with the carbon atom to which they are attached are C=N-O-(3-pyrimidin-2-ylprop-2-ynyl), X and Y taken together with the carbon atom to which they are attached are C=N-Ac, L = CH₂CH₃, Z = H and R₂' = H;
(11) Compound of formula I: A and B taken together with the carbon atom to which they are attached are C=N-O-Ph, X and Y taken together with the carbon atom to which they are attached are C=N-Ac, L = CH₂CH₃, Z = H and R₂' = H;
(12) Compound of formula I: A = NHCH₂-Ph, B = H, X and Y taken together with the carbon atom to which they are attached are C=N-Ac, L = CH₂CH₃, = H and R₂' = H;
(13) Compound of formula I: A = NHCH₂CH₂-Ph, B = H, X and Y taken together with the carbon atom to which they are attached are C=N-Ac, L = CH₂CH₃, Z = H and R₂' = H;
(14) Compound of formula I: A and B taken together with the carbon atom to which they are attached are C=CH₂, X and Y taken together with the carbon atom to which they are attached are C=N-O-CH₂-O-CH₃, L = CH₂CH₃, Z = H, and R₂' = Ac;
(15) Compound of formula I: A and B taken together with the carbon atom to which they are attached are C=CH₂, X and Y taken together with the carbon atom to which they are attached are C=N-O-CH₂-O-CH₃, L = CH₂CH₃, Z = H and R₂' = H;
(16) Compound of formula I: A and B taken together with the carbon atom to which they are attached are C=O, X and Y taken together with the carbon atom to which they are attached are C=N-O-CH₂-O-CH₃, L = CH₂CH₃, Z = H and R₂' = H;
(17) Compound of formula I: A and B taken together with the carbon atom to which they are attached are C=NOCH₂-Ph, X and Y taken together with the carbon atom to which they are attached are C=N-O-CH₂-O-CH₃, L = CH₂CH₃, Z = H and R₂' = H;
(18) Compound of formula I: A and B taken together with the carbon atom to which they are attached are C=O, X and Y taken together with the carbon atom to which they are attached are C=O, L = CH₂CH₃, Z = H, and R₂' = Ac;
(19) Compound of formula I: A and B taken together with the carbon atom to which they are attached are C=CH₂, X and Y taken together with the carbon atom to which they are attached are C=O, L = CH₂CH₃, Z = H, and R₂' = H;
(20) Compound of formula I: A and B taken together with the carbon atom to which they are attached are C=O, X and Y taken together with the carbon atom to which they are attached are C=O, L = CH₂CH₃, Z = H, and R₂' = H;
(21) Compound of formula I: A and B taken together with the carbon atom to which they are attached are C=N-O-CH₂-Ph, X and Y taken together with the carbon atom to which they are attached are C=O, L = CH₂CH₃, Z = H, and R₂' = H;
(22) Compound of formula I: A and B taken together with the carbon atom to which they are attached are C=CH₂, X and Y taken together with the carbon atom to which they are attached are C=NH, L = CH₂CH₃, Z = H, and R₂' = H;
(23) Compound of formula I: A and B taken together with the carbon atom to which they are attached are C=CH-O-CH₂-*p*-NO₂-Ph, X and Y taken together with the carbon atom to which they are attached are C=N-Ac, L = CH₂CH₃, Z = H, and R₂' = H;
(24) Compound of formula I: A and B taken together with the carbon atom to which they are attached are C=N-O-(CH₂)₂-Ph, X and Y taken together with the carbon atom to which they are attached are C=N-Ac, L = CH₂CH₃, Z = H, and R₂' = H;
(25) Compound of formula I: A and B taken together with the carbon atom to which they are attached are C=N-O-(CH₂)₃-Ph, X and Y taken together with the carbon atom to which they are attached are C=N-Ac, L = CH₂CH₃, Z = H, and R₂' = H;
(26) Compound of formula I: A and B taken together with the carbon atom to which they are attached are C=N-O-CH₂-CH=CH-Pₕ X and Y taken together with the carbon atom to which they are attached are C=N-Ac, L = CH₂CH₃, Z = H, and R₂' = H;
(27) Compound of formula I: A is NH-(CH₂)₃-Ph, B is H, X and Y taken together with the carbon atom to which they are attached are C=N-Ac, L = CH₂CH₃, Z = H, and R₂' = H;
(28) Compound of formula I: A is NH-(CH₂)₄-Ph, B is H, X and Y taken together with the carbon atom to which they are attached are C=N-Ac, L = CH₂CH₃, Z = H, and R₂' = H;
(29) Compound of formula I: A is CH₂-CH=CH₂, B is OH, X and Y taken together with the carbon atom to which they are attached are C=N-Ac, L = CH₂CH₃, Z = H, and R₂' = H;
(30) Compound of formula I: A is CH₂-Ph, B is OH, X and Y taken together with the carbon atom to which they are attached are C=N-Ac, L = CH₂CH₃, Z = H, and R₂' = H;
(31) Compound of formula I: A is Ph, B is OH, X and Y taken together with the carbon atom to which they are attached are C=N-Ac, L = CH₂CH₃, Z = H, and R₂' = H;
(33) Compound of formula I: A is CH₂-CH=CH-Ph, B is OH, X and Y taken together with the carbon atom to which they are attached are C=N-Ac, L = CH₂CH₃, Z = H, and R₂' = H;
(34) Compound of formula I: A is (CH₂)₃-Ph, B is OH, X and Y taken together with the carbon atom to which they are attached are C=N-Ac, L = CH₂CH₃, Z = H, and R₂' = H;
(35) Compound of formula I: A and B taken together with the carbon atom to which they are attached are C=CH-CH=CH-Ph, X and Y taken together with the carbon atom to which they are attached are C=N-Ac, L = CH₂CH₃, Z = H, and R₂' = H;
(36) Compound of formula I: A is (CH₂)₃-Ph, B is H, X and Y taken together with the carbon atom to which they are attached are C=N-Ac, L = CH₂CH₃, Z = H, and R₂' = H;
(37) Compound of formula I: A and B taken together with the carbon atom to which they are attached are C=CH-CH=CH-(3-pyridyl), X and Y taken together with the carbon atom to which they are attached are C=N-Ac, L = CH₂CH₃, Z = H, and R₂' = H;
(38) Compound of formula I: A and B taken together with the carbon atom to which they are attached are C=CH-CH=CH-(3-quinolyl), X and Y taken together with the carbon atom to which they are attached are C=N-Ac, L = CH₂CH₃, Z = H, and R₂' = H;
(39) Compound of formula I: A and B taken together with the carbon atom to which they are attached are C=CH-(2-quinolyl), X and Y taken together with the carbon atom to which they are attached are C=N-Ac, L = CH₂CH₃, Z = H, and R₂' = H;
(40) Compound of formula I: A and B taken together with the carbon atom to which they are attached are C=CH-(2-quinolyl), X and Y taken together with the carbon atom to which they are attached are C=N-H, L = CH₂CH₃, Z = H, and R₂' = H;
(41) Compound of formula I: A and B taken together with the carbon atom to which they are attached are C=CH-(4-biphenyl), X and Y taken together with the carbon atom to which they are attached are C=N-Ac, L = CH₂CH₃, Z = H, and R₂' = H;
(42) Compound of formula I: A and B taken together with the carbon atom to which they are attached are C=CH-(3-biphenyl), X and Y taken together with the carbon atom to which they are attached are C=N-Ac, L = CH₂CH₃, Z = H, and R₂' = H;
(43) Compound of formula I: A and B taken together with the carbon atom to which they are attached are C=CH-(4-phenoxyphenyl), X and Y taken together with the carbon atom to which they are attached are C=N-Ac, L = CH₂CH₃, Z = H, and R₂' = H;
(44) Compound of formula I: A and B taken together with the carbon atom to which they are attached are C=CH-Ph, X and Y taken together the carbon atom to which they are attached are C=N-Ac, L = CH₂CH₃, Z = H, and R₂' = H;
(45) Compound of formula I: A and B taken together with the carbon atom to which they are attached are C=CH-(2-(2pyridyl)-thiophen-5-yl), X and Y taken together with the carbon atom to which they are attached are C=N-Ac, L = CH₂CH₃, Z = H, and R₂' = H; or
(46) Compound of formula I: A and B taken together with the carbon atom to which they are attached are C=O, X and Y taken together with the carbon atom to which they are attached are C=N=Ac, L = CH₂CH₃, Z = F. and R₂' = Ac.

10. A compound according to claim 1, of formula A, selected from compounds delineated in Table 10: 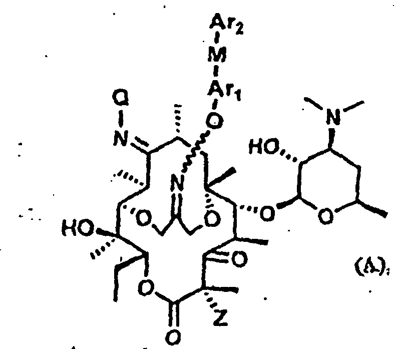
**Table 10**
| Number | Q | -Ar₁-M-Ar₂ | Z |
|---|---|---|---|
| (1). | Ac | 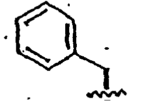 | H |
| (2). | Ac | 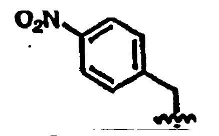 | H |
| (3). | Ac | 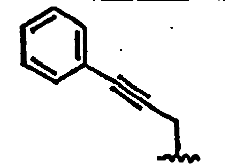 | H |
| (4). | Ac | 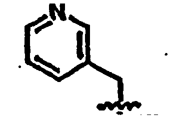 | H |
| (5). | Ac | 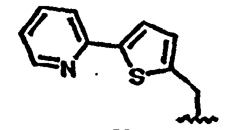 | H |
| (6). | Ac | 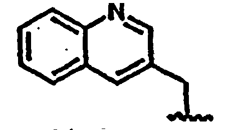 | H |
| (7). | Ac | 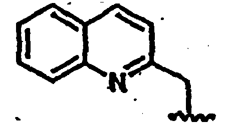 | H |
| (8). | Ac | 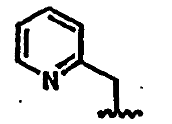 | H |
| (10). | Ac | 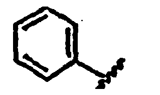 | H |
| (11). | Ac | 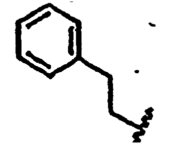 | H |
| (12). | Ac | 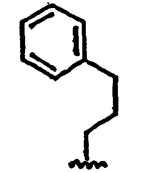 | H |
| (13). | Ac | 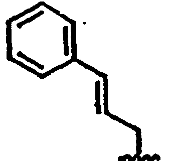 | H |
| (14). | Ac | 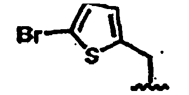 | H |
| (15). | Ac | 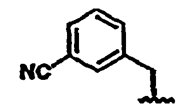 | H |
| (16). | Ac | 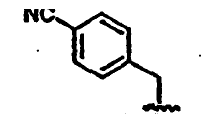 | H |
| (17). | Ac | 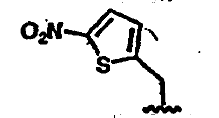 | H |
| (18). | Ac | 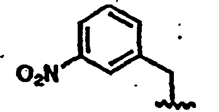 | H |
| (19). | Ac | 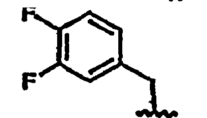 | H |
| (20). | Ac | 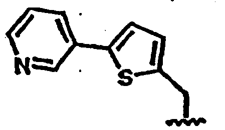 | H |
| (21). | Ac | 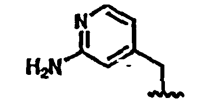 | H |
| (22). | Ac | 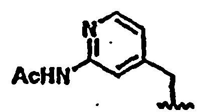 | H |
| (23). | Ac | 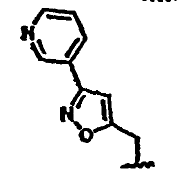 | H |
| (24). | Ac | 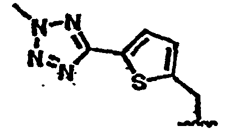 | H |
| (25). | Ac | 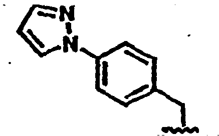 | H |
| (26). | Ac | 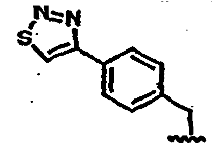 | H |
| (27). | Ac | 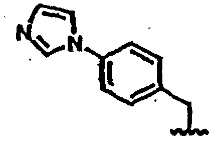 | H |
| (28). | Ac | 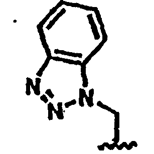 | H |
| (29). | Ac | 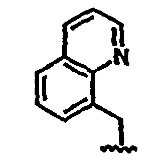 | H |
| (30). | Ac | 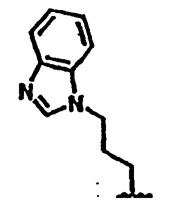 | H |
| (31). | Ac | 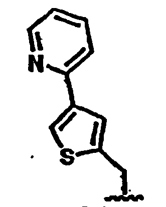 | H |
| (32). | Ac | 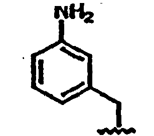 | H |
| (33). | Ac | 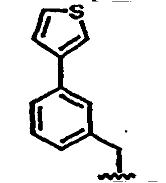 | H |
| (34). | Ac | 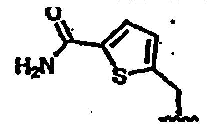 | F |
| (35). | H | 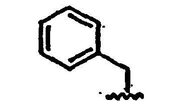 | H |
| (36). | Ac | 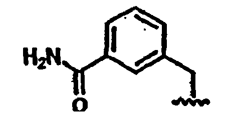 | H |
| (37). | Ac | 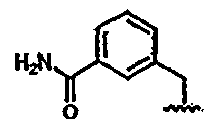 | F |
| (38). | Ac | 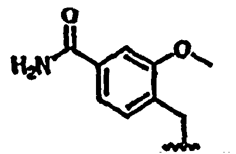 | H |
| (39). | Ac | 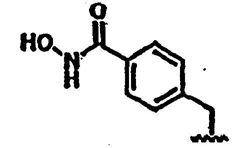 | H |
| (40). | Ac | 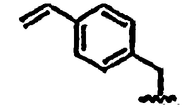 | H |
| (41). | Ac | 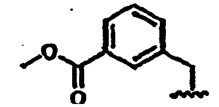 | H |
| (42). | Ac | 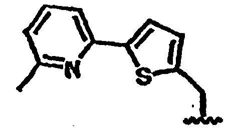 | H |
| (43). | Ac | 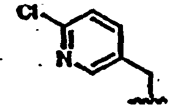 | H |
| (44). | H | 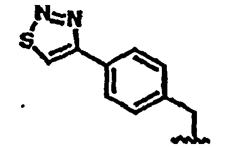 | H |
| (45). | H | 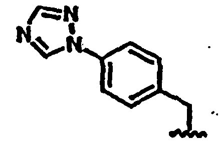 | H |
| (46). | OMe | 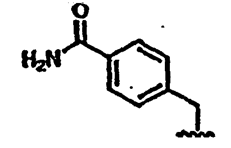 | H |
| (48). | -OCH₂CN | 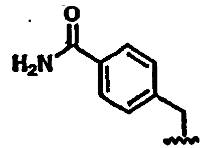 | H |
| (49). | -OCH₂CH₂OH | 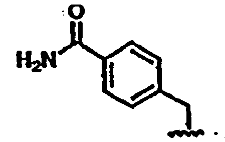 | H |
| (50). | H | 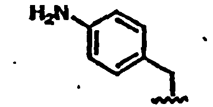 | H |
| (51). | Ac | 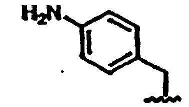 | H |
| (52). | Ac | 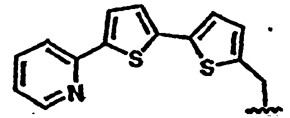 | H |
| (53). | Ac | 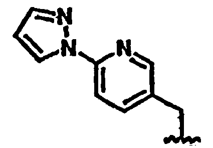 | H |
| (54). | Ac | 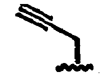 | H |
| (55). | Ac | 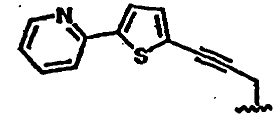 | H |
| (56). | Ac | 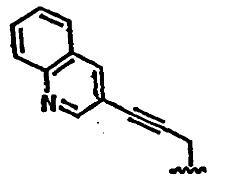 | H |
| (57). | Ac | 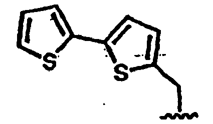 | H |
| (58). | Ac | 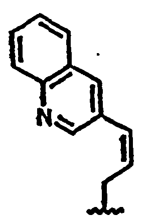 | H |
| (59). | Ac | 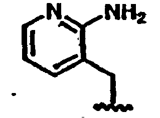 | H |
| (60). | Ac | 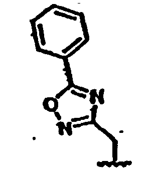 | H |
| (61). | Ac | 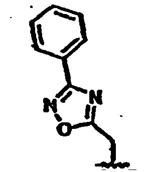 | H |
| (62). | Ac | 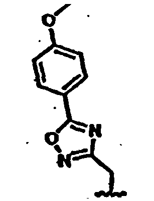 | H |
| (63). | Ac | 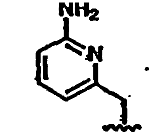 | H |
| (64). | Ac | 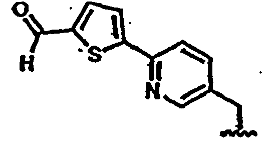 | H |
| (65). | Ac | 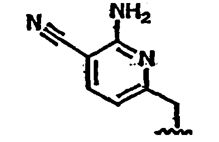 | H |
| (66). | Ac | 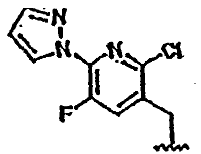 | H |
| (67). | Ac | 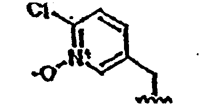 | H |
| (68). | Ac | 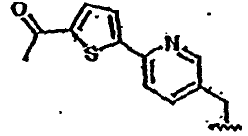 | H |

11. A compound according to claim 1, of formula A1, selected from compounds delineated in Table 11: 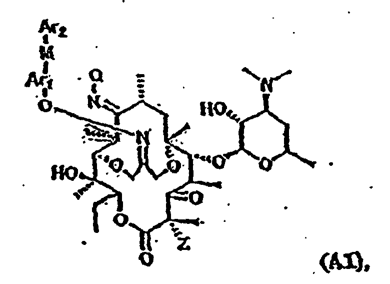
**Table 11**
| Number | Q | -Ar₁-M-Ar₂ | Z |
|---|---|---|---|
| (1). | Ac | 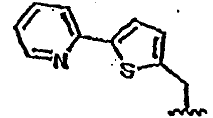 | H |
| (2). | Ac | 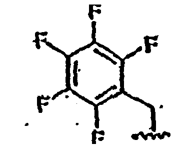 | H |
| (3). | Ac | 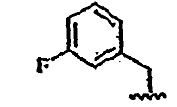 | H |
| (4). | Ac | 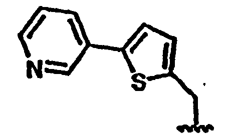 | H |
| (5). | Ac | 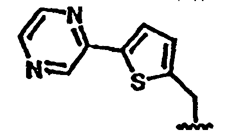 | H |
| (6). | Ac | 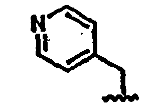 | H |
| (7). | Ac | 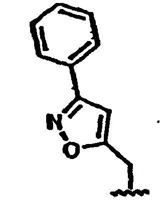 | H |
| (8). | Ac | 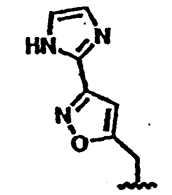 | H |
| (9). | Ac | 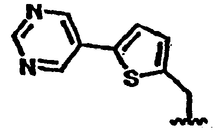 | H |
| (10). | Ac | 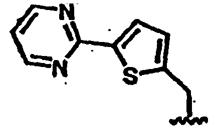 | H |
| (11). | Ac | 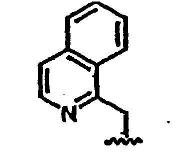 | H |
| (12). | Ac | 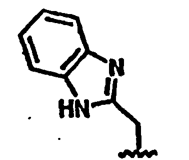 | H |
| (13). | Ac | 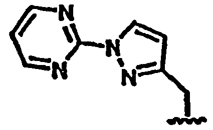 | H |
| (14). | Ac | 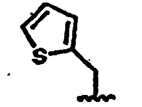 | H |
| (15). | Ac | 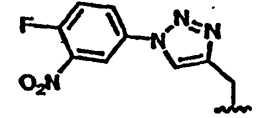 | H |
| (16). | Ac | 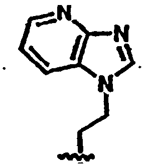 | H |
| (17). | Ac | 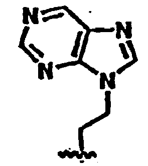 | H |
| (18). | Ac | 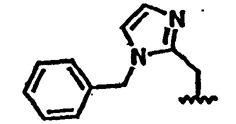 | H |
| (19). | Ac | 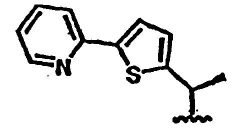 | H |
| (20). | Ac | 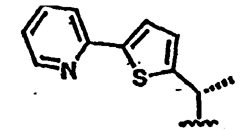 | H |
| (21). | Ac | 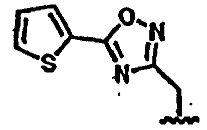 | H |
| (22). | Ac | 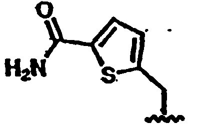 | H |
| (23). | Ac | 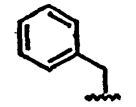 | H |
| (24). | Ac | 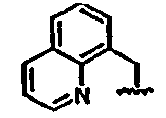 | F |
| (25). | Ac | 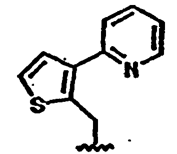 | H |
| (26). | Ac | 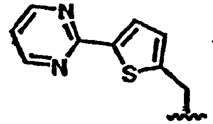 | F |
| (27). | Ac | 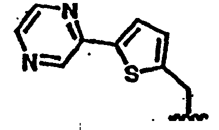 | F |
| (28). | Ac | 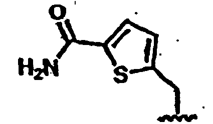 | F |
| (29). | Ac | 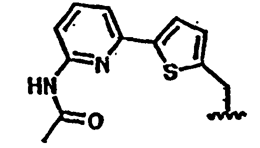 | H |
| (30). | Ac | 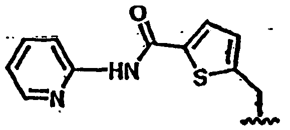 | H |
| (31). | Ac | 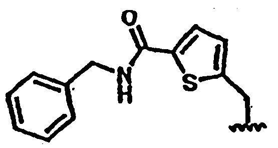 | H |
| (32). | Ac | 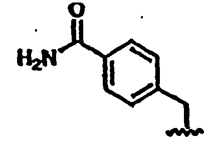 | H |
| (33). | Ac | 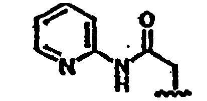 | H |
| (34). | Ac | 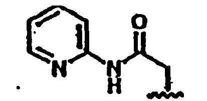 | F |
| (35). | Ac | 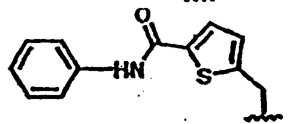 | H |
| (36). | Ac | 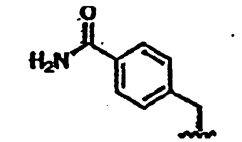 | F |
| (37). | Ac | 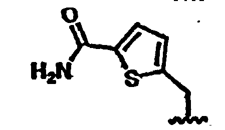 | H |
| (38). | Ac | 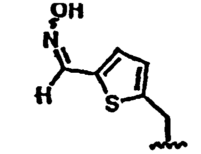 | H |
| (39). | Ac | 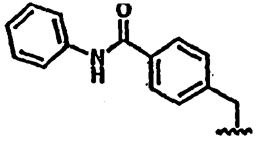 | H |
| (40). | Ac | 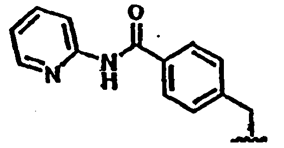 | H |
| (41). | Ac | 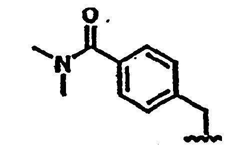 | H |
| (42). | Ac | 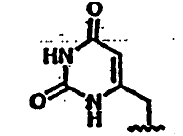 | H |
| (43). | Ac | 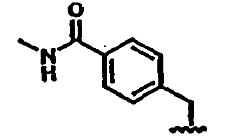 | H |
| (44). | Ac | 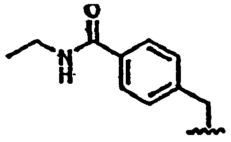 | H |
| (45). | Ac | 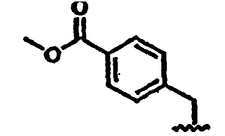 | H |
| (46). | Ac | 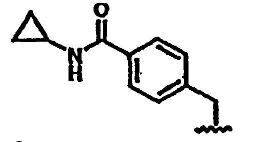 | H |
| (47). | Ac | 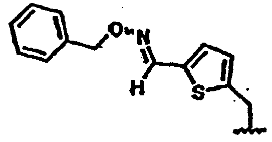 | H |
| (48). | Ac | 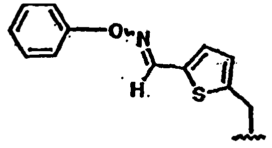 | H |
| (49). | Ac | 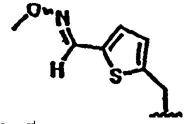 | H |
| (50). | Ac | 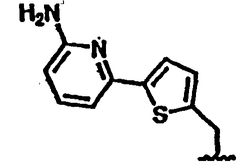 | H |
| (51). | Ac | 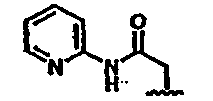 | H |
| (52). | Ac | 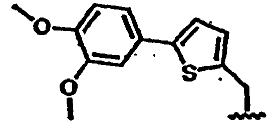 | H |
| (53). | Ac | 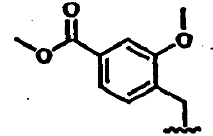 | H |
| (54). | Ac | 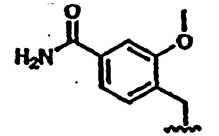 | H |
| (55). | Ac | 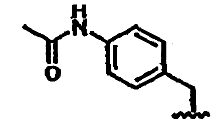 | H |
| (56). | Ac | 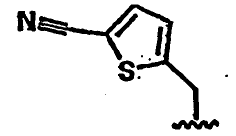 | H |
| (57). | Ac | 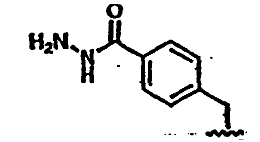 | H |
| (58). | Ac | 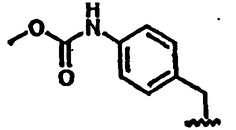 | H |
| (59). | Ac | 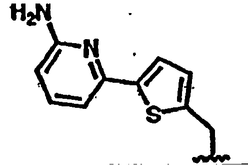 | F |
| (60). | Ac | 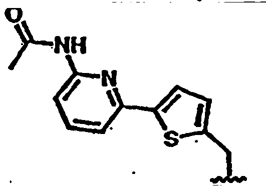 | F |
| (61). | Ac | 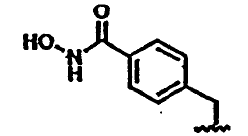 | H |
| (62). | Ac | 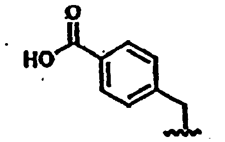 | H |
| (63). | Ac | 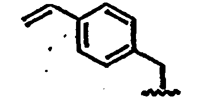 | H |
| (64). | Ac | 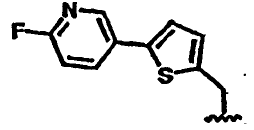 | H |
| (65). | Ac | 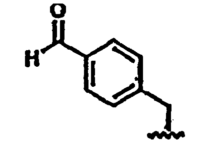 | H |
| (66). | H | 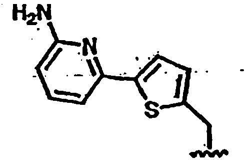 | H |
| (67). | Ac | 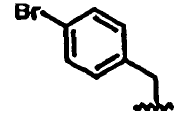 | H |
| (68). | Ac | 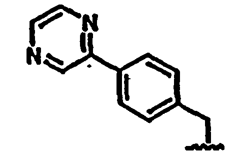 | H |
| (69). | Ac | 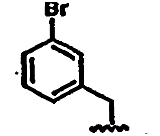 | H |
| (70). | Ac | 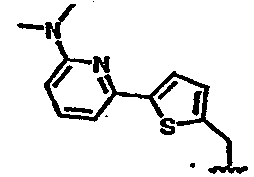 | F |
| (72). | Ac | 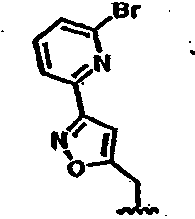 | H |
| (73). | Ac | 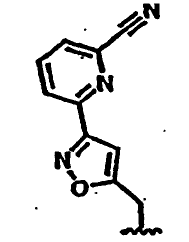 | H |
| (74). | Ac | 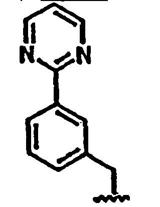 | H |
| (75). | Ac | 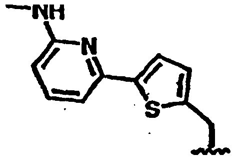 | F |
| (76). | Ac | 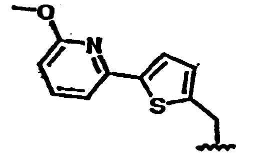 | H |
| (77). | H | 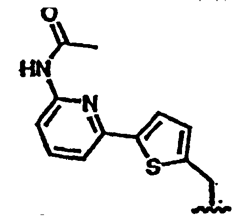 | H |
| (78). | Ac | 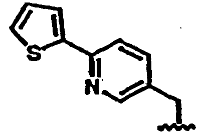 | H |
| (79). | Ac | 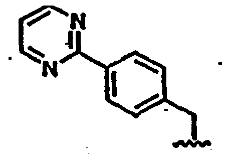 | H |
| (80). | H | 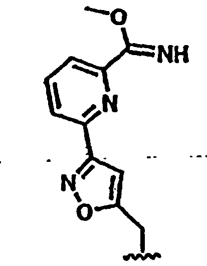 | H |
| (81). | Ac | 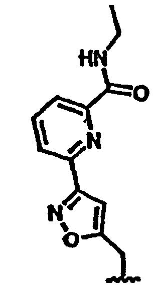 | H |
| (82). | H | 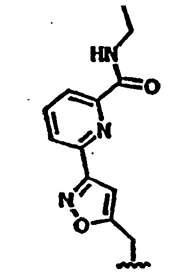 | H |
| (83). | H | 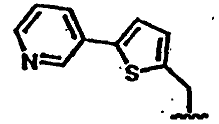 | H |
| (84). | H | 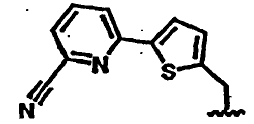 | H |
| (85). | Ac | 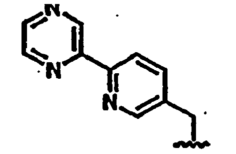 | H |
| (86). | Ac | 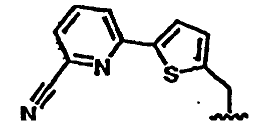 | H |
| (87). | Ac | 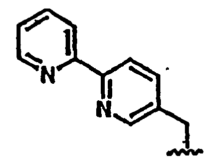 | H |
| (88). | H | 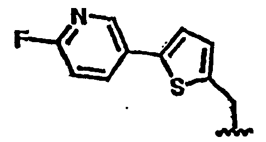 | H |
| (89). | H | 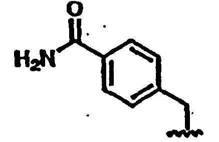 | H |
| (90). | H | 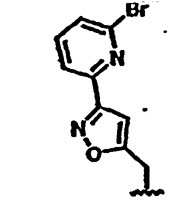 | H |
| (91). | Ac | 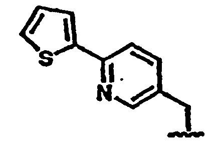 | H |
| (92). | H | 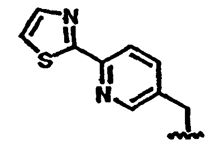 | H |
| (93). | Ac | 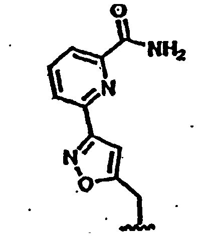 | H |
| (94). | Ac | 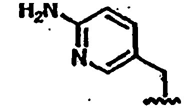 | H |
| (95). | Ac | 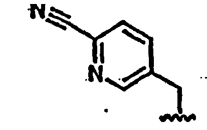 | H |
| (96). | Ac | 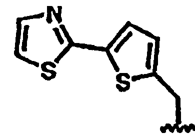 | H |
| (97). | Ac | 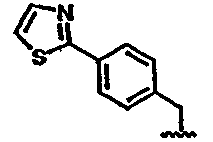 | F |
| (98). | H | 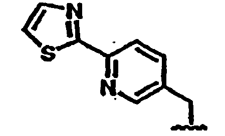 | F |
| (99). | H | 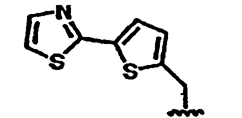 | H |
| (100). | Ac | 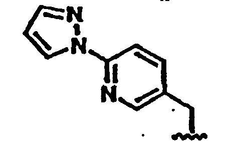 | F |
| (101). | Ac | 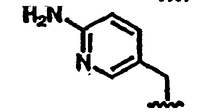 | F |
| (102). | Ac | 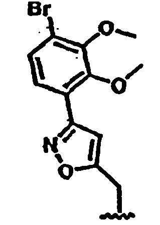 | H |
| (103). | Ac | 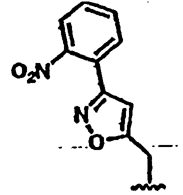 | H |
| (104). | Ac | 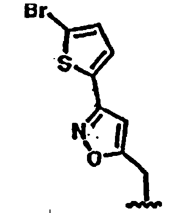 | H |
| (105). | Ac | 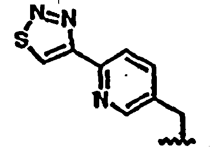 | F |
| (106). | Ac | 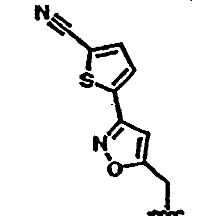 | H |
| (107). | Ac | 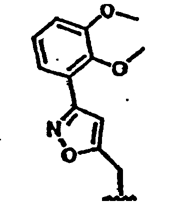 | H |
| (108). | Ac | 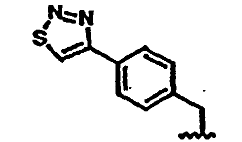 | H |
| (109). | Ac | 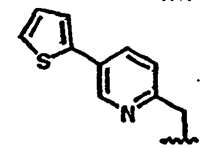 | H |
| (110). | Ac | 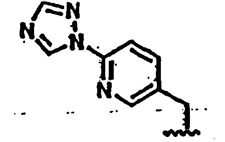 | H |
| (111). | Ac | 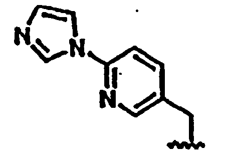 | H |
| (112). | Ac | 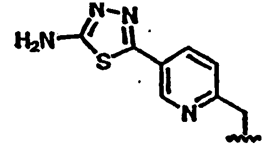 | H |
| (113). | Ac | 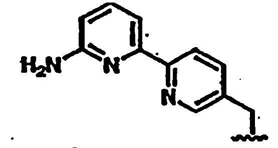 | H |
| (114). | Ac | 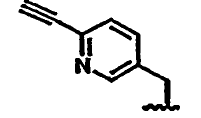 | H |
| (115). | H | 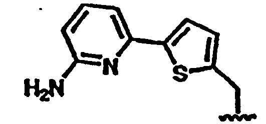 | F |
| (116). | H | 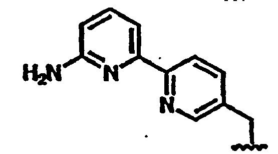 | H |
| (117). | Ac | 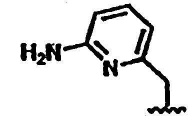 | H |
| (118). | Ac | 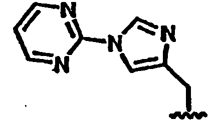 | H |
| (119). | Ac | 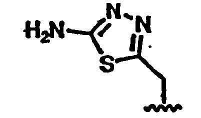 | H |
| (120). | Ac | 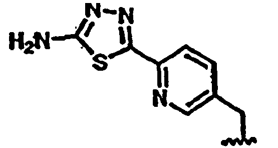 | H |
| (121). | Ac | 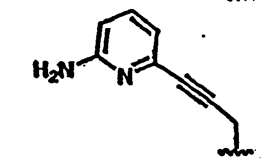 | H |
| (122). | Ac | 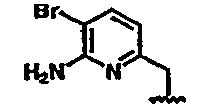 | H |
| (123). | Ac | 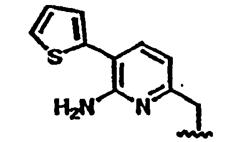 | H |
| (124). | Ac | 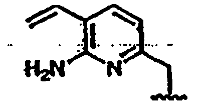 | H |
| (125). | Ac | 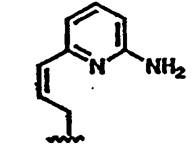 | H |
| (126). | Ac | 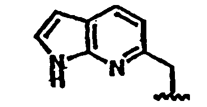 | H |
| (127). | Ac | 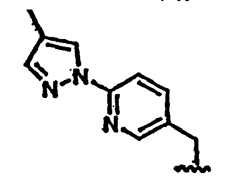 | H |
| (128). | Ac | 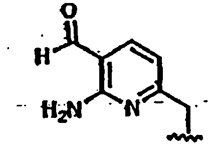 | H |
| (129). | Ac | 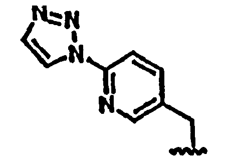 | H |
| (130). | Ac | 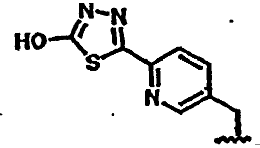 | H |
| (131). | Ac | 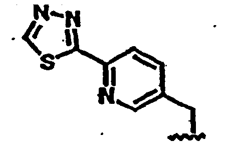 | H |
| (132). | Ac | 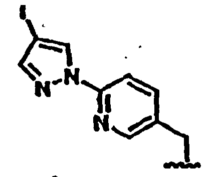 | H |
| (133). | Ac | 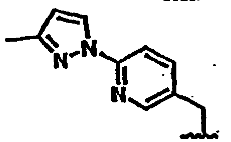 | H |
| (134). | Ac | 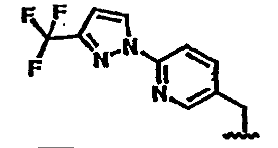 | H |
| (135). | Ac | 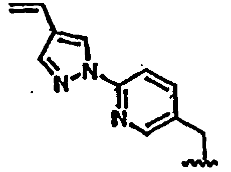 | H |
| (136). | Ac | 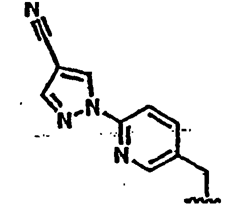 | H |
| (137). | Ac | 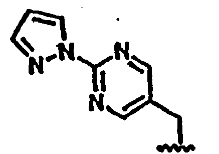 | H |
| (138). | Ac | 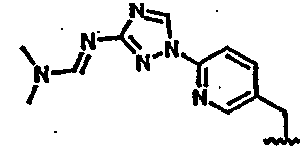 | H |
| (139). | Ac | 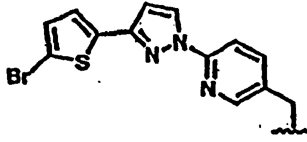 | H |
| (140). | Ac | 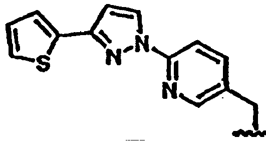 | H |
| (141). | Ac | 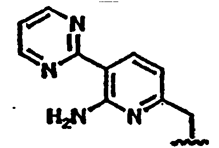 | H |
| (142). | H | 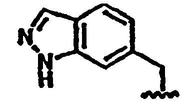 | H |
| (143). | Ac | 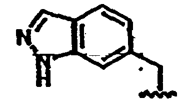 | H |
| (144). | Ac | 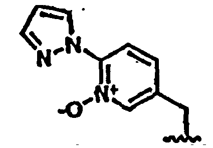 | |
| (145). | Ac | 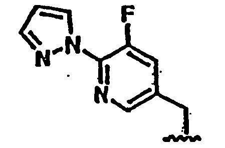 | H |
| (146). | -COCH₂CH₃ | 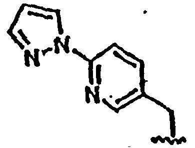 | H |
| (147). | Ac | 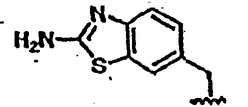 | H |
| (148). | Ac | 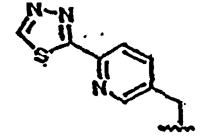 | H |

12. A compound according to claim 1, of formula A2, selected from compounds delineated in Table 12: 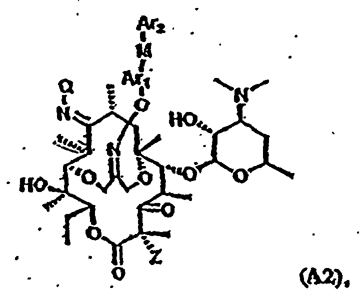
**Table12**
| Number | Q | -Ar₁-M-Ar₂ | Z |
|---|---|---|---|
| (1). | Ac | 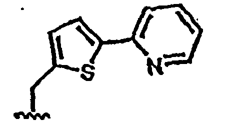 | H |
| (2). | Ac | 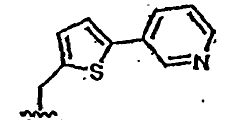 | H |
| (3). | Ac | 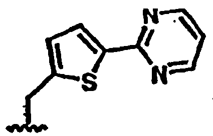 | H |
| (4). | Ac | 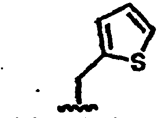 | H |
| (5). | Ac | 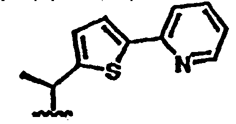 | H |
| (6). | Ac | 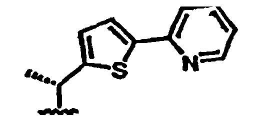 | H |
| (7). | Ac | 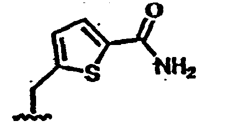 | H |
| (8). | Act | 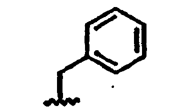 | H |
| (9). | Ac | 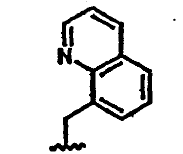 | F |
| (10). | Ac | 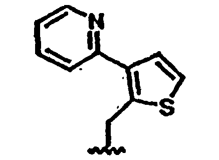 | H |
| (11). | Ac | 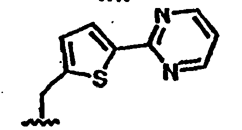 | F |
| (12). | Ac | 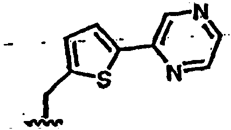 | F |
| (13). | Ac | 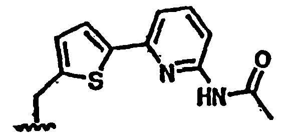 | H |
| (14). | Ac | 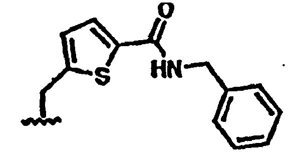 | H |
| (15). | Ac | 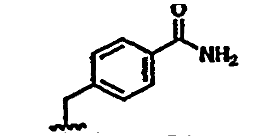 | H |
| (16). | Ac | 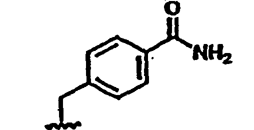 | F |
| (17). | Ac | 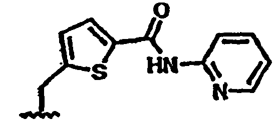 | H |
| (18). | Ac | 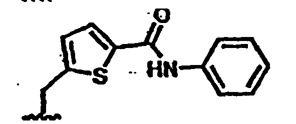 | H |
| (19). | Ac | 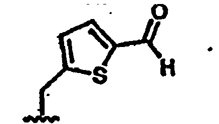 | H |
| (20). | Ac | 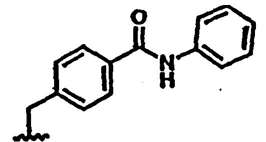 | H |
| (21). | Ac | 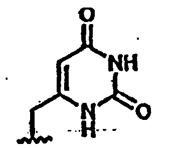 | H |
| (22). | Ac | 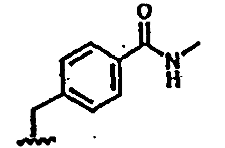 | H |
| (23). | Ac | 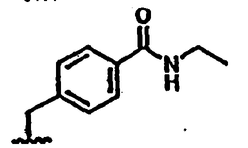 | H |
| (24). | Ac | 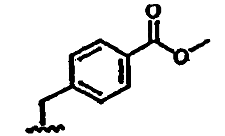 | H |
| (25). | Ac | 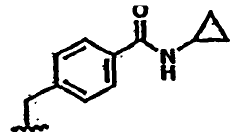 | H |
| (26). | Ac | 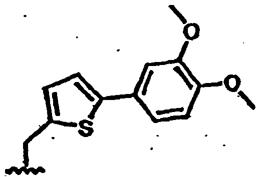 | H |
| (27). | Ac | 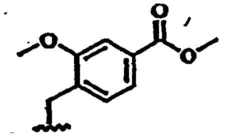 | H |
| (28). | Ac | 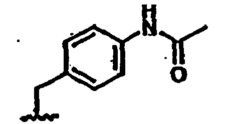 | H |
| (29). | Ac | 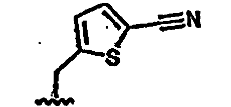 | H |
| (30). | Ac | 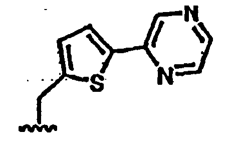 | H |
| (31). | Ac | 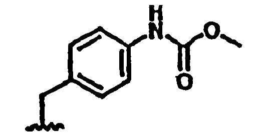 | H |
| (32). | Ac | 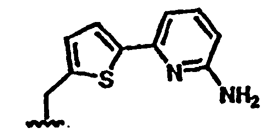 | F |
| (33). | Ac | 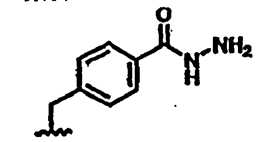 | H |
| (34). | Ac | 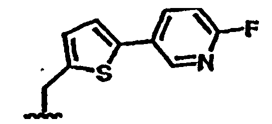 | H |
| (35). | Ac | 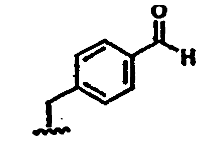 | H |
| (36). | Ac | 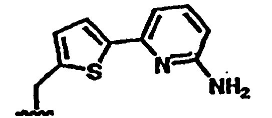 | H |
| (37). | H | 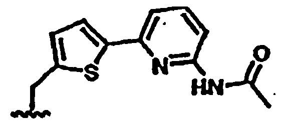 | H |
| (38). | Ac | 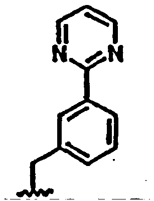 | H |
| (39). | Ac | 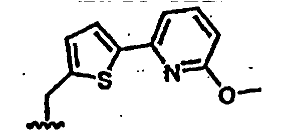 | H |
| (40). | H | 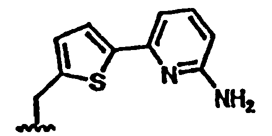 | H |
| (41). | H | 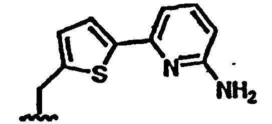 | F |
| (42). | Ac | 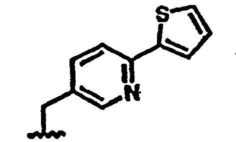 | H |
| (43). | Ac | 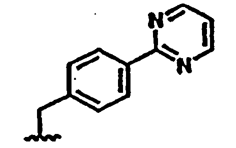 | H |
| (44). | Ac | 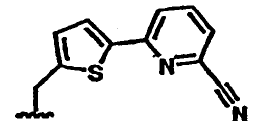 | H |
| (45). | Ac | 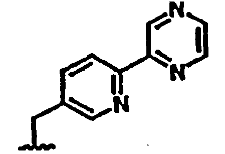 | H |
| (46). | Ac | 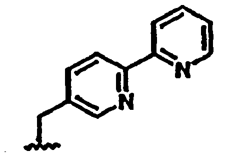 | H |
| (47). | Ac | 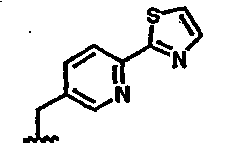 | H |
| (48). | Ac | 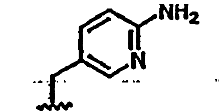 | H |
| (49). | Ac | 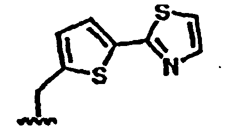 | H |
| (50). | Ac | 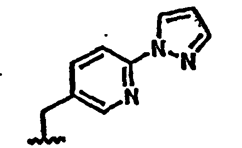 | F |
| (51). | Ac | 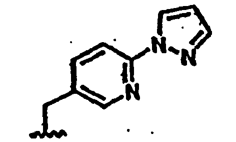 | H |
| (52). | Ac | 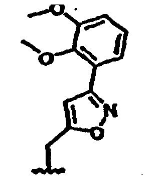 | H |
| (53). | Ac | 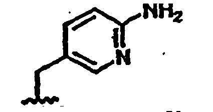 | F |
| (54). | Ac | 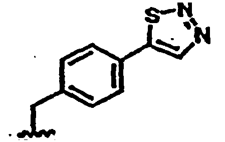 | H |
| (55). | Ac | 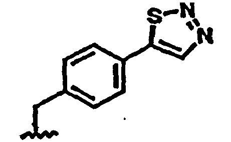 | F |
| (56). | Ac | 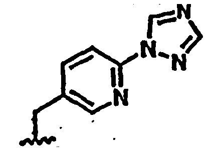 | H |
| (57). | Ac | 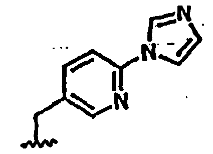 | H |
| (58). | Ac | 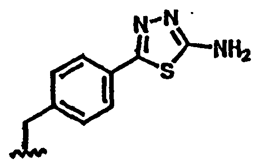 | H |
| (59). | Ac | 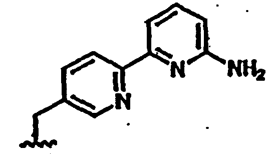 | H |
| (60). | Ac | 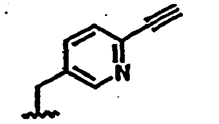 | H |
| (61). | Ac | 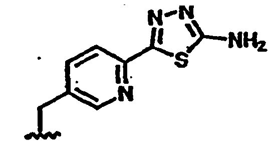 | H |
| (62). | Ac | 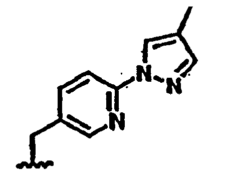 | H |
| (63). | Ac | 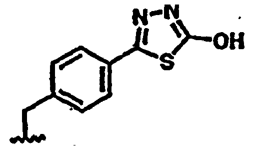 | H |
| (64). | Ac | 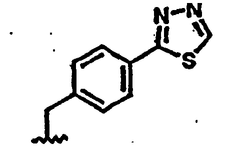 | H |
| (65). | Ac | 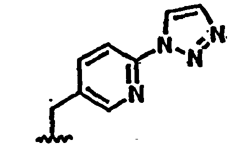 | H |
| (66). | Ac | 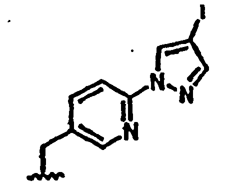 | H |
| (67). | Ac | 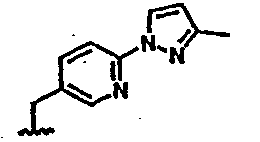 | H |
| (68). | Ac | 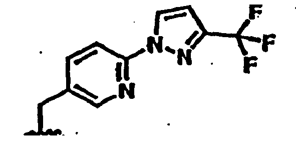 | H |
| (69). | Ac | 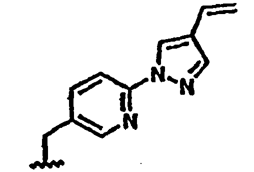 | H |
| (70). | Ac | 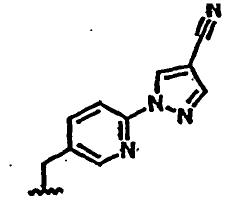 | H |
| (71). | Ac | 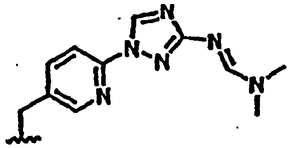 | H |
| (72). | Ac | 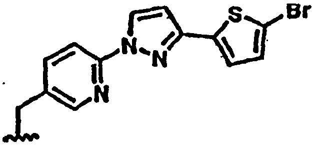 | H |
| (73). | Ac | 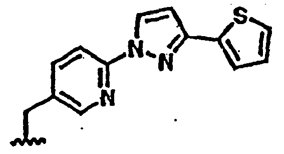 | H |
| (74). | H | 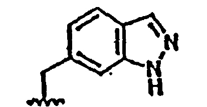 | H |
| (75). | Ac | 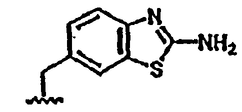 | H |

13. A compound according to claim 1, of formula B, selected from compounds delineated in Table 13: 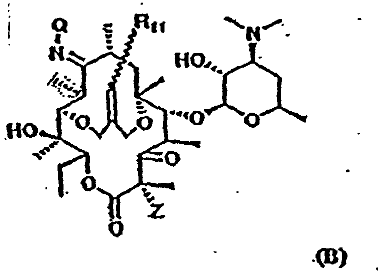
**Table 13**
| Number | Q | R₁₁ | Z |
|---|---|---|---|
| (1). | H | H | H |
| (2). | MOM | H | H |
| (3). | MOM | 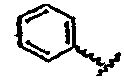 | H |
| (4). | Ac | 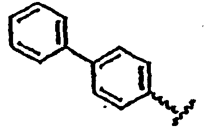 | H |
| (5). | Ac | 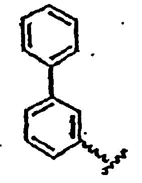 | H |
| (6). | Ac | 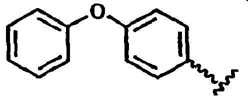 | H |
| (7). | Ac | 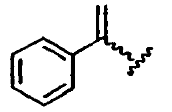 | H |
| (8). | Propionyl | 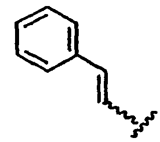 | H |
| (9). | Ac | 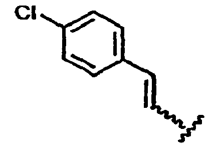 | H |
| (10). | -C(O)OMe | 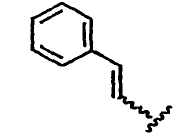 | H |
| (11). | -C(O)NH₂ | H | H |
| (12). | Me | H | H |
| (13). | BOM | H | H |
| (14). | Ac | 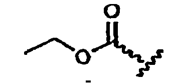 | H |
| (15). | Ac | 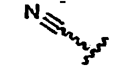 | H |

14. A compound according to claim 1, of formula B1, selected from compounds delineated in Table 14: 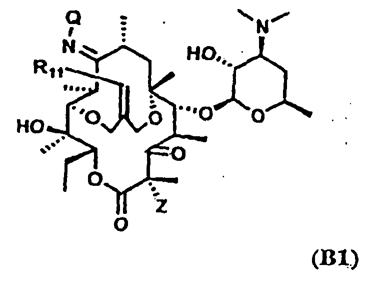
**Table 14**
| Number | Q | R₁₁ | Z |
|---|---|---|---|
| (1). | Ac | 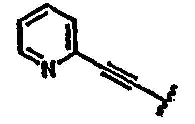 | H |
| (2). | Ac | 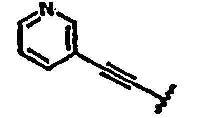 | H |
| (4). | Ac | 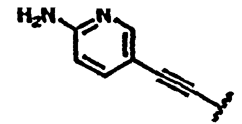 | H |
| (5). | Ac | 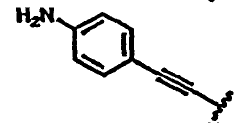 | H |
| (6). | Ac | 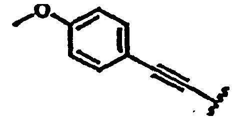 | H |
| (7). | Ac | 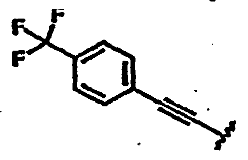 | H |
| (8). | Ac | 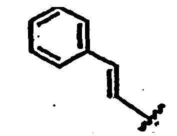 | F |
| (9). | Ac | 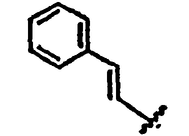 | H |
| (10). | Ac | 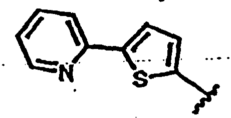 | H |
| (11). | Ac | 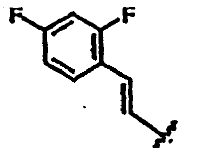 | H |
| (12). | 2-methoxyacetyl | 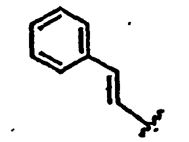 | H |
| (13). | 2-O-acyl-acetyl | 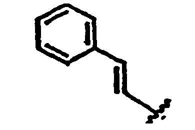 | H |
| (14). | 2-Fmoc-acetyl | 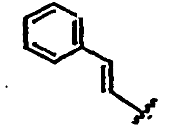 | H |
| (15). | Ac | 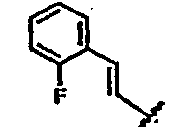 | H |
| (16). | Ac | 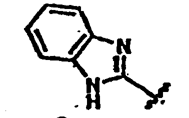 | H |
| (17). | 2-hydroxcy acetyl | 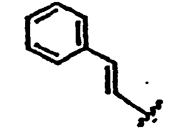 | H |
| (18). | 2-aminoacetyl | 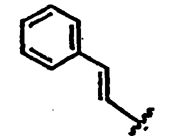 | H |
| (19). | Ac | 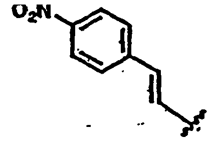 | H |
| (20). | Ac | 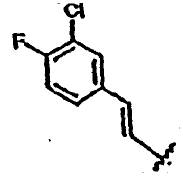 | H |
| (21). | Ac | 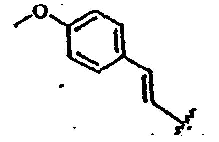 | H |
| (22). | Ac | 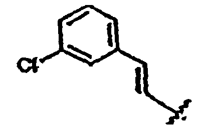 | H |

15. A according to claim 1, of formula B2, selected from compound delineated in Table 15: 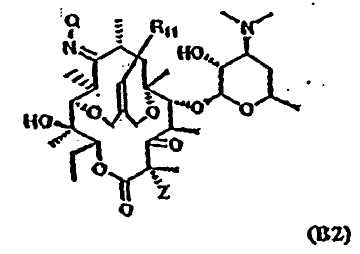
**Table 15**
| Number | Q | R₁₁ | Z |
|---|---|---|---|
| (1). | Ac | 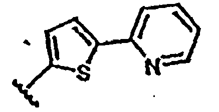 | H |
| (2). | Ac | 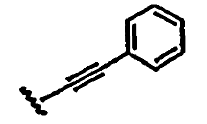 | H |
| (3). | Ac | 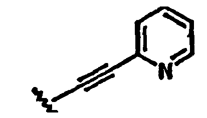 | H |
| (4). | Ac | 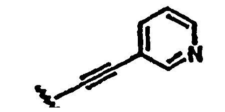 | H |
| (5). | Ac | 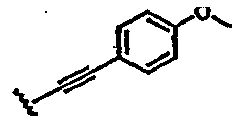 | H |
| (6). | Ac | 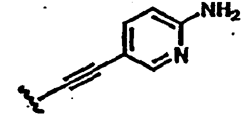 | H |
| (7). | Ac | 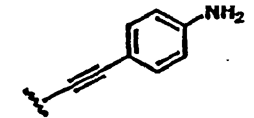 | H |
| (8). | Ac | 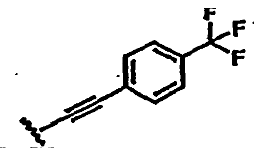 | H |
| (9). | Ac | 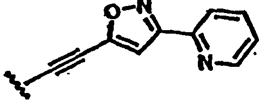 | H |

16. A compound according to claim 1, of formula C, selected from compounds delineated in Table 16: 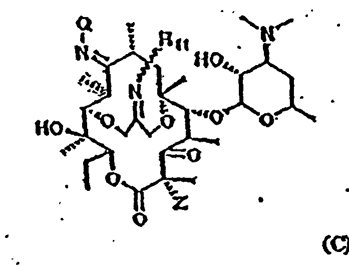
**Table 16**
| Number | Q | R₁₁ | Z |
|---|---|---|---|
| (1). | Ac | 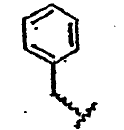 | H |
| (2). | Ac | 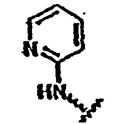 | H |
| (3). | Ac | 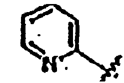 | H |
| (4). | Ac | 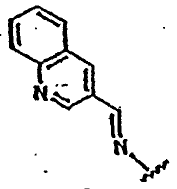 | H |
| (5). | Ac | 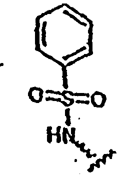 | H |

17. A compound, according to claim 7 or 10, represented by formula: 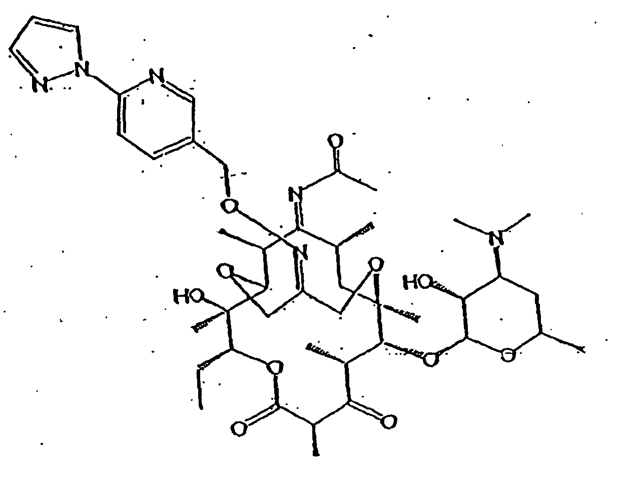 and pharmaceutically acceptable salts, esters, racemic mixtures and stereoisomers thereof.

18. A compound, according to claims 7 or 11, represented by formula: 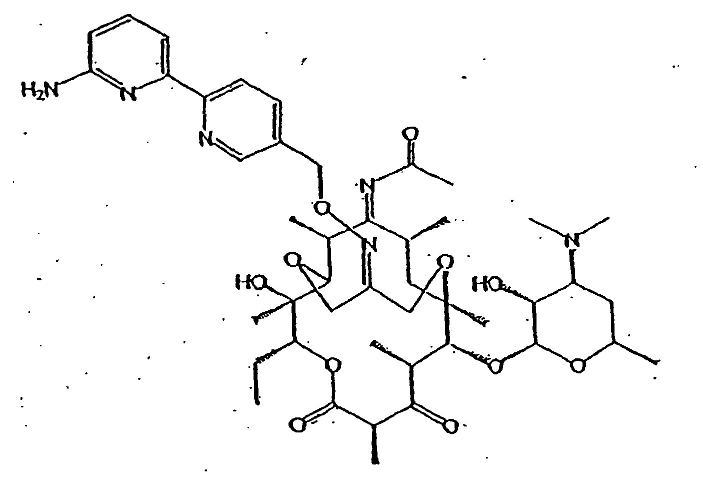 and pharmaceutically acceptable salts, esters, racemic mixtures and stereoisomers thereof.

19. A compound according to any of claims 1 to 18 wherein the compound is a pharmaceutically acceptable salt.

20. A compound according to any of claims 1 to 18 wherein the compound is a free base.

21. A compound according to any of claims 1 to 18 wherein the compound is an ester.

22. A pharmaceutical composition comprising a therapeutically effective amount of a compound of any one of claims 1 to 21 or a pharmaceutically-acceptable salt, or ester thereof, in combination with a pharmaceutically acceptable carrier.

23. The use of the compound of any of claims 1 to 21 for the manufacture of a medicament, for the control of a bacterial infection in a subject in need of such treatment.

24. The use as claimed in claim 23 wherein the medicament is for administration orally, parenterally, by inhalation spray, topically, rectally, nasally, buccally, vaginally or via an implanted reservoir.

25. The use as claimed in claim 24 wherein the medicament is for administration orally or by injection.

26. The use as claimed in claim 23 wherein the medicament is for administration to humans.

27. The use as claimed in any of claims 24 to 26 wherein the medicament is administered in combination with one or more additional therapeutic or prophylactic agents.

28. The use as claimed in claim 27 wherein the additional therapeutic or prophylactic agent is administered separately, as part of a multiple dose regimen, or in single dose form.

29. The use as claimed in any of claims 24 to 28 wherein the medicament is used to treat a protozoa infection or bacterial infection and disorders related to such infections.

30. The use as claimed in claim 29 wherein the infection or disorder is selected from the group consisting of pneumonia, otitis media, sinusitis, bronchitis, tonsillitis, and mastoiditis related to infection Streptococcus pneumoniae, Haemophilus influenzae, Moraxella catarrhalis, Staphylococcus aureus, or Peptostreptococcus app. Pseudomonas spp.; pharynigitis, rheumatic fever, and glomerulonephritis related to infection by Streptococcus pyogenes, Groups C and G streptococci, Clostridium diptheriae, or Actinobacillus haemolyticum; respiratory tract infections related to infection by Mycoplasma pneumoniae, Legionella pneumophila, Streptococcus pneumoniae, Haemophilus influenzae, or Chlamydia pneumoniae, uncomplicated skin and soft tissue infections, abscesses and osteomyelitis, and puerperal fever related to infection by Staphylococcus aureus, coagulase-positive staphylococci (i.e. S. epidermidis, S. hemolyticus, etc.), S. pyogenes, S. agalactiae, Streptococcal groups C-F (minute-colony streptococci), viridans streptococci, Corynebacterium spp., Clostridium spp., or Bartonella henselae; uncomplicated acute urinary tract infections related to infection by S. saprophyticus or Enterococcus spp.; urethritis and cervicitis; and sexually transmitted diseases related to infection by Chlamydia trachomatis, Haemophilus ducreyi, Treponema pallidum, Ureaplasma urealyticum, or Nesseria gonorrhea; toxin diseases related to infection by S. aureus, or Groups A, S. and C streptococci; ulcers related to infection by Helicobacter pylori; systemic febrile syndromes related to infection by Borrelia recurrentis; Lyme disease related to infection by Borrelia burgdorferi; conjunctivitis, keratitis, and dacrocystitis related to infection by C. trachomatis, N. gonorrhoeae, S. aureus, S. pneumoniae, S. pyogenes, H. influenzae, or Listeria spp.; disseminated Mycobacterium avium complex (MAC) disease related to infection by Mycobacterium avium, or Mycobacterium intracellulare; gastroenteritis related to infection by Campylobacter jejuni; intestinal protozoa related to infection by Cryptosporidium spp. odontogenic infection related infection by Bordetella pertussis, gas gangrene related to infection by Clostridium perfringens or Bacteroides spp.; Skin infection by S. aureus, Propionibacterium acne; atherosclerosis related to infection by Helicobacter pylori and Chlamydia pneumoniae.

31. The use as claimed in claim 30 wherein the infection or disorder is pneumonia, otitis media, sinusitis, bronchitis, tonsillitis, *p.acne,* and skin and soft tissue infections.

32. The use as claimed in claim 29 wherein the infection or disorder the medicament is used to treat is selected from the group consisting of bovine respiratory disease related to infection by P. haemolytica., P. multocida, Mycoplasma bovis, or Bordetella spp.; cow enteric disease related to infection by E. coli or protozoa, diary cow mastitis related to infection by S. aureus, S. uberis, S. agalactiae, S. dysgalactiae, Klebsiella spp., Corynebacterium, or Enterococcus spp.; swine respiratory disease related to infection by A. pleuropneumoniae., P. multocida, or Mycoplasma spp.; swine enteric disease related to infection by E. coli, Lawsonia intracellularis, Salmonella spp., or Serpulina hyodyisinteriae; cow footrot related to infection by Fusobacterium spp., cow metritis related to infection by E. coli; cow hairy warts related to Infection by Fusobacterium necrophorum or Bacteroides nodosus; cow pink-eye related to infection by Moraxella bovis, cow premature abortion related to infection by protozoa; urinary tract infection in dogs and coats related to infection by S. epidermidis, S. intermedius, coagulase neg. Staphylococcus or P. multocida; and dental or mouth infections in dogs and cats related to infection by Alcaligenes spp.; Bacteroides spp., Clostridium spp., Enterocater spp., Eubacterium spp., Peptostreptococcus spp., Porphfyromonas spp., Campylobacter spp., Actinomyces spp., Erysipelothrix spp., Rhodococcus spp., Trypanosoma spp., Plasmodium spp., Babesia spp., Toxoplasma spp., Pneumocystis spp., Leishmania spp., and Trichomonas spp. and Prevotella spp.

33. A process for preparing a compound according to claim 1, represented by the formula 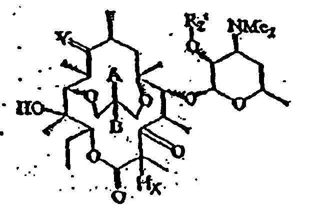 Where V is selected from N-Q or O; Hx is halogen or methyl; and A, B, Q and R₂' are as defined in claim 1, comprising the step of
(a) reacting a compound represented by the formula 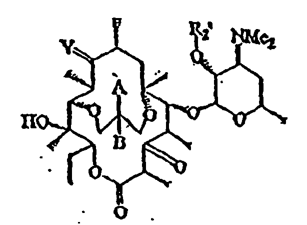 Where A, B, V and R₂' are as previously defined, with a halogentating agent or methylating agent in the presence of a base.

34. A process for preparing a compound according to claim 1, represented by the formula 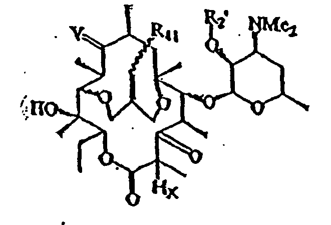 where V, is selected from N-Q or O; Hx is halogen or methyl; and Q, R₁₁ and R₂' are as defined in claim 1, comprising the step of
(a) reacting a compound represented by the formula 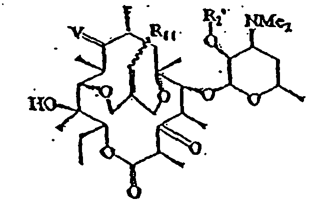 where V, R₁₁ and R₂' are as previously defined, with a halogenating agent in the presence of a base.

35. A process for preparing a compound according to claim 1, represented by the formula 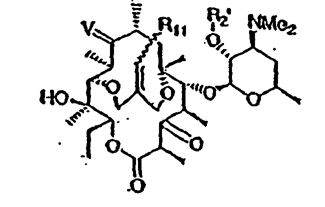 where V is selected from N-Q or O; and R₁₁, Q and R₂' are as defined in claim 1, comprising the step of
(a) reacting a compound represented by the formula 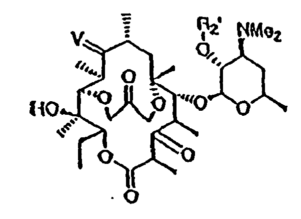 where V and R₂' are as previously defined with a phosphonium compound in the presence of a base.

36. A process for the preparation of a compound according to claim 1, represented by the formula 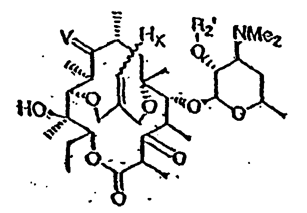 where V is selected from N-Q or O; Hx is halogen; and R₁₁, Q, and R₂' are as defined in claim 1, comprising the step of:
(a) reacting a compound represented by the formula 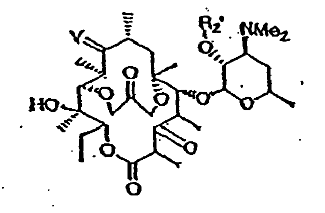 where V and R₂' are as previously defined with a phosphonium salt in the presence of a base.

37. A process for the preparation of a compound according to claim 1, represented by the formula 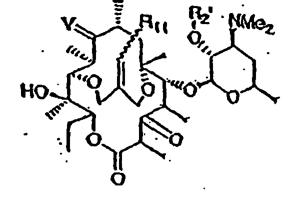 where V is selected from N-Q or Q; and R₁₁, Q and R₂' are as defined in claim 1, comprising the step of:
(a) reacting a compound represented by the formula 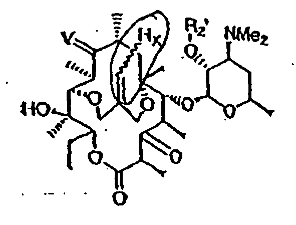 where Hx is halogen and V and R₂' are as previously defined, with an organoboron or an organotin compound in the presence of a palladium catalyst and a base.

38. A process for the preparation of a compound according to claim 1, represented by the formula 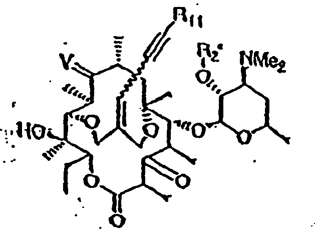 where V is selected from N-Q or O; and R₁₁, Q and R₂' are as defined in claim 1, comprising the step of:
(a) reacting a compound represented by the formula 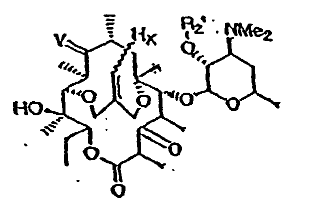 where Hx is halogen and V and R₂' are as previously defined, with a compound represented by the formula 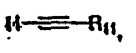 where R₁₁ is previously defined, in the presence of a palladium catalyst, a copper halide and an amine.

39. A process for the preparation of a compound according to claim 1, represented by the formula 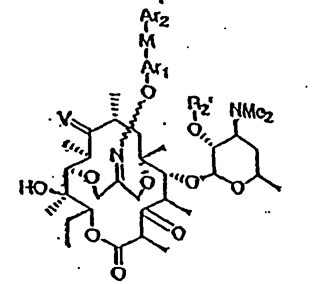 Where V is selected from N-Q or O; and Q, Ar₁, M, Ar₂ and R₂' are as defined in claim 1, comprising the steps of:
(a) reacting a compound represented by the formula (Ia) 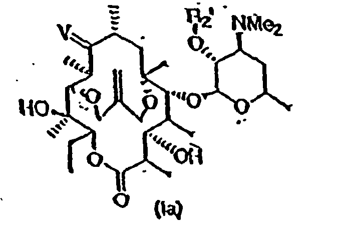 where V and R₂' are as previously defined, with a reagent or reagents capable of performing oxidative cleavage;
(b) reacting a compound from step (a) represented by the formula (Ib) 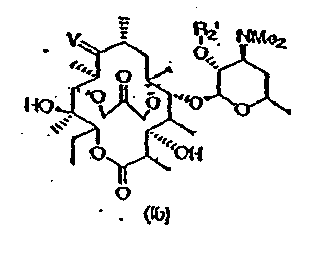 where V and R₂' are as previously defined, with an oxidizing agent to provide 3-keto compound;
(c) reacting a compound from step (b) represented by the formula (Ic) 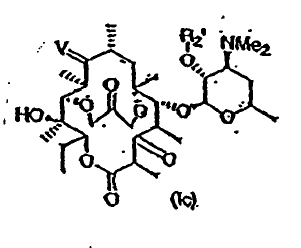 where V and R₂' are as previously defined, with a compound of the formula Ar₂-M-Ar₁-O-NH₂ wherein Ar₁, Ar₂, and M are defined as in claim 1, in the presence of an acid or a base; and
(d) optionally deprotecting the compound from step (c).

40. A process for the preparation of a compound according to claim 1, represented by the formula 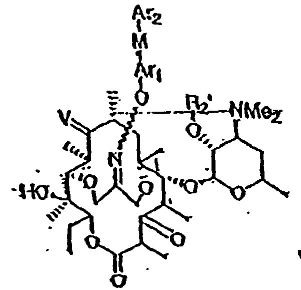 where V is selected from N-Q or O; and Q, Ar₁, M, Ar₂ and R₂' are as defined in claim 1, comprising the steps of:
(a) reacting a compound represented by the formula (Ib) 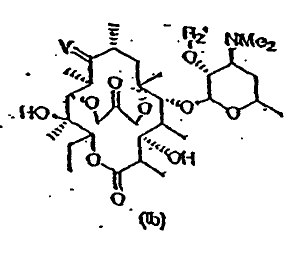 Where V and R₂' are as previously defined, with a compound of the formula Ar₂-M-Ar₁-O-NH₂, wherein Ar₁, Ar₂ and M are defined as in claim 1, in the presence of an acid or a base;
(b) reacting a compound from step (a) with an oxidizing agent to provide 3-keto compound; and
(c) optionally deprotecting the compound formed in step (b).

41. A process for the preparation of a compound according to claim 1, represented by the formula 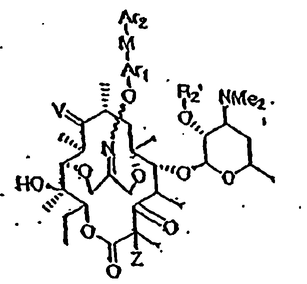 where V is selected from N-Q or O; and Q, Ar₁, M, Ar₂, R₂', and Z are as defined in claim 1, comprising the steps of:
(a) reacting a compound represented by the formula (Ia) 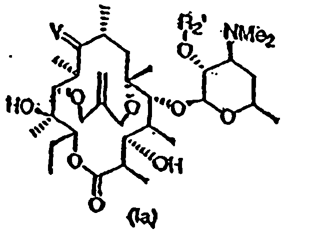 where V and R₂' are as previously defined, with an oxidizing agent to provide 3-keto compound;
(b) reacting a compound from step (a) represented by the formula (Ib) 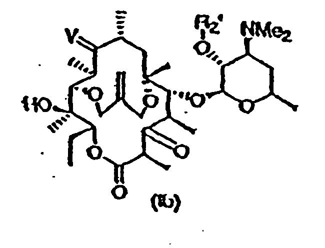 where V and R₂' are as previously defined, with a halogenating agent or a methylating agent in the presence of a base;
(c) reacting a compound from step (b) represented by the formula (Ic) 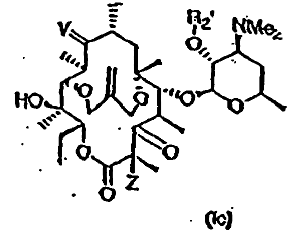 where V, Z and R₂' are as previously defined, with a reagent or reagents capable of performing oxidative cleavage;
(d) reacting a compound from step (c) represented by the formula (Id) 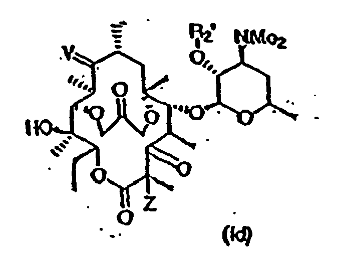 where V, Z, and R₂' are as previously defined, with a compound of the formula Ar₂-M-Ar₁-O-NH₂, wherein Ar₁, Ar₂, and M are defined as in claim 1, in the presence of an acid or a base; and
(e) optionally deprotecting of a compound from step (d).

## Patentansprüche

1. Verbindung, wiedergegeben durch die Formel: 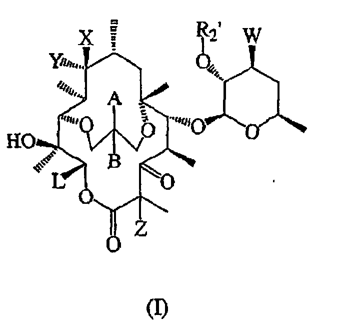 oder ihr pharmazeutisch verträgliches Salz oder ihr Ester, wobei
A ausgewählt ist aus:
a) -OH;
b)-ORp, wobei Rp für eine Hydroxyschutzgruppe steht;
c) -R_{1,} wobei R₁ unabhängig ausgewählt ist aus:
(1) Aryl;
(2) substituiertem Aryl;
(3) Heteroaryl; und
(4) substituiertem Heteroaryl;
d) -OR₁, wobei R₁ wie vorstehend definiert ist;
e) -R₂, wobei R₂ unabhängig ausgewählt ist aus:
(1) Wasserstoff;
(2) Halogen;
(3) -C₁-C₁₂-Alkyl, das gegebenenfalls 0, 1, 2 oder 3 Heteroatome enthält, die aus O, S oder N ausgewählt sind, das gegebenenfalls mit einem oder mehreren Substituenten substituiert ist, die aus Halogen, Aryl, substituiertem Aryl, Heteroaryl und substituiertem Heteroaryl ausgewählt sind;
(4) -C₂-C₁₂-Alkenyl, das gegebenenfalls 0, 1, 2 oder 3 Heteroatome enthält, die aus O, S und N ausgewählt sind, das gegebenenfalls mit einem oder mehreren Substituenten substituiert ist, die aus Halogen, Aryl, substituiertem Aryl, Heteroaryl und substituiertem Heteroaryl ausgewählt sind; und
(5) -C₂-C₁₂-Alkinyl, das gegebenenfalls 0, 1, 2 oder 3 Heteroatome enthält, die aus O, S und N ausgewählt sind, das gegebenenfalls mit einem oder mehreren Substituenten substituiert ist, die aus Halogen, Aryl, substituiertem Aryl, Heteroaryl und substituiertem Heteroaryl ausgewählt sind;
f) -OR₂, wobei R₂ unabhängig wie vorstehend definiert ist;
g) -S(O)ₙR₁₁, wobei n = 0, 1 oder 2 ist, und R₁₁ unabhängig für Wasserstoff, R₁ oder R₂ steht, wobei R₁ und R₂ wie vorstehend definiert sind;
h) -NHC(O)R₁₁, wobei R₁₁ wie vorstehend definiert ist;
i) -NHC(O)NHR₁₁, wobei R₁₁ wie vorstehend definiert ist;
j) -NHS(O)₂R₁₁, wobei R₁₁ wie vorstehend definiert ist;
k) -NR₁₄R₁₅, wobei R₁₄ und R₁₅ jeweils unabhängig voneinander R₁₁ sind, wobei R₁₁ wie vorstehend definiert ist; und
l) -NHR₃, wobei R₃ für eine Aminoschutzgruppe steht;;
B ausgewählt ist aus:
a) Wasserstoff;
b) Deuterium;
c) Halogen;
d) -OH;
e) R₁, wobei R₁ wie vorstehend definiert ist;
f) R₂, wobei R₂ wie vorstehend definiert ist; und
g) -ORp, wobei Rp wie vorstehend definiert ist, unter der Maßgabe, dass, wenn B für Halogen, - OH oder -ORp steht, dann steht A für R₁ oder R₂;
oder wahlweise sind A und B zusammen mit dem Kohlenstoffatom, an das sie gebunden sind, ausgewählt aus:
a) C=O;
b) C(OR₂)₂, wobei R₂ wie vorstehend definiert ist;
c) C(SR₂)₂, wobei R₂ wie vorstehend definiert ist;
d) C[-O(CH₂)ₘ]₂, wobei m = 2 oder 3 ist;
e) C[-S(CH₂)ₘ]₂, wobei m wie vorstehend definiert ist;
f) C=CHR₁₁, wobei R₁₁ wie vorstehend definiert ist;
g) C=N-O-R₁₁, wobei R₁₁ wie vorstehend definiert ist;
h) C=N-O-Ar₁-M-Ar₂, wobei
(1) -Ar₁- für R₃₁ steht, wobei R₃₁ unabhängig ausgewählt ist aus:
(a) R₁, wobei R₁ wie vorstehend definiert ist;
(b) -C₁-C₁₂-Alkyl, das gegebenenfalls 0, 1, 2 oder 3 Heteroatome enthält, die aus O, S oder N ausgewählt sind, das gegebenenfalls mit einem oder mehreren Substituenten substituiert ist, die aus Halogen, Aryl, substituiertem Aryl, Heteroaryl und substituiertem Heteroaryl ausgewählt sind;
(c) -C₂-C₁₂-Alkenyl, das gegebenenfalls 0, 1, 2 oder 3 Heteroatome enthält, die aus O, S und N ausgewählt sind, das gegebenenfalls mit einem oder mehreren Substituenten substituiert ist, die aus Halogen, Aryl, substituiertem Aryl, Heteroaryl und substituiertem Heteroaryl ausgewählt sind;
(d) -C₂-C₁₂-Alkinyl, das gegebenenfalls 0, 1, 2 oder 3 Heteroatome enthält, die aus O, S und N ausgewählt sind, das gegebenenfalls mit einem oder mehreren Substituenten substituiert ist, die aus Halogen, Aryl, substituiertem Aryl, Heteroaryl und substituiertem Heteroaryl ausgewählt sind;
(2) -M- nicht vorhanden ist oder ausgewählt aus:
(a) -C₁-C₁₂-Alkyl, das gegebenenfalls enthält:
0 - 3 Heteroatome, die aus O, S oder N ausgewählt sind; und
0-3 Gruppen, die aus -C=N-, -N=N, -C(O)-ausgewählt sind;
(b) -C₂-C₁₂-Alkenyl, das gegebenenfalls enthält:
0 - 3 Heteroatome, die aus O, S oder N ausgewählt sind; und
0-3 Gruppen, die aus -C=N-, -N=N, -C(O)-ausgewählt sind;
(c) -C₂-C₁₂-Alkinyl, das gegebenenfalls enthält:
0 - 3 Heteroatome, die aus O, S oder N ausgewählt sind; und
0 - 3 Gruppen, die aus -C=N-, -N=N-, -C(O)-ausgewählt sind;
(d) substituiertes Aryl;
(e) substituiertes Heteroaryl; oder
(f) substituiertes Heterocycloalkyl; und
(3) -Ar₂ ausgewählt ist aus:
(a) Aryl;
(b) substituiertes Aryl;
(c) Heteroaryl; oder
(d) substituiertes Heteroaryl;
i) C=NNHR₁₁, wobei R₁₁ wie vorstehend definiert ist;
j) C=NNHC(O)R₁₁, wobei R₁₁ wie vorstehend definiert ist;
k) C=NNHC(O)NHR₁₁, wobei R₁₁ wie vorstehend definiert ist;
l) C=NNHS(O)₂R₁₁, wobei R₁₁ wie vorstehend definiert ist;
m) C=NNHR₃, wobei R₃ wie vorstehend definiert ist;
n) C=NR₁₁, wobei R₁₁ wie vorstehend definiert ist; oder
o) C=N-N=CHR₁₁, wobei R₁₁ wie vorstehend definiert ist;
eines von X und Y für Wasserstoff steht und das andere ausgewählt ist aus:
a) Wasserstoff;
b) Deuterium;
c) -OH;
d) -ORₚ, wobei Rₚ wie vorstehend definiert ist;
e) -NR₄R₅, wobei R₄ und R₅ jeweils unabhängig voneinander ausgewählt sind aus:
(1) Wasserstoff;
(2) -C₁-C₁₂-Alkyl, das gegebenenfalls mit einem oder mehreren Substituenten substituiert ist, die aus Halogen, Aryl, substituiertem Aryl, Heteroaryl und substituiertem Heteroaryl ausgewählt sind;
(3) R₄ und R₅ zusammen mit dem Stickstoffatom, an das sie gebunden sind, einen 3 - 10-gliedrigen Heteroalkylring bilden, der 0 - 2 zusätzliche Heteroatome enthält, die aus O, S und N ausgewählt sind; oder
wahlweise sind X und Y zusammen mit dem Kohlenstoffatom, an das sie gebunden sind, ausgewählt aus:
a) C=O;
b) C=N-Q, wobei Q ausgewählt ist aus:
(1) -R₁₁, wobei R₁₁ wie vorstehend definiert ist;
(2) Aminoschutzgruppe;
(3) -C(O)R₁₁, wobei R₁₁ wie vorstehend definiert ist;
(4) -OR₆, wobei R₆ unabhängig ausgewählt ist aus:
(a) Wasserstoff;
(b) - CH₂O(CH₂)₂OCH₃,
(c) -CH₂O(CH₂O)ₙCH₃, wobei n wie vorstehend definiert ist;
(d) -C₁-C₁₂-Alkyl, das gegebenenfalls mit einem oder mehreren Substituenten substituiert ist, die aus Aryl, substituiertem Aryl, Heteroaryl und substituiertem Heteroaryl ausgewählt sind;
(e) -C₃-C₁₂-Cycloalkyl;
(f) -C(O)-C₁-C₁₂-Alkyl;
(g) -C(O)-C₃-C₁₂-Cycloalkyl;
(h) -C(O)-R₁, wobei R₁ wie vorstehend definiert ist;
(i) -Si(Rₐ)(Rb)(R_{c}), wobei Rₐ, R_{b} und R_{c} jeweils unabhängig voneinander aus -C₁-C₁₂-Alkyl, Aryl und substituiertem Aryl ausgewählt sind; oder
(5) O-C(R₇)(R₈)-O-R₆, wobei R₆ wie vorstehend definiert ist, unter der Maßgabe, dass R₆ nicht für C(O)-C₁-C₁₂-Alkyl, C(O)-C₃-C₁₂-Cycloalkyl oder C(O)-R₁ steht und R₇ und R₈ zusammen mit dem Kohlenstoffatom, an das sie gebunden sind, eine C₃-C₁₂-Cycloalkyl-Gruppe bilden oder sie jeweils unabhängig voneinander ausgewählt sind aus:
(1) Wasserstoff; oder
(2) C₁-C₁₂-Alkyl;
L ausgewählt ist aus:
a) -CH₃;
b) -CH₂CH₃;
c) -CH(OH)CH₃;
d) -C₁-C6-Alkyl, das gegebenenfalls mit einem oder mehreren Substituenten substituiert ist, die aus Aryl, substituiertem Aryl, Heteroaryl und substituiertem Heteroaryl ausgewählt sind;
e) -C₂-C₆-Alkenyl, das gegebenenfalls mit einem oder mehreren Substituenten substituiert ist, die aus Aryl, substituiertem Aryl, Heteroaryl und substituiertem Heteroaryl ausgewählt sind;
f) -C₂-C₆-Alkinyl, das gegebenenfalls mit einem oder mehreren Substituenten substituiert ist, die aus Aryl, substituiertem Aryl, Heteroaryl und substituiertem Heteroaryl ausgewählt sind;
W für -NR₂₀R₂₁ steht, wobei R₂₀ und R₂₁ jeweils unabhängig voneinander ausgewählt sind aus:
a) Wasserstoff;
b) -C₁-C₁₂-Alkyl, das gegebenenfalls mit einem oder mehreren Substituenten substituiert ist, die aus Aryl, substituiertem Aryl, Heteroaryl und substituiertem Heteroaryl ausgewählt sind;
c) -C₂-C₁₂-Alkenyl, das gegebenenfalls mit einem oder mehreren Substituenten substituiert ist, die aus Aryl, substituiertem Aryl, Heteroaryl und substituiertem Heteroaryl ausgewählt sind;
d) -C₂-C₁₂-Alkinyl, das gegebenenfalls mit einem oder mehreren Substituenten substituiert ist, die aus Halogen, Aryl, substituiertem Aryl, Heteroaryl und substituiertem Heteroaryl ausgewählt sind;
e) R₂₀ und R₂₁ zusammen mit dem Stickstoffatom, an das sie gebunden sind, einen Heterocycloalkyl-Rest bilden; oder
Z ausgewählt ist aus:
a) Wasserstoff;
b) Methyl; oder
c) Halogen; und
R₂' für Wasserstoff oder Rp steht, wobei Rp, wie vorstehend definiert ist.

2. Verbindung nach Anspruch 1, die wiedergegeben wird durch die Formel: 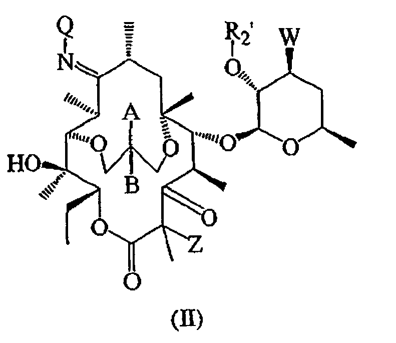 wobei A, B, R₂', Q, W und Z wie in Anspruch 1 definiert sind.

3. Verbindung nach Anspruch 1, die wiedergegeben wird durch die Formel: 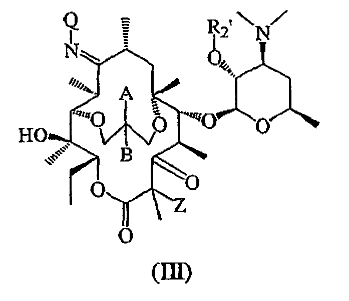 wobei A, B, R₂', Q und Z wie in Anspruch 1 definiert sind.

4. Verbindung nach Anspruch 1, die wiedergegeben wird durch die Formel: 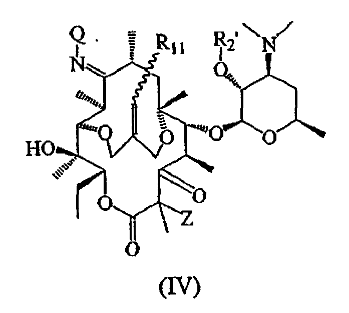 wobei R₁₁, R₂', Q und Z wie in Anspruch 1 definiert sind.

5. Verbindung nach Anspruch 1, die wiedergegeben wird durch die Formel: 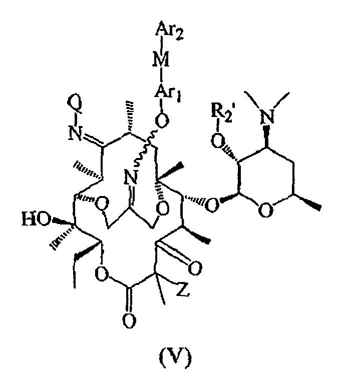 wobei Ar₁, Ar₂, R₂', M, Q, W und Z wie in Anspruch 1 definiert sind.

6. Verbindung nach Anspruch 1, die wiedergegeben wird durch die Formel: 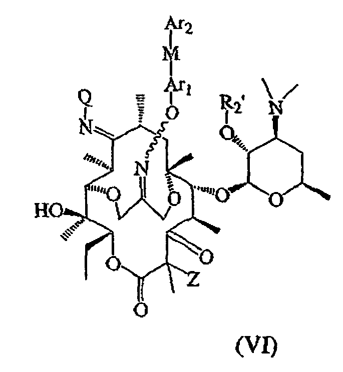 wobei Ar₁, Ar₂, R₂', M, Q und Z wie in Anspruch 1 definiert sind.

7. Verbindung nach Anspruch 1, die wiedergegeben wird durch die Formel: 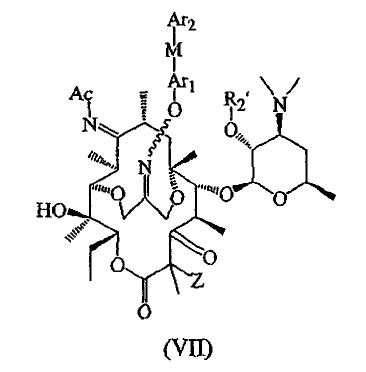 wobei Ar₁, Ar₂, R₂', M und Z wie in Anspruch 1 definiert sind.

8. Verbindung nach Anspruch 1, die wiedergegeben wird durch die Formel: 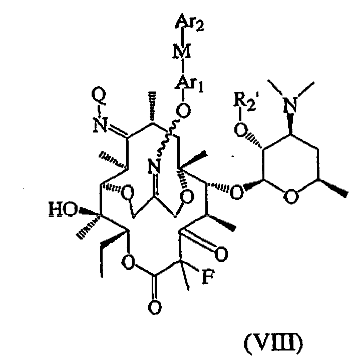 wobei Ar₁, Ar₂, R₂', M und Q wie in Anspruch 1 definiert sind.

9. Verbindung nach Anspruch 1, die ausgewählt ist aus:
(1) Verbindung der Formel (I): A und B stehen zusammen mit dem Kohlenstoffatom, an das sie gebunden sind, für C=CH₂, X und Y stehen zusammen mit dem Kohlenstoffatom, an das sie gebunden sind, für C=N-Ac, L = CH₂CH₃, Z = H und R₂' = Ac;
(2) Verbindung der Formel (I): A und B stehen zusammen mit dem Kohlenstoffatom, an das sie gebunden sind, für C=CH₂, X und Y stehen zusammen mit dem Kohlenstoffatom, an das sie gebunden sind, für C=N-Ac, L = CH₂CH₃, Z = H und R₂' = H;
(3) Verbindung der Formel (I): A und B stehen zusammen mit dem Kohlenstoffatom, an das sie gebunden sind, für C=O, X und Y stehen zusammen mit dem Kohlenstoffatom, an das sie gebunden sind, für C=N-Ac, L = CH₂CH₃, Z = H und R₂' = H;
(4) Verbindung der Formel (I): A und B stehen zusammen mit dem Kohlenstoffatom, an das sie gebunden sind, für C= N-O-CH₂-Ph, X und Y stehen zusammen mit dem Kohlenstoffatom, an das sie gebunden sind, für C=N-Ac, L = CH₂CH₃, Z = H und R₂' = H;
(5) Verbindung der Formel (I): A und B stehen zusammen mit dem Kohlenstoffatom, an das sie gebunden sind, für C= N-O-CH₂-(3-pyridyl), X und Y stehen zusammen mit dem Kohlenstoffatom, an das sie gebunden sind, für C=N-Ac, L = CH₂CH₃, Z = H und R₂' = H;
(6) Verbindung der Formel (I): A und B stehen zusammen mit dem Kohlenstoffatom, an das sie gebunden sind, für C= N-O-CH₂-(2-pyridyl), X und Y stehen zusammen mit dem Kohlenstoffatom, an das sie gebunden sind, für C=N-Ac, L = CH₂CH₃, Z = H und R₂' = H;
(7) Verbindung der Formel (I): A und B stehen zusammen mit dem Kohlenstoffatom, an das sie gebunden sind, für C= N-O-CH₂-(3-quinolyl), X und Y stehen zusammen mit dem Kohlenstoffatom, an das sie gebunden sind, für C=N-Ac, L = CH₂CH₃, Z = H und R₂' = H;
(8) Verbindung der Formel (I): A und B stehen zusammen mit dem Kohlenstoffatom, an das sie gebunden sind, für C= N-O-CH₂-(2-quinolyl), X und Y stehen zusammen mit dem Kohlenstoffatom, an das sie gebunden sind, für C=N-Ac, L = CH₂CH₃, Z = H und R₂' = H;
(9) Verbindung der Formel (I): A und B stehen zusammen mit dem Kohlenstoffatom, an das sie gebunden sind, für C= N-O-(5-pyridin-2-yl-thiophen-2-yl)-methyl, X und Y stehen zusammen mit dem Kohlenstoffatom, an das sie gebunden sind, für C=N-Ac, L = CH₂CH₃, Z = H und R₂' = H;
(10) Verbindung der Formel (I): A und B stehen zusammen mit dem Kohlenstoffatom, an das sie gebunden sind, für C= N-O-(3-pyrimidin-2-yl-prop-2-ynyl), X und Y stehen zusammen mit dem Kohlenstoffatom, an das sie gebunden sind, für C=N-Ac, L = CH₂CH₃, Z = H und R₂' = H;
(11) Verbindung der Formel (I): A und B stehen zusammen mit dem Kohlenstoffatom, an das sie gebunden sind, für C= N-O-Ph, X und Y stehen zusammen mit dem Kohlenstoffatom, an das sie gebunden sind, für C=N-Ac, L = CH₂CH₃, Z = H und R₂' = H;
(12) Verbindung der Formel (I) : A = NHCH₂-Ph, B = H, X und Y stehen zusammen mit dem Kohlenstoffatom, an das sie gebunden sind, für C=N-Ac, L = CH₂CH₃, Z = H und R₂' = H;
(13) Verbindung der Formel (I): A = NHCH₂CH₂-Ph, B = H, X und Y stehen zusammen mit dem Kohlenstoffatom, an das sie gebunden sind, für C=N-Ac, L = CH₂CH₃, Z = H und R₂' = H;
(14) Verbindung der Formel (I): A und B stehen zusammen mit dem Kohlenstoffatom, an das sie gebunden sind, für C=CH₂, X und Y stehen zusammen mit dem Kohlenstoffatom, an das sie gebunden sind, für C=N-O-CH₂-O-CH₃, L = CH₂CH₃, Z = H und R₂' = Ac;
(15) Verbindung der Formel (I): A und B stehen zusammen mit dem Kohlenstoffatom, an das sie gebunden sind, für C=CH₂, X und Y stehen zusammen mit dem Kohlenstoffatom, an das sie gebunden sind, für C=N-O-CH₂-O-CH₃, L = CH₂CH₃, Z = H und R₂' = H;
(16) Verbindung der Formel (I): A und B stehen zusammen mit dem Kohlenstoffatom, an das sie gebunden sind, für C=O, X und Y stehen zusammen mit dem Kohlenstoffatom, an das sie gebunden sind, für C=N-O-CH₂-O-CH₃, L = CH₂CH₃, Z = H und R₂' = H;
(17) Verbindung der Formel (I): A und B stehen zusammen mit dem Kohlenstoffatom, an das sie gebunden sind, für C=NOCH₂-Ph, X und Y stehen zusammen mit dem Kohlenstoffatom, an das sie gebunden sind, für C=N-O-CH₂-O-CH₃, L = CH₂CH₃, Z = H und R₂' = H;
(18) Verbindung der Formel (I): A und B stehen zusammen mit dem Kohlenstoffatom, an das sie gebunden sind, für C=O, X und Y stehen zusammen mit dem Kohlenstoffatom, an das sie gebunden sind, für C=O, L = CH₂CH₃, Z = H und R₂' = Ac;
(19) Verbindung der Formel (I): A und B stehen zusammen mit dem Kohlenstoffatom, an das sie gebunden sind, für C=CH₂, X und Y stehen zusammen mit dem Kohlenstoffatom, an das sie gebunden sind, für C=O, L = CH₂CH₃, Z = H und R₂' = H;
(20) Verbindung der Formel (I): A und B stehen zusammen mit dem Kohlenstoffatom, an das sie gebunden sind, für C=O, X und Y stehen zusammen mit dem Kohlenstoffatom, an das sie gebunden sind, für C=O, L = CH₂CH₃, Z = H und R₂' = H;
(21) Verbindung der Formel (I): A und B stehen zusammen mit dem Kohlenstoffatom, an das sie gebunden sind, für C= N-O-CH₂-Ph, X und Y stehen zusammen mit dem Kohlenstoffatom, an das sie gebunden sind, für C=O, L = CH₂CH₃, Z = H und R₂' = H;
(22) Verbindung der Formel (I): A und B stehen zusammen mit dem Kohlenstoffatom, an das sie gebunden sind, für C=CH₂, X und Y stehen zusammen mit dem Kohlenstoffatom, an das sie gebunden sind, für C=NH, L = CH₂CH₃, Z = H und R₂' = H;
(23) Verbindung der Formel (I): A und B stehen zusammen mit dem Kohlenstoffatom, an das sie gebunden sind, für C= CH-O-CH₂- *p*-NO₂-Ph, X und Y stehen zusammen mit dem Kohlenstoffatom, an das sie gebunden sind, für C=N-Ac, L = CH₂CH₃, Z = H und R₂' = H;
(24) Verbindung der Formel (I): A und B stehen zusammen mit dem Kohlenstoffatom, an das sie gebunden sind, für C= CH-O-(CH₂)₂-Ph, X und Y stehen zusammen mit dem Kohlenstoffatom, an das sie gebunden sind, für C=N-Ac, L = CH₂CH₃, Z = H und R₂' = H;
(25) Verbindung der Formel (I): A und B stehen zusammen mit dem Kohlenstoffatom, an das sie gebunden sind, für C= CH-O-(CH₂)₃-Ph, X und Y stehen zusammen mit dem Kohlenstoffatom, an das sie gebunden sind, für C=N-Ac, L = CH₂CH₃, Z = H und R₂' = H;
(26) Verbindung der Formel (I): A und B stehen zusammen mit dem Kohlenstoffatom, an das sie gebunden sind, für C=CH-O-CH₂-CH=CH-Ph, X und Y stehen zusammen mit dem Kohlenstoffatom, an das sie gebunden sind, für C=N-Ac, L = CH₂CH₃, Z = H und R₂' = H;
(27) Verbindung der Formel (I): A steht für NH-(CH₂)₃-Ph, B steht für H, X und Y stehen zusammen mit dem Kohlenstoffatom, an das sie gebunden sind, für C=N-Ac, L = CH₂CH₃, Z = H und R₂' = H;
(28) Verbindung der Formel (I): A steht für NH-(CH₂)₄-Ph, B steht für H, X und Y stehen zusammen mit dem Kohlenstoffatom, an das sie gebunden sind, für C=N-Ac, L = CH₂CH₃, Z = H und R₂' = H;
(29) Verbindung der Formel (I): A steht für CH₂-CH=CH₂, B steht für OH, X und Y stehen zusammen mit dem Kohlenstoffatom, an das sie gebunden sind, für C=N-Ac, L = CH₂CH₃, Z = H und R₂' = H;
(30) Verbindung der Formel (I): A steht für CH₂-Ph, B steht für OH, X und Y stehen zusammen mit dem Kohlenstoffatom, an das sie gebunden sind, für C=N-Ac, L = CH₂CH₃, Z = H und R₂' = H;
(31) Verbindung der Formel (I): A steht für Ph, B steht für OH, X und Y stehen zusammen mit dem Kohlenstoffatom, an das sie gebunden sind, für C=N-Ac, L = CH₂CH₃, Z = H und R₂' = H;
(33) Verbindung der Formel (I): A steht für CH₂-CH=CH-Ph, B steht für OH, X und Y stehen zusammen mit dem Kohlenstoffatom, an das sie gebunden sind, für C=N-Ac, L = CH₂CH₃, Z = H und R₂' = H;
(34) Verbindung der Formel (I): A steht für (CH₂)₃-Ph, B steht für OH, X und Y stehen zusammen mit dem Kohlenstoffatom, an das sie gebunden sind, für C=N-Ac, L = CH₂CH₃, Z = H und R₂' = H;
(35) Verbindung der Formel (I): A und B stehen zusammen mit dem Kohlenstoffatom, an das sie gebunden sind, für C=CH-CH=CH-Ph, X und Y stehen zusammen mit dem Kohlenstoffatom, an das sie gebunden sind, für C=N-Ac, L = CH₂CH₃, Z = H und R₂' = H;
(36) Verbindung der Formel (I): A steht für (CH₂)₃-Ph, B steht für H, X und Y stehen zusammen mit dem Kohlenstoffatom, an das sie gebunden sind, für C=N-Ac, L = CH₂CH₃, Z = H und R₂' = H;
(37) Verbindung der Formel (I): A und B stehen zusammen mit dem Kohlenstoffatom, an das sie gebunden sind, für C=CH-CH=CH-(3-pyridyl), X und Y stehen zusammen mit dem Kohlenstoffatom, an das sie gebunden sind, für C=N-Ac, L = CH₂CH₃, Z = H und R₂' = H;
(38) Verbindung der Formel (I): A und B stehen zusammen mit dem Kohlenstoffatom, an das sie gebunden sind, für C=CH-CH=CH-(3-quinolyl), X und Y stehen zusammen mit dem Kohlenstoffatom, an das sie gebunden sind, für C=N-Ac, L = CH₂CH₃, Z = H und R₂' = H;
(39) Verbindung der Formel (I): A und B stehen zusammen mit dem Kohlenstoffatom, an das sie gebunden sind, für C=CH-(2-quinolyl), X und Y stehen zusammen mit dem Kohlenstoffatom, an das sie gebunden sind, für C=N-Ac, L = CH₂CH₃, Z = H und R₂' = H;
(40) Verbindung der Formel (I): A und B stehen zusammen mit dem Kohlenstoffatom, an das sie gebunden sind, für C=CH-(2-quinolyl), X und Y stehen zusammen mit dem Kohlenstoffatom, an das sie gebunden sind, für C=N-H, L = CH₂CH₃, Z = H und R₂' = H;
(41) Verbindung der Formel (I): A und B stehen zusammen mit dem Kohlenstoffatom, an das sie gebunden sind, für C=CH-(4-biphenyl), X und Y stehen zusammen mit dem Kohlenstoffatom, an das sie gebunden sind, für C=N-Ac, L = CH₂CH₃, Z = H und R₂' = H;
(42) Verbindung der Formel (I): A und B stehen zusammen mit dem Kohlenstoffatom, an das sie gebunden sind, für C=CH-(3-biphenyl), X und Y stehen zusammen mit dem Kohlenstoffatom, an das sie gebunden sind, für C=N-Ac, L = CH₂CH₃, Z = H und R₂' = H;
(43) Verbindung der Formel (I): A und B stehen zusammen mit dem Kohlenstoffatom, an das sie gebunden sind, für C=CH-(4-phenoxyphenyl), X und Y stehen zusammen mit dem Kohlenstoffatom, an das sie gebunden sind, für C=N-Ac, L = CH₂CH₃, Z = H und R₂' = H;
(44) Verbindung der Formel (I): A und B stehen zusammen mit dem Kohlenstoffatom, an das sie gebunden sind, für C=CH-Ph, X und Y stehen zusammen mit dem Kohlenstoffatom, an das sie gebunden sind, für C=N-Ac, L = CH₂CH₃, Z = H und R₂' = H;
(45) Verbindung der Formel (I): A und B stehen zusammen mit dem Kohlenstoffatom, an das sie gebunden sind, für C=CH-(2-(2-pyridyl)-thiophen-5-yl), X und Y stehen zusammen mit dem Kohlenstoffatom, an das sie gebunden sind, für C=N-Ac, L = CH₂CH₃, Z = H und R₂' = H; oder
(46) Verbindung der Formel (I): A und B stehen zusammen mit dem Kohlenstoffatom, an das sie gebunden sind, für C=O, X und Y stehen zusammen mit dem Kohlenstoffatom, an das sie gebunden sind, für C=N-Ac, L = CH₂CH₃, Z = F und R₂' = Ac;

10. Verbindung nach Anspruch 1 der Formel (A), die aus den Verbindungen ausgewählt ist, die in Tabelle 10 dargestellt sind: 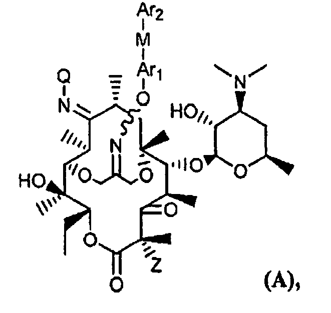
**Tabelle 10**
| **Nummer** | **Q** | **-Ar₁-M-Ar₂** | **Z** |
|---|---|---|---|
| (1). | Ac | 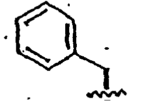 | H |
| (2). | Ac | 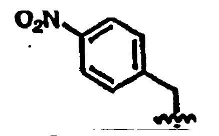 | H |
| (3). | Ac | 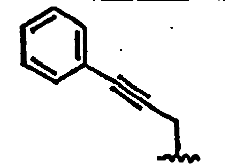 | H |
| (4). | Ac | 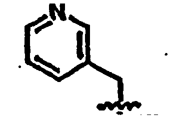 | H |
| (5). | Ac | 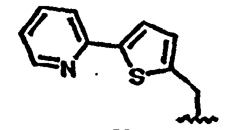 | H |
| (6). | Ac | 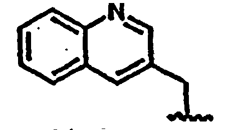 | H |
| (7). | Ac | 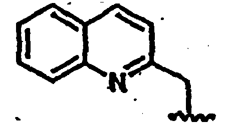 | H |
| (8). | Ac | 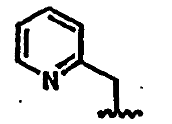 | H |
| (10). | Ac | 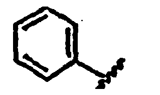 | H |
| (11). | Ac | 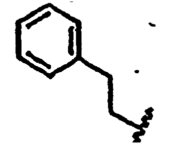 | H |
| (12). | Ac | 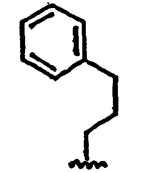 | H |
| (13). | Ac | 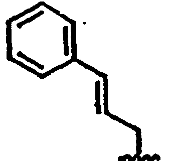 | H |
| (14). | Ac | 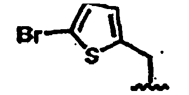 | H |
| (15). | Ac | 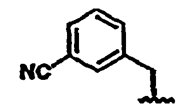 | H |
| (16). | Ac | 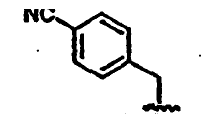 | H |
| (17). | Ac | 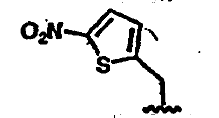 | H |
| (18). | Ac | 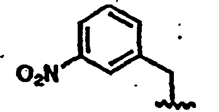 | H |
| (19). | Ac | 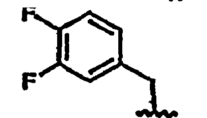 | H |
| (20). | Ac | 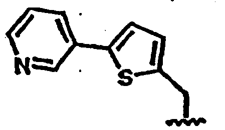 | H |
| (21). | Ac | 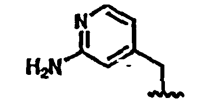 | H |
| (22). | Ac | 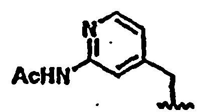 | H |
| (23). | Ac | 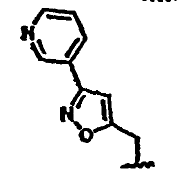 | H |
| (24). | Ac | 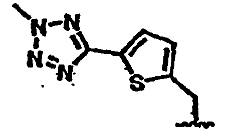 | H |
| (25). | Ac | 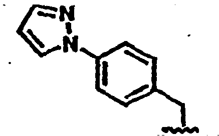 | H |
| (26). | Ac | 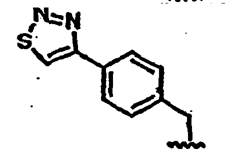 | H |
| (27). | Ac | 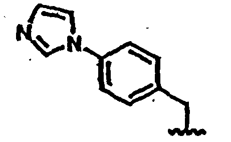 | H |
| (28). | Ac | 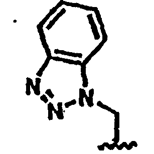 | H |
| (29). | Ac | 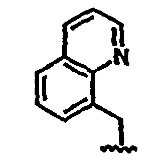 | H |
| (30). | Ac | 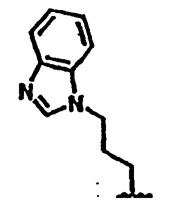 | H |
| (31). | Ac | 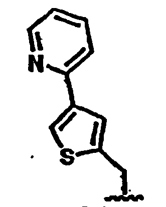 | H |
| (32). | Ac | 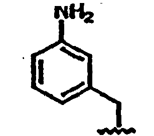 | H |
| (33). | Ac | 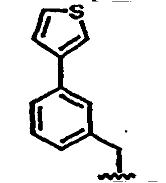 | H |
| (34). | Ac | 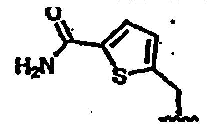 | F |
| (35). | H | 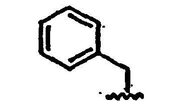 | H |
| (36). | Ac | 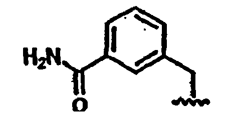 | H |
| (37). | Ac | 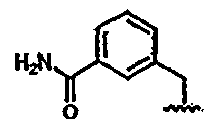 | F |
| (38). | Ac | 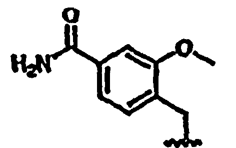 | H |
| (39). | Ac | 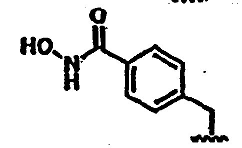 | H |
| (40). | Ac | 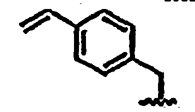 | H |
| (41). | Ac | 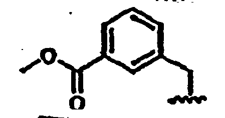 | H |
| (42). | Ac | 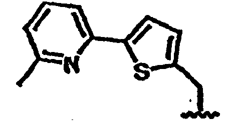 | H |
| (43). | Ac | 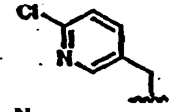 | H |
| (44). | H | 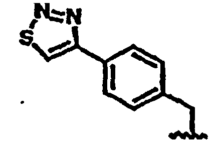 | H |
| (45). | H | 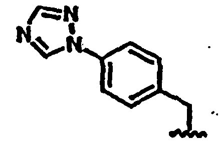 | H |
| (46). | OMe | 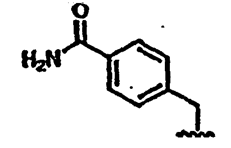 | H |
| (48). | -OCH₂CN | 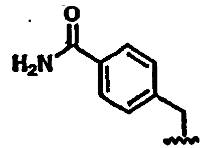 | H |
| (49). | -OCH₂CH₂OH | 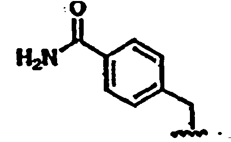 | H |
| (50). | H | 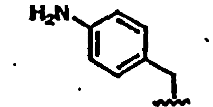 | H |
| (51). | Ac | 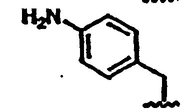 | H |
| (52). | Ac | 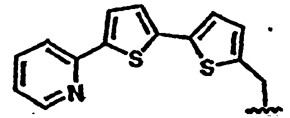 | H |
| (53). | Ac | 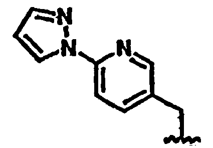 | H |
| (54). | Ac | 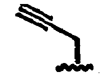 | H |
| (55). | Ac | 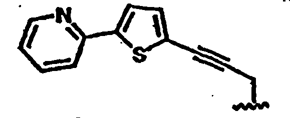 | H |
| (56). | Ac | 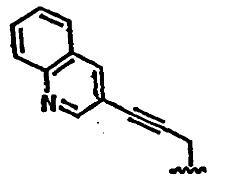 | H |
| (57). | Ac | 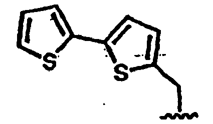 | H |
| (58). | Ac | 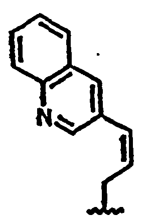 | H |
| (59). | Ac | 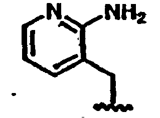 | H |
| (60). | Ac | 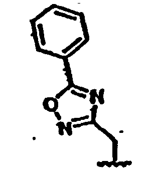 | H |
| (61). | Ac | 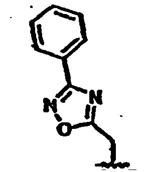 | H |
| (62). | Ac | 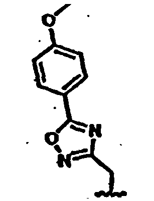 | H |
| (63). | Ac | 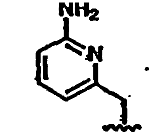 | H |
| (64). | Ac | 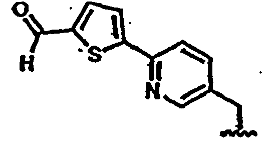 | H |
| (65). | Ac | 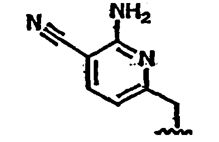 | H |
| (66). | Ac | 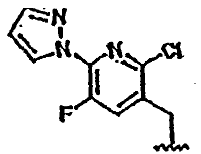 | H |
| (67). | Ac | 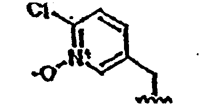 | H |
| (68). | Ac | 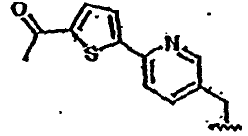 | H |

11. Verbindung nach Anspruch 1 der Formel (A1), die aus den Verbindungen ausgewählt ist, die in Tabelle 11 dargestellt sind: 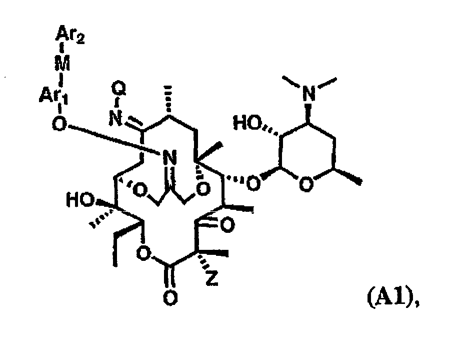
**Tabelle 11**
| **Nummer** | **Q** | **-Ar₁-M-Ar₂** | **Z** |
|---|---|---|---|
| (1). | Ac | 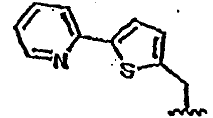 | H |
| (2). | Ac | 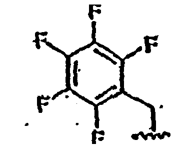 | H |
| (3). | Ac | 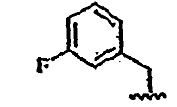 | H |
| (4). | Ac | 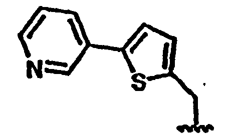 | H |
| (5). | Ac | 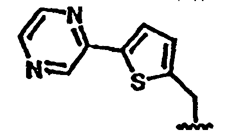 | H |
| (6). | Ac | 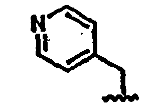 | H |
| (7). | Ac | 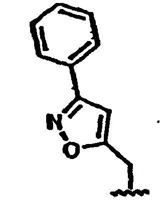 | H |
| (8). | Ac | 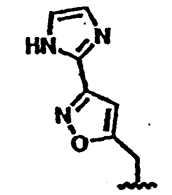 | H |
| (9). | Ac | 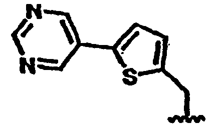 | H |
| (10). | Ac | 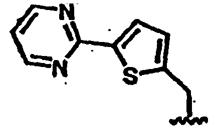 | H |
| (11). | Ac | 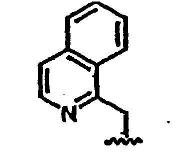 | H |
| (12). | Ac | 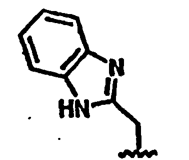 | H |
| (13). | Ac | 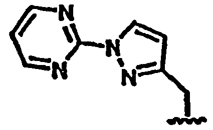 | H |
| (14). | Ac | 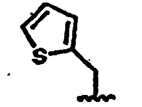 | H |
| (15). | Ac | 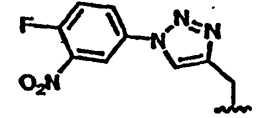 | H |
| (16). | Ac | 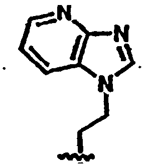 | H |
| (17). | Ac | 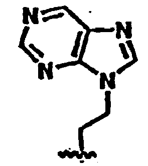 | H |
| (18). | Ac | 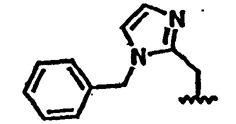 | H |
| (19). | Ac | 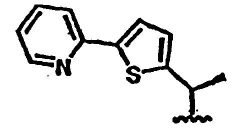 | H |
| (20). | Ac | 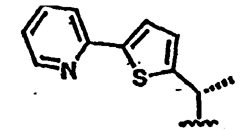 | H |
| (21). | Ac | 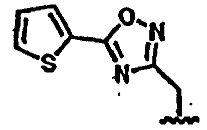 | H |
| (22). | Ac | 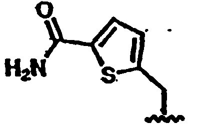 | H |
| (23). | Ac | 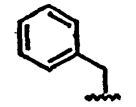 | H |
| (24). | Ac | 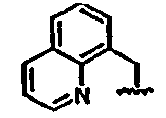 | F |
| (25). | Ac | 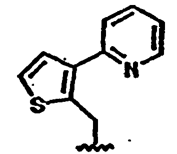 | H |
| (26). | Ac | 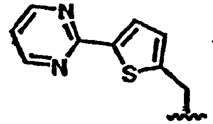 | F |
| (27). | Ac | 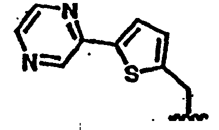 | F |
| (28). | Ac | 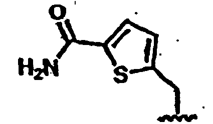 | F |
| (29). | Ac | 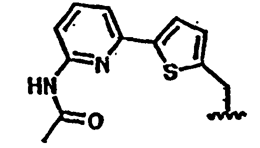 | H |
| (30). | Ac | 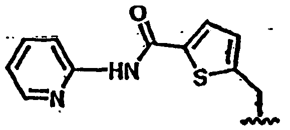 | H |
| (31). | Ac | 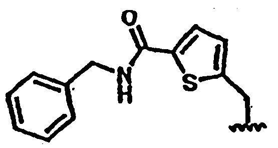 | H |
| (32). | Ac | 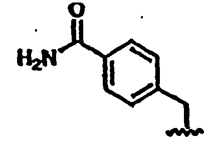 | H |
| (33). | Ac | 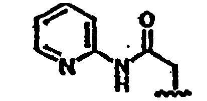 | H |
| (34). | Ac | 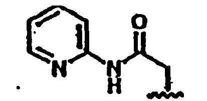 | F |
| (35). | Ac | 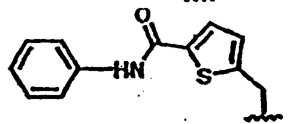 | H |
| (36). | Ac | 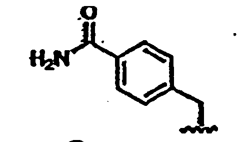 | F |
| (37). | Ac | 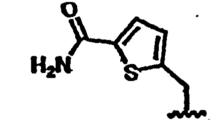 | H |
| (38). | Ac | 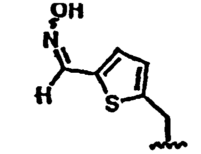 | H |
| (39). | Ac | 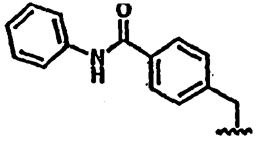 | H |
| (40). | Ac | 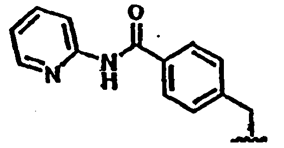 | H |
| (41). | Ac | 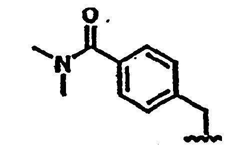 | H |
| (42). | Ac | 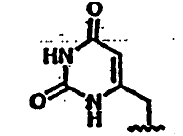 | H |
| (43). | Ac | 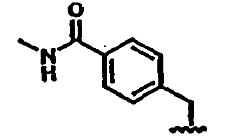 | H |
| (44). | Ac | 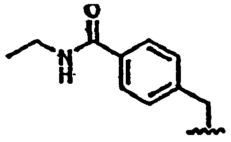 | H |
| (45). | Ac | 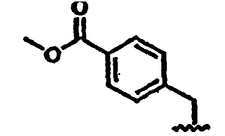 | H |
| (46). | Ac | 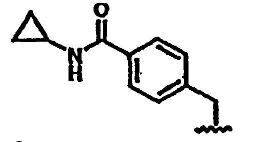 | H |
| (47). | Ac | 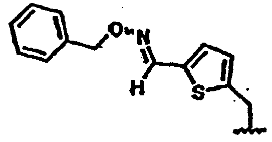 | H |
| (48). | Ac | 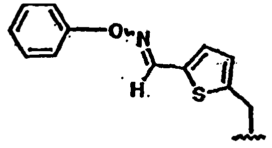 | H |
| (49). | Ac | 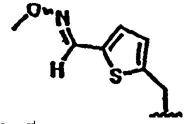 | H |
| (50). | Ac | 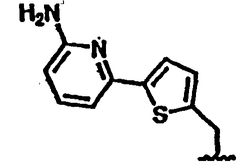 | H |
| (51). | Ac | 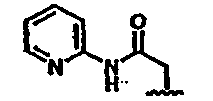 | H |
| (52). | Ac | 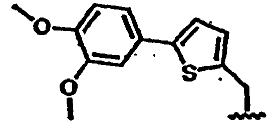 | H |
| (53). | Ac | 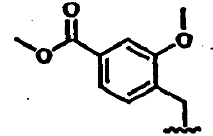 | H |
| (54). | Ac | 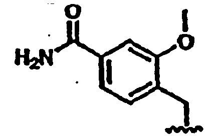 | H |
| (55). | Ac | 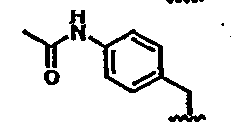 | H |
| (56). | Ac | 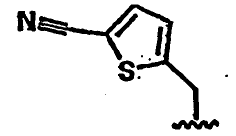 | H |
| (57). | Ac | 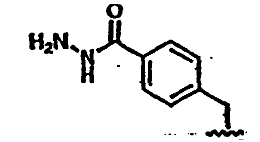 | H |
| (58). | Ac | 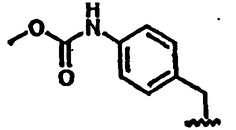 | H |
| (59). | Ac | 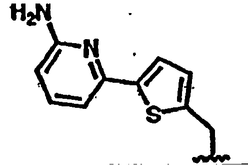 | F |
| (60). | Ac | 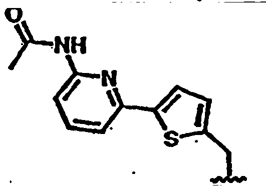 | F |
| (61). | Ac | 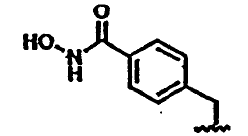 | H |
| (62). | Ac | 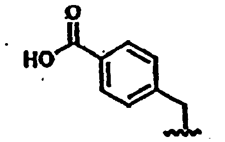 | H |
| (63). | Ac | 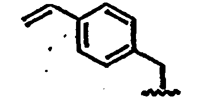 | H |
| (64). | Ac | 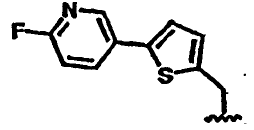 | H |
| (65). | Ac | 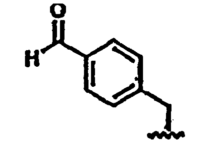 | H |
| (66). | H | 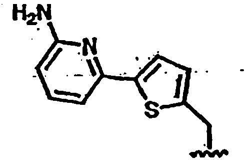 | H |
| (67). | Ac | 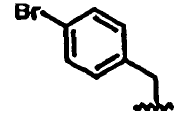 | H |
| (68). | Ac | 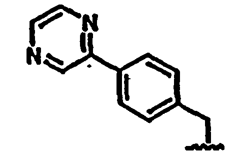 | H |
| (69). | Ac | 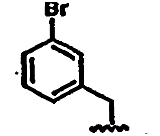 | H |
| (70). | Ac | 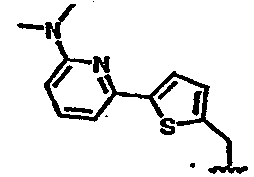 | F |
| (72). | Ac | 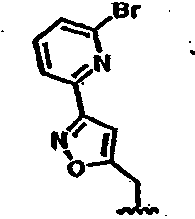 | H |
| (73). | Ac | 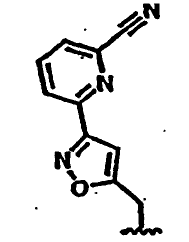 | H |
| (74). | Ac | 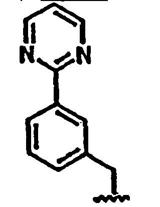 | H |
| (75). | Ac | 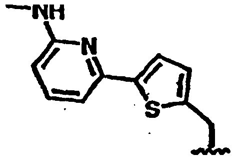 | F |
| (76). | Ac | 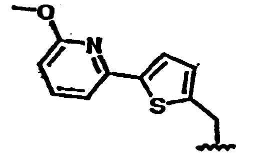 | H |
| (77). | H | 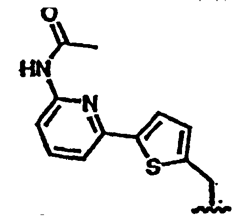 | H |
| (78). | Ac | 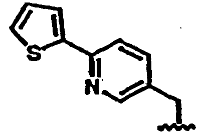 | H |
| (79). | Ac | 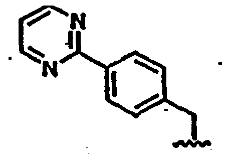 | H |
| (80). | H | 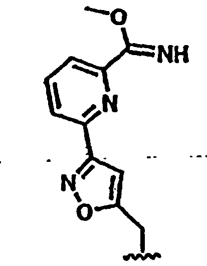 | H |
| (81). | Ac | 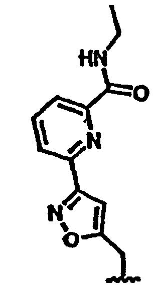 | H |
| (82). | H | 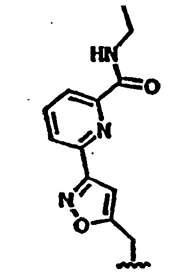 | H |
| (83). | H | 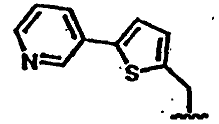 | H |
| (84). | H | 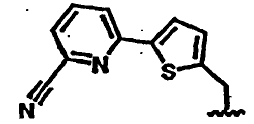 | H |
| (85). | Ac | 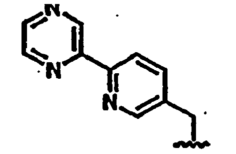 | H |
| (86). | Ac | 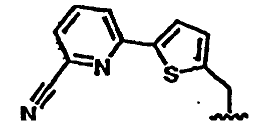 | H |
| (87). | Ac | 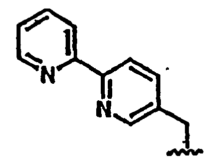 | H |
| (88). | H | 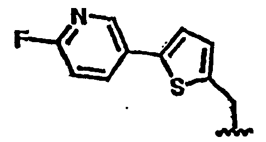 | H |
| (89). | H | 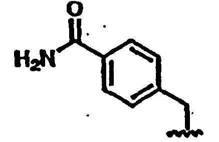 | H |
| (90). | H | 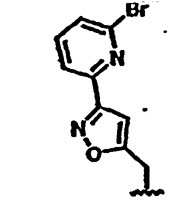 | H |
| (91). | Ac | 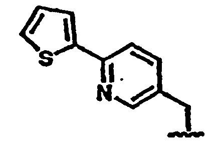 | H |
| (92). | H | 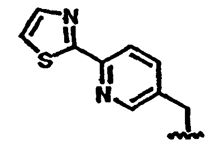 | H |
| (93). | Ac | 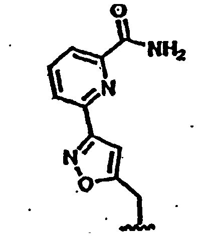 | H |
| (94). | Ac | 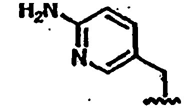 | H |
| (95). | Ac | 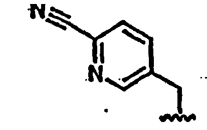 | H |
| (96). | Ac | 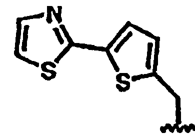 | H |
| (97). | Ac | 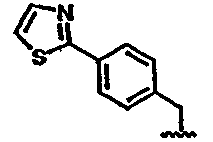 | F |
| (98). | H | 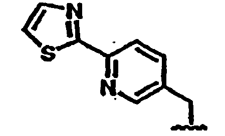 | F |
| (99). | H | 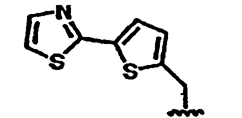 | H |
| (100). | Ac | 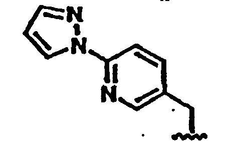 | F |
| (101). | Ac | 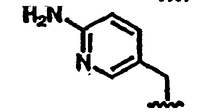 | F |
| (102). | Ac | 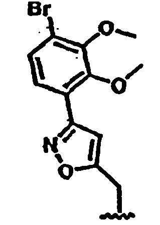 | H |
| (103). | Ac | 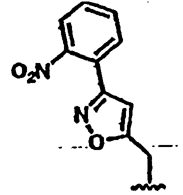 | H |
| (104). | Ac | 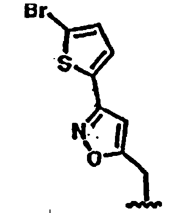 | H |
| (105). | Ac | 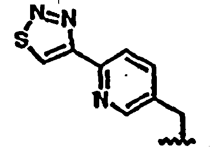 | F |
| (106). | Ac | 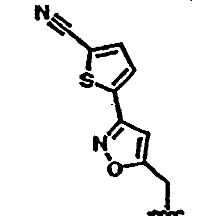 | H |
| (107). | Ac | 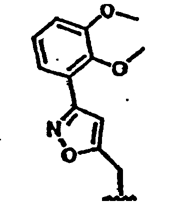 | H |
| (108). | Ac | 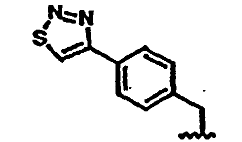 | H |
| (109). | Ac | 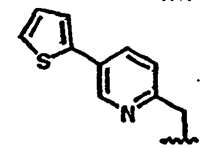 | H |
| (110). | Ac | 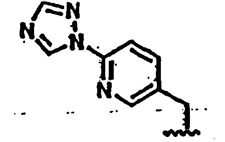 | H |
| (111). | Ac | 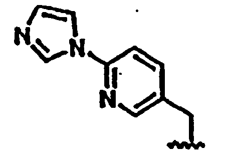 | H |
| (112). | Ac | 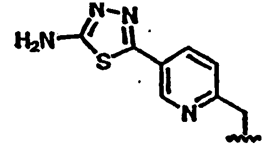 | H |
| (113). | Ac | 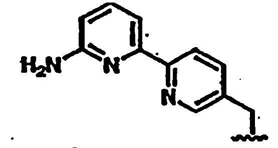 | H |
| (114). | Ac | 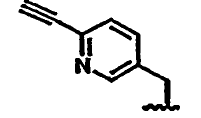 | H |
| (115). | H | 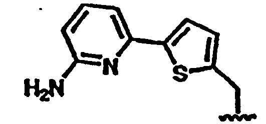 | F |
| (116). | H | 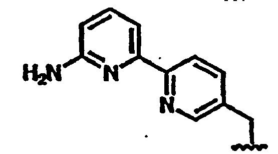 | H |
| (117). | Ac | 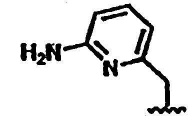 | H |
| (118). | Ac | 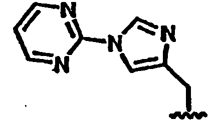 | H |
| (119). | Ac | 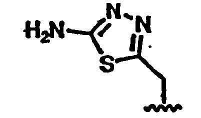 | H |
| (120). | Ac | 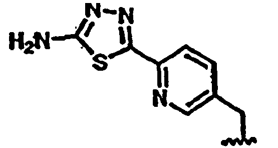 | H |
| (121). | Ac | 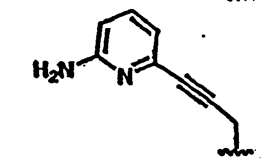 | H |
| (122). | Ac | 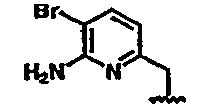 | H |
| (123). | Ac | 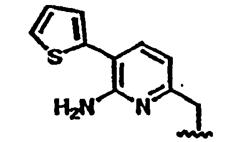 | H |
| (124). | Ac | 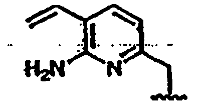 | H |
| (125). | Ac | 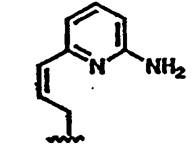 | H |
| (126). | Ac | 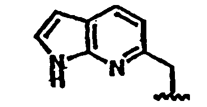 | H |
| (127). | Ac | 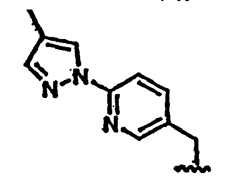 | H |
| (128). | Ac | 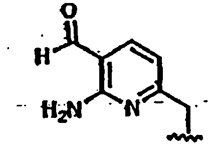 | H |
| (129). | Ac | 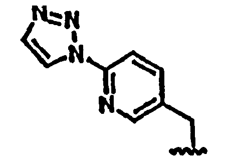 | H |
| (130). | Ac | 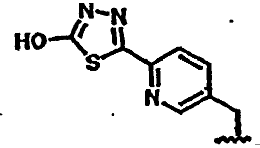 | H |
| (131). | Ac | 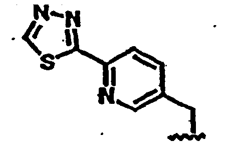 | H |
| (132). | Ac | 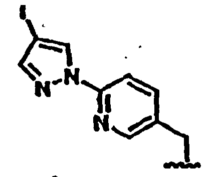 | H |
| (133). | Ac | 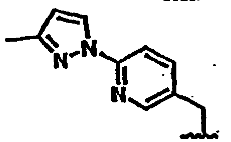 | H |
| (134). | Ac | 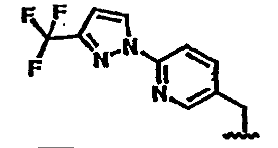 | H |
| (135). | Ac | 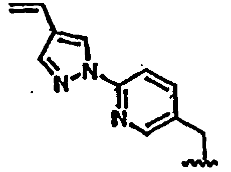 | H |
| (136). | Ac | 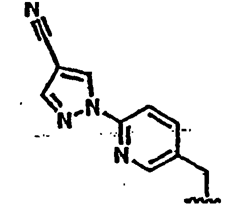 | H |
| (137). | Ac | 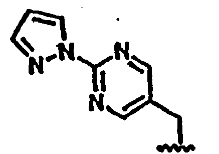 | H |
| (138). | Ac | 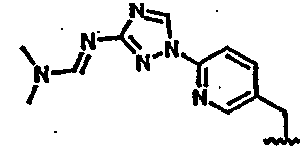 | H |
| (139). | Ac | 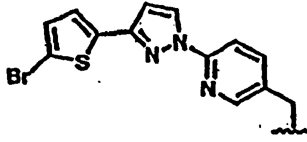 | H |
| (140). | Ac | 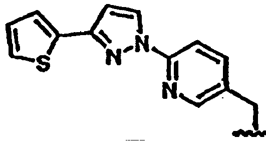 | H |
| (141). | Ac | 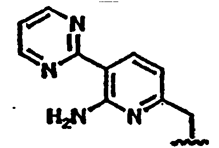 | H |
| (142). | H | 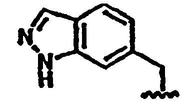 | H |
| (143). | Ac | 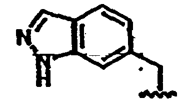 | H |
| (144). | Ac | 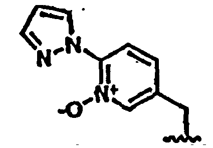 | H |
| (145). | Ac | 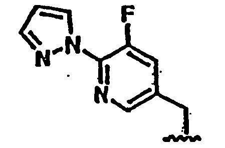 | H |
| (146). | -COCH₂CH₃ | 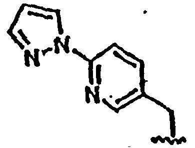 | H |
| (147). | Ac | 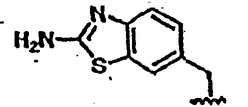 | H |
| (148). | Ac | 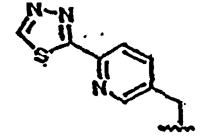 | H |

12. Verbindung nach Anspruch 1 der Formel (A2), die aus den Verbindungen ausgewählt ist, die in Tabelle 12 dargestellt sind: 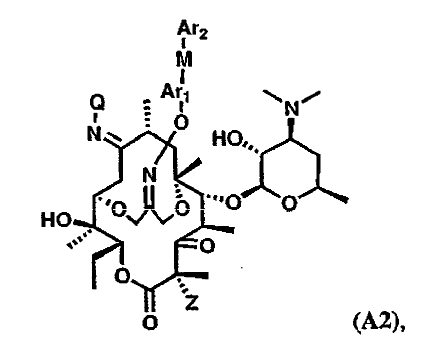
**Tabelle 12**
| **Nummer** | **Q** | **-Ar₁-M-Ar₂** | **Z** |
|---|---|---|---|
| (1). | Ac | 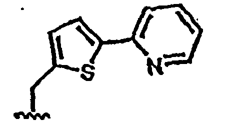 | H |
| (2). | Ac | 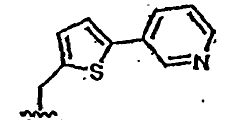 | H |
| (3). | Ac | 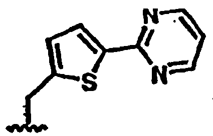 | H |
| (4). | Ac | 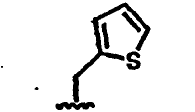 | H |
| (5). | Ac | 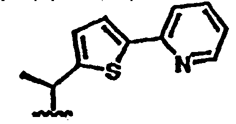 | H |
| (6). | Ac | 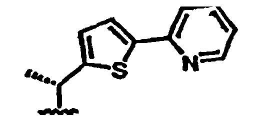 | H |
| (7). | Ac | 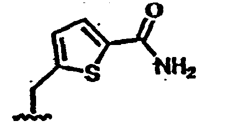 | H |
| (8). | Act | 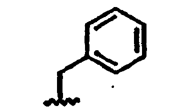 | H |
| (9). | Ac | 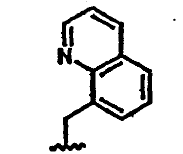 | F |
| (10). | Ac | 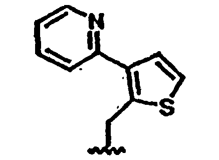 | H |
| (11). | Ac | 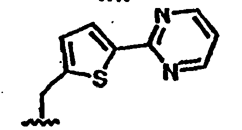 | F |
| (12). | Ac | 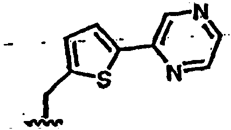 | F |
| (13). | Ac | 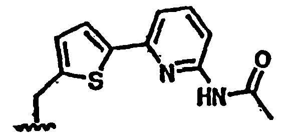 | H |
| (14). | Ac | 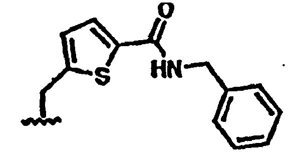 | H |
| (15). | Ac | 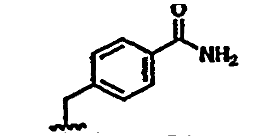 | H |
| (16). | Ac | 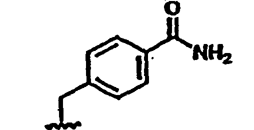 | F |
| (17). | Ac | 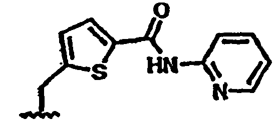 | H |
| (18). | Ac | 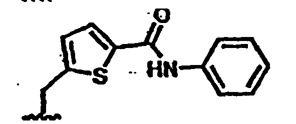 | H |
| (19). | Ac | 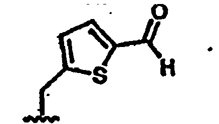 | H |
| (20). | Ac | 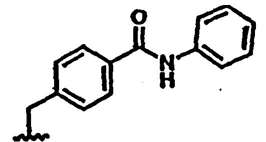 | H |
| (21). | Ac | 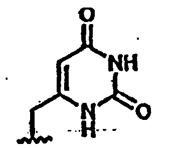 | H |
| (22). | Ac | 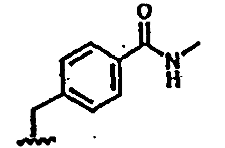 | H |
| (23). | Ac | 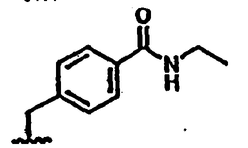 | H |
| (24). | Ac | 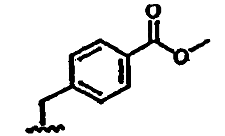 | H |
| (25). | Ac | 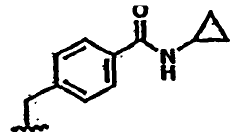 | H |
| (26). | Ac | 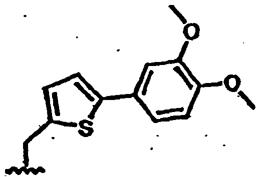 | H |
| (27). | Ac | 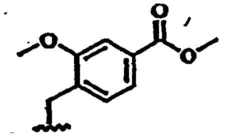 | H |
| (28). | Ac | 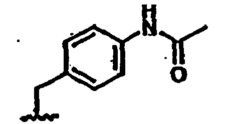 | H |
| (29). | Ac | 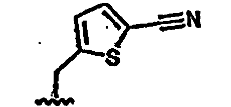 | H |
| (30). | Ac | 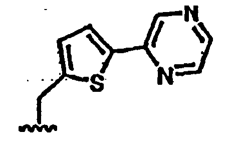 | H |
| (31). | Ac | 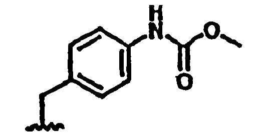 | H |
| (32). | Ac | 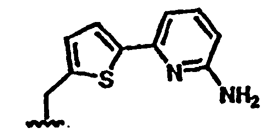 | F |
| (33). | Ac | 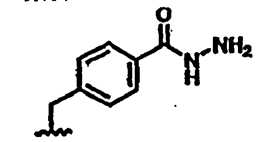 | H |
| (34). | Ac | 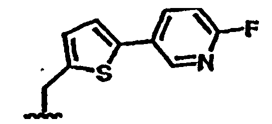 | H |
| (35). | Ac | 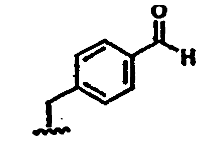 | H |
| (36). | Ac | 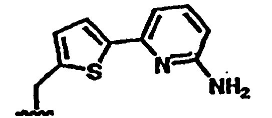 | H |
| (37). | H | 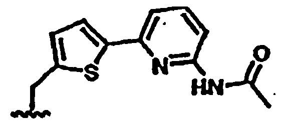 | H |
| (38). | Ac | 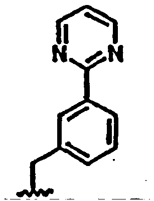 | H |
| (39). | Ac | 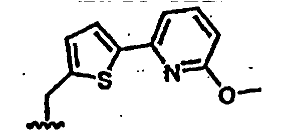 | H |
| (40). | H | 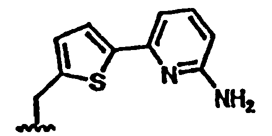 | H |
| (41). | H | 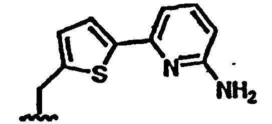 | F |
| (42). | Ac | 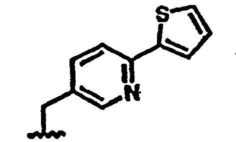 | H |
| (43). | Ac | 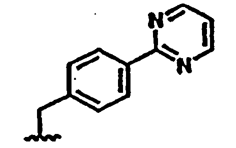 | H |
| (44). | Ac | 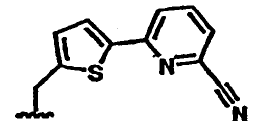 | H |
| (45). | Ac | 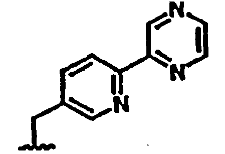 | H |
| (46). | Ac | 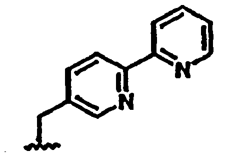 | H |
| (47). | Ac | 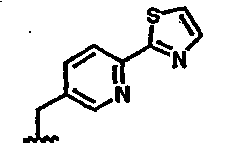 | H |
| (48). | Ac | 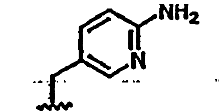 | H |
| (49). | Ac | 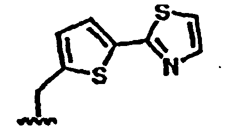 | H |
| (50). | Ac | 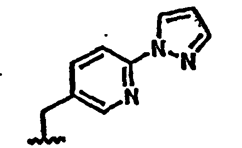 | F |
| (51). | Ac | 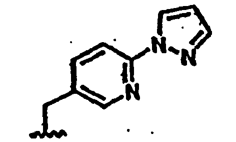 | H |
| (52). | Ac | 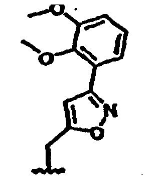 | H |
| (53). | Ac | 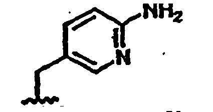 | F |
| (54). | Ac | 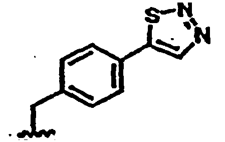 | H |
| (55). | Ac | 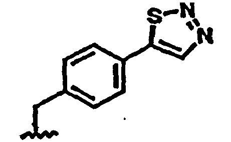 | F |
| (56). | Ac | 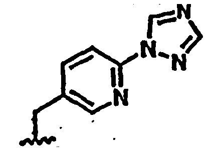 | H |
| (57). | Ac | 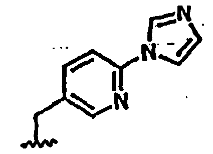 | H |
| (58). | Ac | 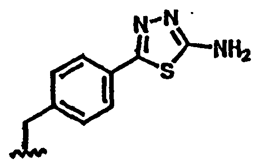 | H |
| (59). | Ac | 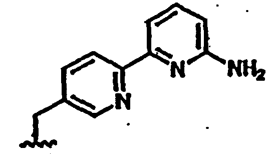 | H |
| (60). | Ac | 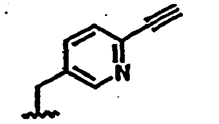 | H |
| (61). | Ac | 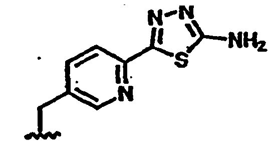 | H |
| (62). | Ac | 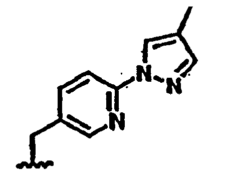 | H |
| (63). | Ac | 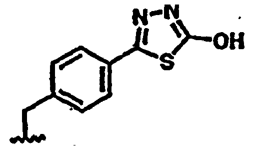 | H |
| (64). | Ac | 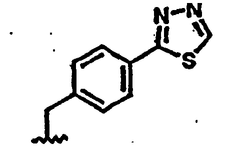 | H |
| (65). | Ac | 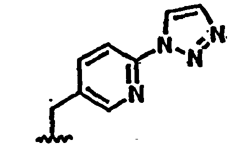 | H |
| (66). | Ac | 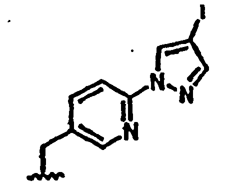 | H |
| (67). | Ac | 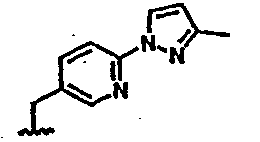 | H |
| (68). | Ac | 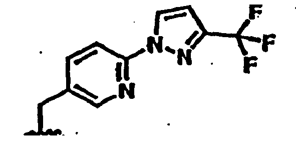 | H |
| (69). | Ac | 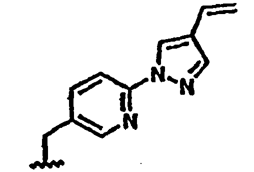 | H |
| (70). | Ac | 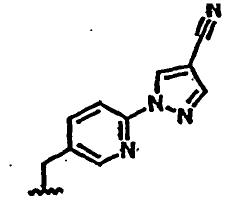 | H |
| (71). | Ac | 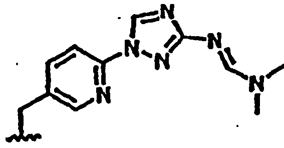 | H |
| (72). | Ac | 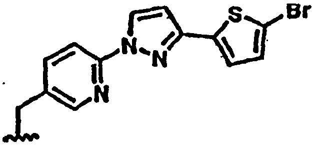 | H |
| (73). | Ac | 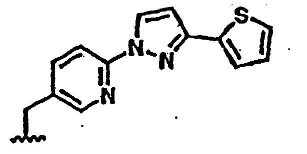 | H |
| (74). | H | 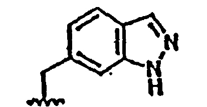 | H |
| (75). | Ac | 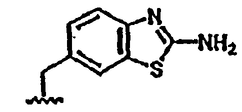 | H |

13. Verbindung nach Anspruch 1 der Formel (B), die aus den Verbindungen ausgewählt ist, die in Tabelle 13 dargestellt sind: 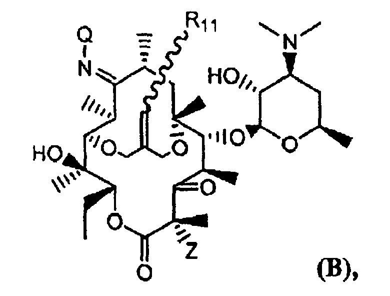
**Tabelle 13**
| **Nummer** | **Q** | **R₁₁** | **Z** |
|---|---|---|---|
| (1). | H | H | H |
| (2). | MOM | H | H |
| (3). | MOM | 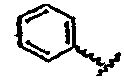 | H |
| (4). | Ac | 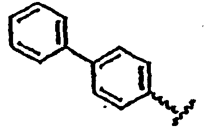 | H |
| (5). | Ac | 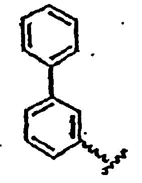 | H |
| (6). | Ac | 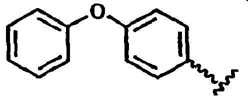 | H |
| (7). | Ac | 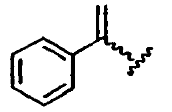 | H |
| (8). | Propionyl | 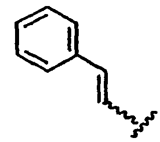 | H |
| (9). | Ac | 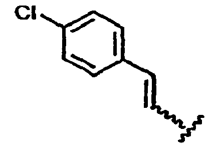 | H |
| (10). | -C(O)OMe | 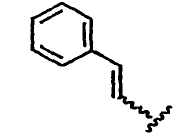 | H |
| (11). | -C(O)NH₂ | H | H |
| (12). | Me | H | H |
| (13). | BOM | H | H |
| (14). | Ac | 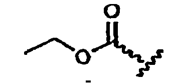 | H |
| (15). | Ac | 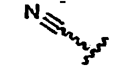 | H |

14. Verbindung nach Anspruch 1 der Formel (B1), die aus den Verbindungen ausgewählt ist, die in Tabelle 14 dargestellt sind: 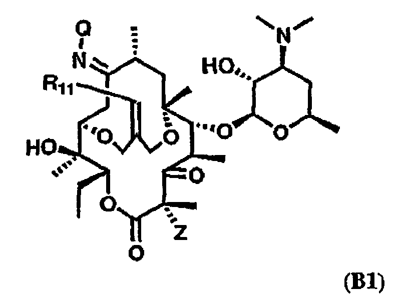
**Tabelle 14**
| **Nummer** | **Q** | **R₁₁** | **Z** |
|---|---|---|---|
| (1). | Ac | 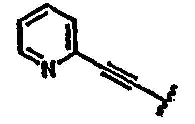 | H |
| (2). | Ac | 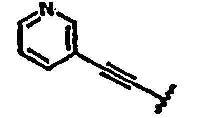 | H |
| (4). | Ac | 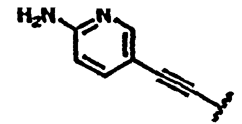 | H |
| (5). | Ac | 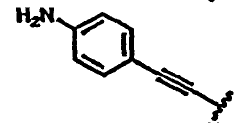 | H |
| (6). | Ac | 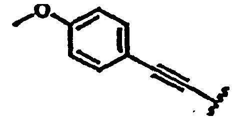 | H |
| (7). | Ac | 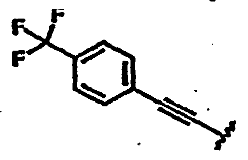 | H |
| (8). | Ac | 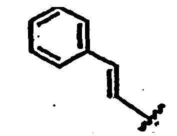 | F |
| (9). | Ac | 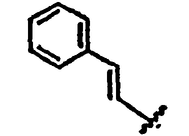 | H |
| (10). | Ac | 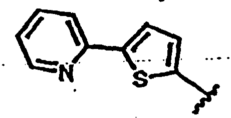 | H |
| (11). | Ac | 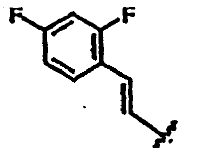 | H |
| (12). | 2-methoxyacetyl | 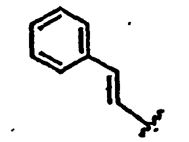 | H |
| (13). | 2-O-acyl-acetyl | 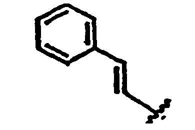 | H |
| (14). | 2-Fmoc-acetyl | 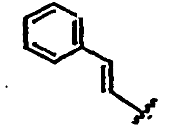 | H |
| (15). | Ac | 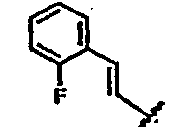 | H |
| (16). | Ac | 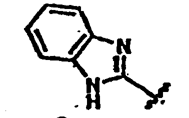 | H |
| (17). | 2-hydroxcy acetyl | 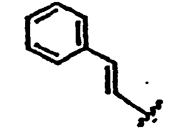 | H |
| (18). | 2-aminoacetyl | 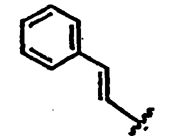 | H |
| (19). | Ac | 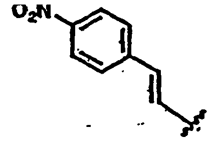 | H |
| (20). | Ac | 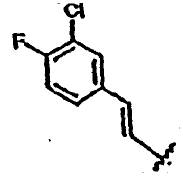 | H |
| (21). | Ac | 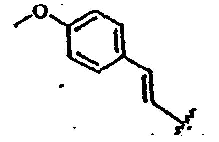 | H |
| (22). | Ac | 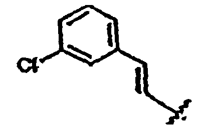 | H |

15. Verbindung nach Anspruch 1 der Formel (B2), die aus den Verbindungen ausgewählt ist, die in Tabelle 15 dargestellt sind: 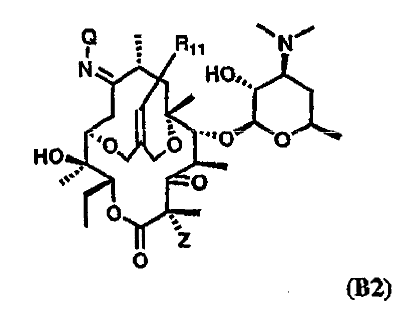
**Tabelle 15**
| **Nummer** | **Q** | **R₁₁** | **Z** |
|---|---|---|---|
| (1). | Ac | 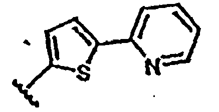 | H |
| (2). | Ac | 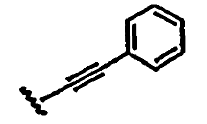 | H |
| (3). | Ac | 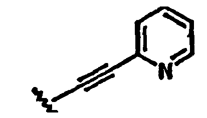 | H |
| (4). | Ac | 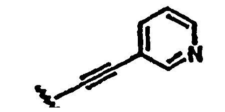 | H |
| (5). | Ac | 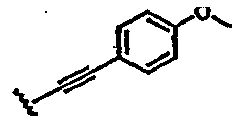 | H |
| (6). | Ac | 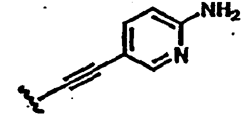 | H |
| (7). | Ac | 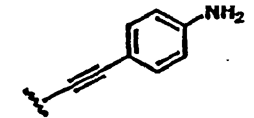 | H |
| (8). | Ac | 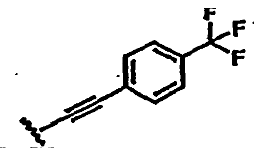 | H |
| (9). | Ac | 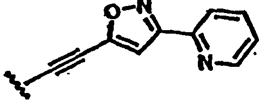 | H |

16. Verbindung nach Anspruch 1 der Formel (C), die aus den Verbindungen ausgewählt ist, die in Tabelle 16 dargestellt sind: 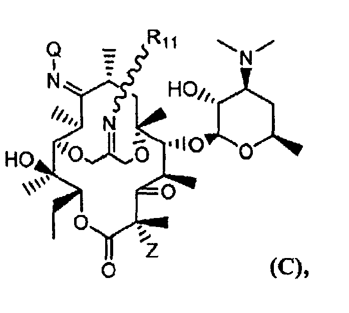
**Tabelle 16**
| **Nummer** | **Q** | **R₁₁** | **Z** |
|---|---|---|---|
| (1). | Ac | 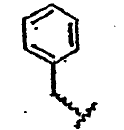 | H |
| (2). | Ac | 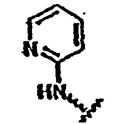 | H |
| (3). | Ac | 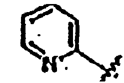 | H |
| (4). | Ac | 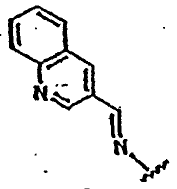 | H |
| (5). | Ac | 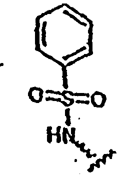 | H |

17. Verbindung nach Anspruch 7 oder 10, die wiedergegeben wird durch die Formel: 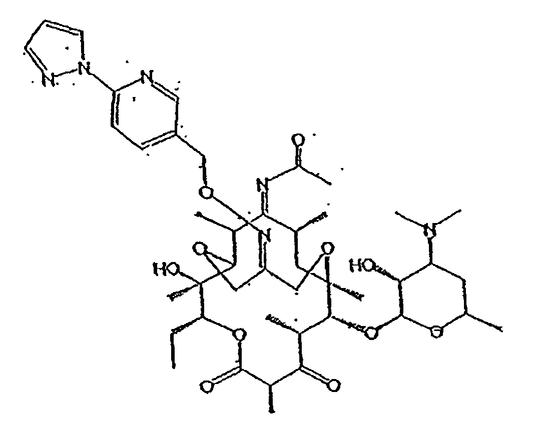 und pharmazeutisch verträgliche Salze, Ester, racemische Mischungen und Stereoisomere davon.

18. Verbindung nach Anspruch 7 oder 11, die wiedergegeben wird durch die Formel: 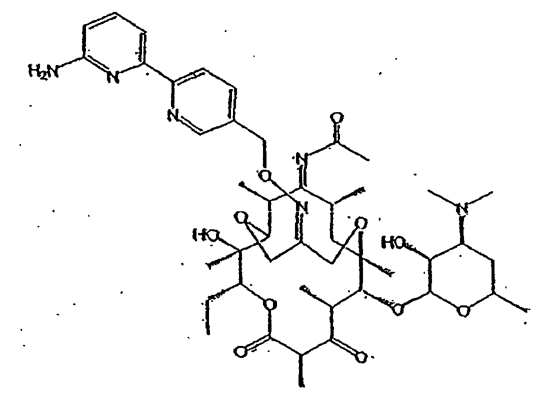 und pharmazeutisch verträgliche Salze, Ester, racemische Mischungen und Stereoisomere davon.

19. Verbindung nach einem der Ansprüche 1 bis 18, wobei die Verbindung ein pharmazeutisch verträgliches Salz ist.

20. Verbindung nach einem der Ansprüche 1 bis 18, wobei die Verbindung eine freie Base ist.

21. Verbindung nach einem der Ansprüche 1 bis 18, wobei die Verbindung ein Ester ist.

22. Pharmazeutische Zusammensetzung, die eine therapeutisch wirksame Menge einer Verbindung nach einem der Ansprüche 1 bis 21 oder ein pharmazeutisch verträgliches Salz oder ein Ester davon, in Kombination mit einer pharmazeutisch verträglichen Trägersubstanz umfasst.

23. Verwendung der Verbindung nach einem der Ansprüche 1 bis 21 für die Herstellung von einem Arzneimittel für die Bekämpfung von einer bakteriellen Infektion bei einer Person, die einer solchen Behandlung bedarf.

24. Verwendung nach Anspruch 23, wobei das Arzneimittel für die orale, parenterale Verabreichung, Verabreichung durch Inhalationsspray, topische, rektale, nasale, bukkale, vaginale Verabreichung oder eine Verabreichung über ein implantiertes Reservoir geeignet ist.

25. Verwendung nach Anspruch 24, wobei das Arzneimittel für die orale Verabreichung oder die Verabreichung durch Injektion ist.

26. Verwendung nach Anspruch 23, wobei das Arzneimittel für die Verabreichung an Menschen ist.

27. Verwendung nach einem der Ansprüche 24 bis 26, wobei das Arzneimittel in Kombination mit einem oder mehreren zusätzlichen therapeutischen oder prophylaktischen Wirkstoffen verabreicht wird.

28. Verwendung nach Anspruch 27, wobei der zusätzliche therapeutische oder prophylaktische Wirkstoff getrennt, als Anteil einer Mehrfachdosierung oder in einer Darreichungsform einer Einzeldosis verabreicht wird.

29. Verwendung nach einem der Ansprüche 24 bis 28, wobei das Arzneimittel für die Behandlung einer Protozoen-Infektion oder einer bakteriellen Infektion und von gesundheitlichen Störungen, die mit derartigen Infektionen zusammenhängen, verwendet wird.

30. Verwendung nach Anspruch 29, wobei die Infektion oder die gesundheitliche Störung ausgewählt ist aus der Gruppe bestehend aus Lungenentzündung, Mittelohrentzündung, Sinusitis, Bronchitis, Mandelentzündung und Mastoiditis, die in Verbindung mit einer Infektion durch Streptococcus pneumoniae, Haemophilus influenzae, Moraxella catarrhalis, Staphylococcus aureus oder Peptostreptococcus spp. und Pseudomonas spp. stehen; Rachenentzündung, rheumatisches Fieber und Glomerulonephritis, die in Verbindung mit einer Infektion durch Streptococcus pyogenes, Gruppe C- und G-Streptokokken, Clostridium diptheriae oder Actinobacillus haemolyticum stehen; Atemwegsinfektionen, die in Verbindung mit einer Infektion durch Mycoplasma pneumoniae, Legionella pneumophila, Streptococcus pneumoniae, Haemophilus influenzae oder Chlamydia pneumoniae stehen; unkomplizierte Haut- und Weichgewebeinfektionen, Abszesse sowie Osteomyelitis und Kindbettfieber, die mit einer Infektion durch Staphylococcus aureus, Koagulase-positiven Staphylokokken (d. h., S. epidermidis, S. hemolyticus, usw.), S. pyogenes, S. agalactiae, Streptokokken der Gruppen C - F (Streptokokken, die winzige Kolonien bilden), Viridans-Streptokokken, Corynebacterium spp., Clostridium spp. oder Bartonella henselae in Verbindung stehen; unkomplizierte, akute Harnwegsinfektionen, die mit einer Infektion durch S. saprophyticus oder Enterococcus spp. in Verbindung stehen; Harnröhrenentzündung und Gebärmutterhalsentzündung; und sexuell übertragene Erkrankungen, die mit einer Infektion durch Chlamydia trachomatis, Haemophilus ducreyi, Treponema pallidum, Ureaplasma urealyticum oder Nesseria gonorrhea in Verbindung stehen; durch Toxine verursachte Erkrankungen, die mit einer Infektion durch S. aureus oder durch Streptokokken der Gruppen A, S und C in Verbindung stehen: Geschwüre, die mit einer Infektion durch Helicobacter pylori in Verbindung stehen; systemische Fiebersyndrome, die mit einer Infektion durch Borrelia recurrentis in Verbindung stehen; Lyme-Borreliose, die mit einer Infektion durch Borrelia burgdorferi in Verbindung steht; Bindehautentzündungen, Hornhautentzündungen und Dakryozystitis, die mit einer Infektion durch C. trachomatis, N. gonorrhoeae, S. aureus, S. pneumoniae, S. pyogenes, H. influenzae oder Listeria spp. in Verbindung stehen; disseminierte Infektion mit Mycobacterium avium-Komplex (MAC-Erkrankung), die mit einer Infektion durch Mycobacterium avium oder Mycobacterium intracellulare in Verbindung steht; Magen-Darm-Entzündung, die mit einer Infektion durch Campylobacter jejuni in Verbindung steht; Darmprotozoen, die mit einer Infektion durch Cryptosporidium spp. in Verbindung stehen; odontogene Infektion, die mit einer Infektion durch Viridans-Streptokokken in Verbindung steht; anhaltender Husten, der mit einer Infektion durch Bordetella pertussis in Verbindung steht; Gasbrand, der mit einer Infektion durch Clostridium perfringens oder Bacteroides spp. in Verbindung steht; Hautinfektionen durch S. aureus und Propionibacterium acne, sowie Atherosklerose, die mit einer Infektion durch Helicobacter pylori und Chlamydia pneumoniae in Verbindung steht.

31. Verwendung nach Anspruch 30, wobei die Infektion oder die gesundheitliche Störung Lungenentzündung, Mittelohrentzündung, Sinusitis, Mandelentzündung, Akne durch *P. acne* und Haut- und Weichgewebeinfektionen sind.

32. Verwendung nach Anspruch 29, wobei die Infektion oder die gesundheitliche Störung, für die das Arzneimittel zur Behandlung verwendet wird, ausgewählt ist aus der Gruppe bestehend aus respiratorischen Erkrankungen beim Rind, die mit einer Infektion durch P. haemolytica., P. multocida, Mycoplasma bovis oder Bordetella spp. in Verbindung stehen; Darmerkrankung bei Kühen, die mit einer Infektion durch E. coli oder Protozoen in Verbindung steht; Mastitis der Milchkuh, die mit einer Infektion durch S. aureus, S. uberis, S. agalactiae, S. dysgalactiae, Klebsiella spp., Corynebacterium oder Enterococcus spp. in Verbindung steht; Atemwegserkrankungen bei Schweinen, die mit einer Infektion durch A. pleuropneumoniae., P. multocida oder Mycoplasma spp. in Verbindung stehen; Darmerkrankungen bei Schweinen, die mit einer Infektion durch E. coli, Lawsonia intracellularis, Salmonella spp. oder Serpulina hyodysinteriae in Verbindung stehen; Fußfäule bei Kühen, die mit einer Infektion durch Fusobacterium spp. in Verbindung steht; Gebärmutterentzündung bei Kühen, die mit einer Infektion durch E. coli in Verbindung steht; haarige Warzen bei Rindern, die mit einer Infektion durch Fusobacterium necrophorum oder Bacteroides nodosus in Verbindung stehen; Weideblindheit bei Rindern, die mit einer Infektion durch Moraxella bovis in Verbindung steht; vorzeitiger Abort bei Rindern, der mit einer Infektion durch Protozoen in Verbindung steht; Harnwegsinfektionen bei Hunden und Katzen, die mit einer Infektion durch E. coli in Verbindung stehen; Haut- und Weichgewebeinfektionen bei Hunden und Katzen die mit einer Infektion durch S. epidermidis, S. intermedius, Koagulase-negativen Staphylokokken oder P. multocida in Verbindung stehen; und Zahn- oder Maulinfektionen bei Hunden und Katzen, die mit einer Infektion durch Alcaligenes spp.; Bacteroides spp., Clostridium spp., Enterobacter spp., Eubacterium spp., Peptostreptococcus spp., Porphyromonas spp., Campylobacter spp., Actinomyces spp., Erysipelothrix spp., Rhodococcus spp., Trypanosoma spp., Plasmodium spp., Babesia spp., Toxoplasma spp., Pneumocystis spp., Leishmania spp., sowie Trichomonas spp. und Prevotella spp. in Verbindung stehen.

33. Verfahren zur Darstellung einer Verbindung nach Anspruch 1, wiedergegeben durch die Formel 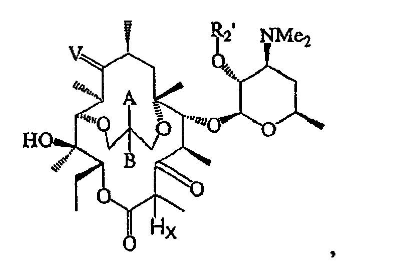 wobei V ausgewählt ist aus N-Q oder O; Hx für Halogen oder Methyl steht; und A, B, Q und R₂' wie in Anspruch 1 definiert sind, umfassend den Schritt des
(a) Umsetzens einer Verbindung, wiedergegeben durch die Formel 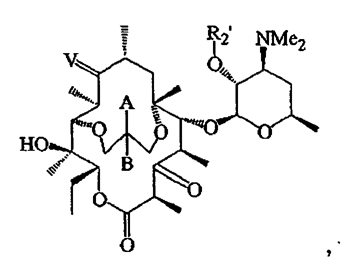 wobei A, B, V und R₂' wie vorstehend definiert sind, mit einem Halogenierungsmittel oder einem Methylierungsmittel in der Gegenwart von einer Base.

34. Verfahren zur Darstellung einer Verbindung nach Anspruch 1, wiedergegeben durch die Formel 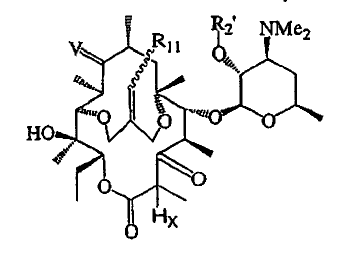 wobei V ausgewählt ist aus N-Q oder O; Hx für Halogen oder Methyl steht; und Q, R₁₁ und R₂' wie in Anspruch 1 definiert sind, umfassend den Schritt des
(a) Umsetzens einer Verbindung, wiedergegeben durch die Formel 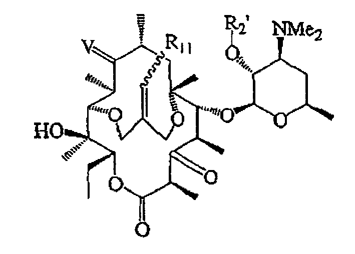 wobei V, R₁₁ und R₂' wie vorstehend definiert sind, mit einem Halogenierungsmittel in der Gegenwart von einer Base.

35. Verfahren zur Darstellung einer Verbindung nach Anspruch 1, wiedergegeben durch die Formel 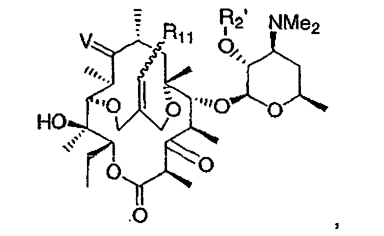 wobei V ausgewählt ist aus N-Q oder O; und Q, R₁₁ und R₂' wie in Anspruch 1 definiert sind, umfassend den Schritt des
(a) Umsetzens einer Verbindung, wiedergegeben durch die Formel 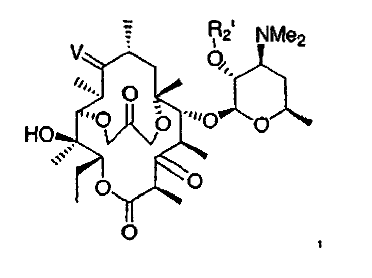 wobei V und R₂' wie vorstehend definiert sind, mit einer Phosphoniumverbindung in der Gegenwart von einer Base.

36. Verfahren zur Darstellung einer Verbindung nach Anspruch 1, wiedergegeben durch die Formel 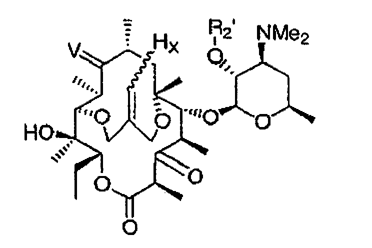 wobei V ausgewählt ist aus N-Q oder O; Hx für Halogen steht und Q, R₁₁ und R₂' wie in Anspruch 1 definiert sind, umfassend den Schritt des:
(a) Umsetzens einer Verbindung, wiedergegeben durch die Formel 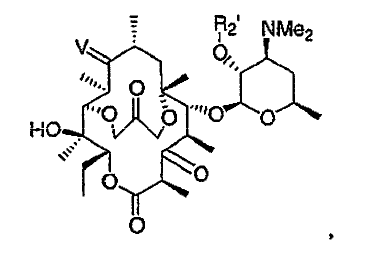 wobei V und R₂' wie vorstehend definiert sind, mit einem Phosphoniumsalz in der Gegenwart von einer Base.

37. Verfahren zur Darstellung einer Verbindung nach Anspruch 1, wiedergegeben durch die Formel 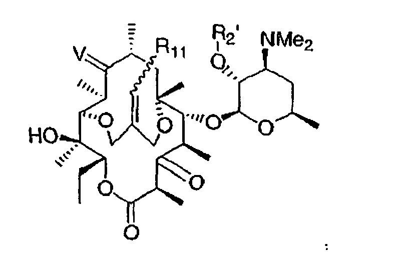 wobei V ausgewählt ist aus N-Q oder O; und Q, R₁₁ und R₂' wie in Anspruch 1 definiert sind, umfassend den Schritt des:
(a) Umsetzens einer Verbindung, wiedergegeben durch die Formel 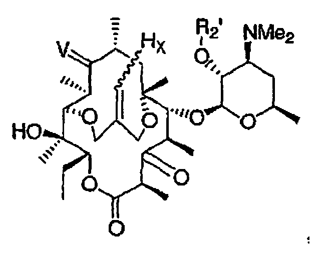 wobei Hx für Halogen steht und V und R₂' wie vorstehend definiert sind, mit einer Organobor- oder einer Oragnozinn-Verbindung in der Gegenwart von einem Palladium-Katalysator und einer Base.

38. Verfahren zur Darstellung einer Verbindung nach Anspruch 1, wiedergegeben durch die Formel 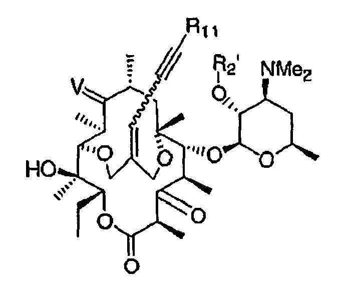 wobei V ausgewählt ist aus N-Q oder O; und Q, R₁₁ und R₂' wie in Anspruch 1 definiert sind, umfassend den Schritt des:
(a) Umsetzens einer Verbindung, wiedergegeben durch die Formel 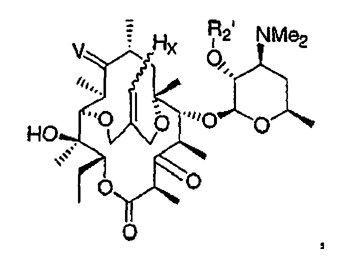 wobei Hx für Halogen steht und V und R₂' wie vorstehend definiert sind, mit einer Verbindung, die durch die Formel H 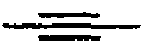 R₁₁ wiedergegeben wird, wobei R₁₁ wie vorstehend definiert ist, in der Gegenwart von einem Palladium-Katalysator, einem Kupferhalogenid und einem Amin.

39. Verfahren zur Darstellung einer Verbindung nach Anspruch 1, wiedergegeben durch die Formel 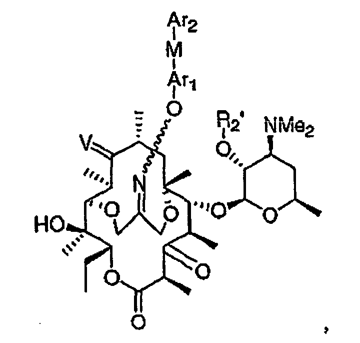 wobei V ausgewählt ist aus N-Q oder O; und Q, Ar₁, M, Ar₂ und R₂' wie in Anspruch 1 definiert sind, umfassend die Schritte:
(a) Umsetzen einer Verbindung, wiedergegeben durch die Formel (Ia), 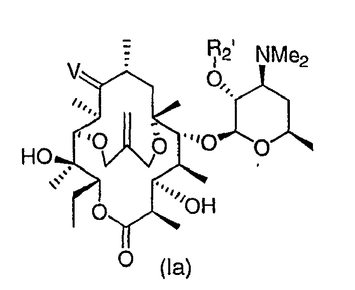 wobei V und R₂' wie vorstehend definiert sind, mit einem Reagenz oder Reagenzien, das/die in der Lage ist/sind, eine oxidative Spaltung durchzuführen;
(b) Umsetzen einer Verbindung aus Schritt (a), wiedergegeben durch die Formel (Ib), 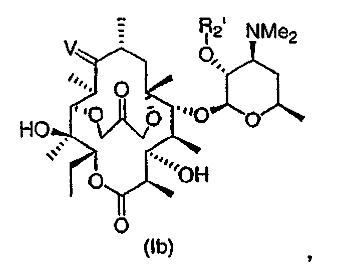 wobei V und R₂' wie vorstehend definiert sind, mit einem Oxidationsmittel, um eine 3-Keto-Verbindung bereitzustellen;
(c) Umsetzen einer Verbindung aus Schritt (b), wiedergegeben durch die Formel (Ic), 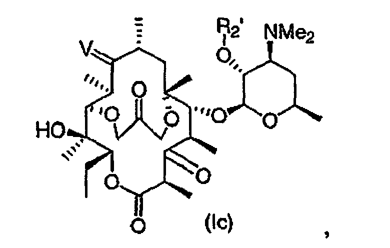 wobei V und R₂' wie vorstehend definiert sind, mit einer Verbindung der Formel Ar₂-M-Ar₁-O-NH₂, wobei Ar₁, Ar₂ und M wie vorstehend in Anspruch 1 definiert sind, in der Gegenwart von einer Säure oder einer Base; und
(d) gegebenenfalls Entschützen der Verbindung aus Schritt (c).

40. Verfahren zur Darstellung einer Verbindung nach Anspruch 1, wiedergegeben durch die Formel 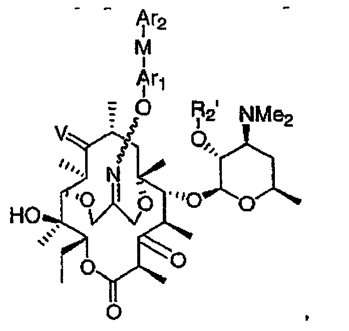 wobei V ausgewählt ist aus N-Q oder O; und Q, Ar₁, M, Ar₂ und R₂' wie in Anspruch 1 definiert sind, umfassend die Schritte:
(a) Umsetzen einer Verbindung, wiedergegeben durch die Formel (Ib), 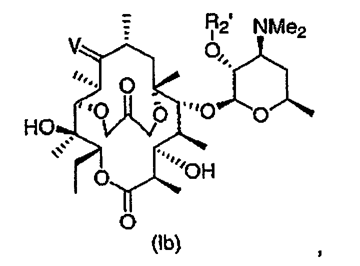 wobei V und R₂' wie vorstehend definiert sind, mit einer Verbindung der Formel Ar₂-M-Ar₁-O-NH₂, wobei Ar₁, Ar₂ und M wie vorstehend in Anspruch 1 definiert sind, in der Gegenwart von einer Säure oder einer Base;
(b)Umsetzen einer Verbindung aus Schritt (a) mit einem Oxidationsmittel, um eine 3-Keto-Verbindung bereitzustellen; und
(c) gegebenenfalls Entschützen der Verbindung, die in Schritt (b) gebildet wurde.

41. Verfahren zur Darstellung einer Verbindung nach Anspruch 1, wiedergegeben durch die Formel 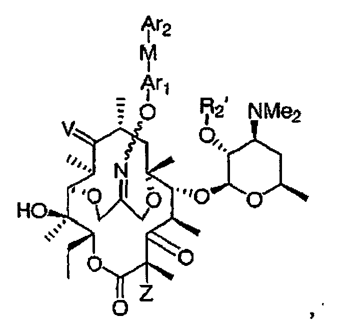 wobei V ausgewählt ist aus N-Q oder O; und Q, Ar₁, M, Ar₂ R₂' und Z wie in Anspruch 1 definiert sind, umfassend die Schritte:
(a) Umsetzen einer Verbindung, wiedergegeben durch die Formel (Ia), 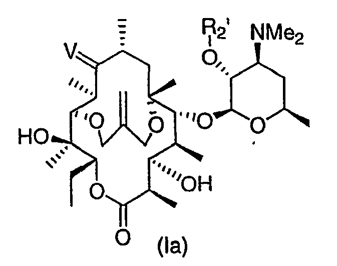 wobei V und R₂' wie vorstehend definiert sind, mit einem Oxidationsmittel, um eine 3-Keto-Verbindung bereitzustellen;
(b) Umsetzen einer Verbindung aus Schritt (a), wiedergegeben durch die Formel (Ib), 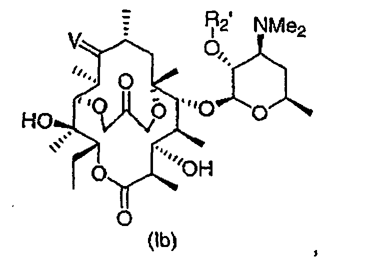 wobei V und R₂' wie vorstehend definiert sind, mit einem Halogenierungsmittel oder einem Methylierungsmittel in der Gegenwart von einer Base.
(c)Umsetzen einer Verbindung aus Schritt (b), wiedergegeben durch die Formel (Ic), 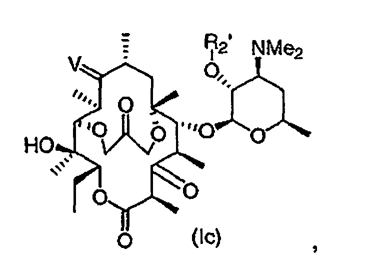 wobei V, Z und R₂' wie vorstehend definiert sind, mit einem Reagenz oder Reagenzien, das/die in der Lage ist/sind, eine oxidative Spaltung durchzuführen;
(d)Umsetzen einer Verbindung aus Schritt (c), wiedergegeben durch die Formel (Id), 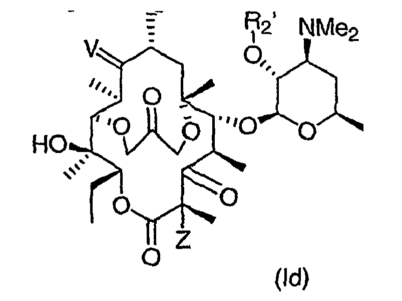 wobei V, Z und R₂' wie vorstehend definiert sind, mit einer Verbindung der Formel Ar₂-M-Ar₁-O-NH₂, wobei Ar₁, Ar₂ und M wie vorstehend in Anspruch 1 definiert sind, in der Gegenwart von einer Säure oder einer Base; und
(e) gegebenenfalls Entschützen von einer Verbindung aus Schritt (d)

## Revendications

1. Composé représenté par la formule: 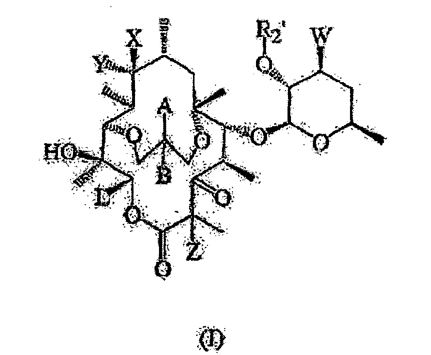 ou son sel ou ester acceptable sur le plan pharmaceutique,
dans laquelle
A est choisi parmi :
a) -OH ;
b) -ORₚ, où Rp est un groupe de protection hydroxy ;
c) -R₁, où R₁ est indépendamment choisi parmi :
(1) un aryle ;
(2) un aryle substitué;
(3) un hétéroaryle ; et
(4) un hétéroaryle substitué;
d) -OR₁, où R₁ est tel que défini précédemment ;
e) -R₂, où R₂ est choisi parmi :
(1) un hydrogène;
(2) un halogène;
(3) un alkyle en C₁ à C₁₂ contenant éventuellement 0, 1, 2 ou 3 hétéroatomes choisis parmi O, S ou N, éventuellement substitué avec un ou plusieurs substituants choisis parmi un halogène, un aryle, un aryle substitué, un hétéroaryle et un hétéroaryle substitué ;
(4) un alcényle en C₂ à C₁₂ contenant éventuellement 0, 1, 2 ou 3 hétéroatomes choisis parmi O, S et N, éventuellement substitué avec un ou plusieurs substituants choisis parmi un halogène, un aryle, un aryle substitué, un hétéroaryle et un hétéroaryle substitué ; et
(5) un alcynyle en C₂ à C₁₂ contenant éventuellement 0, 1, 2 ou 3 hétéroatomes choisis parmi O, S et N, éventuellement substitué avec un ou plusieurs substituants choisis parmi un halogène, un aryle, un aryle substitué, un hétéroaryle et un hétéroaryle substitué ;
f) -OR₂, où R₂ est indépendamment défini précédemment ;
g) -S(O)ₙR₁₁, où n = 0, 1 ou 2, et R₁₁ est indépendamment un hydrogène, R₁ ou R₂, où R₁ et R₂ sont tels que définis précédemment ;
h) -NHC(O)R₁₁, où R₁₁ est tel que défini précédemment ;
i) -NHC(O)NHR₁₁, où R₁₁ est tel que défini précédemment ;
j) -NHS(O)₂R₁₁, où R₁₁ est tel que défini précédemment ;
k) -NR₁₄R₁₅, où R₁₄ et R₁₅ sont chacun indépendamment R₁₁, où R₁₁ est tel que défini précédemment ; et
l) -NHR₃, où R₃ est un groupe de protection amino;
B est choisi parmi :
a) un hydrogène;
b) un deutérium;
c) un halogène;
d) -OH;
e) R₁, où R₁ est tel que défini précédemment;
f) R₂, où R₂ est tel que défini précédemment; et
g) -ORₚ, où Rp est tel que défini précédemment,
pour autant que lorsque B est un halogène, -OH, ou -ORₚ, A est R₁ ou R₂;
ou selon une autre possibilité, A et B pris ensemble avec l'atome de carbone auquel ils sont fixés sont choisis parmi :
a) C=O ;
b) C(OR₂)₂, où R₂ est tel que défini précédemment;
c) C(SR₂)₂, où R₂ est tel que défini précédemment;
d) C[-O(CH₂)ₘ]₂, où m = 2 ou 3;
e) C[-S(CH₂)ₘ]₂, où m est tel que défini précédemment;
f) C=CHR₁₁, où R₁₁ est tel que défini précédemment;
g) C=N-O-R₁₁, où R₁₁ est tel que défini précédemment;
h) C=N-O-Ar₁-M-Ar₂, où
(1) -Ar₁- est R₃₁, où R₃₁ est indépendamment choisi parmi :
(a) R₁, où R₁ est tel que défini précédemment;
(b) un alkyle en C₁ à C₁₂ contenant éventuellement 0, 1, 2 ou 3 hétéroatomes choisis parmi O, S ou N, éventuellement substitué avec un ou plusieurs substituants choisis parmi un halogène, un aryle, un aryle substitué, un hétéroaryle et un hétéroaryle substitué ;
(c) un alcényle en C₂ à C₁₂ contenant éventuellement 0, 1, 2 ou 3 hétéroatomes choisis parmi O, S et N, éventuellement substitué avec un ou plusieurs substituants choisis parmi un halogène, un aryle, un aryle substitué, un hétéroaryle et un hétéroaryle substitué ; ou
(d) un alcynyle en C₂ à C₁₂ contenant éventuellement 0, 1, 2 ou 3 hétéroatomes choisis parmi O, S et N, éventuellement substitué avec un ou plusieurs substituants choisis parmi un halogène, un aryle, un aryle substitué, un hétéroaryle et un hétéroaryle substitué ;
(2) -M- est absent ou est choisi parmi :
(a) un alkyle en C₁ à C₁₂ contenant éventuellement:
0 à 3 hétéroatomes choisis parmi O, S ou N ; et
0 à 3 groupes choisis parmi -C=N-, -N=N, -C(O)- ;
(b) un alcényle en C₂ à C₁₂ contenant éventuellement :
0 à 3 hétéroatomes choisis parmi O, S ou N ; et
0 à 3 groupes choisis parmi -C=N-, -N=N, -C(O)- ;
(c) un alcynyle en C₂ à C₁₂ contenant éventuellement:
0 à 3 hétéroatomes choisis parmi 0, S ou N ;
0 à 3 groupes choisis parmi -C=N-, -N=N, -C(O)- ;
(d) un aryle substitué ;
(e) un hétéroaryle substitué ; ou
(f) un hétérocycloalkyle substitué ; et
(3) -Ar₂ est choisi parmi :
(a) un aryle ;
(b) un aryle substitué ;
(c) un hétéroaryle ; ou
(d) un hétéroaryle substitué ;
i) C=NNHR₁₁, où R₁₁ est tel que défini précédemment ;
j) C=NNHC(O)R₁₁, où R₁₁ est tel que défini précédemment ;
k) C=NNHC(O)NHR₁₁, où R₁₁ est tel que défini précédemment ;
l) C=NNHS(O)₂R₁₁, où R₁₁ est tel que défini précédemment ;
m) C=NNHR₃, où R₃ est tel que défini précédemment ;
n) C=NR₁₁, où R₁₁ est tel que défini précédemment ; ou
o) C=N-N=CHR₁₁, où R₁₁ est tel que défini précédemment ;
un de X et Y est un hydrogène et l'autre est choisi parmi :
a) un hydrogène;
b) un deutérium ;
c) -OH ;
d) -ORₚ, où Rp est tel que défini précédemment ;
e) -NR₄R₅, où R₄ et R₅ sont chacun indépendamment choisis parmi :
(1) un hydrogène ;
(2) un alkyle en C₁ à C₁₂, éventuellement substitué avec un ou plusieurs substituants choisis parmi un halogène, un aryle, un aryle substitué, un hétéroaryle et un hétéroaryle substitué ; ou
(3) R₄ et R₅, pris ensemble avec l'atome d'azote auquel ils sont fixés forment un cycle hétéroalkyle de 3 à 10 membres contenant 0 à 2 hétéroatomes supplémentaires choisis parmi O, S et N ; ou
selon une autre possibilité, X et Y pris ensemble avec l'atome de carbone auquel ils sont fixés sont choisis parmi :
a) C=O ;
b) C=N-Q, où Q est choisi parmi :
(1) -R₁₁, où R₁₁ est tel que défini précédemment ;
(2) un groupe de protection amino ;
(5) -C(O)R₁₁, où R₁₁ est tel que défini précédemment ;
(6) -OR₆, où R₆ est indépendamment choisi parmi :
(a) un hydrogène ;
(b) -CH₂O(CH₂)₂OCH₃,
(c) -CH₂O(CH₂O)ₙCH₃, où n est tel que défini précédemment ;
(d) un alkyle en C₁ à C₁₂, éventuellement substitué avec un ou plusieurs substituants choisis parmi un aryle, un aryle substitué, un hétéroaryle et un hétéroaryle substitué ;
(e) un cycloalkyle en C₃ à C₁₂ ;
(f) -C(O)- alkyle en C₁ à C₁₂ ;
(g) -C(O)- cycloalkyle en C₃ à C₁₂ ;
(h) -C(O)-R₁, où R₁ est tel que défini précédemment ; ou
(i) -Si(Rₐ)(R_{b})(R_{c}), où Rₐ, R_{b} et R_{c} sont chacun indépendamment choisis parmi un alkyle en C₁ à C₁₂, un aryle et un aryle substitué ; ou
(5) O-C(R₇)(R₈)-O-R₆, où R₆ est tel que défini précédemment, pour autant que R₆ ne soit pas C(O)- alkyle en C₁ à C₂, C(O)- cycloalkyle en C₃ à C₁₂, ou C(O)-R₁, et R₇ et R₈ pris ensemble avec l'atome de carbone auquel ils sont fixés forment un groupe cycloalkyle en C₃ à C₁₂ ou chacun est indépendamment choisi parmi :
(1) un hydrogène ; ou
(2) un alkyle en C₁ à C₁₂ ;
L est choisi parmi :
a) -CH₃ ;
b) -CH₂CH₃ ;
c) -CH(OH)CH₃ ;
d) un alkyle en C₁ à C₆, éventuellement substitué avec un ou plusieurs substituants choisis parmi un aryle, un aryle substitué, un hétéroaryle et un hétéroaryle substitué;
e) un alcényle en C₂ à C₆, éventuellement substitué avec un ou plusieurs substituants choisis parmi un aryle, un aryle substitué, un hétéroaryle et un hétéroaryle substitué ; ou
f) un alcynyle en C2 à C6, éventuellement substitué avec un ou plusieurs substituants choisis parmi un aryle, un aryle substitué, un hétéroaryle et un hétéroaryle substitué ;
W est -NR₂₀R₂₁, où R₂₀ et R₂₁ sont chacun indépendamment choisis parmi :
a) un hydrogène ;
b) un alkyle en C₁ à C₁₂, éventuellement substitué avec un ou plusieurs substituants choisis parmi un halogène, un aryle, un aryle substitué, un hétéroaryle et un hétéroaryle substitué ;
c) un alcényle en C₂ à C₁₂, éventuellement substitué avec un ou plusieurs substituants choisis parmi un aryle, un aryle substitué, un hétéroaryle et un hétéroaryle substitué ;
d) un alcynyle en C₂ à C₁₂, éventuellement substitué avec un ou plusieurs substituants choisis parmi un aryle, un aryle substitué, un hétéroaryle et un hétéroaryle substitué ;
e) R₂₀ et R₂₁, pris ensemble avec l'atome d'azote auquel ils sont fixés forment un fragment hétérocycloalkyle ; ou
Z est choisi parmi :
a) un hydrogène ;
b) un méthyle ; ou
c) un halogène ; et
R₂' est un hydrogène ou Rp, où Rp est tel que défini précédemment.

2. Composé selon la revendication 1, qui est représenté par la formule: 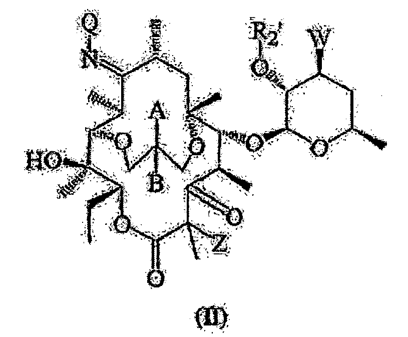 dans laquelle A, B, R₂' , Q, W, et Z sont tels que définis dans la revendication 1.

3. Composé selon la revendication 1, qui est représenté par la formule: 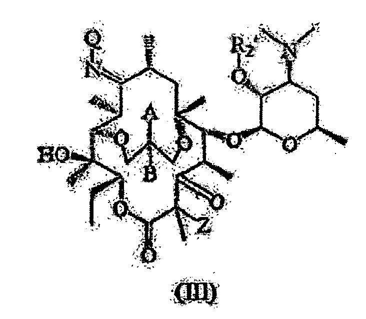 dans laquelle A, B, R₂', Q, et Z sont tels que définis dans la revendication 1.

4. Composé selon la revendication 1, qui est représenté par la formule: 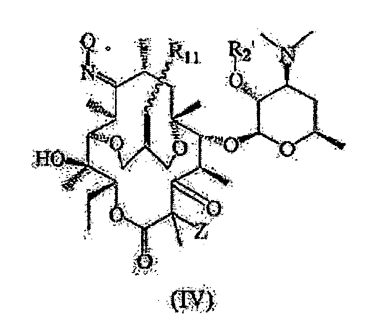 dans laquelle R₁₁, R₂', Q, et Z sont tels que définis dans la revendication 1.

5. Composé selon la revendication 1, qui est représenté par la formule: 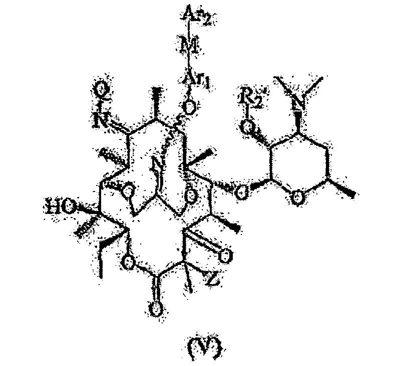 dans laquelle Ar₁, Ar₂, R₂', M, Q, W, et Z sont tels que définis dans la revendication 1.

6. Composé selon la revendication 1, qui est représenté par la formule: 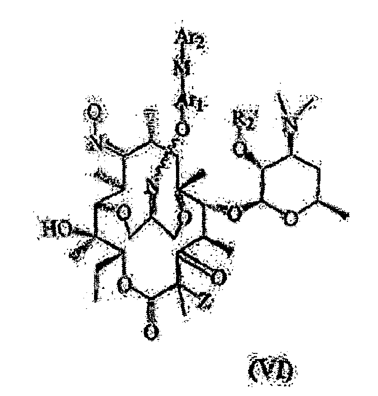 dans laquelle Ar₁, Ar₂, R₂', M, Q et Z sont tels que définis dans la revendication 1.

7. Composé selon la revendication 1, qui est représenté par la formule: 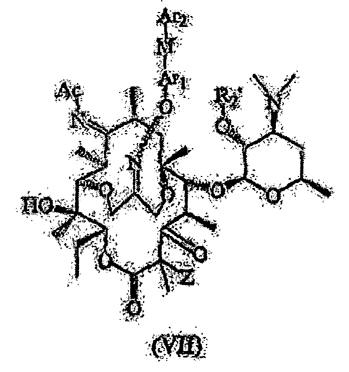 dans laquelle Ar₁, Ar₂, R₂', M et Z sont tels que définis dans la revendication 1.

8. Composé selon la revendication 1, qui est représenté par la formule: 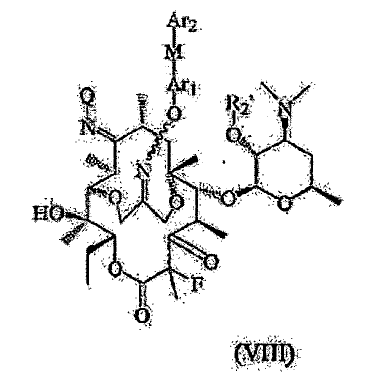 dans laquelle Ar₁, Ar₂, R₂', M et Q sont tels que définis dans la revendication 1.

9. Composé selon la revendication 1, qui est choisi parmi :
(1) Composé de formule I : A et B pris ensemble avec l'atome de carbone auquel ils sont fixés sont C=CH₂, X et Y pris ensemble avec l'atome de carbone auquel ils sont fixés sont C=N-Ac, L = CH₂CH₃, Z = H, et R₂' = Ac ;
(2) Composé de formule I : A et B pris ensemble avec l'atome de carbone auquel ils sont fixés sont C=CH₂, X et Y pris ensemble avec l'atome de carbone auquel ils sont fixés sont C=N-Ac, L = CH₂CH₃, Z = H, et R₂' = H ;
(3) Composé de formule I : A et B pris ensemble avec l'atome de carbone auquel ils sont fixés sont C=O, X et Y pris ensemble avec l'atome de carbone auquel ils sont fixés sont C=N-Ac, L = CH₂CH₃, Z = H, et R₂' = H;
(4) Composé de formule I : A et B pris ensemble avec l'atome de carbone auquel ils sont fixés sont C=N-O-CH₂-Ph, X et Y pris ensemble avec l'atome de carbone auquel ils sont fixés sont C=N-Ac, L = CH₂CH₃, Z = H et R₂'=H;
(5) Composé de formule I : A et B pris ensemble avec l'atome de carbone auquel ils sont fixés sont C=N-O-CH₂-(3-pyridyle), X et Y pris ensemble avec l'atome de carbone auquel ils sont fixés sont C=N-Ac, L = CH₂CH₃, Z= H et R₂' = H ;
(6) Composé de formule I : A et B pris ensemble avec l'atome de carbone auquel ils sont fixés sont C=N-O-CH₂-(2-pyridyle), X et Y pris ensemble avec l'atome de carbone auquel ils sont fixés sont C=N-Ac, L = CH₂CH₃, Z= H et R₂' = H ;
(7) Composé de formule I : A et B pris ensemble avec l'atome de carbone auquel ils sont fixés sont C=N-O-CH₂-(3-quinolyle), X et Y pris ensemble avec l'atome de carbone auquel ils sont fixés sont C=N-Ac, L = CH₂CH₃, Z = H et R₂' = H ;
(8) Composé de formule I : A et B pris ensemble avec l'atome de carbone auquel ils sont fixés sont C=N-O-CH₂-(2-quinolyle), X et Y pris ensemble avec l'atome de carbone auquel ils sont fixés sont C=N-Ac, L = CH₂CH₃, Z = H et R₂' = H ;
(9) Composé de formule I : A et B pris ensemble avec l'atome de carbone auquel ils sont fixés sont C=N-O-(5-pyridin-2-ylthiophén-2yle)-méthyle, X et Y pris ensemble avec l'atome de carbone auquel ils sont fixés sont C=N-Ac, L = CH₂CH₃, Z = H et R₂' = H ;
(10) Composé de formule I : A et B pris ensemble avec l'atome de carbone auquel ils sont fixés sont C=N-O-(3-pyrimidin-2-ylprop-2-ynyle), X et Y pris ensemble avec l'atome de carbone auquel ils sont fixés sont C=N-Ac, L = CH₂CH₃, Z = H et R₂' = H ;
(11) Composé de formule I : A et B pris ensemble avec l'atome de carbone auquel ils sont fixés sont C=N-O-Ph, X et Y pris ensemble avec l'atome de carbone auquel ils sont fixés sont C=N-Ac, L = CH₂CH₃, Z = H et R₂'=H ;
(12) Composé de formule I : A = NHCH₂-Ph, B = H, X et Y pris ensemble avec l'atome de carbone auquel ils sont fixés sont C=N-Ac, L = CH₂CH₃, Z = H et R₂' = H ;
(13) Composé de formule I : A = NHCH₂CH₂-Ph, B = H, X et Y pris ensemble avec l'atome de carbone auquel ils sont fixés sont C=N-Ac, L = CH₂CH₃, Z = H et R₂' = H ;
(14) Composé de formule I : A et B pris ensemble avec l'atome de carbone auquel ils sont fixés sont C=CH₂, X et Y pris ensemble avec l'atome de carbone auquel ils sont fixés sont C=N-O-CH₂-O-CH₃, L = CH₂CH₃, Z = H et R₂' = Ac ;
(15) Composé de formule I : A et B pris ensemble avec l'atome de carbone auquel ils sont fixés sont C=CH₂, X et Y pris ensemble avec l'atome de carbone auquel ils sont fixés sont C=N-O-CH₂-O-CH₃, L = CH₂CH₃, Z = H et R₂' = H ;
(16) Composé de formule I : A et B pris ensemble avec l'atome de carbone auquel ils sont fixés sont C=O, X et Y pris ensemble avec l'atome de carbone auquel ils sont fixés sont C=N-O-CH₂-O-CH₃, L = CH₂CH₃, Z = H et R₂' = H ;
(17) Composé de formule I : A et B pris ensemble avec l'atome de carbone auquel ils sont fixés sont C=NOCH₂-Ph, X et Y pris ensemble avec l'atome de carbone auquel ils sont fixés sont C=N-O-CH₂-O-CH₃, L = CH₂CH₃, Z = H et R₂' = H;
(18) Composé de formule I : A et B pris ensemble avec l'atome de carbone auquel ils sont fixés sont C=O, X et Y pris ensemble avec l'atome de carbone auquel ils sont fixés sont C=O, L = CH₂CH₃, Z = H, et R₂' = Ac ;
(19) Composé de formule I : A et B pris ensemble avec l'atome de carbone auquel ils sont fixés sont C=CH₂, X et Y pris ensemble avec l'atome de carbone auquel ils sont fixés sont C=O, L = CH₂CH₃, Z = H, et R₂' = H ;
(20) Composé de formule I : A et B pris ensemble avec l'atome de carbone auquel ils sont fixés sont C=O, X et Y pris ensemble avec l'atome de carbone auquel ils sont fixés sont C=O, L = CH₂CH₃, Z = H et R₂' = H ;
(21) Composé de formule I : A et B pris ensemble avec l'atome de carbone auquel ils sont fixés sont C=N-O-CH₂-Ph, X et Y pris ensemble avec l'atome de carbone auquel ils sont fixés sont C=O, L = CH₂CH₃, Z = H et R₂' =H ;
(22) Composé de formule I : A et B pris ensemble avec l'atome de carbone auquel ils sont fixés sont C=CH₂, X et Y pris ensemble avec l'atome de carbone auquel ils sont fixés sont C=NH, L = CH₂CH₃, Z = H et R₂' = H ;
(23) Composé de formule I : A et B pris ensemble avec l'atome de carbone auquel ils sont fixés sont C=CH-O-CH₂-p-NO₂-Ph, X et Y pris ensemble avec l'atome de carbone auquel ils sont fixés sont C=N-Ac, L = CH₂CH₃, Z = H et R₂' = H ;
(24) Composé de formule I : A et B pris ensemble avec l'atome de carbone auquel ils sont fixés sont C=N-O-(CH₂)₂-Ph, X et Y pris ensemble avec l'atome de carbone auquel ils sont fixés sont C=N-Ac, L = CH₂CH₃, Z = H et R₂' = H ;
(25) Composé de formule I : A et B pris ensemble avec l'atome de carbone auquel ils sont fixés sont C=N-O-(CH₂)₃-Ph, X et Y pris ensemble avec l'atome de carbone auquel ils sont fixés sont C=N-Ac, L = CH₂CH₃, Z = H et R₂' = H ;
(26) Composé de formule I : A et B pris ensemble avec l'atome de carbone auquel ils sont fixés sont C=N-O-CH₂-CH=CH-Ph, X et Y pris ensemble avec l'atome de carbone auquel ils sont fixés sont C=N-Ac, L = CH₂CH₃, Z = H et R₂' = H ;
(27) Composé de formule I : A est NH-(CH₂)₃-Ph, B est H, X et Y pris ensemble avec l'atome de carbone auquel ils sont fixés sont C=N-Ac, L = CH₂CH₃, Z = H et R₂' = H ;
(28) Composé de formule I : A est NH-(CH₂)₄-Ph, B est H, X et Y pris ensemble avec l'atome de carbone auquel ils sont fixés sont C=N-Ac, L = CH₂CH₃, Z = H et R₂' = H ;
(29) Composé de formule I : A est CH₂-CH=CH₂, B est OH, X et Y pris ensemble avec l'atome de carbone auquel ils sont fixés sont C=N-Ac, L = CH₂CH₃, Z = H et R₂' = H ;
(30) Composé de formule I : A est CH₂-Ph, B est OH, X et Y pris ensemble avec l'atome de carbone auquel ils sont fixés sont C=N-Ac, L = CH₂CH₃, Z = H et R₂' = H ;
(31) Composé de formule I : A est Ph, B est OH, X et Y pris ensemble avec l'atome de carbone auquel ils sont fixés sont C=N-Ac, L = CH₂CH₃, Z = H et R₂' = H ;
(33) Composé de formule I : A est CH₂-CH=CH-Ph, B est OH, X et Y pris ensemble avec l'atome de carbone auquel ils sont fixés sont C=N-Ac, L = CH₂CH₃, Z = H et R₂' = H ;
(34) Composé de formule I : A est (CH₂)₃-Ph, B est OH, X et Y pris ensemble avec l'atome de carbone auquel ils sont fixés sont C=N-Ac, L = CH₂CH₃, Z = H et R₂' = H ;
(35) Composé de formule I : A et B pris ensemble avec l'atome de carbone auquel ils sont fixés sont C=CH-CH=CH-Ph, X et Y pris ensemble avec l'atome de carbone auquel ils sont fixés sont C=N-Ac, L = CH₂CH₃, Z = H et R₂' = H ;
(36) Composé de formule I : A est (CH₂)₃-Ph, B est H, X et Y pris ensemble avec l'atome de carbone auquel ils sont fixés sont C=N-Ac, L = CH₂CH₃, Z = H, et R₂' = H ;
(37) Composé de formule I : A et B pris ensemble avec l'atome de carbone auquel ils sont fixés sont C=CH-CH=CH-(3-pyridyle), X et Y pris ensemble avec l'atome de carbone auquel ils sont fixés sont C=N-Ac, L = CH₂CH₃, Z = H et R₂' = H ;
(38) Composé de formule I : A et B pris ensemble avec l'atome de carbone auquel ils sont fixés sont C=CH-CH=CH-(3-quinolyle), X et Y pris ensemble avec l'atome de carbone auquel ils sont fixés sont C=N-Ac, L = CH₂CH₃, Z = H et R₂' = H ;
(39) Composé de formule I : A et B pris ensemble avec l'atome de carbone auquel ils sont fixés sont C=CH-(2-quinolyle), X et Y pris ensemble avec l'atome de carbone auquel ils sont fixés sont C=N-Ac, L = CH₂CH₃, Z = H et R₂' = H ;
(40) Composé de formule I : A et B pris ensemble avec l'atome de carbone auquel ils sont fixés sont C=CH-(2-quinolyle), X et Y pris ensemble avec l'atome de carbone auquel ils sont fixés sont C=N-H, L = CH₂CH₃, Z = H et R₂' = H ;
(41) Composé de formule I : A et B pris ensemble avec l'atome de carbone auquel ils sont fixés sont C=CH-(4-biphényle), X et Y pris ensemble avec l'atome de carbone auquel ils sont fixés sont C=N-Ac, L = CH₂CH₃, Z = H et R₂' = H ;
(42) Composé de formule I : A et B pris ensemble avec l'atome de carbone auquel ils sont fixés sont C=CH-(3-biphényle), X et Y pris ensemble avec l'atome de carbone auquel ils sont fixés sont C=N-Ac, L = CH₂CH₃, Z = H et R₂' = H ;
(43) Composé de formule I : A et B pris ensemble avec l'atome de carbone auquel ils sont fixés sont C=CH-(4-phenoxyphenyl), X et Y pris ensemble avec l'atome de carbone auquel ils sont fixés sont C=N-Ac, L = CH₂CH₃, Z = H et R₂' = H ;
(44) Composé de formule I : A et B pris ensemble avec l'atome de carbone auquel ils sont fixés sont C=CH-Ph, X et Y pris ensemble avec l'atome de carbone auquel ils sont fixés sont C=N-Ac, L = CH₂CH₃, Z = H et R₂' = H ;
(45) Composé de formule I : A et B pris ensemble avec l'atome de carbone auquel ils sont fixés sont C=CH-(2-(2pyridyl)-thiophén-5-yle), X et Y pris ensemble avec l'atome de carbone auquel ils sont fixés sont C=N-Ac, L = CH₂CH₃, Z = H et R₂' = H ; ou
(46) Composé de formule I : A et B pris ensemble avec l'atome de carbone auquel ils sont fixés sont C=O, X et Y pris ensemble avec l'atome de carbone auquel ils sont fixés sont C=N-Ac, L = CH₂CH₃, Z = H et R₂' = Ac.

10. Composé de formule A, choisi parmi les composés représentés dans le tableau 10 : 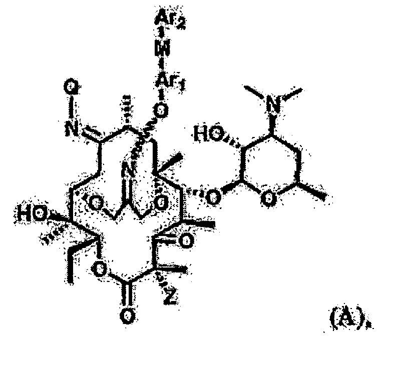
**Tableau 10**
| **Numéro** | **Q** | **-Ar₁-M-Ar₂** | **Z** |
|---|---|---|---|
| (1). | Ac | 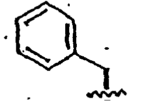 | H |
| (2). | Ac | 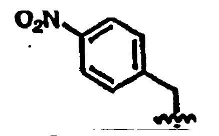 | H |
| (3). | Ac | 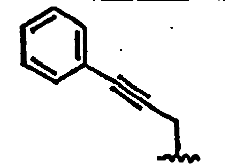 | H |
| (4). | Ac | 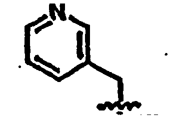 | H |
| (5). | Ac | 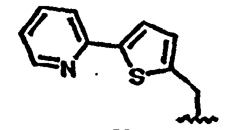 | H |
| (6). | Ac | 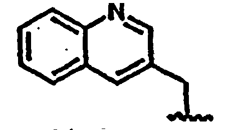 | H |
| (7). | Ac | 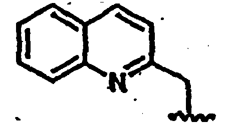 | H |
| (8). | Ac | 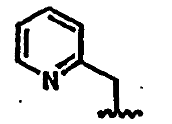 | H |
| (10). | Ac | 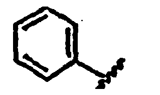 | H |
| (11). | Ac | 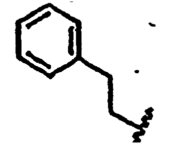 | H |
| (12). | Ac | 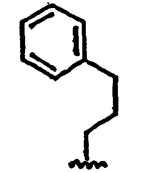 | H |
| (13). | Ac | 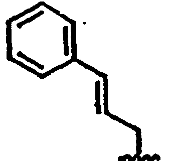 | H |
| (14). | Ac | 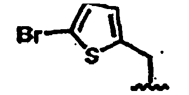 | H |
| (15). | Ac | 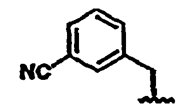 | H |
| (16). | Ac | 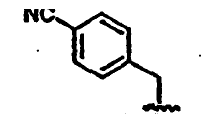 | H |
| (17). | Ac | 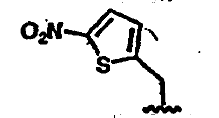 | H |
| (18). | Ac | 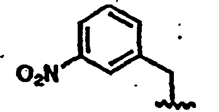 | H |
| (19). | Ac | 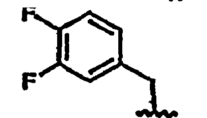 | H |
| (20). | Ac | 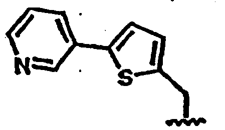 | H |
| (21). | Ac | 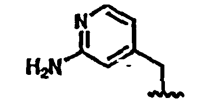 | H |
| (22). | Ac | 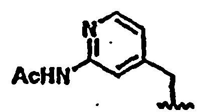 | H |
| (23). | Ac | 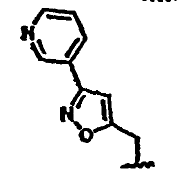 | H |
| (24). | Ac | 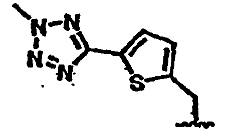 | H |
| (25). | Ac | 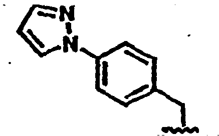 | H |
| (26). | Ac | 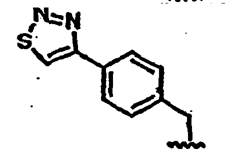 | H |
| (27). | Ac | 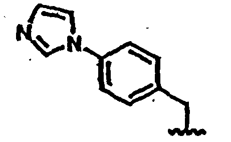 | H |
| (28). | Ac | 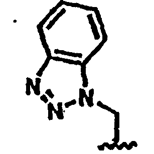 | H |
| (29). | Ac | 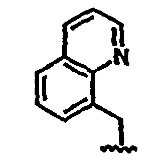 | H |
| (30). | Ac | 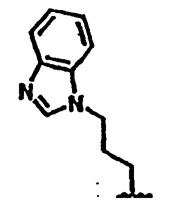 | H |
| (31). | Ac | 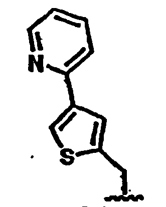 | H |
| (32). | Ac | 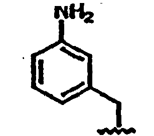 | H |
| (33). | Ac | 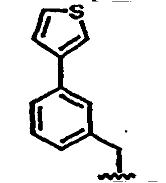 | H |
| (34). | Ac | 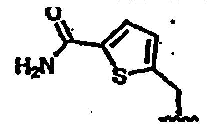 | F |
| (35). | H | 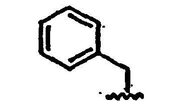 | H |
| (36). | Ac | 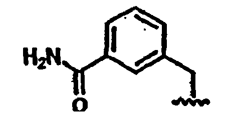 | H |
| (37). | Ac | 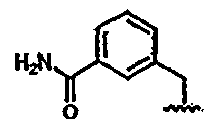 | F |
| (38). | Ac | 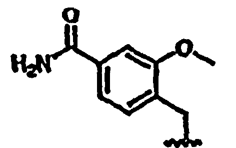 | H |
| (39). | Ac | 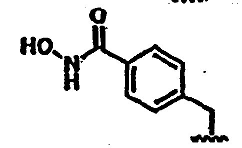 | H |
| (40). | Ac | 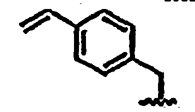 | H |
| (41). | Ac | 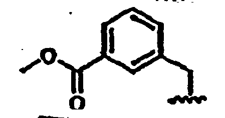 | H |
| (42). | Ac | 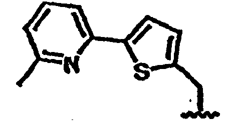 | H |
| (43). | Ac | 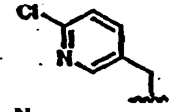 | H |
| (44). | H | 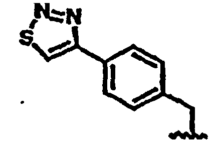 | H |
| (45). | H | 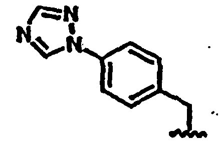 | H |
| (46). | OMe | 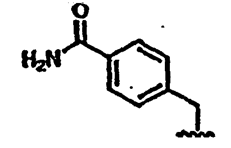 | H |
| (48). | -OCH₂CN | 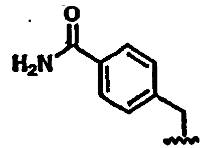 | H |
| (49). | -OCH₂CH₂OH | 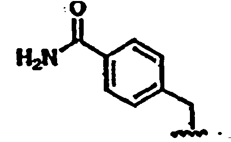 | H |
| (50). | H | 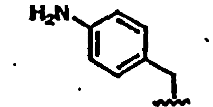 | H |
| (51). | Ac | 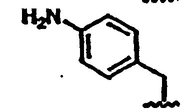 | H |
| (52). | Ac | 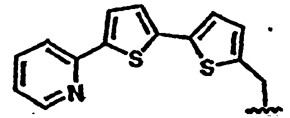 | H |
| (53). | Ac | 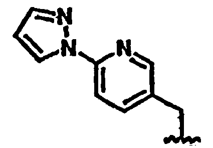 | H |
| (54). | Ac | 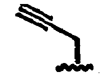 | H |
| (55). | Ac | 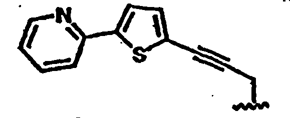 | H |
| (56). | Ac | 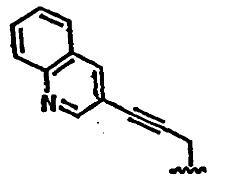 | H |
| (57). | Ac | 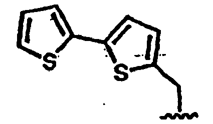 | H |
| (58). | Ac | 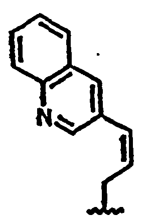 | H |
| (59). | Ac | 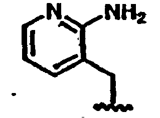 | H |
| (60). | Ac | 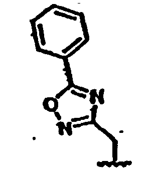 | H |
| (61). | Ac | 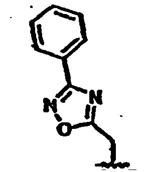 | H |
| (62). | Ac | 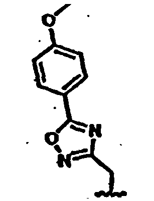 | H |
| (63). | Ac | 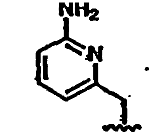 | H |
| (64). | Ac | 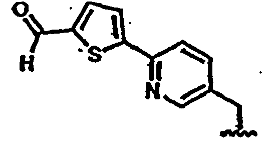 | H |
| (65). | Ac | 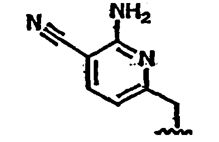 | H |

11. Composé selon la revendication 1, de formule A1, choisi parmi les composés représentés dans le tableau 11 : 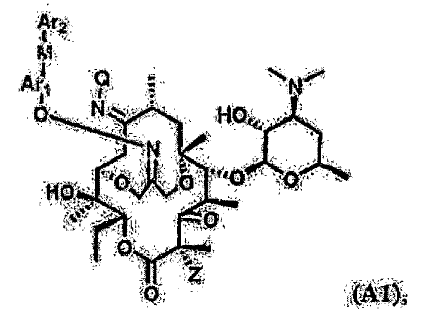
**Tableau 11**
| **Numéro** | **Q** | **-Ar₁-M-Ar₂** | **Z** |
|---|---|---|---|
| (1). | Ac | 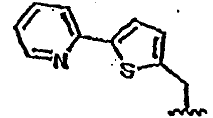 | H |
| (2). | Ac | 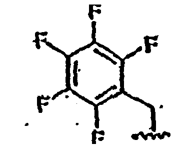 | H |
| (3). | Ac | 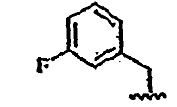 | H |
| (4). | Ac | 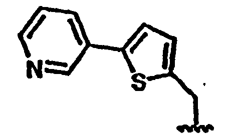 | H |
| (5). | Ac | 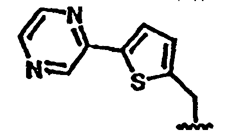 | H |
| (6). | Ac | 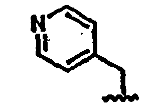 | H |
| (7). | Ac | 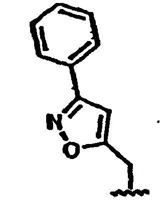 | H |
| (8). | Ac | 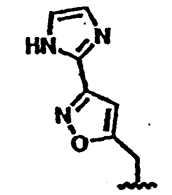 | H |
| (9). | Ac | 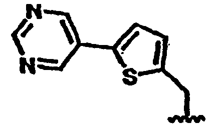 | H |
| (10). | Ac | 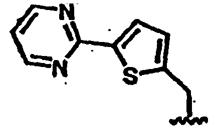 | H |
| (11). | Ac | 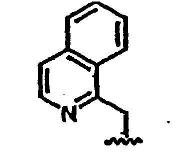 | H |
| (12). | Ac | 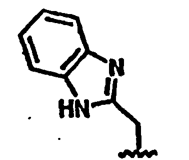 | H |
| (13). | Ac | 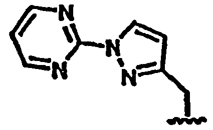 | H |
| (14). | Ac | 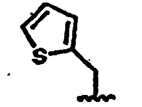 | H |
| (15). | Ac | 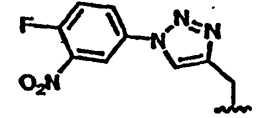 | H |
| (16). | Ac | 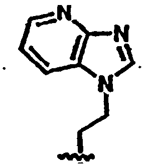 | H |
| (17). | Ac | 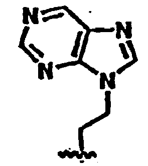 | H |
| (18). | Ac | 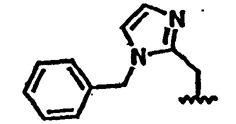 | H |
| (19). | Ac | 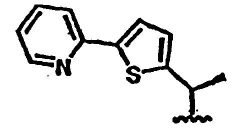 | H |
| (20). | Ac | 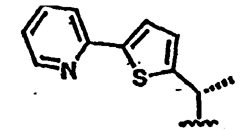 | H |
| (21). | Ac | 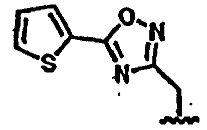 | H |
| (22). | Ac | 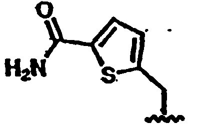 | H |
| (23). | Ac | 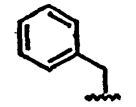 | H |
| (24). | Ac | 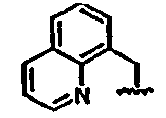 | F |
| (25). | Ac | 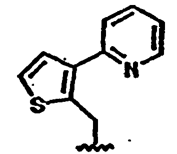 | H |
| (26). | Ac | 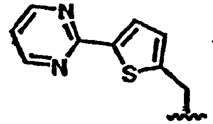 | F |
| (27). | Ac | 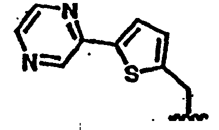 | F |
| (28). | Ac | 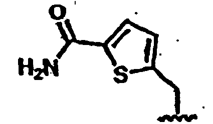 | F |
| (29). | Ac | 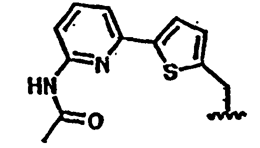 | H |
| (30). | Ac | 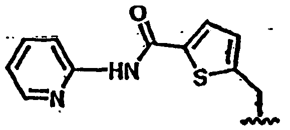 | H |
| (31). | Ac | 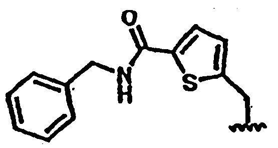 | H |
| (32). | Ac | 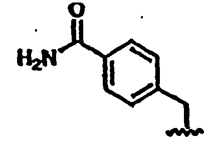 | H |
| (33). | Ac | 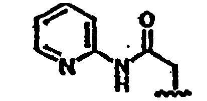 | H |
| (34). | Ac | 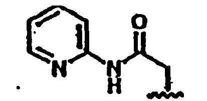 | F |
| (35). | Ac | 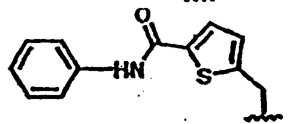 | H |
| (36). | Ac | 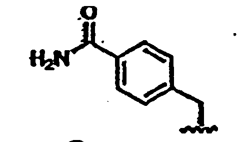 | F |
| (37). | Ac | 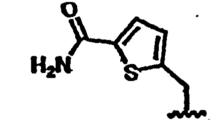 | H |
| (38). | Ac | 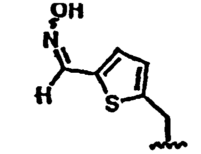 | H |
| (39). | Ac | 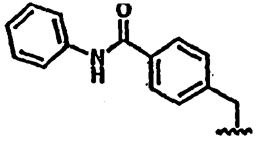 | H |
| (40). | Ac | 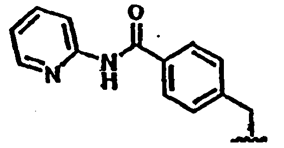 | H |
| (41). | Ac | 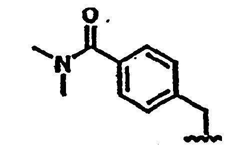 | H |
| (42). | Ac | 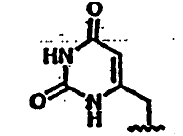 | H |
| (43). | Ac | 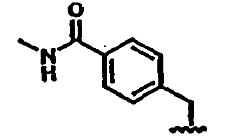 | H |
| (44). | Ac | 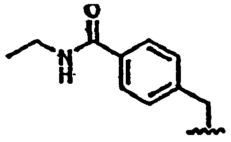 | H |
| (45). | Ac | 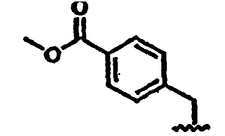 | H |
| (46). | Ac | 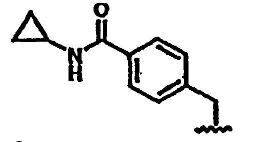 | H |
| (47). | Ac | 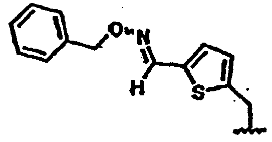 | H |
| (48). | Ac | 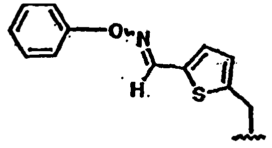 | H |
| (49). | Ac | 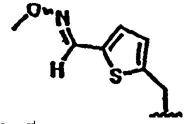 | H |
| (50). | Ac | 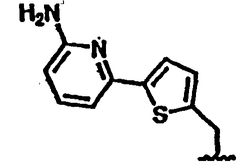 | H |
| (51). | Ac | 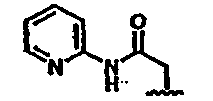 | H |
| (52). | Ac | 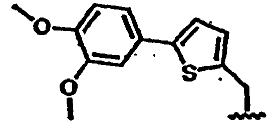 | H |
| (53). | Ac | 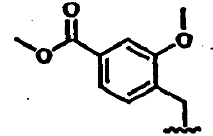 | H |
| (54). | Ac | 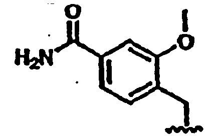 | H |
| (55). | Ac | 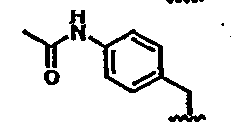 | H |
| (56). | Ac | 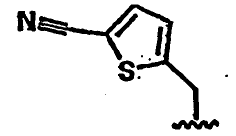 | H |
| (57). | Ac | 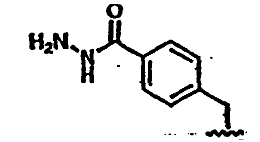 | H |
| (58). | Ac | 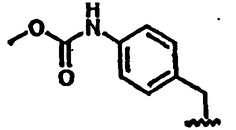 | H |
| (59). | Ac | 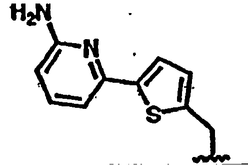 | F |
| (60). | Ac | 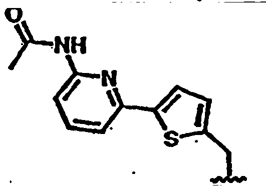 | F |
| (61). | Ac | 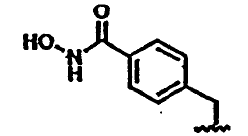 | H |
| (62). | Ac | 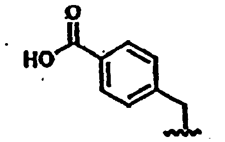 | H |
| (63). | Ac | 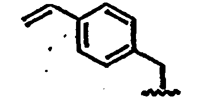 | H |
| (64). | Ac | 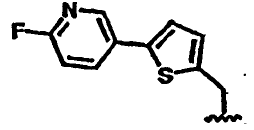 | H |
| (65). | Ac | 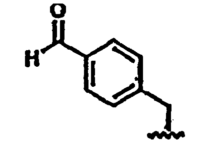 | H |
| (66). | H | 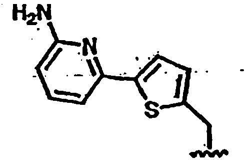 | H |
| (67). | Ac | 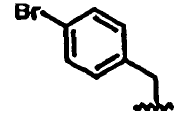 | H |
| (68). | Ac | 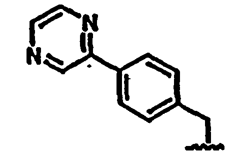 | H |
| (69). | Ac | 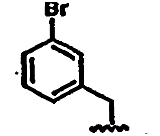 | H |
| (70). | Ac | 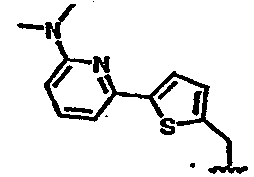 | F |
| (72). | Ac | 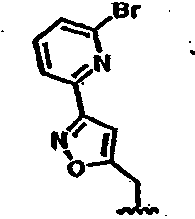 | H |
| (73). | Ac | 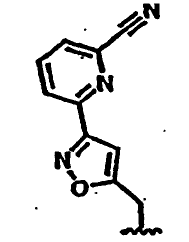 | H |
| (74). | Ac | 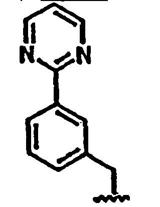 | H |
| (75). | Ac | 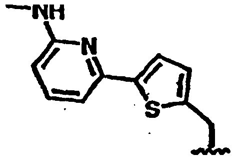 | F |
| (76). | Ac | 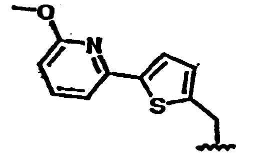 | H |
| (77). | H | 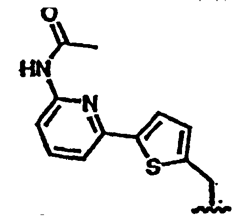 | H |
| (78). | Ac | 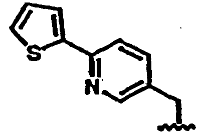 | H |
| (79). | Ac | 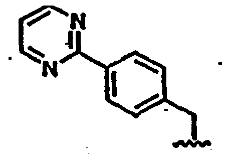 | H |
| (80). | H | 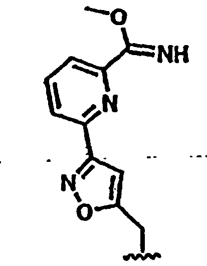 | H |
| (81). | Ac | 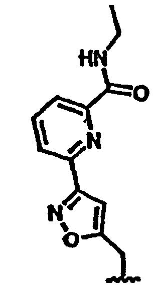 | H |
| (82). | H | 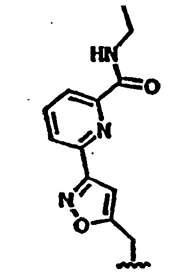 | H |
| (83). | H | 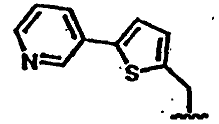 | H |
| (84). | H | 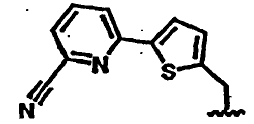 | H |
| (85). | Ac | 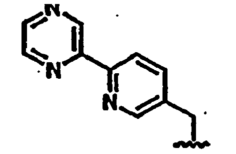 | H |
| (86). | Ac | 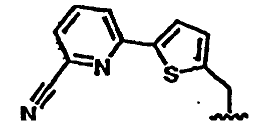 | H |
| (87). | Ac | 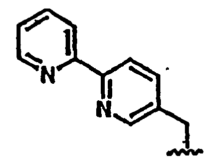 | H |
| (88). | H | 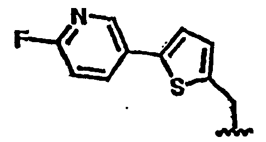 | H |
| (89). | H | 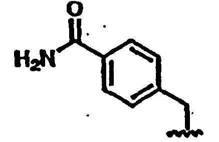 | H |
| (90). | H | 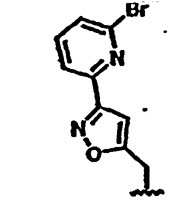 | H |
| (91). | Ac | 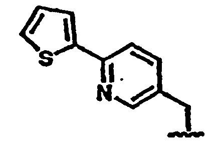 | H |
| (92). | H | 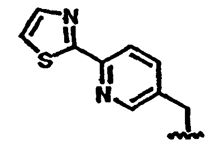 | H |
| (93). | Ac | 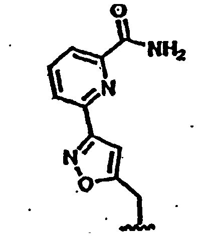 | H |
| (94). | Ac | 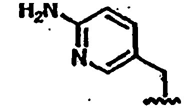 | H |
| (95). | Ac | 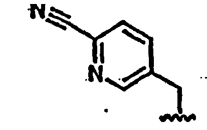 | H |
| (96). | Ac | 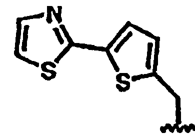 | H |
| (97). | Ac | 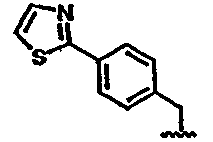 | F |
| (98). | H | 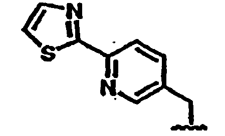 | F |
| (99). | H | 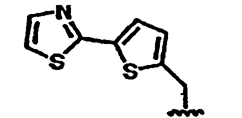 | H |
| (100). | Ac | 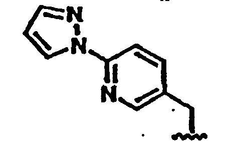 | F |
| (101). | Ac | 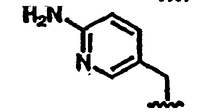 | F |
| (102). | Ac | 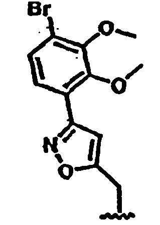 | H |
| (103). | Ac | 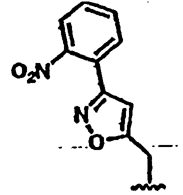 | H |
| (104). | Ac | 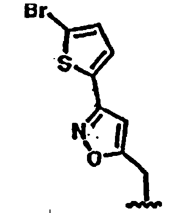 | H |
| (105). | Ac | 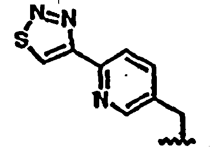 | F |
| (106). | Ac | 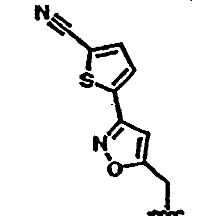 | H |
| (107). | Ac | 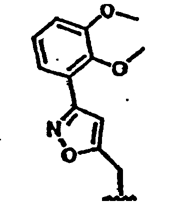 | H |
| (108). | Ac | 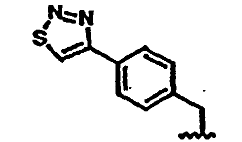 | H |
| (109). | Ac | 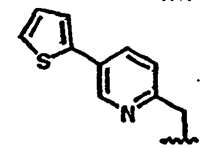 | H |
| (110). | Ac | 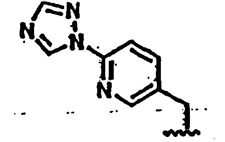 | H |
| (111). | Ac | 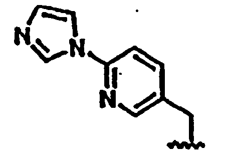 | H |
| (112). | Ac | 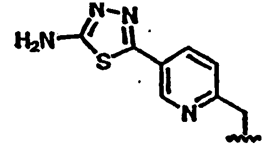 | H |
| (113). | Ac | 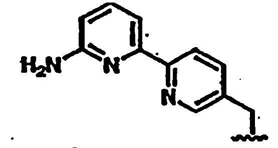 | H |
| (114). | Ac | 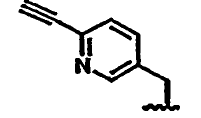 | H |
| (115). | H | 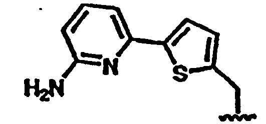 | F |
| (116). | H | 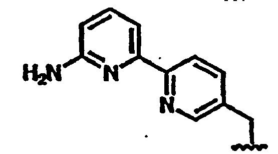 | H |
| (117). | Ac | 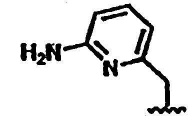 | H |
| (118). | Ac | 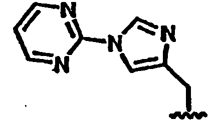 | H |
| (119). | Ac | 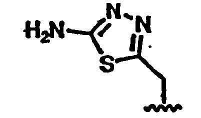 | H |
| (120). | Ac | 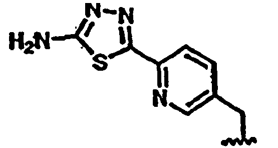 | H |
| (121). | Ac | 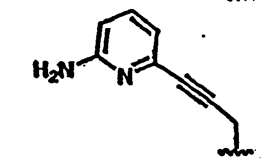 | H |
| (122). | Ac | 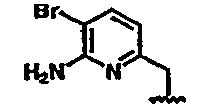 | H |
| (123). | Ac | 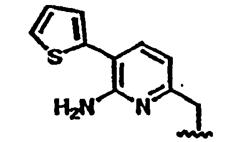 | H |
| (124). | Ac | 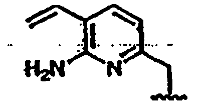 | H |
| (125). | Ac | 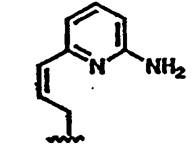 | H |
| (126). | Ac | 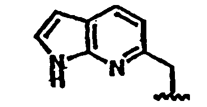 | H |
| (127). | Ac | 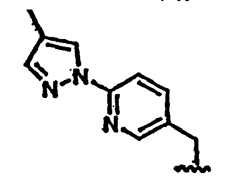 | H |
| (128). | Ac | 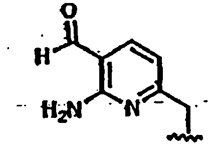 | H |
| (129). | Ac | 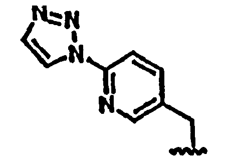 | H |
| (130). | Ac | 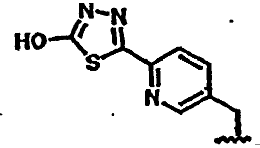 | H |
| (131). | Ac | 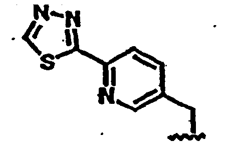 | H |
| (132). | Ac | 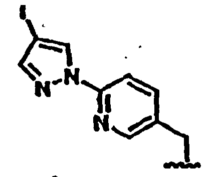 | H |
| (133). | Ac | 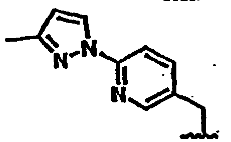 | H |
| (134). | Ac | 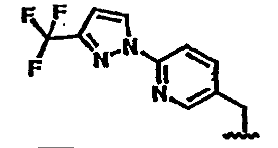 | H |
| (135). | Ac | 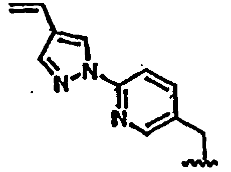 | H |
| (136). | Ac | 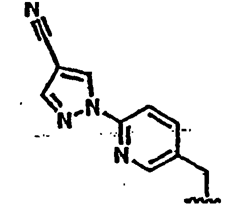 | H |
| (137). | Ac | 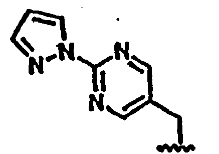 | H |
| (138). | Ac | 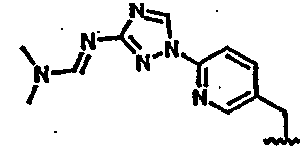 | H |
| (139). | Ac | 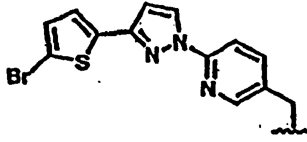 | H |
| (140). | Ac | 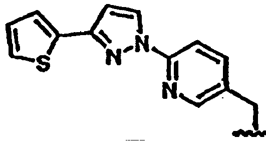 | H |
| (141). | Ac | 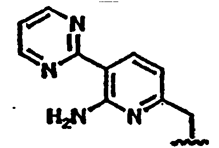 | H |
| (142). | H | 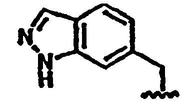 | H |
| (143). | Ac | 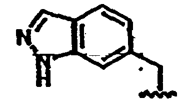 | H |
| (144). | Ac | 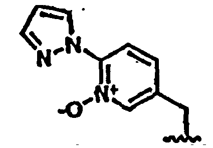 | H |
| (145). | Ac | 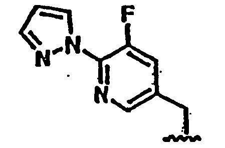 | H |
| (146). | -COCH₂CH₃ | 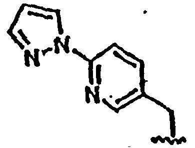 | H |
| (147). | Ac | 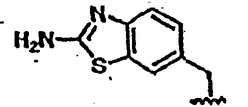 | H |
| (148). | Ac | 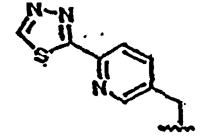 | H |

12. Composé selon la revendication 1, de formule A2, choisi parmi les composés représentés dans le tableau 12 : 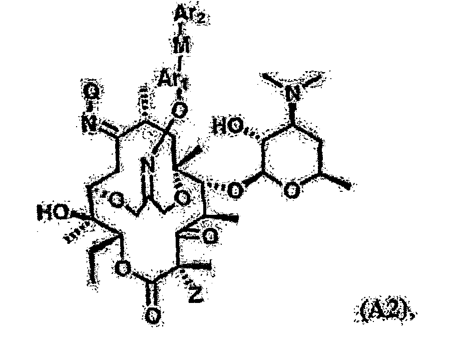
**Tableau 12**
| **Numéro** | **Q** | **-Ar₁-M-Ar₂** | **Z** |
|---|---|---|---|
| (1). | Ac | 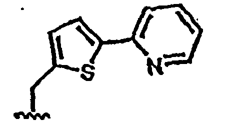 | H |
| (2). | Ac | 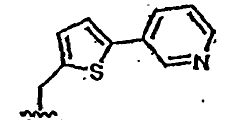 | H |
| (3). | Ac | 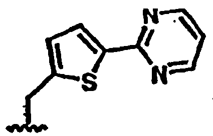 | H |
| (4). | Ac | 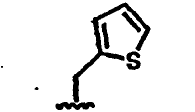 | H |
| (5). | Ac | 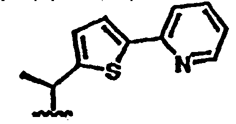 | H |
| (6). | Ac | 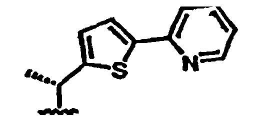 | H |
| (7). | Ac | 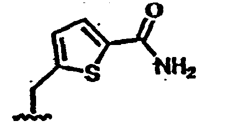 | H |
| (8). | Act | 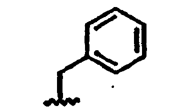 | H |
| (9). | Ac | 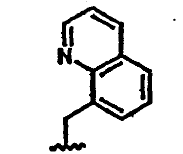 | F |
| (10). | Ac | 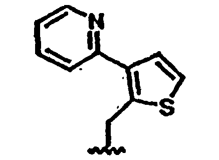 | H |
| (11). | Ac | 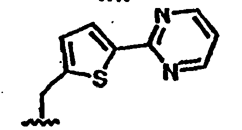 | F |
| (12). | Ac | 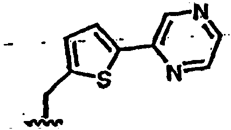 | F |
| (13). | Ac | 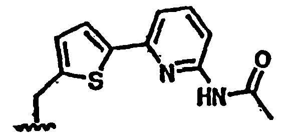 | H |
| (14). | Ac | 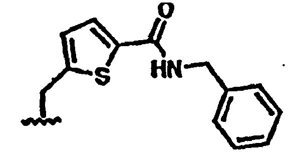 | H |
| (15). | Ac | 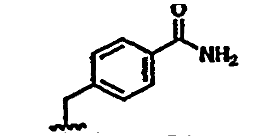 | H |
| (16). | Ac | 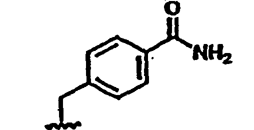 | F |
| (17). | Ac | 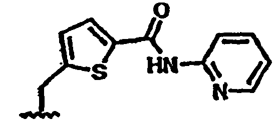 | H |
| (18). | Ac | 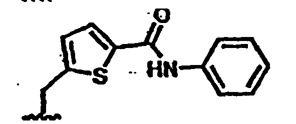 | H |
| (19). | Ac | 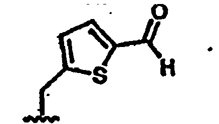 | H |
| (20). | Ac | 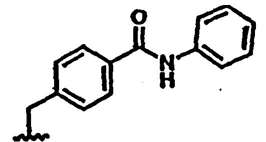 | H |
| (21). | Ac | 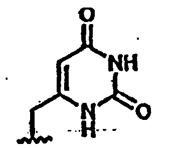 | H |
| (22). | Ac | 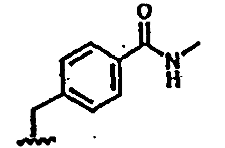 | H |
| (23). | Ac | 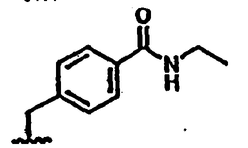 | H |
| (24). | Ac | 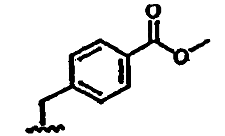 | H |
| (25). | Ac | 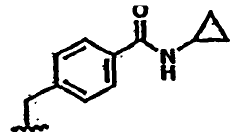 | H |
| (26). | Ac | 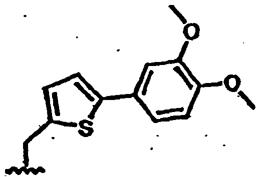 | H |
| (27). | Ac | 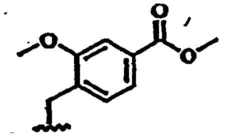 | H |
| (28). | Ac | 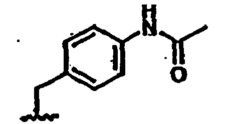 | H |
| (29). | Ac | 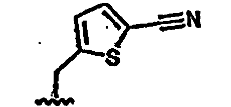 | H |
| (30). | Ac | 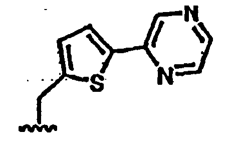 | H |
| (31). | Ac | 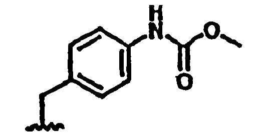 | H |
| (32). | Ac | 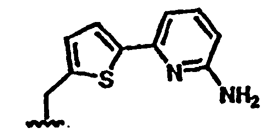 | F |
| (33). | Ac | 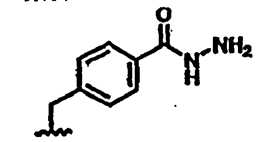 | H |
| (34). | Ac | 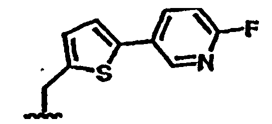 | H |
| (35). | Ac | 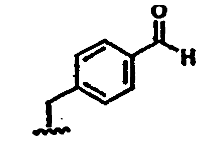 | H |
| (36). | Ac | 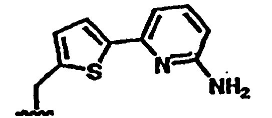 | H |
| (37). | H | 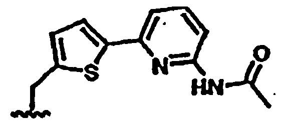 | H |
| (38). | Ac | 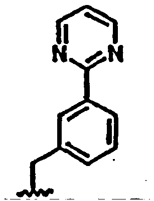 | H |
| (39). | Ac | 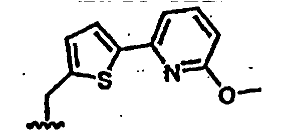 | H |
| (40). | H | 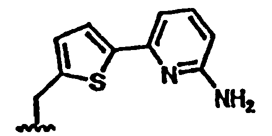 | H |
| (41). | H | 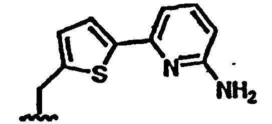 | F |
| (42). | Ac | 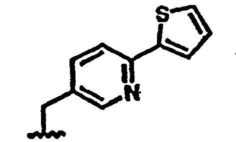 | H |
| (43). | Ac | 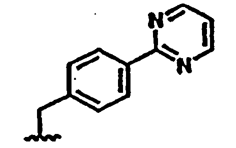 | H |
| (44). | Ac | 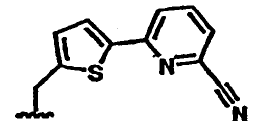 | H |
| (45). | Ac | 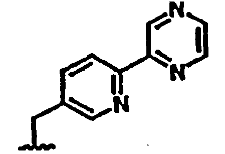 | H |
| (46). | Ac | 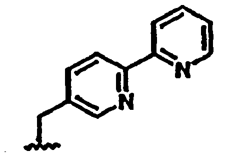 | H |
| (47). | Ac | 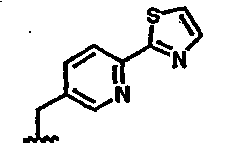 | H |
| (48). | Ac | 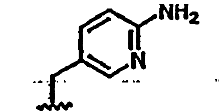 | H |
| (49). | Ac | 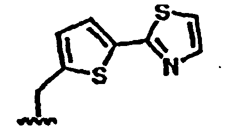 | H |
| (50). | Ac | 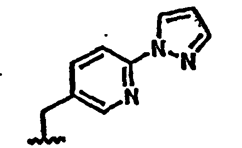 | F |
| (51). | Ac | 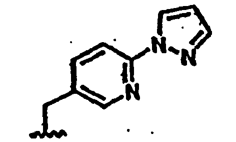 | H |
| (52). | Ac | 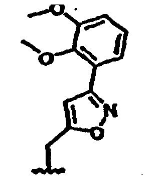 | H |
| (53). | Ac | 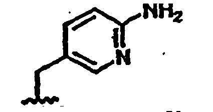 | F |
| (54). | Ac | 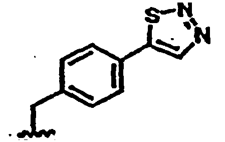 | H |
| (55). | Ac | 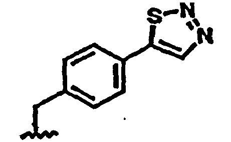 | F |
| (56). | Ac | 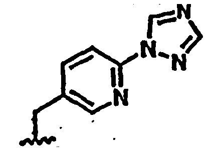 | H |
| (57). | Ac | 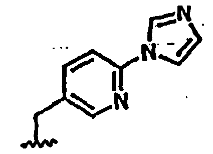 | H |
| (58). | Ac | 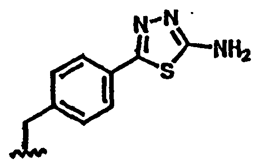 | H |
| (59). | Ac | 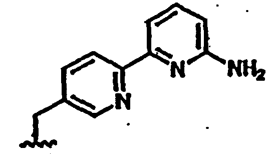 | H |
| (60). | Ac | 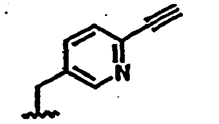 | H |
| (61). | Ac | 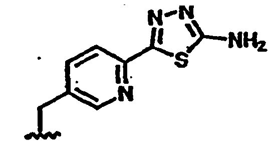 | H |
| (62). | Ac | 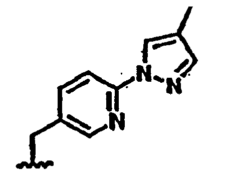 | H |
| (63). | Ac | 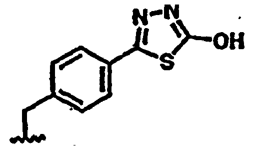 | H |
| (64). | Ac | 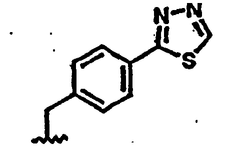 | H |
| (65). | Ac | 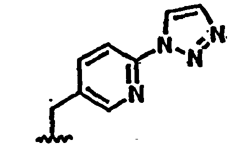 | H |
| (66). | Ac | 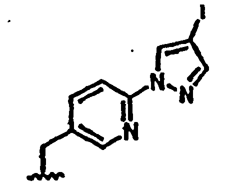 | H |
| (67). | Ac | 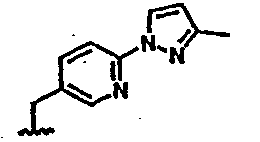 | H |
| (68). | Ac | 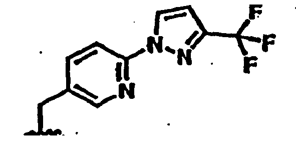 | H |
| (69). | Ac | 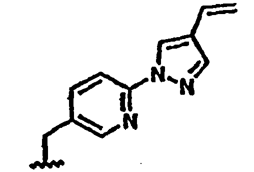 | H |
| (70). | Ac | 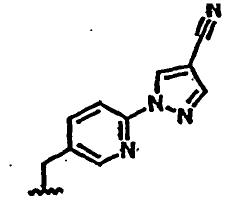 | H |
| (71). | Ac | 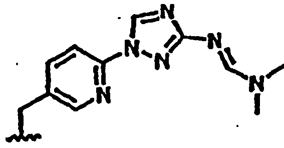 | H |
| (72). | Ac | 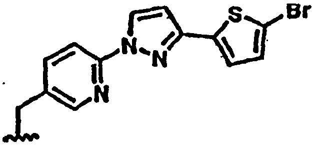 | H |
| (73). | Ac | 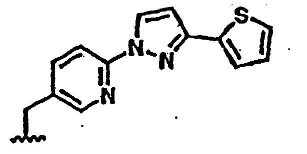 | H |
| (74). | H | 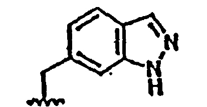 | H |
| (75). | Ac | 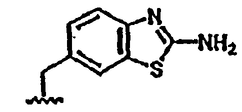 | H |

13. Composé selon la revendication 1, de formule B, choisi parmi les composés représentés dans le tableau 13 : 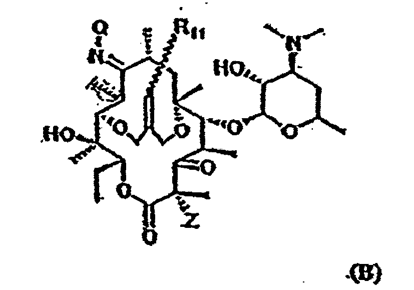
**Tableau 13**
| **Numéro** | **Q** | **R₁₁** | **Z** |
|---|---|---|---|
| (1). | H | H | H |
| (2). | MOM | H | H |
| (3). | MOM | 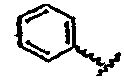 | H |
| (4). | Ac | 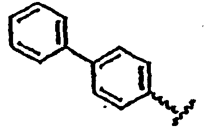 | H |
| (5). | Ac | 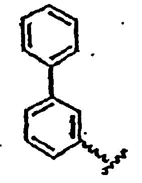 | H |
| (6). | Ac | 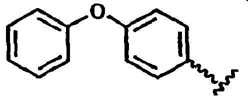 | H |
| (7). | Ac | 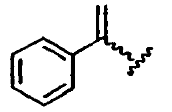 | H |
| (8). | Propionyl | 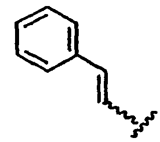 | H |
| (9). | Ac | 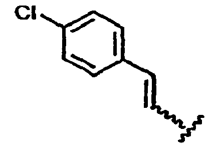 | H |
| (10). | -C(O)OMe | 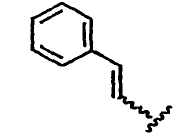 | H |
| (11). | -C(O)NH₂ | H | H |
| (12). | Me | H | H |
| (13). | BOM | H | H |
| (14). | Ac | 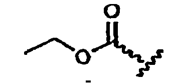 | H |
| (15). | Ac | 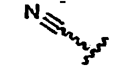 | H |

14. Composé selon la revendication 1, de formule B1, choisi parmi les composés représentés dans le tableau 14 : 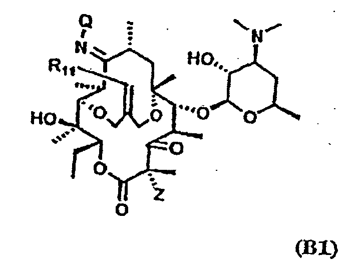
**Tableau 14**
| **Numéro** | **Q** | **R₁₁** | **Z** |
|---|---|---|---|
| (1). | Ac | 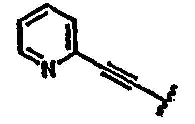 | H |
| (2). | Ac | 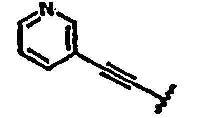 | H |
| (4). | Ac | 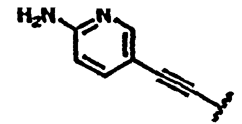 | H |
| (5). | Ac | 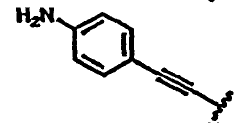 | H |
| (6). | Ac | 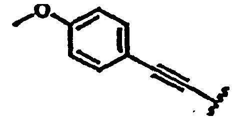 | H |
| (7). | Ac | 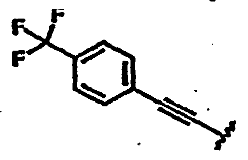 | H |
| (8). | Ac | 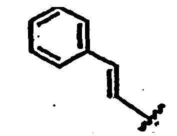 | F |
| (9). | Ac | 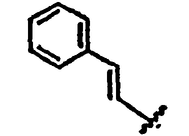 | H |
| (10). | Ac | 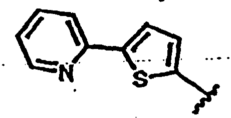 | H |
| (11). | Ac | 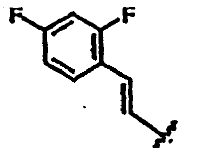 | H |
| (12). | 2-methoxyacetyl | 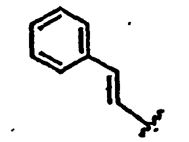 | H |
| (13). | 2-O-acyl-acetyl | 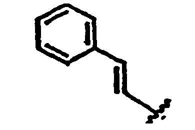 | H |
| (14). | 2-Fmoc-acetyl | 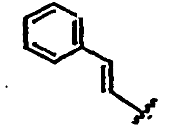 | H |
| (15). | Ac | 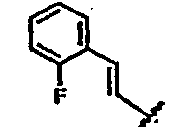 | H |
| (16). | Ac | 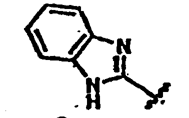 | H |
| (17). | 2-hydroxcy acetyl | 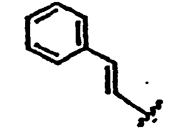 | H |
| (18). | 2-aminoacetyl | 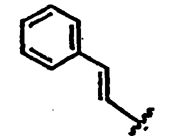 | H |
| (19). | Ac | 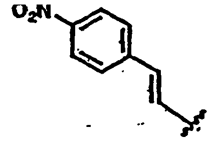 | H |
| (20). | Ac | 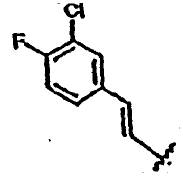 | H |
| (21). | Ac | 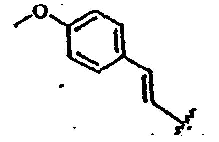 | H |
| (22). | Ac | 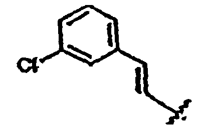 | H |

15. Composé selon la revendication 1, de formule B2, choisi parmi les composés représentés dans le tableau 15 : 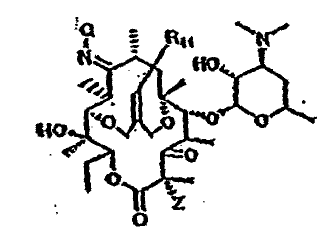
**Tableau 15**
| **Numéro** | **Q** | **R₁₁** | **Z** |
|---|---|---|---|
| (1). | Ac | 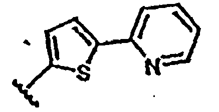 | H |
| (2). | Ac | 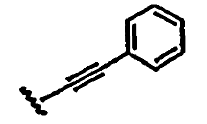 | H |
| (3). | Ac | 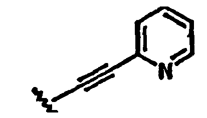 | H |
| (4). | Ac | 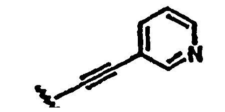 | H |
| (5). | Ac | 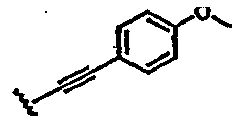 | H |
| (6). | Ac | 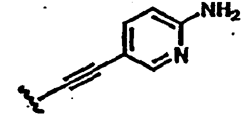 | H |
| (7). | Ac | 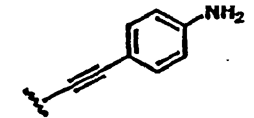 | H |
| (8). | Ac | 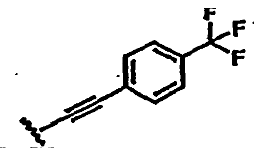 | H |
| (9). | Ac | 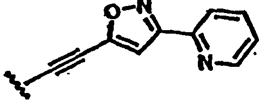 | H |

16. Composé selon la revendication 1, de formule C, choisi parmi les composés représentés dans le Tableau 16 : 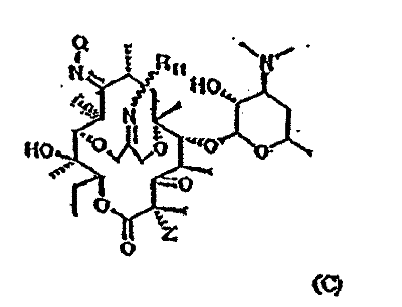
**Tableau 16**
| **Numéro** | **Q** | **R₁₁** | **Z** |
|---|---|---|---|
| (1). | Ac | 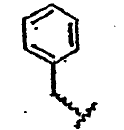 | H |
| (2). | Ac | 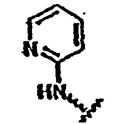 | H |
| (3). | Ac | 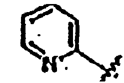 | H |
| (4). | Ac | 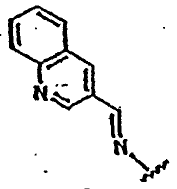 | H |
| (5). | Ac | 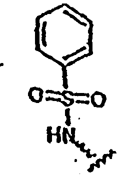 | H |

17. Composé, selon la revendication 7 ou 10, représenté par la formule : 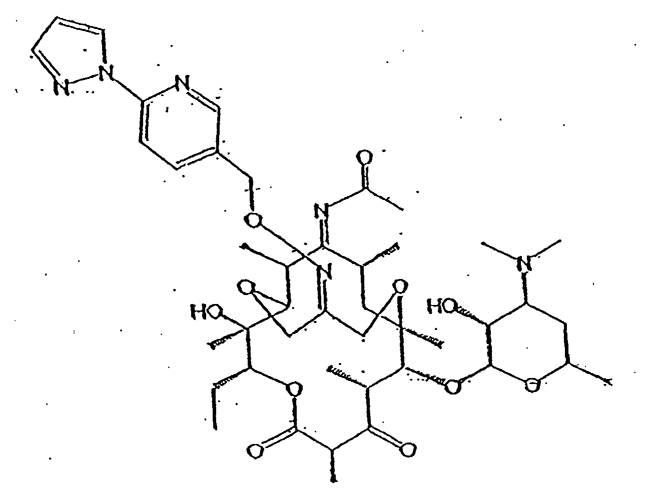 et ses sels, esters, mélanges racémiques et stéréo-isomères acceptables sur le plan pharmaceutique.

18. Composé, selon la revendication 7 ou 11, représenté par la formule : 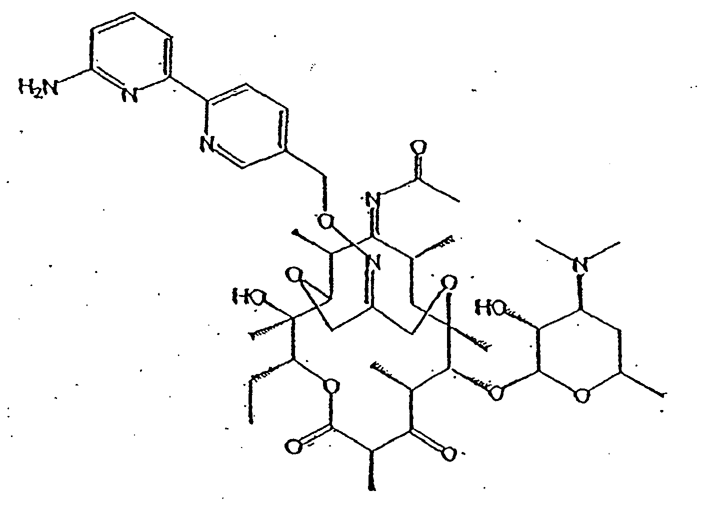 et ses sels, esters, mélanges racémiques et stéréo-isomères acceptables sur le plan pharmaceutique.

19. Composé selon l'une quelconque des revendications 1 à 18, où le composé est un sel acceptable sur le plan pharmaceutique.

20. Composé selon l'une quelconque des revendications 1 à 18, où le composé est une base libre.

21. Composé selon l'une quelconque des revendications 1 à 18, où le composé est un ester.

22. Composition pharmaceutique comprenant une quantité efficace sur le plan thérapeutique d'un composé selon l'une quelconque des revendications 1 à 21 ou d'un sel ou ester de celui-ci acceptable sur le plan pharmaceutique, en combinaison avec un vecteur acceptable sur le plan pharmaceutique.

23. Utilisation du composé selon l'une quelconque des revendications 1 à 21 pour la fabrication d'un médicament, pour le contrôle d'une infection bactérienne chez un sujet en besoin d'un tel traitement.

24. Utilisation selon la revendication 23, dans laquelle le médicament est pour administration par voie orale, par voie parentérale, par pulvérisation pour inhalation, par voie topique, par voie recale, par voie nasale, par voie buccale, par voie vaginale ou via un réservoir implanté.

25. Utilisation selon la revendication 24, dans laquelle le médicament est pour administration par voie orale ou par injection.

26. Utilisation selon la revendication 23, dans laquelle le médicament est prévu pour une administration à des humains.

27. Utilisation selon l'une quelconque des revendications 24 à 26, dans laquelle le médicament est administré en combinaison avec un ou plusieurs agents thérapeutiques ou prophylactiques supplémentaires.

28. Utilisation selon la revendication 27, dans laquelle l'agent thérapeutique ou prophylactique supplémentaire est administré séparément, dans le cadre d'un régime à doses multiples, ou sous une forme à dose unique.

29. Utilisation selon l'une quelconque des revendications 24 à 28, dans laquelle le médicament est utilisé pour traiter une infection aux protozoaires ou une infection bactérienne et des troubles se rapportant à de telles infections.

30. Utilisation selon la revendication 29, dans laquelle l'infection ou le trouble est choisi dans le groupe constitué d'une pneumonie, une otite moyenne, une sinusite, une bronchite, une amygdalite et une mastoïdite se rapportant à une infection par Streptococcus pneumoniae, Haemophilus influenzae, Moraxella catarrhalis, Staphylococcus aureus ou Peptostreptococcus app. Pseudomonas spp. ; une pharynigite, une fièvre rhumatique et une glomérulonéphrite se rapportant à une infection par Streptococcus pyogenes, des streptocoques des Groupes C et G, Clostridium diptheriae ou Actinobacillus haemolyticum ; des infections du système respiratoire se rapportant à une infection par Mycoplasma pneumoniae, Legionella pneumophila, Streptococcus pneumoniae, Haemophilus influenzae ou Chlamydia pneumoniae, des infections de la peau et des tissus mous sans complications, des abcès et ostéomyélites, et la fièvre puerpérale se rapportant à une infection par Staphylococcus aureus, staphylocoques coagulase-positifs (c'est-à-dire, S. epidermidis, S. hemolyticus, etc.), S. pyogenes, S. agalactiae, les groupes de streptocoques C-F (streptocoques à colonies miniatures), viridans streptococci, Corynebacterium spp., Clostridium spp., ou Bartonella henselae ; des infections aiguës du système urinaire sans complications se rapportant à une infection par S. saprophyticus ou Enterococcus spp. ; une urétrite et une cervicite ; et des maladies sexuellement transmissibles se rapportant à une infection par Chlamydia trachomatis, Haemophilus ducreyi, Treponema pallidum, Ureaplasma urealyticum ou Nesseria gonorrhea ; des troubles liés à une toxine se rapportant à une infection par S. aureus, ou des streptocoques des Groupes A, S et C ; des ulcères se rapportant à une infection par Helicobacter pylori ; des syndromes fébriles systémiques se rapportant à une infection par Borrelia recurrentis ; une maladie de Lyme se rapportant à une infection par Borrelia burgdorferi ; une conjonctivite, une kératite et une dacryocystite se rapportant à une infection par C. trachomatis, N. gonorrhoeae, S. aureus, S. pneumoniae, S. pyogenes, H. influenzae ou Listeria spp. ; un trouble de souches disséminées du Mycobactérium avium (MAC) se rapportant à une infection par Mycobacterium avium ou Mycobacterium intracellulare ; une gastroentérite se rapportant à une infection par Campylobacter jejuni ; un protozoaire intestinal se rapportant à une infection par Cryptosporidium spp., une infection d'origine dentaire se rapportant à une infection par Bordetella pertussis, une gangrène gazeuse se rapportant à une infection par Clostridium perfringens ou Bacteroides spp. ; une infection cutanée par S. aureus, Propionibacterium acne ; une athérosclérose se rapportant à une infection par Helicobacter pylori et Chlamydia pneumoniae.

31. Utilisation selon la revendication 30, dans laquelle l'infection ou le trouble est une pneumonie, une otite moyenne, une sinusite, une bronchite, une amygdalite, p. acne, et des infections de la peau et des tissus mous.

32. Utilisation selon la revendication 29, dans laquelle l'infection ou le trouble pour le traitement duquel le médicament est utilisé est choisi dans le groupe constitué d'une maladie respiratoire bovine se rapportant à une infection par P. haemolytica., P. multocida, Mycoplasma bovis ou Bordetella spp. ; une maladie entérique de la vache se rapportant à une infection par E. coli ou protozoaires, une mastite des vaches laitières se rapportant à une infection par S. aureus, S. uberis, S. agalactiae, S. dysgalactiae, Klebsiella spp., Corynebacterium ou Enterococcus spp. ; une maladie respiratoire porcine se rapportant à une infection par A. pleuropneumoniae., P. multocida ou Mycoplasma spp. ; une maladie entérique porcine se rapportant à une infection par E. coli, Lawsonia intracellularis, Salmonella spp. ou Serpulina hyodyisinteriae ; le piétin de la vache se rapportant à une infection par Fusobacterium spp., une métrite de la vache se rapportant à une infection par E. coli ; des verrues pileuses chez la vache se rapportant à une infection par Fusobacterium necrophorum ou Bacteroides nodosus ; une conjonctivite chez la vache se rapportant à une infection par Moraxella bovis, un avortement prématuré chez la vache se rapportant à une infection par des protozoaires ; une infection du système urinaire chez les chiens et les chats se rapportant à une infection par S. epidermidis, S. intermedius, coagulase neg. Staphylococcus ou P. multocida ; et des infections des dents ou de la bouche chez les chiens et les chats se rapportant à une infection par Alcaligenes spp. ; Bacteroides spp., Clostridium spp., Enterocater spp, Eubacterium spp., Peptostreptococcus spp., Porphfyromonas spp, Campylobacter spp., Actinomyces spp., Erysipelothrix spp., Rhodococcus spp., Trypanosoma spp., Plasmodium spp., Babesia spp., Toxoplasma spp., Pneumocystis spp., Leishmania spp., et Trichomonas spp. et Prevotella spp.

33. Procédé pour préparer un composé selon la revendication 1, représenté par la formule 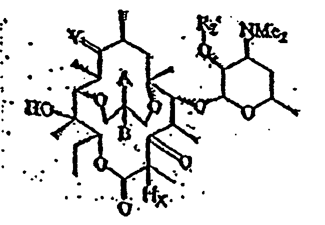 où V est choisi parmi N-Q ou O ; Hx est un halogène ou un méthyle ; et A, B, Q et R₂' sont tels que définis dans la revendication 1, comprenant l'étape consistant à
(a) faire réagir un composé représenté par la formule 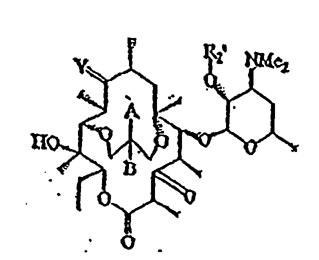 où A, B, V et R₂' sont tels que définis précédemment, avec un agent d'halogénation ou un agent de méthylation en présence d'une base .

34. Procédé pour préparer un composé selon la revendication 1, représenté par la formule 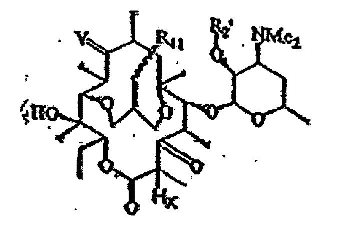 où V est choisi parmi N-Q ou O ; Hx est un halogène ou un méthyle ; et Q, R₁₁ et R₂' sont tels que définis dans la revendication 1, comprenant l'étape consistant à
(a) faire réagir un composé représenté par la formule 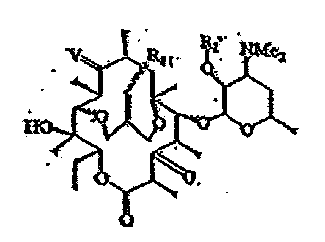 où V, R₁₁ et R₂' sont tels que définis précédemment, avec un agent d'halogénation en présence d'une base.

35. Procédé pour préparer un composé selon la revendication 1, représenté par la formule 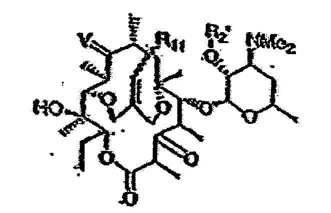 où V est choisi parmi N-Q ou O ; et R₁₁, Q, et R₂' sont tels que définis dans la revendication 1, comprenant l'étape consistant à :
(a) faire réagir un composé représenté par la formule 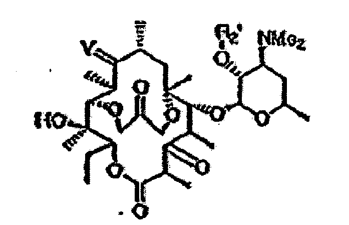 où V et R₂' sont tels que définis précédemment avec un composé phosphonium en présence d'une base.

36. Procédé pour la préparation d'un composé selon la revendication 1, représenté par la formule 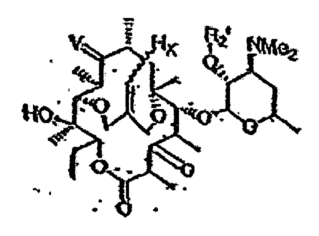 où V est choisi parmi N-Q ou O ; Hx est un halogène ; et R₁₁, Q, et R₂' sont tels que définis dans la revendication 1, comprenant l'étape consistant à :
(a) faire réagir un composé représenté par la formule 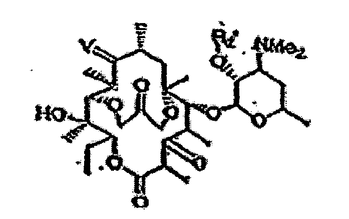 où V et R₂' sont tels que définis précédemment avec un sel de phosphonium en présence d'une base.

37. Procédé pour la préparation d'un composé selon la revendication 1, représenté par la formule 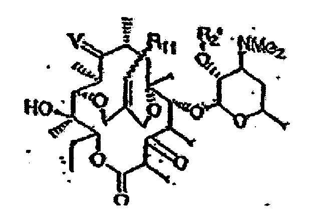 où V est choisi parmi N-Q ou O ; et R₁₁, Q, et R₂' sont tels que définis dans la revendication 1, comprenant l'étape consistant à :
(a) faire réagir un composé représenté par la formule 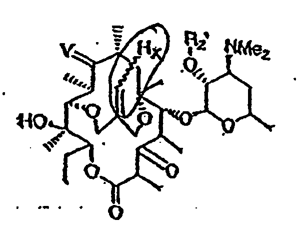 où Hx est un halogène et V et R₂' sont tels que définis précédemment, avec un composé organobore ou organotine en présence d'un catalyseur au palladium et d'une base.

38. Procédé pour la préparation d'un composé selon la revendication 1, représenté par la formule 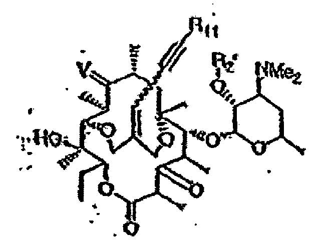 où V est choisi parmi N-Q ou O ; et R₁₁, Q, et R₂' sont tels que définis dans la revendication 1, comprenant l'étape consistant à :
(a) faire réagir un composé représenté par la formule 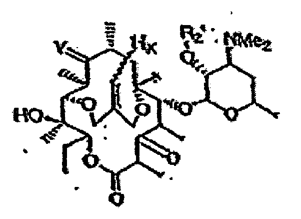 où Hx est un halogène et V et R₂' sont tels que définis précédemment, avec un composé représenté par la formule 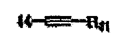 où R₁₁ est défini précédemment, en présence d'un catalyseur au palladium, d'un halogénure de cuivre et d'une amine.

39. Procédé pour la préparation d'un composé selon la revendication 1, représenté par la formule 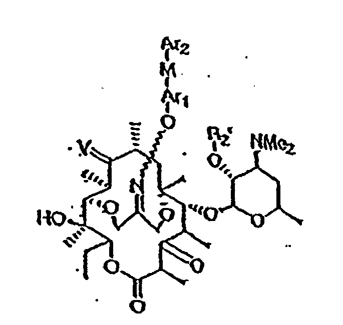 où V est choisi parmi N-Q ou O ; et Q, Ar₁, M, Ar₂ et R₂' sont tels que définis dans la revendication 1, comprenant les étapes consistant à :
(a) faire réagir un composé représenté par la formule (Ia) 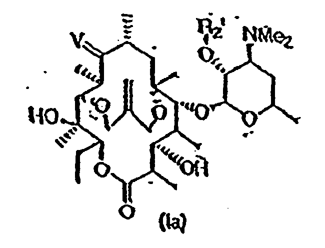 où V et R₂' sont tels que définis précédemment, avec un réactif ou des réactifs susceptibles d'effectuer un clivage oxydant ;
(b) faire réagir un composé de l'étape (a) représenté par la formule (Ib) 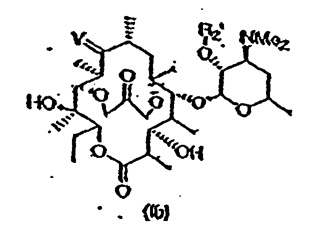 où V et R₂' sont tels que définis précédemment, avec un agent oxydant pour fournir un composé de type 3-céto ;
(c) faire réagir un composé de l'étape (b) représenté par la formule (Ic) 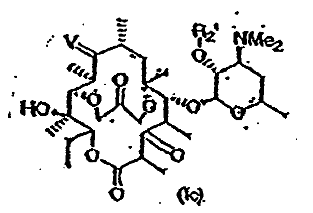 où V et R₂' sont tels que définis précédemment, avec un composé de formule Ar₂-M-Ar₁O-NH₂, dans laquelle Ar₁, Ar₂, et M sont tels que définis dans la revendication 1, en présence d'un acide ou d'une base ; et
(d) éventuellement déprotéger le composé du procédé de l'étape (c).

40. Procédé pour la préparation d'un composé selon la revendication 1, représenté par la formule 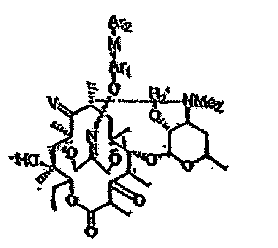 où V est choisi parmi N-Q ou 0 ; et Q, Ar₁, M, Ar₂ et R₂' sont tels que définis dans la revendication 1, comprenant les étapes consistant à :
(a) faire réagir un composé représenté par la formule (Ib) 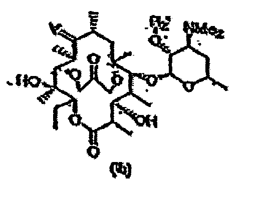 où V et R₂' sont tels que définis précédemment, avec un composé de formule Ar₂-M-Ar₁O-NH₂, dans laquelle Ar₁, Ar₂, et M sont tels que définis dans la revendication 1, en présence d'un acide ou d'une base ;
(b) faire réagir un composé de l'étape (a) avec un agent oxydant pour fournir un composé de type 3-céto ; et
(c) éventuellement déprotéger le composé du procédé de l'étape (b).

41. Procédé pour la préparation d'un composé selon la revendication 1, représenté par la formule 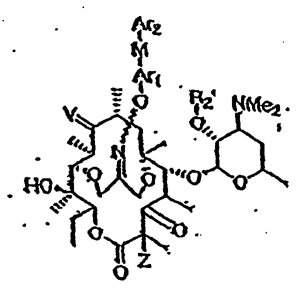 où V est choisi parmi N-Q ou O ; et Q, Ar₁, M, Ar₂, R₂' et Z sont tels que définis dans la revendication 1, comprenant les étapes consistant à :
(a) faire réagir un composé représenté par la formule (Ia) 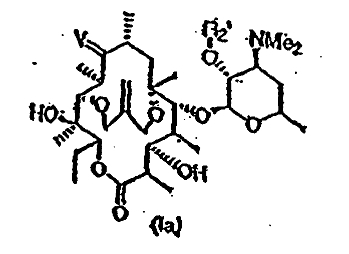 où V et R₂' sont tels que définis précédemment, avec un agent oxydant pour fournir un composé de type 3-céto ;
(b) faire réagir un composé de l'étape (a) représenté par la formule (Ib) 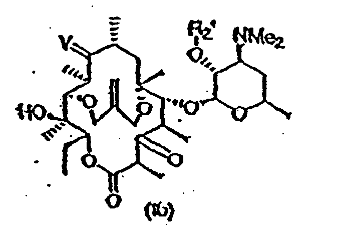 où V et R₂' sont tels que définis précédemment, avec un agent d'halogénation ou un agent de méthylation en présence d'une base ;
(c) faire réagir un composé de l'étape (b) représenté par la formule (Ic) 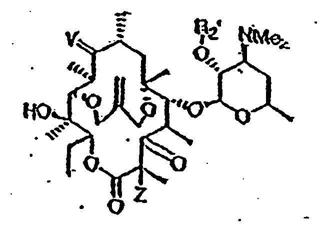 où V, Z et R₂' sont tels que définis précédemment, avec un réactif ou des réactifs susceptibles d'effectuer un clivage oxydant ;
(d) faire réagir un composé de l'étape (c) représenté par la formule (Id) 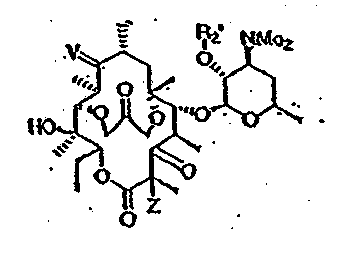 où V, Z, et R₂' sont tels que définis précédemment, avec un composé de formule Ar₂-M-Ar₁-O-NH₂, dans laquelle Ar₁, Ar₂, et M sont tels que définis dans la revendication 1, en présence d'un acide ou d'une base ; et
(e) éventuellement déprotéger un composé de l'étape (d).
